# EUROPEAN PATENT APPLICATION

(11) **EP 1 099 690 A1**
(43) Date of publication of application: **16.05.2001**
(21) Application number: 99929887.0
(22) Date of filing: 16.07.1999
(51) Int. Cl.: C07D 205/08, C07D 401/12, C07D 403/12, C07D 405/06, C07D 405/12, C07D 405/14, A61K 31/395, A61K 31/40, A61K 31/44, A61K 31/445, A61K 31/495, A61K 31/505, A61K 31/535

(54) **MONOCYCLIC $g(b)-LACTAM COMPOUNDS AND CHYMASE INHIBITORS CONTAINING THE SAME**

(30) Priority: 23.07.1998 JP 20754098
(71) Applicant: SHIONOGI & CO., LTD., Osaka-shi, Osaka 541-0045 (JP)
(72) Inventor: UENAKA, Masaaki, Toyono-gun, Osaka 563-0131 (JP); KII, Makoto, Amagasaki-shi, Hyogo 660-0881 (JP); NAKAJIMA, Masatoshi, Kanzaki-gun, Shiga 521-1231 (JP)
(74) Representative: Baverstock, Michael George Douglas
(86) International application number: JP9903864
(87) International publication number: WO0005204

(57) **Abstract**

Chymase inhibitors and cytokine production inhibitors containing compounds represented by general formula (I), prodrugs of the same, pharmaceutically acceptable salts thereof or hydrates of them, wherein A is -CO-, -CONH- or the like; R¹ is optionally substituted lower alkyl, optionally substituted aryl or the like; R² and R³ are each independently hydrogen, optionally substituted lower alkyl or the like; B is -S-, -O- or the like; and R⁴ is optionally substituted aryl or the like.

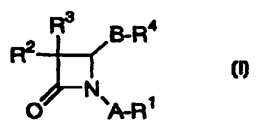

## Description

### Technical Field

The present invention relates to the use of compounds having chymase inhibitory activity and/or cytokine production inhibitory activity and novel compounds having çhymase inhibitory activity and/or cytokine production inhibitory activity. In detail, the invention relates to chymase inhibiting compositions containing monocyclic *β*-lactam compounds having chymase inhibitory activity and/or cytokine production inhibitory activity, and novel monocyclic *β*-lactam compounds.

### Background Art

Human chymase is a neutral serine protease whose molecular weight is about 30,000. It is known that chymase is generally synthesized, stored in, and secreted from mast cells and mainly exists e.g. in the heart, blood vessels and skin.

One of the main activities is, for example, the production of Angiotensin II. The production of Angiotensin II was considered to be caused by angiotensin converting enzyme (hereinafter referred to as ACE). Recently, however, it has been revealed that ACE effects only 10 to 15 % of Angiotensin II production in the human heart, and 80 % or more is caused by human chymase (Circulation Research, Vol. 66, p. 883, 1990 and Journal of Biological Chemistry, Vol.266, p. 17173, 1991).

Chymase is supposed to be concerned with the acceleration of histamine release from mast cells (Journal of Biochemistry, Vol. 103, p. 820 - 822, 1988) and chymase inhibitors are expected to be a new type of anti-inflammatory agent or anti-allergic agent.

Further, human chymase has been revealed to possess other activities: acceleration of macrophage foam cell formation, production of active collagenase from procollagenase, limited degradation of extracellular matrix such as collagen, fibronectine, vitronectin, etc., conversion of big-endothelin to endothelin, limited degradation of thrombin or IgG.

Pathophysiologically, chymase activity is known to be raised in blood vessels after balloon injury or heart myocardosis.

Peptide chymase inhibitors are described in WO93/25574, WO95/27053 and WO95/27055. Examples of non-peptide chymase inhibitors are imidazolidine derivatives in WO96/04248, pyridine and pyrimidine derivatives in WO96/33974 and triazine derivatives in EP713876A. These are quite different from the compounds of the present invention in chemical structure.

Some compounds having similar structures to that of the compounds of the present invention are described, for example, in GB 2266527 A, Japan Patent No. 2736113, J. Med. Chem., 1995, 38, 2449-2462 and USP 5,747,485. All of these have an elastase inhibitory activity, differing from the present invention. In JP 9-263577 A, other similar compounds having an elastase inhibitory activity and cytokine production inhibitory activity are described.

### Disclosure of Invention

The object of the present invention is to provide a pharmaceutical composition for use as a chymase inhibitor and/or cytokine production inhibitor having a potent activity and novel compounds having chymase inhibition and/or cytokine production inhibitory activity.

The present invention provides a pharmaceutical composition for use as a chymase inhibitor and/or cytokine production inhibitor, specifically an anti-inflammatory agent, comprising
1) a compound of the formula (I): 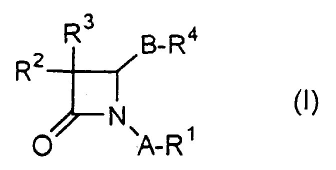
   wherein A is a bond, -CO-, -COO-, -COCO-, -CONH- or -SO₂-,
   R¹ is optionally substituted lower alkyl, optionally substituted lower alkenyl, optionally substituted lower alkynyl, optionally substituted cycloalkyl, optionally substituted cycloalkenyl or optionally substituted aryl, and R¹ may be hydrogen when A is a bond, -CO-, -COCO-, -CONH- or -SO₂-,
   R² and R³ are each independently hydrogen, halogen, optionally substituted lower alkyl, optionally substituted lower alkoxycarbonyl, optionally substituted acyl, optionally substituted amino, optionally substituted carbamoyl or optionally substituted aryl,
   B is a bond, -S-, -O-, -S-S-, -SO- or -SO₂-, and
   R⁴ is hydrogen, optionally substituted lower alkyl, optionally substituted aryl or optionally substituted heterocyclyl and R⁴ may be optionally substituted acyl when B is a bond, -S-, -O-, -SO- or -SO₂- (hereinafter referred to as compound (I)).
2) the compound described in 1) wherein A-R¹ is 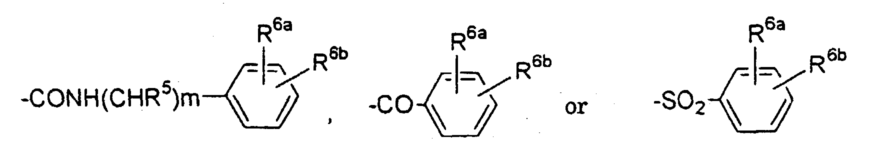
   wherein R⁵ is hydrogen, optionally substituted lower alkyl, optionally substituted lower alkenyl, optionally substituted lower alkoxy or optionally substituted aryl, R^{6a} and R^{6b} are each independently hydrogen, halogen, hydroxy, lower alkyl, carboxy, lower alkoxycarbonyl, lower alkoxy, aryl, acyl, optionally substituted amino, aryloxy, lower alkylthio or heterocyclyl and R^{6a} and R^{6b} taken together may form lower alkylenedioxy, and m is 0 or 1,
   R² and R³ are each independently hydrogen, optionally substituted phenyl or optionally substituted benzyl, and
   B-R⁴ is hydrogen, optionally substituted acyloxy, 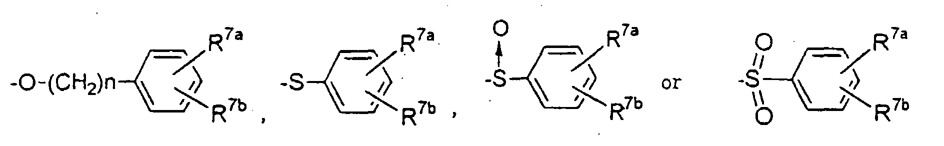
   wherein R^{7a} and R^{7b} are each independently hydrogen, halogen, lower alkyl, lower alkoxy, lower alkenyl, amino, acylamino, 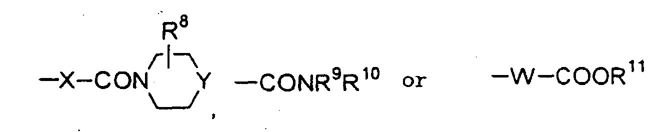
   wherein X and W are each independently a bond, lower alkylene or lower alkenylene, Y is a bond, -CH₂-, -NR¹²- (wherein R¹² is hydrogen, cycloalkyl, heterocyclyl or lower alkyl optionally substituted with methylenedioxyphenyl) or -O-, R⁸ is hydrogen, optionally substituted lower alkyl or optionally substituted carbamoyl, R⁹, R¹⁰ and R¹¹ are each independently hydrogen, optionally substituted lower alkyl, optionally substituted lower alkenyl, optionally substituted amino, optionally substituted aryl or optionally substituted arylsulfonyl and n is an integer of 0 to 6,
3) the compound described in 1) wherein A-R¹ is 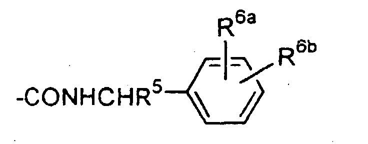
   wherein R⁵ is C1 to C3 alkyl or optionally substituted phenyl wherein the substituent is halogen, lower alkyl or lower alkoxy, R^{6a} and R^{6b} are each independently hydrogen, halogen, lower alkyl or lower alkoxy,
   R² is benzyl optionally substituted with lower alkoxy,
   R³ is hydrogen,
   B-R⁴ is acyloxy, 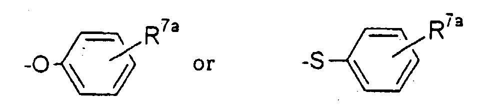
   wherein R^{7a} is hydrogen, 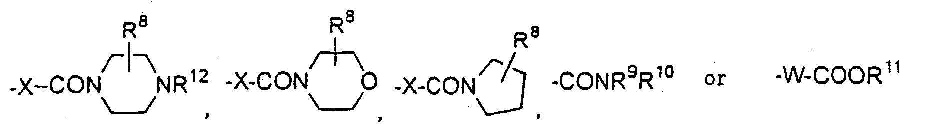
   wherein X and W are each independently a bond, methylene or vinylene, R⁸ is lower alkyl or carbamoyl, R⁹ is hydrogen or optionally substituted lower alkyl, R¹⁰ is hydrogen, optionally substituted lower alkyl, lower alkenyl, lower alkylamino, arylamino, phenyl or arylsulfonyl, R¹¹ is hydrogen, optionally substituted lower alkyl or optionally substituted phenyl and R¹² is cycloalkyl or lower alkyl optionally substituted with methylenedioxyphenyl or,
4) the compound described in 1) wherein A-R¹ is 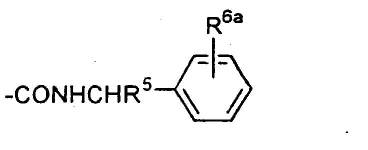
   wherein R⁵ is C1 to C3 alkyl or 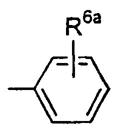 and all R^{6a} are the same and hydrogen, halogen, lower alkyl or lower alkoxy, or
5) the compound described in 1) wherein A-R¹ is -CONHR⁵Ph wherein Ph is phenyl, R² is benzyl, R³ is C1 to C3 alkyl, B-R⁴ is 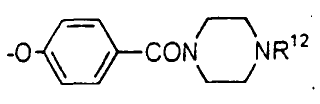 and R⁵ and R¹² are each independently C1 to C3 alkyl, prodrug, pharmaceutically acceptable salt or hydrate thereof.

The present invention provides a method for preventing and/or treating diseases caused by chymase, for example, cardiovascular diseases, inflammation, allergic diseases, rheumatics, asthma and atopy, comprising administering the compound (I) described in 1), prodrug, pharmaceutically acceptable salt or hydrate thereof.

In one of other embodiments, the present invention provides use of the compound (I), prodrug, pharmaceutically acceptable salt or hydrate thereof for manufacturing a medicament for preventing and/or treating diseases caused by chymase.

In one of other embodiments, the present invention provides 6) a compound of the formula (I'): 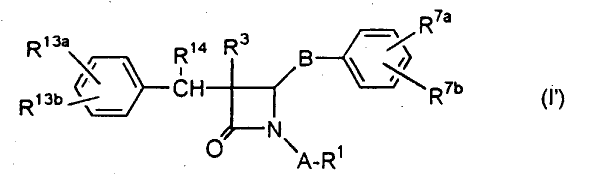
wherein A and R¹ are the same as defined in 1),
R³ is hydrogen, halogen, optionally substituted lower alkoxycarbonyl, optionally substituted acyl, optionally substituted amino, optionally substituted aryl or optionally substituted benzyl,
R^{13a} and R^{13b} are each independently hydrogen, halogen, hydroxy, optionally substituted lower alkyl, optionally substituted lower alkoxy, optionally substituted amino or optionally substituted lower alkylthio, and R^{13a} and R^{13b} taken together may form lower alkylenedioxy,
R¹⁴ is hydrogen, hydroxy, lower alkyl, lower alkoxy or acyloxy,
R^{7a} is hydrogen, 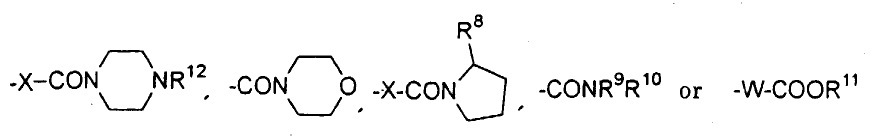
wherein X and W are each independently a bond, methylene or vinylene, R⁸ is methyl or carbamoyl, R⁹ is hydrogen or lower alkyl, R¹⁰ is optionally substituted lower alkyl wherein the substituent is lower alkylamino; phenyl optionally substituted with halogen; carboxy; or lower alkoxycarbonyl optionally substituted with aryl, lower alkenyl, lower alkylamino, phenylamino, phenyl or benzenesulfonyl, R¹¹ is hydrogen or optionally substituted lower alkyl (wherein the substituent is lower alkylamino; acyloxy; phenyl optionally substituted with halogen or methylenedioxy; or heterocyclyl) and R¹² is C1 to C3 alkyl or cyclohexyl,
R^{7b} is hydrogen and B is O or S
   (hereinafter referred to as a compound (I')), prodrug, pharmaceutically acceptable salt or hydrate thereof.

The present invention provides the following compounds, prodrugs, pharmaceutically acceptable salts or hydrates thereof:
7) a compound of the formula (I''): 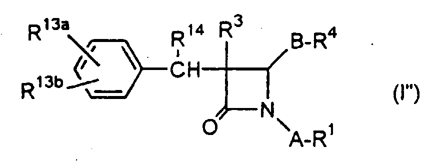
   wherein B and R⁴ are the same as defined in 1),
   A is -CO-, -CONH- or -SO₂-,
   R¹ is optionally substituted lower alkyl or optionally substituted aryl,
   R³ is hydrogen, halogen, lower alkyl, optionally substituted lower alkoxycarbonyl, optionally substituted acyl, optionally substituted amino, optionally substituted aryl or optionally substituted benzyl,
   R^{13a} and R^{13b} are each independently hydrogen, halogen, hydroxy, optionally substituted lower alkyl, optionally substituted lower alkoxy, optionally substituted amino or optionally substituted lower alkylthio and R^{13a} and R^{13b} taken together may form lower alkylenedioxy and
   R¹⁴ is hydrogen, hydroxy, lower alkyl, lower alkoxy or acyloxy, excluding a compound wherein B-R⁴ is optionally substituted aryloxy or optionally substituted acylthio and A is CONH (hereinafter referred to as Compound (I'')),
8) the compound described in 7) wherein B-R⁴ is acyloxy, 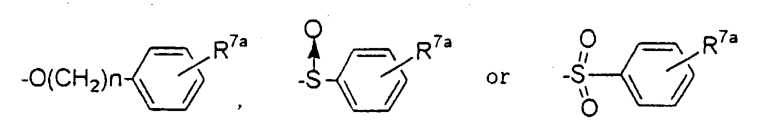
   wherein n is 0 or 1, R^{7a} is hydrogen, 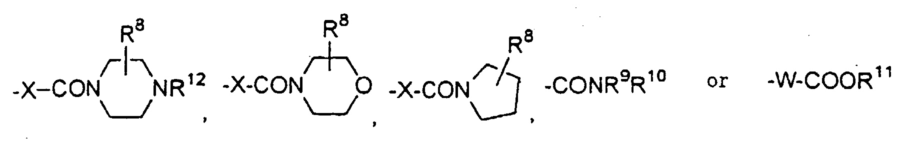
   wherein X and W are each independently a bond, methylene or vinylene, R⁸ is lower alkyl or carbamoyl, R⁹ is hydrogen or optionally substituted lower alkyl, R¹⁰ is hydrogen, optionally substituted lower alkyl, lower alkenyl, lower alkylamino, arylamino, phenyl or arylsulfonyl, R¹¹ is hydrogen, optionally substituted alkyl or optionally substituted phenyl and R¹² is cycloalkyl or lower alkyl optionally substituted with methylenedioxyphenyl,
9) the compound described in 6) or 7) wherein R³ is hydrogen,
10) the compound described in 6) or 7) wherein R^{13a} is hydrogen or C1 to C3 lower alkoxy at the *o*-position and R^{13b} is hydrogen,
11) Any one of the compounds selected from the group of
   (a) 4-[3-Benzyl-4-oxo-1-(1-phenyl-ethylcarbamoyl)-azetidin-2-yloxy]-benzoic acid,
   (b) 3-Benzyl-2-[4-(4-methyl-piperazine-1-carbonyl)-phenoxy]-4-oxo-azetidine-1-carboxylic acid (1-phenyl-ethyl)-amide,
   (c) 3-Benzyl-2-[4-(2-carbamoyl-pyrrolidine-1-carbonyl)-phenoxy)-4-oxo-azetidine-1-carboxylic acid (1-phenyl-ethyl)-amide,
   (d) 3-Benzyl-2-[4-(2-methyl-pyrrolidine-1-carbonyl)-phenoxy]-4-oxo-azetidine-1-carboxylic acid (1-phenyl-ethyl)-amide,
   (e) 4-[3-(2-Methoxy-benzyl)-4-oxo-1-(1-phenyl-ethylcarbamoyl)-azetidin-2-yloxy]-benzoic acid,
   (f) 4-[3-(2-Methoxy-benzyl)-4-oxo-1-(1-phenyl-ethylcarbamoyl)-azetidin-2-yloxy]-benzoic acid pyridin-4-ylmethyl ester,
   (g) 4-[3-(2-Methoxy-benzyl)-4-oxo-1-(1-phenyl-ethylcarbamoyl)-azetidin-2-yloxy]-benzoic acid benzyl ester,
   (h) 3-(2-Methoxy-benzyl)-2-oxo-4-[4-(4-pyrimidin-2-yl-piperazine-1-carbony)-phenoxy]-azetidine-1-carboxylic acid (1-phenyl-ethyl)-amide,
   (i) 2-[4-(4-Cyclohexyl-piperazine-1-carbonyl)-phenoxy]-3-(2-methoxy-benzyl)-4-oxo-azetidine-1-carboxylic acid (1-phenyl-ethyl)-amide,
   (j) 3-(2-Methoxy-benzyl)-2-[4-(4-methyl-piperazine-1-carbonyl)-phenoxy]-4-oxo-azetidine-1-carboxylic acid (1-phenyl-ethyl)-amide,
   (k) 4-[1-(Benzhydryl-carbamoyl)-3-(2-ethoxy-benzyl)-4-oxo-azetidin-2-yloxy]-benzoic acid,
   (l) 2-[4-(4-Cyclohexyl-piperazine-1-carbonyl)-phenoxy]-3-(2-ethoxy-benzyl)-4-oxo-azetidine-1-carboxylic acid benzhydryl-amide,
   (m) 3-(2-Ethoxy-benzyl)-2-[4-(morpholine-4-carbonyl)-phenoxy]-4-oxo-azetidine-1-carboxylic acid benzhydryl-amide,
   (n) {4-[1-(Benzhydryl-carbamoyl)-3-(2-ethoxy-benzyl)-4-oxo-azetidin-2-yloxy]-phenyl}-acetic acid,
   (o) 3-{4-[1-(Benzhydryl-carbamoyl)-3-(2-ethoxy-benzyl)-4-oxo-azetidin-2-yloxy]-phenyl}-acrylic acid,
   (p) 4-[1-(Di-p-tolylmethyl-carbamoyl)-3-(2-ethoxy-benzyl)-4-oxo-azetidin-2-yloxy]-benzoic acid,
   (q) 4-[1-(Bis-4-fluoro-phenyl)-methyl-carbamoyl)-3-(2-ethoxy-benzyl)-4-oxo-azetidin-2-yloxy]-benzoic acid and
   (r) 4-[1-{[Bis-(4-methoxy-phenyl)-methyl]-carbamoyl}-3-(2-ethoxy-benzyl)-4-oxo-azetidin-2-yloxy]-benzoic acid.

The present invention provides a pharmaceutical composition, specifically a pharmaceutical composition for use as a chymase inhibitor and/or cytokine production inhibitor, more specifically, a pharmaceutical composition for use as an anti-inflammatory agent comprising the compound described in any one of 6) to 11), prodrug, pharmaceutically acceptable salt or hydrate thereof.

In other embodiment, the present invention provides a method for preventing and/or treating diseases caused by chymase comprising administering the compound described in any one of 6) to 11), prodrug, pharmaceutically acceptable salt or hydrate thereof, and use of the compound described in any one of 6) to 11), prodrug, pharmaceutically acceptable salt or hydrate thereof for manufacturing a medicament for preventing and/or treating diseases caused by chymase.

In the present specification, the term "halogen" includes fluorine, chlorine, bromine and iodine. Chlorine or bromine is preferable.

The term "lower alkyl" includes straight or branched chain alkyl having 1 to 10 carbon atoms, preferably 1 to 6 carbon atoms, more preferably 1 to 3 carbon atoms. For example, included are methyl, ethyl, n-propyl, isopropyl, n-butyl, isobutyl, sec-butyl, tert-butyl, n-pentyl, isopentyl, neopentyl, hexyl, isohexyl, n-heptyl, isoheptyl, n-octyl, isooctyl, n-nonyl and n-decyl.

The term "optionally substituted lower alkyl" includes lower alkyl optionally substituted with one or more of substituents at any possible positions. As the substituents, exemplified are hydroxy; halogen; lower alkoxy; carboxy; acyl; acyloxy; cycloalkyl; optionally substituted lower alkoxycarbonyl (wherein the substituent is amino optionally substituted with e.g. lower alkyl or aryl; optionally substituted amino wherein the substituent is e.g. lower alkyl or acyl; carbamoyl; optionally substituted aryl wherein the substituent is i)halogen, ii) optionally substituted lower alkyl wherein the substituent is carboxy, optionally substituted lower alkoxycarbonyl wherein the substituent is e.g. aryl or alkylamino, optionally substituted lower alkenyloxycarbonyl wherein the substituent is e.g. aryl or alkylamino, optionally substituted aryloxycarbonyl wherein the substituent is e.g. aryl or alkylamino, or optionally substituted heterocyclylcarbonyl wherein the substituent is e.g. lower alkyl or carbamoyl, iii) optionally substituted lower alkenyl wherein the substituent is carboxy, optionally substituted lower alkoxycarbonyl wherein the substituent is e.g. aryl or alkylamino, lower alkenyloxycarbonyl, aryloxycarbonyl, optionally substituted heterocyclylcarbonyl wherein the substituent is e.g. lower alkyl or carbamoyl iv) lower alkoxy, v) carboxy, vi) lower alkoxycarbonyl, vii) aryl, viii) acyl, ix) optionally substituted amino wherein the substituent is e.g. lower alkyl, optionally substituted carbamoyl wherein the substituent is optionally substituted lower alkyl wherein the substituent is e.g. lower alkylamino or aryl, optionally substituted lower alkenyl wherein the substituent is e.g. lower alkylamino or aryl, optionally substituted aryl wherein the substituent is e.g. lower alkylamino or aryl, xi) aryloxy, xii) heterocyclyl, xiii) optionally substituted heterocyclylcarbonyl wherein the substituent is e.g. lower alkyl or carbamoyl, xiv) lower alkylenedioxy, heterocyclyl; or optionally substituted heterocyclylcarbonyl wherein the substituent is e.g. lower alkyl. Preferable examples of "lower alkyl substituted with optionally substituted aryl" are unsubstituted benzyl, lower alkoxy benzyl and diphenylmethyl.

The alkyl parts of "lower alkoxy", "lower alkoxycarbonyl", "lower alkylamino" and "lower alkylthio" are the same as the above "lower alkyl", and their optional substituents are the same as the substituents for the above "optionally substituted alkyl".

The term "lower alkylene" includes straight or branched alkylene having 1 to 6 carbon atoms. For example, methylene, ethylene, trimethylene, tetramethylene, propylene and ethylethylene are included, and preferred is methylene.

The term "lower alkylenedioxy" includes methylenedioxy and ethylenedioxy and a preferable example is methylenedioxy.

The term "lower alkenyl" includes straight or branched alkenyl having 2 to 10 carbon atoms, preferably 2 to 6 carbon atoms, more preferably 2 to 4 carbon atoms. For example, vinyl, 1-propenyl, allyl, isopropenyl, butenyl, isobutenyl, butadienyl, pentenyl, isopentenyl, pentadienyl, hexenyl, isohexenyl, hexadienyl, heptenyl, octenyl, nonenyl and decenyl are included and these have at least one double bond at any possible position. As substituents for "optionally substituted lower alkenyl", exemplified are hydroxy, halogen, lower alkoxy, carboxy, acyl, acyloxy, cycloalkyl, lower alkoxycarbonyl, aryl, heterocyclyl, optionally substituted heterocyclylcarbonyl wherein the substituent is e.g. lower alkyl or carbamoyl and the lower alkenyl may be substituted with one or more of these substituents at any possible position.

The lower alkenyl part of "lower alkenyloxycarbonyl" and the substituents for "optionally substituted lower alkenyloxycarbonyl" are the same as the above.

The term "lower alkenylene" includes groups having one or more double bonds at any possible position in the above "lower alkylene" having 2 to 6 carbon atoms, preferably 2 to 4 carbon atoms. For example, vinylene, propenylene, butenylene, pentenylene and methylpropenylene are exemplified.

The term "lower alkynyl" means straight or branched alkynyl having 2 to 10 carbon atoms, preferably 2 to 6 carbon atoms, more preferably 2 to 4 carbon atoms and included are, for example, ethynyl, propynyl, butynyl, pentynyl, hexynyl, heptynyl, octynyl, nonynyl and decynyl. Lower alkynyl have at least one triple bond and further may have some double bonds at any possible position. The substituents for "optionally substituted lower alkynyl" are the same as those for the above "optionally substituted lower alkenyl".

The term "acyl" includes aliphatic acyl having 1 to 10 carbon atoms, preferably 1 to 6 carbon atoms, more preferably 1 to 3 carbon atoms or aroyl. For example, formyl, acetyl, propionyl, butyryl, isobutyryl, valeryl, pivaloyl, hexanoyl, acryloyl, propioloyl, methacryloyl, crotonoyl, cyclohexanecarbonyl and benzoyl are included. The substituents for "optionally substituted acyl" include hydroxy, halogen, lower alkoxy, carboxy, lower alkoxycarbonyl, aryl, and heterocyclyl, and the acyl may be substituted with one or more of these substituents at any possible position.

The acyl parts of "acyloxy" and "acylamino" and the substituents for "optionally substituted acyloxy" and "optionally substituted acylamino" are the same as the above "acyl" and the substituents for the above "optionally substituted acyl", respectively. A preferable example of "acyloxy" is acetyloxy.

The term "cycloalkyl" includes carbocyclyl having 3 to 6 carbon atoms and for example, cyclopropyl, cyclobutyl, cyclopentyl and cyclohexyl are included. As the substituents for "optionally substituted cycloalkyl", hydroxy, halogen, lower alkoxycarbonyl, lower alkoxy, aryl and heterocyclyl are exemplified and the cycloalkyl may be substituted with one or more of these substituents at any possible position.

The term "cycloalkenyl" includes a group having one or more double bonds at any possible position in the above "cycloalkyl". For example, cyclopropenyl, cyclobutenyl, cyclopentenyl, cyclohexenyl and cyclohexadienyl are included. The substituents for "optionally substituted cycloalkenyl" are the same as those for the above "cycloalkyl" and the cycloalkenyl may be substituted with one or more of these substituents at any possible position.

The term "optionally substituted amino" includes substituted amino and unsubstituted amino. The amino may have one or more substituents such as hydroxy, halogen, lower alkyl, lower alkylamino, acyl, carbamoyl, aryl and heterocyclyl.

The term "optionally substituted carbamoyl" includes substituted carbamoyl and unsubstituted carbamoyl. Examples of the substituents are optionally substituted lower alkyl such as unsubstituted lower alkyl, optionally substituted lower alkenyl such as unsubstituted lower alkenyl, lower alkylsulfonyl, sulfamoyl, acyl optionally substituted with halogen, and amino, optionally substituted aryl such as unsubstituted aryl.

The term "aryl" includes phenyl, naphthyl, anthrathenyl, indenyl and phenanthrenyl. Phenyl is preferable.

As the substituents for "optionally substituted aryl", exemplified are i) hydroxy, ii) halogen, iii) optionally substituted lower alkyl wherein the substituent is halogen; carboxy; optionally substituted lower alkoxycarbonyl wherein the substituent is e.g. lower alkylamino or aryl; optionally substituted lower alkenyloxycarbonyl wherein the substituent is e.g. lower alkylamino or aryl; optionally substituted aryloxycarbonyl wherein the substituent is e.g. lower alkylamino or aryl; optionally substituted heterocyclylcarbonyl wherein the substituent is e.g. lower alkyl or carbamoyl, iv) optionally substituted lower alkenyl wherein the substituent is halogen; carboxy; optionally substituted lower alkoxycarbonyl wherein the substituent is e.g. lower alkylamino or aryl; optionally substituted lower alkenyloxycarbonyl wherein the substituent is e.g. lower alkylamino or aryl; optionally substituted aryloxycarbonyl wherein the substituent is e.g. lower alkylamino or aryl; optionally substituted heterocyclylcarbonyl wherein the substituent is e.g. lower alkyl or carbamoyl v) optionally substituted lower alkoxy wherein the substituent is e.g. hydroxy, halogen, lower alkoxy, carboxyl, lower alkoxycarbonyl, amino or lower alkylamino, vi) carboxy, vii) optionally substituted lower alkoxycarbonyl wherein the substituent is acyloxy, lower alkylamino, optionally substituted aryl wherein the substituent is alkylenedioxy or halogen, heterocyclyl, viii) lower alkenyloxycarbonyl, ix) lower alkylenedioxy, x) acyl, xi) acyloxy, xii) optionally substituted amino wherein the substituent is e.g. lower alkyl, acyl, xiii) nitro, xiv) optionally substituted carbamoyl wherein the substituent is a) lower alkyl optionally substituted with carboxy; amino optionally substituted with lower alkyl or aroyl; lower alkoxycarbonyl optionally substituted with aryl; aryl optionally substituted with halogen, lower alkyl or lower alkoxy; b) cycloalkyl optionally substituted with e.g. aryl, c) lower alkenyl optionally substituted with e.g. lower alkylamino or aryl, d) amino optionally substituted with e.g. lower alkyl or aryl, e) aryl optionally substituted with e.g. lower alkylamino or aryl, f) arylsulfonyl xv) aryl, xvi) aryloxy, xvii) heterocyclyl or xviii) optionally substituted heterocyclylcarbonyl wherein the substituent is lower alkyl, arylalkyl optionally substituted with lower alkylenedioxy, cycloalkyl, carbamoyl, or heterocyclyl. The aryl may be substituted with one or more of these substituents at any possible position.

The aryl parts of "aryloxy", "arylsulfonyl" and "arylamino" are the same as the above "aryl", and the substituents for "optionally substituted aryloxy" and "optionally substituted arylsulfonyl" are the same as those for the above "optionally substituted aryl".

The term "optionally substituted benzyl" includes benzyl which may be substituted with lower alkyl or the same substituents as those for the above "optionally substituted lower alkyl" at the methylene part and may be substituted with the same substituents as those for the above "optionally substituted aryl" at the phenylene part. As the substituents for methylene part, lower alkyl and aryl are exemplified.

The term "heterocyclyl" includes heterocyclyl containing at least one hetero atom arbitrarily selected from a group of O, S and N. Examples of heterocyclyl include 5 to 6-membered aromatic heterocyclyl such as pyrrolyl, imidazolyl, pyrazolyl, pyridyl, pyridazinyl, pyrimidinyl, pyrazinyl, triazinyl, isoxazolyl, oxazolyl, oxadiazolyl, isothiazolyl, thiazolyl, thiadiazolyl, furyl and thienyl, fused aromatic heterocyclyl such as indolyl, benzimidazolyl, indazolyl, indolizinyl, quinolyl, isoquinolyl, cinnolinyl, phthalazinyl, quinazolinyl, naphthyridinyl, quinoxalinyl, pteridinyl, benzisoxazolyl, benzoxazolyl, benzoxadiazolyl, benzisothiazolyl, benzothiazolyl, benzothiadiazolyl, benzofuryl and benzothienyl, aliphatic heterocycle such as ethylene oxidyl, dioxanyl, thiiranyl, oxathioranyl, azetidinyl, thianyl, pyrrolidinyl, imidazolidinyl, pyrazolidinyl, piperidinyl, piperazinyl and morpholinyl.

As substituents for "optionally substituted heterocyclyl", exemplified are hydroxy, halogen, optionally substituted lower alkyl such as unsubstituted lower alkyl etc., lower alkenyl, lower alkoxy, carboxy, lower alkoxycarbonyl, optionally substituted carbamoyl such as unsubstituted carbamoyl etc., aryl and heterocyclyl. The heterocyclyl may be substituted with one or more of these substituents at any possible position.

The heterocyclyl part of "heterocyclylcarbonyl" and the substituents for "optionally substituted heterocyclylcarbonyl" are the same as the above "heterocyclyl" and those for "optionally substituted heterocyclyl", respectively. Preferable examples of "heterocyclylcarbonyl" are morpholylcarbonyl, piperazinylcarbonyl, methylpiperazinylcarbonyl, pyrimidinyl piperazinylcarbonyl, cyclohexylpiperazinylcarbonyl, piperidylcarbonyl and bipiperidylcarbonyl.

As pharmaceutically acceptable salt of the compound (I), exemplified are salts with mineral acids such as hydrochloric acid, sulfuric acid, nitric acid, phosphoric acid, hydrofluoric acid and hydrobromic acid; salts with organic acids such as formic acid, acetic acid, tartaric acid, lactic acid, citric acid, fumaric acid, maleic acid and succinic acid; salts with organic bases such as ammonium, trimethylammonium and triethylammonium; salts with alkali metals such as sodium and potassium and salts with alkaline earth metals such as calcium and magnesium.

The compound of the present invention includes hydrates, wherein arbitrary numbers of water molecules may coordinate to the compound (I), (I') or (I'').

The compound of the present invention includes racemates, all enantiomers and all stereoisomers such as diastereomers, epimers, and enantiomers thereof.

### Best Mode for Carrying Out the Invention

All of the compounds (I), (I') and (I'') have chymase inhibitory activity and/or cytokine production inhibitory activity and the following compounds are specifically preferable.

In the above formula (I), (I') or (I'')
1) the compound wherein A is -CO-, -CONH- or -SO₂-, and
   R¹ is optionally substituted lower alkyl or optionally substituted aryl (hereinafter referred to as "A and R¹ are AR¹-1"),
   preferably the compound wherein A-R¹ is 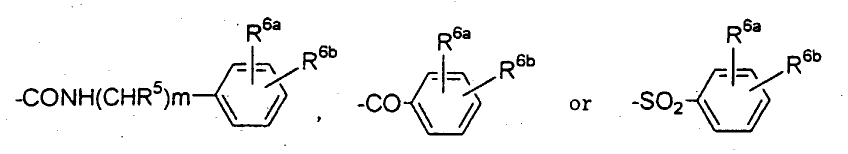
   wherein R⁵ is hydrogen, optionally substituted lower alkyl, optionally substituted lower alkenyl, optionally substituted lower alkoxy or optionally substituted aryl, R^{6a} and R^{6b} are each independently hydrogen, halogen, hydroxy, lower alkyl, carboxy, lower alkoxycarbonyl, lower alkoxy, aryl, acyl, optionally substituted amino, aryloxy, lower alkylthio or heterocyclyl and R^{6a} and R^{6b} taken together may form lower alkylenedioxy and m is 0 or 1 (hereinafter referred to as "A and R¹ are AR¹-2"), preferably the compound wherein A-R¹ is 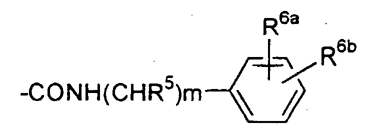
   wherein R⁵ is hydrogen, lower alkyl or optionally substituted phenyl wherein the substituent is halogen, lower alkyl or lower alkoxy, R^{6a} and R^{6b} are each independently hydrogen, halogen, lower alkyl or lower alkoxy and R^{6a} and R^{6b} taken together may form methylenedioxy and m is 1 (hereinafter referred to as "A and R¹ are AR¹-3"), preferably the compound wherein A-R¹ is 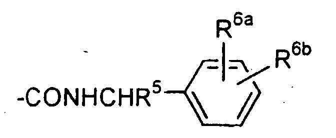
   wherein R⁵ is C1 to C3 alkyl or optionally substituted phenyl wherein the substituent is halogen, lower alkyl or lower alkoxy and R^{6a} and R^{6b} are each independently hydrogen, halogen, lower alkyl or lower alkoxy (hereinafter referred to as "A and R¹ are AR¹-4"), preferably the compound wherein A-R¹ is 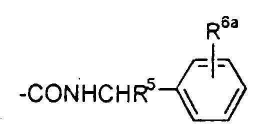
   wherein R⁵ is C1 to C3 alkyl or 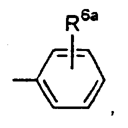 all R^{6a} are the same and hydrogen, halogen, lower alkyl or lower alkoxy (hereinafter referred to as "A and R¹ are AR¹-5"), preferably the compound wherein A-R¹ is 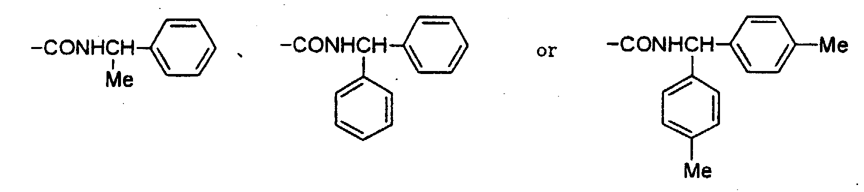 (hereinafter referred to as "A and R¹ are AR¹-5"),
2) the compound wherein R² is hydrogen, optionally substituted lower alkyl or optionally substituted aryl (hereinafter referred to as "R² is R²-1"), preferably the compound wherein R² is hydrogen, optionally substituted phenyl or optionally substituted benzyl (hereinafter referred to as R² is R²-2), preferably the compound wherein R² is 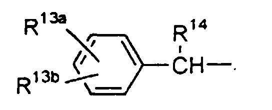
   wherein R^{13a} and R^{13b} are each independently hydrogen, halogen, hydroxy, optionally substituted lower alkyl, optionally substituted lower alkoxy, optionally substituted aryloxy, optionally substituted heterocyclyloxy, optionally substituted amino such as unsubstituted amino, lower alkylamino, arylamino, heterocyclylamino etc., optionally substituted lower alkylthio, optionally substituted arylthio, optionally substituted heterocyclylthio, aryl or heterocyclyl and R^{13a} and R^{13b} taken together may form lower alkylenedioxy, R¹⁴ is hydrogen, hydroxy, lower alkyl, lower alkoxy or acyloxy (hereinafter referred to as "R² is R²-3"),
   preferably the compound wherein R² is 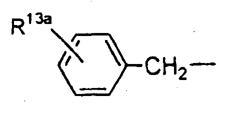
   wherein R^{13a} is hydrogen, lower alkyl, lower alkoxy, phenyloxy, lower alkylamino, phenylamino, lower alkylthio, phenylthio or phenyl (hereinafter referred to as "R² is R²-4"),
   preferably the compound wherein R² is 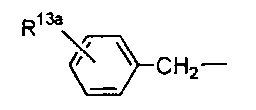
   wherein R^{13a} is hydrogen, lower alkyl, lower alkoxy, lower alkylamino or lower alkylthio (hereinafter referred to as "R² is R²-5"),
   preferably the compound wherein R² is benzyl optionally substituted with lower alkoxy (hereinafter referred to as "R² is R²-6"), and
   more preferably the compound wherein R² is benzyl optionally substituted with lower alkoxy at *o*-position (hereinafter referred to as "R² is R²-7"),
3) the compound wherein R³ is hydrogen, halogen, optionally substituted lower alkoxycarbonyl, optionally substituted acyl, optionally substituted amino, optionally substituted aryl or optionally substituted benzyl (hereinafter referred to as "R³ is R³-1"),
   preferably the compound wherein R³ is hydrogen, optionally substituted phenyl or optionally substituted benzyl (hereinafter referred to as "R³ is R³-2"), and
   preferably the compound wherein R³ is hydrogen (hereinafter referred to as "R³ is R³-3"),
4) the compound wherein B-R⁴ is hydrogen, optionally substituted acyloxy, 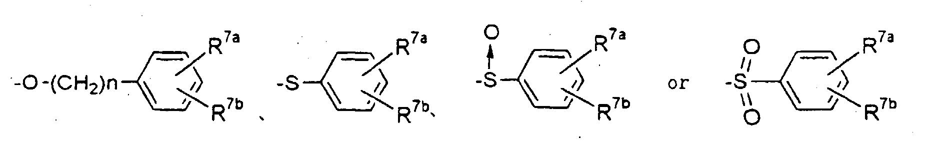
   wherein R^{7a} and R^{7b} are each independently hydrogen, halogen, lower alkyl, lower alkoxy, lower alkenyl, amino, acylamino, 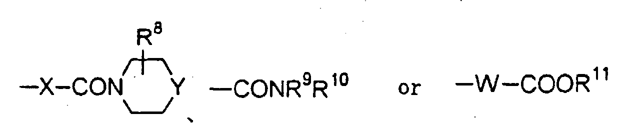
   wherein X and W are each independently a bond, lower alkylene or lower alkenylene, Y is a bond, -CH₂-, -NR¹²- (wherein R¹² is hydrogen, cycloalkyl, heterocyclyl or lower alkyl optionally substituted with methylenedioxyphenyl), or -O-, R⁸ is hydrogen, optionally substituted lower alkyl or optionally substituted carbamoyl, R⁹, R¹⁰ and R¹¹ are each independently hydrogen, optionally substituted lower alkyl, optionally substituted lower alkenyl, optionally substituted amino, optionally substituted aryl or optionally substituted arylsulfonyl, and n is an integer of 0 to 6 (hereinafter referred to as "B and R⁴ are B-R⁴-1"),
   preferably the compound wherein B-R⁴ is acyloxy, 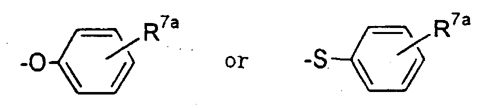
   wherein R^{7a} is hydrogen, 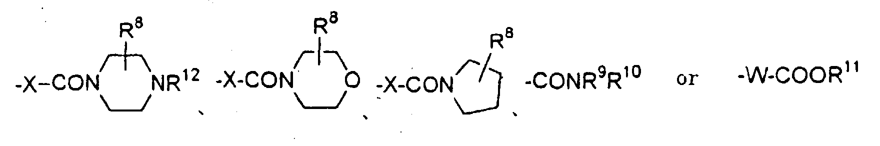
   wherein X and W are each independently a bond, methylene or vinylene, R⁸ is lower alkyl or carbamoyl, R⁹ is hydrogen or optionally substituted lower alkyl, R¹⁰ is hydrogen, optionally substituted lower alkyl, lower alkenyl, lower alkylamino, arylamino, phenyl or arylsulfonyl, R¹¹ is hydrogen, optionally substituted alkyl or optionally substituted phenyl, R¹² is cycloalkyl or lower alkyl optionally substituted with methylenedioxypheny (hereinafter referred to as "B and R⁴ are BR⁴-2"), preferably the compo 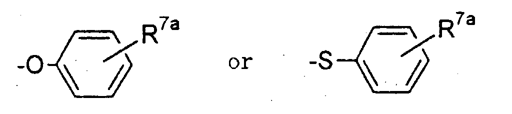 und wherein B-R⁴ is
   wherein R^{7a} is hydrogen, 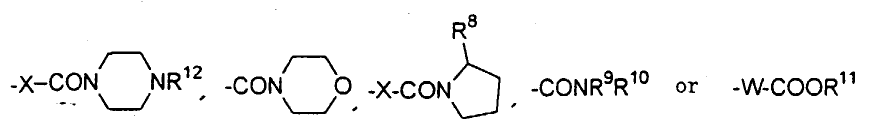
   wherein X and W are each independently a bond, methylene or vinylene, R⁸ is methyl or carbamoyl, R⁹ is hydrogen or lower alkyl, R¹⁰ is optionally substituted lower alkyl (wherein the substituent is lower alkylamino, phenyl optionally substituted with halogen, carboxy, or lower alkoxycarbonyl optionally substituted with aryl), lower alkenyl, lower alkylamino, phenylamino, phenyl, or benzenesulfonyl,
   R¹¹ is hydrogen or optionally substituted lower alkyl (wherein the substituent is lower alkylamino, acyloxy, phenyl optionally substituted with halogen or methylenedioxy, or heterocyclyl), and
   R¹² is C1 to C3 alkyl or cyclohexyl (hereinafter referred to as "B and R⁴ are BR⁴-3"), preferably the compound wherein B-R⁴ is 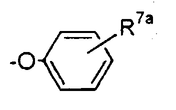
   wherein R^{7a} is hydrogen, 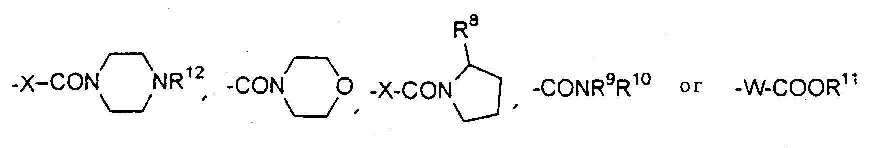
   wherein X and W are each independently a bond, methylene or vinylene, R⁸ is methyl or carbamoyl, R⁹ is hydrogen or lower alkyl, R¹⁰ is lower alkylamino(lower)alkyl; phenyl(lower)alkyl optionally substituted with halogen: lower alkenyl; phenylamino; or benzenesulfonyl; R¹¹ is hydrogen or lower alkyl optionally substituted with phenyl or heterocyclyl, and R¹² is C1 to C3 alkyl or cyclohexyl,
      (hereinafter referred to as "B and R⁴ are BR⁴-4")
   preferably the compound wherein B-R⁴ is 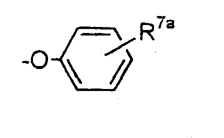
   wherein R^{7a} is 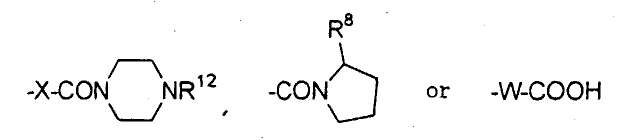
   wherein X is a bond or methylene, R⁸ is methyl or carbamoyl, W is a bond, methylene or vinylene, R¹² is methyl or cyclohexyl (hereinafter referred to as "B and R⁴ are BR⁴-5"), and
   most preferably the compound wherein B-R⁴ is 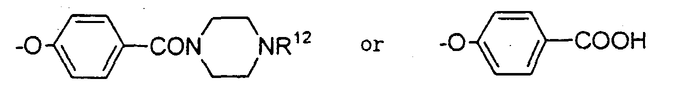
   wherein R¹² is methyl or cyclohexyl (hereinafter referred to as "B and R⁴ are BR⁴-6"),
5) the compound wherein R⁵ is hydrogen, optionally substituted lower alkyl, optionally substituted lower alkenyl, optionally substituted lower alkoxy or optionally substituted aryl (hereinafter referred to as "R⁵ is R⁵-1"),
   the compound wherein R⁵ is hydrogen, lower alkyl or phenyl optionally substituted with halogen, lower alkyl or lower alkoxy (hereinafter referred to as "R⁵ is R⁵-2"),
   the compound wherein R⁵ is C1 to C3 alkyl or phenyl optionally substituted with halogen, lower alkyl or lower alkoxy (hereinafter referred to as "R⁵ is R⁵-3"), and
   the compound wherein R⁵ is methyl or phenyl optionally substituted with lower alkyl (hereinafter referred to as "R⁵ is R⁵-4"),
6) the compound wherein R^{6a} and R^{6b} are each independently hydrogen, halogen, lower alkyl, lower alkoxycarbonyl or lower alkoxy, or R^{6a} and R^{6b} taken together may form lower alkylenedioxy (hereinafter referred to as "R⁶ is R⁶-1"),
   the compound wherein both of R^{6a} and R^{6b} are hydrogen, halogen, C1 to C3 alkyl or C1 to C3 alkoxy or R^{6a} and R^{6b} taken together may form methylenedioxy (hereinafter referred to as "R⁶ is R⁶-2"),
   the compound wherein both of R^{6a} and R^{6b} are hydrogen or C1 to C3 alkyl (hereinafter referred to as "R⁶ is R⁶-3"), and
   the compound wherein both of R^{6a} and R^{6b} are hydrogen (hereinafter referred to as "R⁶ is R⁶-4"),
7) the compound wherein R^{7a} is hydrogen, 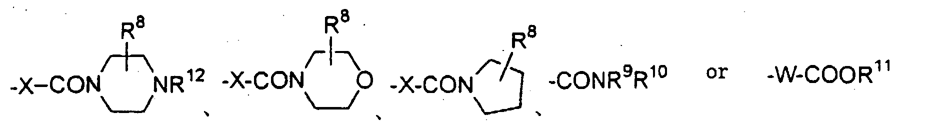
   wherein X and W are each independently a bond, methylene or vinylene, R⁸ is lower alkyl or carbamoyl, R⁹ is hydrogen or optionally substituted lower alkyl, R¹⁰ is hydrogen, optionally substituted lower alkyl, lower alkenyl, lower alkylamino, arylamino, phenyl or arylsulfonyl, R¹¹ is hydrogen, optionally substituted lower alkyl or optionally substituted phenyl, R¹² is cycloalkyl or lower alkyl optionally substituted with methylenedioxyphenyl and
   R^{7b} is hydrogen (hereinafter R^{7a} and R^{7b} are referred to as "R⁷ is R⁷-1"), the compound wherein R^{7a} is hydrogen, 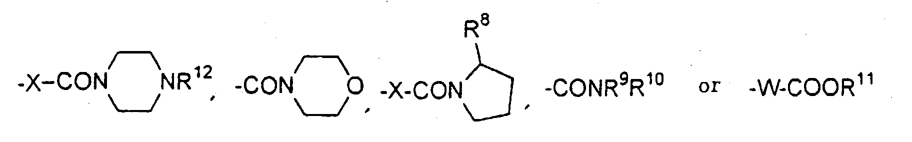
   wherein X and W are each independently a bond, methylene or vinylene, R⁸ is methyl or carbamoyl, R⁹ is hydrogen or lower alkyl, R¹⁰ is optionally substituted lower alkyl (wherein the substituent is lower alkylamino, phenyl optionally substituted with halogen, carboxy, or lower alkoxycarbonyl optionally substituted with aryl), lower alkenyl, lower alkylamino, phenylamino, phenyl or benzenesulfonyl, R¹¹ is hydrogen or optionally substituted lower alkyl (wherein the substituent is lower alkylamino, acyloxy, phenyl optionally substituted with halogen or methylenedioxy, or heterocyclyl) and R¹² is cyclohexyl or C1 to C3 alkyl, and
   R^{7b} is hydrogen (hereinafter R^{7a} and R^{7b} are referred to as "R⁷ is R⁷-2"), preferably the compound wherein R^{7a} is 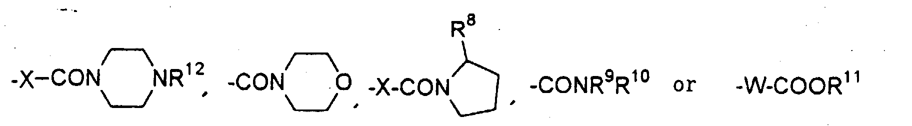
   wherein X and W are each independently a bond, methylene or vinylene, R⁸ is methyl or carbamoyl, R⁹ is hydrogen or lower alkyl, R¹⁰ is lower alkylamino(lower)alkyl or lower alkenyl, R¹¹ is hydrogen, lower alkylamino(lower)alkyl or benzyl, and R¹² is methyl or cyclohexyl, and
   R^{7b} is hydrogen (hereinafter R^{7a} and R^{7b} are referred to as "R⁷ is R⁷-3"), preferably the compound wherein R^{7a} is 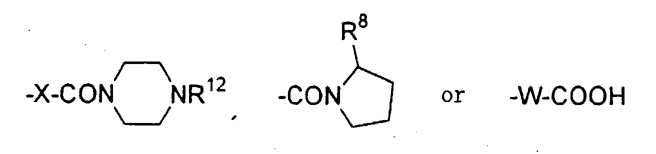
   wherein X is a bond or methylene, R⁸ is methyl or carbamoyl, W is a bond, methylene or vinylene, R¹² is methyl or cyclohexyl and
   R^{7b} is hydrogen (hereinafter R^{7a} and R^{7b} are referred to as "R⁷ is R⁷-4"), and most preferably the compound wherein R^{7a} is 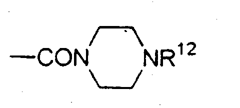 or -COOH
   wherein R¹² is methyl or cyclohexyl (hereinafter R^{7a} and R^{7b} are referred to as "R⁷ is R⁷-5"),
8) the compound wherein R⁸ is lower alkyl or carbamoyl (hereinafter referred to as R⁸ is R⁸-1"),
   preferably the compound wherein R⁸ is C1 to C3 alkyl or carbamoyl (hereinafter referred to as "R⁸ is R⁸-2"), and
   preferably the compound wherein R⁸ is methyl or carbamoyl (hereinafter referred to as "R⁸ is R⁸-3"),
9) the compound wherein R⁹ is hydrogen or optionally substituted lower alkyl (hereinafter referred to as "R⁹ is R⁹-1"),
   preferably the compound wherein R⁹ is hydrogen or lower alkyl (hereinafter referred to as "R⁹ is R⁹-2"), and
   preferably the compound wherein R⁹ is hydrogen or C1 to C3 alkyl (hereinafter referred to as "R⁹ is R⁹-3"),
10) the compound wherein R¹⁰ is hydrogen, optionally substituted lower alkyl, lower alkenyl, lower alkylamino, arylamino, phenyl or arylsulfonyl (hereinafter referred to as "R¹⁰ is R¹⁰-1"),
   preferably the compound wherein R¹⁰ is optionally substituted lower alkyl (wherein the substituent is lower alkylamino, phenyl optionally substituted with halogen, carboxy, or lower alkoxycarbonyl optionally substituted with aryl); lower alkenyl; lower alkylamino; phenylamino; phenyl; or benzenesulfonyl (hereinafter referred to as "R¹⁰ is R¹⁰-2"), more preferably the compound wherein R¹⁰ is lower alkyl optionally substituted with lower alkylamino, phenyl optionally substituted with halogen, carboxy or aryl(lower)alkoxycarbonyl; lower alkenyl; or phenylamino (hereinafter referred to as "R¹⁰ is R¹⁰-3"), and
   preferably the compound wherein R¹⁰ is lower alkylamino(lower)alkyl,
   phenyl(lower)alkyl, halogenophenyl(lower)alkyl or phenylamino (hereinafter referred to as "R¹⁰ is R¹⁰-4")
11) the compound wherein R¹¹ is hydrogen, optionally substituted lower alkyl or optionally substituted phenyl (hereinafter referred to as "R¹¹ is R¹¹-1"),
   preferably the compound wherein R¹¹ is hydrogen or optionally substituted lower alkyl wherein the substituent is lower alkylamino, acyloxy, optionally substituted phenyl wherein the substituent is halogen or methylenedioxy, or heterocyclyl (hereinafter referred to as "R¹¹ is R¹¹-2"),
   preferably the compound wherein R¹¹ is hydrogen, lower alkylamino(lower)alkyl or phenylalkyl (hereinafter referred to as "R¹¹ is R¹¹-3"), and
   most preferably the compound wherein R¹¹ is hydrogen (hereinafter referred to as "R¹¹ is R¹¹-4"),
12) the compound wherein R¹² is cycloalkyl, pyrimidyl or lower alkyl optionally substituted with methylenedioxyophenyl (hereinafter referred to as "R¹² is R¹²-1"),
   the compound wherein R¹² is cycloalkyl or lower alkyl optionally substituted with methylenedioxyphenyl (hereinafter referred to as "R¹² is R¹²-2"),
   the compound wherein R¹² is C1 to C3 alkyl or cycloalkyl (hereinafter referred to as "R¹² is R¹²-3"), and
   most preferably the compound wherein R¹² is methyl or cyclohexyl (hereinafter referred to as "R¹² is R¹²-4"),
13) the compound wherein R^{13a} and R^{13b} are each independently hydrogen, halogen, hydroxy, optionally substituted lower alkyl, optionally substituted lower alkoxy, optionally substituted amino or optionally substituted lower alkylthio, and R^{13a} and R^{13b} taken together may form methylenedioxy (hereinafter referred to as "R¹³ is R¹³-1"),
   preferably the compound wherein R^{13a} is hydrogen, lower alkyl, lower alkoxy, lower alkylamino or lower alkylthio and R^{13b} is hydrogen (hereinafter referred to as "R¹³ is R¹³-2"),
   the compound wherein R^{13a} and R^{13b} are each independently hydrogen or lower alkoxy (hereinafter referred to as "R¹³ is R¹³-3"),
   the compound wherein R^{13a} is hydrogen or C1 to C3 lower alkoxy at *o*-position and R^{13b} is hydrogen (hereinafter referred to as "R¹³ is R¹³-4"), and
   the compound wherein both of R^{13a} and R^{13b} are hydrogen (hereinafter referred to as "R¹³ is R¹³-5"),
14) the compound wherein R¹⁴ is hydrogen (hereinafter referred to as "R¹⁴ is R¹⁴-1"),
15) the compound wherein A and R¹ are AR¹-1, R² is R²-1 and R³ is R³-1,
   preferably the compound wherein A and R¹ are AR¹-2, R² is R²-2 and R³ is R³-2,
   more preferably the compound wherein A and R¹ are AR¹-3, R² is R²-3 and R³ is R³-3,
   preferably the compound wherein A and R¹ are AR¹-4, R² is R²-4 and R³ is R³-3,
   preferably the compound wherein A and R¹ are AR¹-5, R² is R²-5 and R³ is R³-3,
   preferably the compound wherein A and R¹ are AR¹-5, R² is R²-6 and R³ is R³-3, and
   most preferably the compound wherein A and R¹ are AR¹-6, R² is R²-7 and R³ is R³-3,
16) the compound wherein A and R1 are AR¹-1, R² is R²-1 and BR⁴ is BR⁴-1,
   preferably the compound wherein A and R¹ are AR¹-2, R² is R²-2 and BR⁴ is BR⁴-2,
   preferably the compound wherein A and R¹ are AR¹-3, R² is R²-3 and BR⁴ is BR⁴-3,
   preferably the compound wherein A and R¹ are AR¹-4, R² is R²-4 and BR⁴ is BR⁴-4,
   preferably the compound wherein A and R¹ are AR¹-5, R² is R²-5 and BR⁴ is BR⁴-5,
   preferably the compound wherein A and R¹ are AR¹-5, R² is R²-6 and BR⁴ is BR⁴-5,
   most preferably the compound wherein A and R¹ are AR¹-5, R² is R²-7 and BR⁴ is BR⁴-5,
17) the compound wherein A and R¹ are AR¹-1, R³ is R³-1 and BR⁴ is BR⁴-1,
   preferably the compound wherein A and R¹ are AR¹-2, R³ is R³-2 and BR⁴ is BR⁴-2,
   preferably the compound wherein A and R¹ are AR¹-3, R³ is R³-3 and BR⁴ is BR⁴-3,
   preferably the compound wherein A and R¹ are AR¹-4, R³ is R³-3 and BR⁴ is BR⁴-4,
   mots preferably the compound whereinA and R¹ are AR¹-5, R³ is R³-3 and BR⁴ is BR⁴-5,
18) the compound wherein R² is R²-1, R³ is R³-1 and BR⁴ is BR⁴-1,
   preferably the compound wherein R² is R²-2, R³ is R³-2 and BR⁴ is BR⁴-2,
   preferably the compound wherein R² is R²-3, R³ is R³-3 and BR⁴ is BR⁴-3,
   preferably the compound wherein R² is R²-4, R³ is R³-3 and BR⁴ is BR⁴-4,
   preferably the compound wherein R² is R²-5, R³ is R³-3 and BR⁴ is BR⁴-4,
   preferably the compound wherein R² is R²-6, R³ is R³-3 and BR⁴ is BR⁴-4,
   preferably the compound wherein R² is R²-7, R³ is R³-3 and BR⁴ is BR⁴-4,
   most preferably the compound wherein R² is R²-7, R³ is R³-3 and BR⁴ is BR⁴-5,
19) the compound wherein the combination of A and R¹, R², R³, and B and R⁴ is as follows:
   (AR¹-1, R²-1, R³-2, BR⁴-2), (AR¹-1, R²-1, R³-3, BR⁴-3),
   (AR¹-1, R²-2, R³-1, BR⁴-2), (AR¹-1, R²-2, R³-1, BR⁴-3), (AR¹-1, R²-2, R³-1, BR⁴-4),
   (AR¹-1, R²-2, R³-1, BR⁴-5), (AR¹-1, R²-2, R³-2, BR⁴-1), (AR¹-1, R²-2, R³-2, BR⁴-2),
   (AR¹-1, R²-2, R³-2, BR⁴-3), (AR¹-1, R²-2, R³-2, BR⁴-4), (AR¹-1, R²-2, R³-2, BR⁴-5),
   (AR¹-1, R²-2, R³-3, BR⁴-2), (AR¹-1, R²-2, R³-3, BR⁴-3), (AR¹-1, R²-2, R³-3, BR⁴-4),
   (AR¹-1, R²-2, R³-3, BR⁴-5),
   (AR¹-1, R²-3, R³-1, BR⁴-2), (AR¹-1, R²-3, R³-1, BR⁴-3), (AR¹-1, R²-3, R³-1, BR⁴-4),
   (AR¹-1, R²-3, R³-1, BR⁴-5), (AR¹-1, R²-3, R³-2, BR⁴-2), (AR¹-1, R²-3, R³-2, BR⁴-3),
   (AR¹-1, R²-3, R³-2, BR⁴-4), (AR¹-1, R²-3, R³-2, BR⁴-5), (AR¹-1, R²-3, R³-3, BR⁴-1),
   (AR¹-1, R²-3, R³-3, BR⁴-2), (AR¹-1, R²-3, R³-3, BR⁴-3), (AR¹-1, R²-3, R³-3, BR⁴-4),
   (AR¹-1 R²-3, R³-3, BR⁴-5),
   (AR¹-1, R²-4, R³-1, BR⁴-2), (AR¹-1, R²-4, R³-1, BR⁴-3), (AR¹-1, R²-4, R³-1, BR⁴-4),
   (AR¹-1, R²-4, R³-1, BR⁴-5), (AR¹-1, R²-4, R³-2, BR⁴-2), (AR¹-1, R²-4, R³-2, BR⁴-3),
   (AR¹-1, R²-4, R³-2, BR⁴-4), (AR¹-1, R²-4, R³-2, BR⁴-5), (AR¹-1, R²-4, R³-3, BR⁴-2),
   (AR¹-1, R²-4, R³-3, BR⁴-3), (AR¹-1, R²-4, R³-3, BR⁴-4), (AR¹-1, R²-4, R³-3, BR⁴-5),
   (AR¹-1, R²-6, R³-1, BR⁴-2), (AR¹-1, R²-6, R³-1, BR⁴-3), (AR¹-1, R²-6, R³-1, BR⁴-4),
   (AR¹-1, R²-6, R³-1, BR⁴-5), (AR¹-1, R²-6, R³-2, BR⁴-2), (AR¹-1, R²-6, R³-2, BR⁴-3),
   (AR¹-1, R²-6, R³-2, BR⁴-4), (AR¹-1, R²-6, R³-2, BR⁴-5), (AR^{¹}-1, R²-6, R³-3, BR⁴-2),
   (AR¹-1, R²-6, R³-3, BR⁴-3), (AR¹-1, R²-6, R³-3, BR⁴-4), (AR¹-1, R²-6, R³-3, BR⁴-5),
   (AR¹-2, R²-1, R³-1, BR⁴-2), (AR¹-2, R²-1, R³-2, BR⁴-1),
   (AR¹-2, R²-2, R³-1, BR⁴-1), (AR¹-2, R²-2, R³-3, BR⁴-3),
   AR¹-2, R²-3, R³-2, BR⁴-3), (AR¹-2, R²-3, R³-3, BR⁴-2),
   (AR¹-2, R²-4, R³-2, BR⁴-5),
   (AR¹-2, R²-6, R³-3, BR⁴-3),
   (AR¹-2, R²-7, R³-3, BR⁴-3),
   (AR¹-3, R²-2, R³-1, BR⁴-2), (AR¹-3, R²-2, R³-1, BR⁴-3), (AR¹-3, R²-2, R³-1, BR⁴-4),
   (AR¹-3, R²-2, R³-1, BR⁴-5), (AR¹-3, R²-2, R³-2, BR⁴-2), (AR¹-3, R²-2, R³-2, BR⁴-3),
   (AR¹-3, R²-2, R³-2, BR⁴-4), (AR¹-3, R²-2, R³-2, BR⁴-5), (AR¹-3, R²-2, R³-3, BR⁴-2),
   (AR¹-3, R²-2, R³-3, BR⁴-3), (AR¹-3, R²-2, R³-3, BR⁴-4), (AR¹-3, R²-2, R³-3, BR⁴-5),
   (AR¹-3, R²-3, R³-1, BR⁴-2), (AR¹-3, R²-3, R³-1, BR⁴-3), (AR¹-3, R²-3, R³-1, BR⁴-4),
   (AR¹-3, R²-3, R³-1, BR⁴-5), (AR¹-3, R²-3, R³-2, BR⁴-2), (AR¹-3 R²-3, R³-2, BR⁴-3),
   (AR¹-3, R²-3, R³-2, BR⁴-4), (AR¹-3, R²-3, R³-2, BR⁴-5), (AR¹-3, R²-3, R³-3, BR⁴-2),
   (AR¹-3, R²-3, R³-3, BR⁴-3), (AR¹-3, R²-3, R³-3, BR⁴-4), (AR¹-3, R²-3, R³-3, BR⁴-5),
   (AR¹-3, R²-4, R³-1, BR⁴-2), (AR¹-3, R²-4, R³-1, BR⁴-3), (AR¹-3, R²-4, R³-1, BR⁴-4),
   (AR¹-3, R²-4, R³-1, BR⁴-5), (AR¹-3, R²-4, R³-2, BR⁴-2), (AR¹-3, R²-4 R³-2, BR⁴-3),
   (AR¹-3, R²-4, R³-2, BR⁴-4), (AR¹-3, R²-4, R³-2, BR⁴-5), (AR¹-3, R²-4, R³-3, BR⁴-2),
   (AR¹-3, R²-4, R³-3, BR⁴-3), (AR¹-3, R²-4, R³-3, BR⁴-4), (AR¹-3, R²-4, R³-3, BR⁴-5),
   (AR¹-3, R²-5, R³-3, BR⁴-3),
   (AR¹-3, R²-6, R³-1, BR⁴-2), (AR¹-3, R²-6, R³-1, BR⁴-3), (AR¹-3, R²-6, R³-1, BR⁴-4),
   (AR¹-3, R²-6, R³-1, BR⁴-5), (AR¹-3, R²-6, R³-2, BR⁴-2), (AR¹-3, R²-6, R³-2, BR⁴-3),
   (AR¹-3, R²-6, R³-2, BR⁴-4), (AR¹-3, R²-6, R³-2, BR⁴-5), (AR¹-3, R²-6, R³-3, BR⁴-2),
   (AR¹-3, R²-6, R³-3, BR⁴-3), (AR¹-3, R²-6, R³-3, BR⁴-4), (AR¹-3, R²-6, R³-3, BR⁴-5),
   (AR¹-3, R²-7 R³-3, BR⁴-6),
   (AR¹-4, R²-7, R³-3, BR⁴-1), (AR¹-4, R²-7, R³-3, BR⁴-3),
   (AR¹-5, R²-2, R³-1, BR⁴-2), (AR¹-5, R²-2, R³-1, BR⁴-3), (AR¹-5, R²-2, R³-1, BR⁴-4),
   (AR¹-5, R²-2, R³-1, BR⁴-5), (AR¹-5, R²-2, R³-2, BR⁴-2), (AR¹-5, R²-2, R³-2, BR⁴-3),
   (AR¹-5, R²-2, R³-2, BR⁴-4), (AR¹-5, R²-2, R³-2, BR⁴-5), (AR¹-5, R²-2, R³-3, BR⁴-2),
   (AR¹-5, R²-2, R³-3, BR⁴-3), (AR¹-5, R²-2, R³-3, BR⁴-4), (AR¹-5, R²-2, R³-3, BR⁴-5),
   (AR¹-5, R²-3, R³-1, BR⁴-2), (AR¹-5, R²-3, R³-1, BR⁴-3), (AR¹-5, R²-3, R³-1, BR⁴-4),
   (AR¹-5, R²-3, R³-1, BR⁴-5), (AR¹-5, R²-3, R³-2, BR⁴-2), (AR¹-5, R²-3, R³-2, BR⁴-3),
   (AR¹-5, R²-3, R³-2, BR⁴-4), (AR¹-5, R²-3, R³-2, BR⁴-5), (AR¹-5, R²-3, R³-3, BR⁴-2),
   (AR¹-5, R²-3, R³-3, BR⁴-3), (AR¹-5, R²-3, R³-3, BR⁴-4), (AR¹-5, R²-3, R³-3, BR⁴-5),
   (AR¹-5, R²-4, R³-1, BR⁴-2), (AR¹-5, R²-4, R³-1, BR⁴-3), (AR¹-5, R²-4, R³-1, BR⁴-4),
   (AR¹-5, R²-4, R³-1, BR⁴-5), (AR¹-5, R²-4, R³-2, BR⁴-2), (AR¹-5, R²-4, R³-2, BR⁴-3),
   (AR¹-5, R²-4, R³-2, BR⁴-4), (AR¹-5, R²-4, R³-2, BR⁴-5), (AR¹-5, R²-4, R³-3, BR⁴-2),
   (AR¹-5, R²-4, R³-3, BR⁴-3), (AR¹-5, R²-4, R³-3, BR⁴-4), (AR¹-5, R²-4, R³-3, BR⁴-5),
   (AR¹-5, R²-6, R³-1, BR⁴-2), (AR¹-5, R²-6, R³-1, BR⁴-3), (AR¹-5, R²-6, R³-1, BR⁴-4),
   (AR¹-5, R²-6, R³-1, BR⁴-5), (AR¹-5, R²-6, R³-2, BR⁴-2), (AR¹-5, R²-6, R³-2, BR⁴-3),
   (AR¹-5, R²-6, R³-2, BR⁴-4), (AR¹-5, R²-6, R³-2, BR⁴-5), (AR¹-5, R²-6, R³-3, BR⁴-2),
   (AR¹-5, R²-6, R³-3, BR⁴-3), (AR¹-5, R²-6, R³-3, BR⁴-4), (AR¹-5, R²-6, R³-3, BR⁴-5),
   (AR¹-5, R²-6, R³-3, BR⁴-6),
   (AR¹-5, R²-7, R³-3, BR⁴-1), (AR¹-5, R²-7, R³-3, BR⁴-3), (AR¹-5, R²-7, R³-3, BR⁴-6),
   (AR¹-6, R²-1, R³-1, BR⁴-3), (AR¹-6, R²-1, R³-3, BR⁴-1),
   (AR¹-6, R²-2, R³-2, BR⁴-3), (AR¹-6, R²-2, R³-3, BR⁴-2),
   (AR¹-6, R²-3, R³-1, BR⁴-1), (AR¹-6, R²-3, R³-2, BR⁴-2),
   (AR¹-6, R²-4, R³-3, BR⁴-5),
   (AR¹-6, R²-6, R³-3, BR⁴-6)
   (AR¹-6, R²-7, R³-3, BR⁴-1), (AR¹-6, R²-7, R³-3, BR⁴-2), (AR¹-6, R²-7, R³-3, BR⁴-3),
   (AR¹-6, R²-7, R³-3, BR⁴-4), (AR¹-6, R²-7, R³-3, BR⁴-5) and (AR¹-6, R²-7 R³-3, BR⁴-6)
20) the compound wherein A-R¹ is -CONHCHR⁵Ph and R² is benzyl, R³ is C1 to C3 alkyl, B-R⁴ is 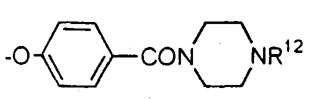 and R⁵ and R¹² are each independently C1 to C3 alkyl,
21) the compound wherein carbon atoms at the 3- and 4-positions are asymmetric carbon atoms and the configuration is 3-β, preferably the compound wherein the configuration is 3-β, 4-β.

The compound (I) of the present invention can be synthesized by obtaining azetidin-2-on compound from vinyl acetate according to the method described in Org. Synth. 1986, 65, 135, followed by introducing objective substituents by the usual methods.

For example, the compound of the above formula (I) wherein A is -CO- can be obtained by synthesizing an azetidin-2-on compound wherein A-R¹ is hydrogen to react with an acid anhydride or a halogenide having an objective substituent R¹. The reaction may be carried out in a solvent such as dimethylformamide, tetrahydrofuran, dichloromethane or dioxane, in the presence of an organic base such as pyridine, DMAP, triethylamine or diisopropylethylamine or a base such as sodium hydride, lithium hydride, potassium hydride, or lithium bis(trimethylsilyl)amide, under ―60 °C to heating, preferably ―50 °C to 50 °C for several minutes to several hours, preferably 1 to 3 hours.

A compound wherein A is -COO- can be obtained by a reaction of a compound wherein A-R¹ is hydrogen with Hal-COOR¹ in the presence of a base such as potassium carbonate, sodium hydride, LiHMDS or LDA. The reaction may be carried out in a solvent such as dimethylformamide, tetrahydrofuran, dichloromethane or dioxane at ―60 °C to room temperature for several minutes to several hours to obtain the objective compound.

A compound wherein A is -COCO- can be obtained by a reaction of a compound wherein A-R¹ is hydrogen with di-ketone-halogen compound having the substituent R¹. The reaction may be carried out in a solvent such as dimethylformamide, tetrahydrofuran, dichloromethane or dioxane at ―60 °C to room temperature for several minutes to several hours.

A compound wherein A is -CONH- may be obtained by a reaction of a compound wherein A-R¹ is hydrogen with an isocyanate compound having an objective substituent R¹. The reaction may be carried out in a solvent such as methylene chloride, acetonitrile, dimethylformamide, tetrahydrofuran or dioxane in the presence of an organic base such as DBU, pyridine, DMAP, triethylamine or diisopropylethylamine or a base such as sodium hydride, lithium hydride, or potassium hydride under ice-cooling to heating, preferably at 5 °C to 25 °C for several minutes to several hours, preferably 5 to 16 hours.

A compound wherein A is -CONH- can be obtained by a reaction of, R¹COOH with an azide compound such as diphenylphosphoryl azide or sodium azide to give an isocyanate compound via acyl azide, followed by a reaction with an azetizine-2-one compound wherein A-R¹ is hydrogen (Curtius rearrangement). R¹COOH and the azide compound may be reacted in a solvent such as methylene chloride, acetonitrile, toluene, t-butyl alcohol, benzyl alcohol, tetrahydrofuran or dioxane in the presence of an organic base such as pyridine, DBU, DMAP, triethylamine or diisopropylethylamine, or a base such as sodium hydride, lithium hydride or potassium hydride under ice-cooling to heating, preferably at 0 °C to 50 °C for several minutes to several hours, preferably 1 to 16 hours.

A compound wherein A is -SO₂- can be obtained by a reaction of an azetizine-2-one compound wherein A-R¹ is hydrogen with a sulfonyl halide compound having an objective substituent R¹. The reaction may be carried out in a solvent such as dichloromethane, dimethylformamide, toluene. tetorahydrofuran or dioxane, in the presence of an organic base such as pyridine, DMAP, triethylamine, or diisopropylethylamine or a base such as sodium hydride, lithium hydride, potassium hydride or lithium bis(trimethylsilyl)amide at ―80°C to heating, preferably ―60°C to 25°C for several minutes to several hours, preferably about 2 hours to obtain the objective compound.

Alternatively, the objective compound can be synthesized by reacting sulfonylisocyanate with silylenol ether according to the method described in J. Organomet. Chem., 164 (1979) 123 ― 134.

A compound wherein BR⁴ is -S- or -O- can be obtained by a reaction of a compound wherein BR⁴ is acyloxy, which can be synthesized according to the method described in the above-mentioned Org. Synth. 1986, 65, 135, with a mercapt compound or a hydroxy compound having an objective substituent R⁴. The reaction may be carried out in a solvent such as acetone, methanol, ethanol, dimethylformamide, tetrahydrofuran or dioxane in the presence of an organic base such as pyridine, DMAP, triethylamine or diisopropylethylamine or a base such a sodium hydride, lithium hydride, potassium hydride or sodium hydroxide under ic-cooling to heating, preferably at 0 °C to 50 °C for several minutes to several hours, preferably 3 hours.

A compound wherein B is -SO₂- or -SO- can be synthesized by oxidation of the above-mentioned compound wherein B is -S- obtained. The oxidation may be carried out in a solvent such as methylene chloride or tetrahydrofuran, with an oxidizing agent such as m-chloro perbenzoic acid, peracetic acid, perbenzoic acid, hydrogen peroxide, pertrifluoroacetic acid, sodium periodic acid, sodium hypochlorite, or potassium permanganic acid under ice-cooling to heating, preferably at 0 °C to 50 °C for several minutes to several hours, preferably 3 hours.

A compound wherein BR⁴ is hydrogen can be synthesized by reducing the compound wherein R⁴ is phenylthio which is obtained by the above-mentioned method or the usual methods. The compound may be reduced in a solvent such as benzene or toluene with an reducing agent such as tributyltin under ice-cooling to heating, preferably at 0 °C to 150 °C for several minutes to several hours, preferably 1 hour. In this reaction, the objective compound can be preferably synthesized in the presence of a free radical initiator such as AIBN or dibenzoylperoxide.

A compound wherein R² or R³ is the substituent other than hydrogen can be obtained by a reaction of an azetizine-2-one compound wherein R² and R³ are simultaneously hydrogen with a halogen compound having the objective substituent R² or R³. These compounds may be reacted in a solvent such as tetrahydrofuran or diethyl ether at -80°C to room temperature, preferably -60°C to 0°C for several minutes to several hours, preferably 2 hours.

Each substituent of thus obtained compounds can be converted into a suitable substituent by the usual methods.

When a compound has a substituent interfering with the above reaction, the substituent may be protected with a suitable protecting group in advance and the protecting group may be removed in a suitable step by the usual methods. For example, lower alkoxycarbonyl such as t-butyloxycarbonyl, lower alkenyloxycarbonyl such as vinyloxycarbonyl, or aryloxycarbonyl, aralkyloxycarbonyl such as benzyloxycarbonyl, p-methoxybenzylcarbonyl, o-nitrobenzyloxycarbonyl, p-nitrobenzyloxycarbonyl, or phenyloxycarbonyl, tri(lower)alkylsilyl such as trimethylsilyl, triehylsilyl or t-butyldimethylsilyl, acyl such as acetyl, halogenoacetyl, pivaloyl, benzoyl, or toluoyl, lower alkylsulfonyl such as methanesulfonyl, trifluoroethanesulfonyl, toluenesulfonyl, or 4-t-butylbenzenesulfonyl can be used as an amino protecting group.

Thus obtained compound of the present invention can be converted into a prodrug thereof. The term "prodrug" includes derivatives of the compounds of the present invention which have a chemically or metabolically decomposable group and can be converted into pharmaceutically active compounds of the present invention in vivo by solvolysis or under the physiological conditions. The methods for selecting and producing suitable prodrugs are described in Design of Prodrugs, Elsevier, Amsterdam 1985.

When a compound of the present invention has carboxy, examples of prodrugs are an ester derivative, or an amide derivative, which can be produced by reacting a carboxy compound with a suitable alcohol or a suitable amine, respectively. More preferable ester derivatives as prodrugs are methyl ester, ethyl ester, n-propyl ester, isopropyl ester, n-butyl ester, isobutyl ester, tert-butyl ester, molpholinoethyl ester, and N, N-diethylglycolamide ester.

When a compound of the present invention has hydroxy, an example of a prodrug is an acyloxy derivative, which can be synthesized by reacting a compound having hydroxy with a suitable acyl halide or a suitable acid anhydride. Acyloxy groups preferable for prodrugs are -OCOC₂H₅, -OCO(t-Bu), -OCOC₁₅H₃₁, -OCO(m-COONa-Ph), -OCOCH₂CH₂COONa, -OCOCH(NH₂)CH₃, and -OCOCH₂N(CH₃)₂.

When a compound of the present invention has amino, an example of a prodrug is an amide derivative, which can be synthesized by reacting a compound having amino with a suitable acid halogeno compound or a suitable mixed acid anhydride. Amide groups preferable for prodrugs are -NHCO(CH₂)₂₀CH₃, -NHCOCH(NH₂)CH₃, and -NHCOOCH₂OCOCH₃.

Chymase inhibitors of the present invention have a high oral absorbability and stability in blood as well as potent chymase inhibitory activity, and they are effective in all of the diseases caused by angiotensin II or chymase. Additionally, they have cytokine production inhibitory activity and show potent preventive and/or therapeutic effect for inflammatory diseases, allergic diseases and circulatory system diseases.

The objective diseases are, for example, various postoperative organ adhesions, stenosis after vascular transplantation, dysfunction or insufficiency of a transplanted tissue, aberrant growth or hyperplasia of a transplanted organ and peripheral tissue, formation of keloid and cicatrice, chronic inflammatory diseases with fibrosis such as cardiac insufficiency or myocardosis after myocardial infarction, cystic fibrosis, interstitial fibrosis, rheumatics, asthma, atopic dermatitis, non-atopic dermatitis, arthritis, psoriasis, hepatitis, hepatocirrhosis, inflammatory eye diseases such as conjunctivitis, scleroderma, nephritis, colitis, Crohn disease, septic shock, myocardial infarction, cardiac insufficiency, hypercardia, cardiac myopathy, congestive heat diseases, hypertension, vascular intimal hyperplasia after PTCA (percutaneous transluminal coronary angioplasty), peripheral circulatory disorder, vasculitis, arteriosclerosis, revascularization, diabetic or non-diabetic nephropathy, stroke and Alzheimer's disease. The chymase inhibitor can be used also as an immunosuppressive agent.

While having potent chymase inhibitory activity, the chymase inhibitor of the present invention has no or very weak inhibitory activity on elastase, trypsin, thrombin, and plasmin which are serine proteases like chymase. Thus, the chymase inhibitor of the present invention has a high selectivity for chymase and can be a reagent useful for physiological research of chymase.

A compound of the present invention can be administered orally or parenterally as a chymase inhibitor and/or cytokine production inhibitor. In the case of oral administration, it may be in any usual form such as tablets, granules, powders, capsules, pills, solutions, syrups, buccal tablets and sublingual tablets. When the compound is parenterally administered, any usual form is preferable, for example, injections (e.g., intravenous, intramuscular), suppositories, endermic agents, vapors and ophthalmic solutions. Oral administration is particularly preferable because the compounds of the present invention show high oral absorbability.

A pharmaceutical composition may be manufactured by mixing an effective amount of a compound of the present invention with various pharmaceutical additives suitable for the administration form, such as excipients, binders, moistening agents, disintegrators, lubricants and diluents. When the composition is for injection, an active ingredient can be sterilized with a suitable carrier to give a pharmaceutical composition.

Examples of excipients include lactose, saccharose, glucose, starch, calcium carbonate and crystalline cellulose. Examples of binders include methylcellulose, carboxymethylcellulose, hydroxypropylcellulose, gelatin and polyvinylpyrrolidone. Examples of disintegrators include carboxymethylcellulose, sodium carboxymethylcellulose, starch, sodium alginate, agar and sodium lauryl sulfate. Examples of lubricants include talc, magnesium stearate and macrogol. Cacao oil macrogol and methyl cellulose may be used as base materials for suppositories. When the composition is manufactured as solutions, emulsified injections or suspended injections, dissolving accelerators, suspending agents, emulsifiers, stabilizers, preservatives and isotonic agents may be added. For oral administration, sweetening agents and flavors may be added.

Although the dosage of a compound of the present invention as a chymase inhibitor and/or cytokine production inhibitor should be determined taking into account the patient's age and body weight, the type and degree of disease and the administration route, a usual oral dosage for adults is 0.05-100 mg/kg/day and preferably 0.01-10 mg/kg/day. For parenteral administration, although the dosage varies highly with administration route, a usual dosage os 0.005-10 mg/kg/day, preferably, 0.01-1 mg/kg/day. The dosage may be administered in a single or several divisions per day.

The present invention is further explained by the following Examples and Experiments, which are not intended to limit the scope of the present invention.

### Examples

### Reference Example Compound (6)

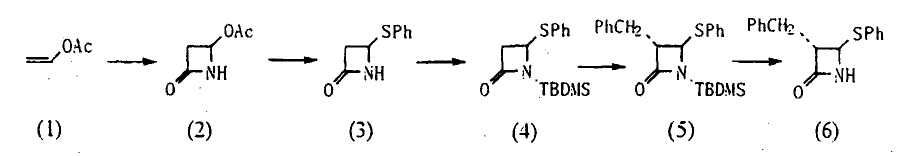

### (Step 1) 4-Acetoxy-azetidine-2-one(2)

Title compound was synthesized by the method described in Org. Synth. 1986, 65, 135. Submitted by S. J. Mickel and modified by Chi-Nung Hsiao and M. J. Miller.

### (Step 2) 4-Phenylthio-azetidine-2-one(3)

To a solution of 20.7 ml of thiophenol (1.3 eq) in acetone (40 ml) was added dropwise 185 ml of N-NaOH (1.2 eq) at 5 to 10 °C and the mixture was stirred at the same temperature for 10 minutes. A solution of 20 g of Compound (2) (155 mmol) in acetone (80 ml) was added dropwise thereto at the same temperature. The mixture was stirred at 10 to 15 °C for 3 hours, poured into ice-cooling water and extracted with ethyl acetate. The organic layer was washed with water, dried and filtered, and the solvent was removed to give 31 g of an oil residue (3).
NMR:H¹,CDCl₃(δ),2.85-2.94(m,1H),3.22-3.45(m,1H),4.99-5.03(m,1H),6.31(br, 1H),7.34-7.60(m,5H)

### (Step 3) 4-Phenylthio-N-(t-butyldimethylsilyl)-azetidine-2-one(4)

To a solution of 31 g of Compound (3) (155 mmol) in methylene chloride (200 ml) were added 29.2 g of t-butyldimethylsilyl chloride (1.25 eq) and 27 ml of triethylamine (1.25 eq) at 5 °C. The mixture was stirred for 16 hours at the same temperature, poured into a diluted aqueous solution of ammonium chloride and extracted with methylene chloride. The organic layer was washed with water, dried and filtered, and the solvent was removed to give 50 g of an oil residue. The obtained residue was chromatographed on silica gel (n-hexane : ethyl acetate) to give 37.9 g of an oil material (4) (83 % from (2)).
NMR:H¹,CDCl₃(δ),0.02(s,6H),0.70(s,9H),2.70,2.77 (d,J=2.4Hz,1H),3.15,3.23 (d,J=5.0Hz,1H), 4.59-4.63(m,1H),7.01-7.18(m,5H)

### (Step 4) 3-Benzyl-4-phenylthio-N-(t-butyldimethylsilyl)-azetidine-2-one(5)

To a solution of 16.4 g of Compound (4) (56 mmol) in tetrahydrofuran (164 ml) were added 10 ml of benzyl bromide (1.5 eq) and added dropwise 42 ml of 2M LDA (1.5 eq) over 10 minutes at ―76 °C. The mixture was stirred at the same temperature for 10 minutes, poured into diluted hydrochloric acid and extracted with ethyl acetate. The organic layer was washed with water, dried and filtered, and the solvent was removed to give 27 g of an oil residue. The obtained residue was chromatographed on silica gel (n-hexane : ethyl acetate) to give 12.3 g of an oil material (5) (59 %).
NMR:H¹,CDCl₃(δ), 0.22(s,6H),0.69(s,9H),2.78(d,J=6.4Hz,2H), 3.31,3.44 (d,d,J=2.3,6.4Hz,1H), 4.37(d,J=2.3Hz,1H),6.90-7.15(m,10H)
IR: ν;CHCl₃;1742 cm⁻¹

### (Step 5) 3-Benzyl-4-phenylthio-azetidine-2-one(6)

To a solution of 11.5 g of Compound (5) (31 mmol) in tetrahydrofuran (77 ml) were added 2.12 ml of acetic acid (1.2 eq) and 77 ml of 1M n-Bu₄NF/THF (1.2 eq). The mixture was stirred at 25 °C for 30 minutes, poured into diluted hydrochloric acid and extracted with ethyl acetate. The organic layer was washed with water, dried and filtered, and the solvent was removed to give 9.78 g of a crystalline residue. The residue was recrystallized from n-hexane: ethyl acetate to give 7.22 g of Compound (6) (87 %:mp. 119-120 °C).
NMR:H¹,CDCl₃(δ)2.90.3.20(m,2H),3.35-3.40(m,1H),4.68(d,J=2.2Hz,1H),6.20(br, 1H),7.20-7.50(m,10H)
IR: ν;CHCl₃;3400,1766 cm⁻¹

### Example 1 Compound (I-1)

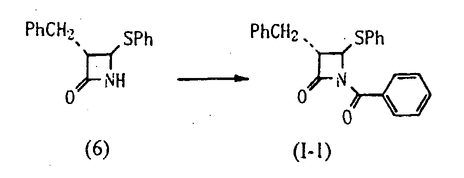

To a solution of 454 mg of Compound (6) (1.65 mmol) in dimethylformamide (5.0 ml) was added 0.23 ml of benzyl chloride (1.2 eq), followed by addition of 80 mg of 60 % NaH (1.2 eq) at 5 °C. The mixture was stirred at the same temperature for 3 hours, poured into diluted hydrochloric acid and extracted with ethyl acetate. The organic layer was washed with water, dried and filtered, and the solvent was removed to give 0.85 g of an oil residue. The obtained residue was chromatographed on silica gel (n-hexane : ethyl acetate) to give 314 mg of an oil material (I-1) (58 %).

### Example 2 Compound (I-7)

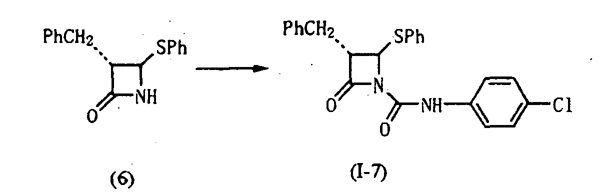

To a solution of 350 mg of Compound (6) (1.30 mmol) in methylene chloride (4.0 ml) were added 441 mg of p-chlorophenylisocyanate (2.0 eq), 0.36 ml of triethylamine(2.0 eq) and catalytic amounts of DMAP. The mixture was stirred at 25 °C for 16 hours, poured into diluted hydrochloric acid and extracted with ethyl acetate. The organic layer was washed with water, dried and filtered, and the solvent was removed to give 0.85 g of an oil residue. The residue was chromatographed on silica gel (n-hexane : ethyl acetate) to give 150 mg of a crystalline material (I-7) (26 %).

### Example 3 Compound (I-14)

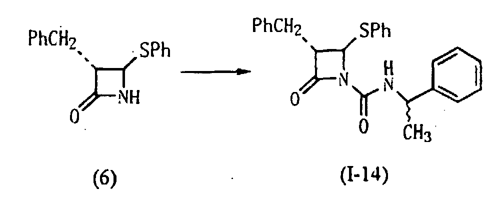

To a solution of 3.50 mg of Compound (6) (1.30 mmol) in methylene chloride (4.0 ml) were added 0.38 ml of 1-phenyl-ethylisocyanate (2.0 eq), 0.36 ml of triethylamine (2.0 eq) and catalytic amounts of DMAP. The mixture was stirred at 25 °C for 16 hours, poured into diluted hydrochloric acid and extracted with ethyl acetate. The organic layer was washed with water, dried and filtered, and the solvent was removed to give 0.80 g of an oil residue. The obtained residue was chromatographed on silica gel (toluene : ethyl acetate) to give 520 mg of an oil material (I-14) (96%).

### Example 4 Compound (I-21)

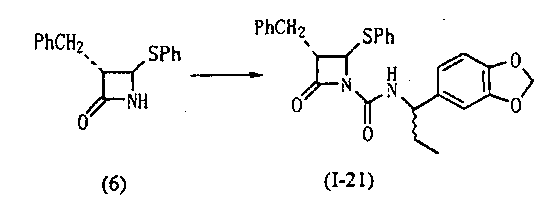

To a solution of 0.52 g of 2-(3,4-methylenedioxyphenyl) butyric acid (2.5 eq) in methylene chloride (5.0 ml) were added 0.54 ml of diphenylphosphoryl azide (2.5 eq) and 0.35 ml of triethylamine (2.5 eq) at 25 °C and the mixture was stirred for 2 hours. To the mixture were added 269 mg of Compound (6) (1.0 mmol), 0.35 ml of triethylamine (2.5 eq) and catalytic amounts of DMAP. The mixture was stirred at 45 °C for 4 hours, poured into diluted hydrochloric acid and extracted with ethyl acetate. The organic layer was washed with water, dried and filtered, and the solvent was removed to give 0.80 g of an oil residue. The obtained residue was chromatographed on silica gel (toluene : ethyl acetate) to give 440 mg of an oil material (I-21) (96 %).

### Example 5 Compound (I-28)

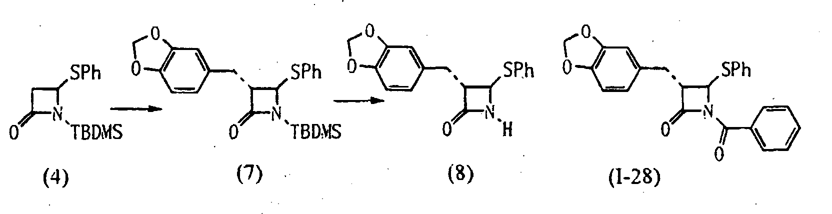

### (Step 1) 3-(3,4-Methylenedioxy benzyl)-4-phenylthio-N-(t-butyldimethyl silyl)-azetidine-2-one(7)

To a solution of 2.94 g of Compound (4) (10 mmol) in tetrahydrofuran (30 ml) was added 2.8 g of 3,4-methylenedioxybenzyl bromide (1.3 eq) and was added dropwise 8.8 ml of 2M LDA (1.76 eq) over 10 minutes at ―76 °C. The mixture was stirred for 2 hours at the same temperature, poured into diluted hydrochloric acid and extracted with ethyl acetate. The organic layer was washed with water, dried and filtered, and the solvent was removed to give 5.5 g of an oil residue. The obtained residue was chromatographed on silica gel (n-hexane : ethyl acetate) to give 1.42 g of an oil material (7) (33 %).
NMR:H¹,CDCl₃(δ), 0.23(m,6H),0.91(m,9H)2.85-3.00(m,2H),3.42-3.50(m,1H), 4.57(d, J=2.2 Hz,1H),5.95(m,2H),6.50-7.50(m,8H)

### (Step 2) 3-(3,4-Methylenedioxy benzyl)-4-phenylthio-azetidine-2-one(8)

To a solution of 1.32 g of Compound (7) (3.09 mmol) in tetrahydrofuran (7 ml) were added 0.22 ml of acetic acid (1.2 eq) and 3.7 ml of 1 M n-Bu₄NF/THF (1.2 eq). The mixture was stirred for 45 minutes at 25 °C, poured into diluted hydrochloric acid and extracted with ethyl acetate. The organic layer was washed with water, dried and filtered, and the solvent was removed to give 0.85 g of an oil residue. The obtained residue was chromatographed on silica gel (n-hexane : ethyl acetate) to give 488 mg of a crystalline material (8) (50 %).
NMR:H¹,CDCl₃(δ),2.85-3.10(m,2H),3.28-3.38(m,1H),4.67(d,J=2.2Hz,1H),5.94 (m,2H),6.10(br,1H),6.70-7.40(m,8H)

### (Step 3) Compound (I-28)

To a solution of 407 mg of Compound (8) (1.3 mmol) in dimethylformamide (4.0 ml) was added 0.18 ml of benzyl chloride (1.3 eq), followed by addition of 7.5 mg of 60% NaH (1.4 eq) at 5 °C. The mixture was stirred for 2 hours at the same temperature, poured into diluted hydrochloric acid and extracted with ethyl acetate. The organic layer was washed with water, dried and filtered, and the solvent was removed to give 0.60 g of an oil residue. The obtained residue was chromatographed on silica gel (n-hexane : ethyl acetate) to give 270 mg of an oil material (I-28) (49.7 %).

### Example 6 Compound (I-27)

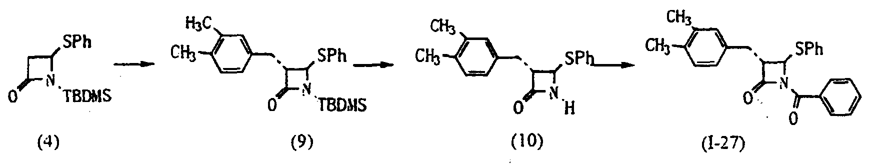

### (Step 1) 3-(2,3-Dimethyl benzyl)-4-phenylthio-N-(t-butyldimethylsilyl)-azetidine-2-one(9)

To a solution of 2.94 g of Compound (4) (10 mmol) in tetrahydrofuran (30 ml) was added 4.57 g of 2,3-dimethylbenzyl iodide (1.3 eq) and was added dropwise 7.5 ml of 2M LDA (1.50 eq) over 10 minutes at ―76 °C. The mixture was stirred for 0.5 hours at the same temperature, poured into diluted hydrochloric acid and extracted with ethyl acetate. The organic layer was washed with water, dried and filtered, and the solvent was removed to give 7.8 g of an oil residue. The obtained residue was chromatographed on silica gel(n-hexane : ethyl acetate) to give 4.06 g of an oil material (9) (99 %).

### (Step 2) 3-(3,4-Dimethyl-benzyl)-4-phenylthio-azetidine-2-one(10)

To a solution of 3.71 g of Compound (9) (9.0 mmol) in tetrahydrofuran (25 ml) were added 0.62 ml of acetic acid (1.2 eq) and 10.8 ml of 1 M n-Bu₄NF/THF (1.2 eq). The mixture was stirred for 30 minutes at 25 °C, poured into diluted hydrochloric acid and extracted with ethyl acetate. The organic layer was washed with water, dried and filtered, and the solvent was removed to give 3.30 g of an oil residue. The obtained residue was chromatographed on silica gel (n-hexane : ethyl acetate) to give 1.99 g of a crystalline material (10), followed by recrystallization from n-hexane : ethyl acetate to give 1.07 g of Compound (10) (63 %). As a byproduct, 0.40 g of 3,3-Bis-(3,4-dimethylbenzyl)-4-phenylthio-azetidine-2-one(11) (10/7 %) was obtained by chromatography.
Compound (10) NMR:H¹,CDCl₃(δ),2.23.2.28(m,6H),2.90-3.25(m,2H),3.30-3.42(m, 1H), 4.67(d,J=2.2Hz,1H),6.20(br,1H),6.97-7.35(m,8H)
Compound (11) NMR:H¹,CDCl₃(δ),2.15-2.30(m,12H),2.60-3.50(m,4H),4.84,4.90 (s,1H),5.89(s,1H),6.82-7.40(m,11H)

### (Step 3) Compound (I-27)

To a solution of 446 mg of Compound (10) (1.5 mmol) in dimethylformamide (4.0 ml) was added 0.21 ml of benzoyl chloride (1.3 eq), followed by addition of 81 mg of 60% NaH (1.4 eq) at 5 °C. The mixture was stirred for 1.5 hours at the same temperature, poured into diluted hydrochloric acid and extracted with ethyl acetate. The organic layer was washed with water, dried and filtered, and the solvent was removed to give 0.65 g of an oil residue. The obtained residue was chromatographed on silica gel (n-hexane : ethyl acetate) to give 350 mg of a crystalline material (I-27) (58.1 %).

### Example 7 Compound (I-29)

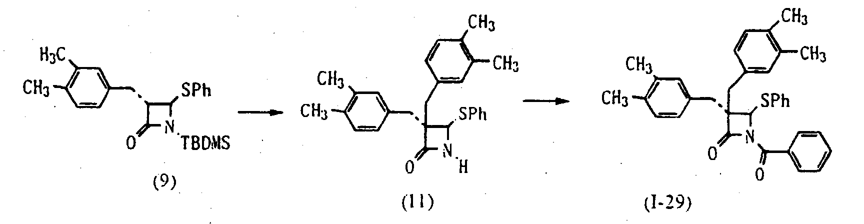

To a solution of 350 mg of Compound (11) (0.84 mmol) obtained in Example 6 Step 2 in dimethylformamide (3.5 ml) was added 0.12 ml of benzyl chloride (1.2 eq), followed by addition of 49 mg of 60% NaH (1.4 eq) at 5 °C. The mixture was stirred for 1.5 hours at the same temperature, poured into diluted hydrochloric acid and extracted with ethyl acetate. The organic layer was washed with water, dried and filtered, and the solvent was removed to give 0.45 g of an oil residue. The obtained residue was chromatographed on silica gel (n-hexane : ethyl acetate) to give 240 mg of a crystalline material (I-29) (55 %).

### Example 8 Compound (I-33)

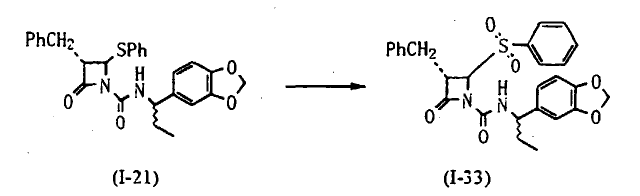

To a solution of 0.30 g of Compound (I-21) (0.65 mmol) in methylene chloride (5.0 ml) was added 310 mg of m-chloroperbenzoic acid (2.0 eq) at 5 °C, followed by stirring for 2 hours at the same temperature and for 1 hour at 25 °C. The reaction mixture was poured into a dilute aqueous solution of sodium bicarbonate and extracted with methylene chloride. The organic layer was washed with water, dried and filtered, and the solvent was removed to give 0.35 g of an oil residue. The obtained residue was chromatographed on silica gel (n-hexane : ethyl acetate) to give 290 mg of an oil material (I-33) (91 %).

### Example 9 Compound (I-31)

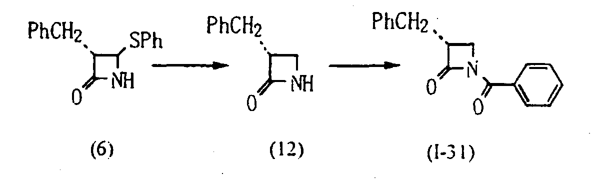

### (Step 1) 3-Benzyl-azetidine-2-one(12)

To a solution of 3.77 g of Compound (6) (14 mmol) in benzene (15 ml) were added 7.53 ml of nBu₃SnH (2.0 eq) and 0.46 g of AIBN (2 eq). The mixture was stirred for 5.5 hours at 100 °C and 0.46 g of AIBN was added thereto in every 2 hours. After the solvent was removed under reduced pressure and a soluble material was removed by n-hexane, the residue was chromatographed on silica gel (n-hexane : ethyl acetate) to give 2.2 g of a crystalline residue. The obtained residue was recrystallized from n-hexane : ethyl acetate to give 2.10 g of Compound (12) (92%:mp. 86 to 87°C).
NMR:H¹,CDCl₃(δ),2.88-3.22(m,3H),3.38(t,J=5.4Hz,1H),3.50-3.60(m,1H),5.85 (br,1H),20-7.40(m,5H)
IR: ν;CHCl₃;3420,1753 cm⁻¹

### (Step 2) Compound (I-31)

To a solution of 387 mg of Compound (12) (2.40 mmol) in dimethylformamide (4.0 ml) was added 0.34 ml of benzoyl chloride (1.2 eq), followed by addition of 0.12 g of 60 % NaH (1.2 eq) at 5 °C. The mixture was stirred for 1.0 hours at the same temperature, poured into diluted hydrochloric acid and extracted with ethyl acetate. The organic layer was washed with water, dried and filtered, and the solvent was removed to give 0.65 g of an oil residue. The obtained residue was chromatographed on silica gel (n-hexane : ethyl acetate) to give 505 mg of an oil material (I-31) (83.7 %).

### Example 10 Compound (I-34)

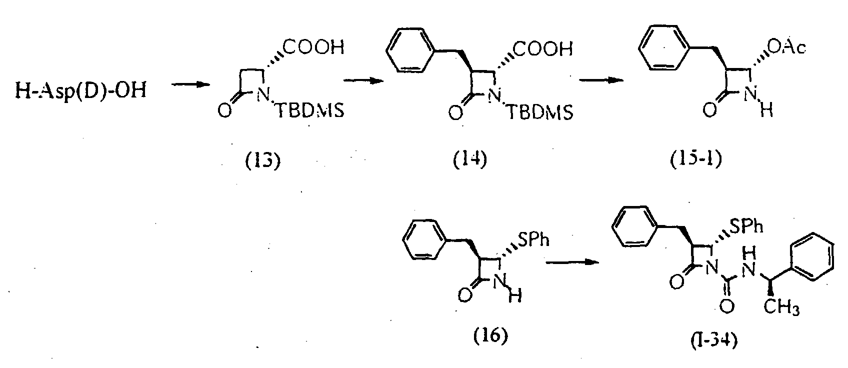

### (Step 1) (4R)-Carboxyl-N-(t-butyldimethylsilyl)-azetidine-2-one(13)

The title compound was synthesized from (D)-aspartic acid according to the method described in Tetrahedron Vol.46 No.13/14 PP.4733-4748 1990, J. E. Boldwin et al.

### (Step 2) (3S)-Benzyl-(4R)-carboxyl-N-(t-butyldimethylsilyl)-azetidine-2-one(14)

To a solution of 12.84 g of Compound (13) (56 mmol) in tetrahydrofuran (64 ml) was added dropwise 58.8 ml of 2 M LDA (2.15 eq) over 15 minutes at ―55 to ―40 °C. The mixture was stirred for 20 minutes at the same temperature and 14.65 ml of benzyl bromide (2.2 eq) was added thereto at ―55 to ―40 °C. The mixture was stirred for 1.5 hours at ―40 to ―15 °C, poured into an aqueous solution of M-NaHSO₄ and extracted with ethyl acetate. The objective material was extracted with an aqueous solution of sodium bicarbonate and ethyl acetate at pH 3.0 successively. The organic layer was washed with water, dried and filtered, and the solvent was removed to give 17.45 g of a crystalline residue (14) (98 %).
NMR:H¹,CDCl₃(δ),0.21(s,6H),0.78(s,9H),2.95-3.20(m,2H),3.60-3.70(m,1H), 3.77(d,J=2.8Hz,1H),7.20-7.40(m,5H),7.80(br,1H)

### (Step 3) (3S)-Benzyl-4-acetoxy-azetidine-2-one(15)

To a solution of 17.25 g of Compound (14) (54 mmol) in dimethylformamide (50 ml) was added 10 ml of acetic acid and was added 25.2 g of Pb(OAc)₄ (1.0 eq) at 25 °C. The mixture was stirred for 40 minutes at 50 to 55 °C and 43 ml of 1M n-Bu₄NF/THF (0.8eq) was added to the mixture at 20 to 25 °C. The mixture was stirred for 1 hour at the same temperature, poured into diluted hydrochloric acid and extracted with ethyl acetate. The organic layer was washed with diluted aqueous solution of sodium bicarbonate and water successively, dried and filtered, and the solvent was removed to give 11.44 g of an oil residue. The obtained residue was chromatographed on silica gel (toluene : ethyl acetate) to give 6.24 g of an oil material. i.e., (3S)-benzyl-(4S)-acetoxy-azetidine-2-one
(15-1) (53 %), 0.65 g of (3S)-benzyl-(4R)-acetoxy-azetidine-2-one (15-2) (6%) and 1.07 g of mixture thereof (9 %).
(15-1) NMR:H¹,CDCl₃(δ),2.07(s,3H),2.96-3.19(m,2H),3.47-3.54(m,1H),5.15 (d,J=1.0Hz,1H),6.49(br,1H),7.20-7.40(m,5H)
(15-2) NMR:H¹,CDCl₃(δ),2.12(s,3H),3.08-3.15(m,2H),3.63-3.77(m, 1H), 5.89(d,J=4.3Hz,1H), 6.61(br,1H),7.20-7.40(m,5H)

### (Step 4) (3S)-Benzyl-(4S)-phenylthio-azetidine-2-one(16)

To a solution of 0.61 ml of thiophenol (1.3 eq) in acetone (6 ml) was added dropwise 5 ml of N-NaOH (1.2 eq) at 5 to 10 °C, and the mixture was stirred for 10 minutes at the same temperature. To the mixture was added dropwise a solution of 1.0 g of Compound (15-1) (4.56 mmol) in acetone (7 ml) at the same temperature. The mixture was stirred for 3 hours at 10 to 15 °C, poured into ice-cooled water and extracted with ethyl acetate. The organic layer was washed with water, dried and filtered, and the solvent was removed to give 1.35 g of a crystalline residue. The obtained residue was recrystallized from n-hexane : ethyl acetate to give 1.13 g of Compound (16) (92 %).
NMR:H¹,CDCl₃(δ),2.95-3.19(m,2H),3.34-3.45(m,1H) 4.68(d,J=2.2Hz,1H), 6.14 (br,1H), 7.18-7.35(m,10H)

### (Step 5) Compound (I-34)

To a solution of 162 mg of Compound (16) (0.6 mmol) in methylene chloride (2.0 ml) were added 0.17 ml of R-(+)-Phenyl-ethyl-isocyanate (2.0eq), 0.18 ml of triethylamine (2.0 eq) and catalytic amounts of DMAP. The mixture was stirred at 25 °C for 16 hours, poured into diluted hydrochloric acid and extracted with ethyl acetate. The organic layer was washed with water, dried and filtered, and the solvent was removed to give 0.37 g of a crystalline residue. The residue was chromatographed on silica gel (n-hexane : ethyl acetate) to give 145 mg of a crystalline material (I-34) (58 %).

### Example 11 Compound (I-48)

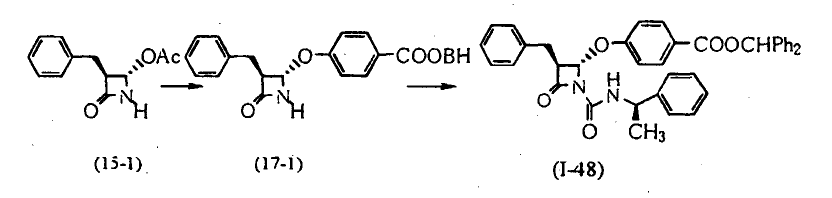

### (Step 1) (3S)-Benzyl-(4S)-(4-benzhydrylcarboxyphenyl)oxy-azetidine-2-one(17-1)

To a solution of 2.07 g of benzhydryl-4-hydroxy benzoate (1.3 eq) in acetone (8 ml) was added dropwise 6 ml of N-NaOH (1.2 eq) at 5 to 10 °C. At the same temperature, the mixture was stirred for 10 minutes and a solution of 1.1 g of Compound (15-1) (5.0 mmol) in acetone (6 ml) was added dropwise to the mixture. The mixture was stirred for 3 hours at 10 to 15 °C, poured into ice-cooled water and extracted with ethyl acetate. The organic layer was washed with water, dried and filtered, and the solvent was removed to give 2.75 g of an oil residue. The obtained residue was chromatographed on silica gel (toluene : ethyl acetate) to give 1.87 g of Compound (17-1) (80 %).

From another fraction, 0.14 g of a crystalline material, i.e., (3S)-benzyl-(4R)-(4-benzhydrylcarboxyphenyl)oxy-azetidine-2-one(17-2), was obtained (6%).
(17-1) NMR:H¹,CDCl₃(δ),3.02-3.28(m,2H),3.59-3.66(m,1H),5.40(s,1H),(br,1H), 7.08(s,1H),7.15-7.48(m,15H) 7.36,7.96 (ABq,J=8.0 Hz, 4H).
(17-2) NMR:H¹,CDCl₃(δ),3.19 (d,J=7.6Hz,2H),3.73-3.84(m,1H),5.75(d, J= 4.2Hz,1H),6.70(br,1H),6.86,8.08(ABq,J=8.0Hz,2H),7.09(s,1H),7.15-7.48(m,15H)

### (Step 2) Compound (I-48)

To a solution of 1.85 g of Compound (17-1) (4.0 mmol) in methylene chloride (18.0 ml) were added 1.13 ml of R-(+)-Phenyl-ethyl-isocyanate (2.0eq), 1.12 ml of triethylamine (2.0 eq) and catalytic amounts of DMAP. The mixture was stirred at 25 °C for 16 hours, poured into diluted hydrochloric acid and extracted with ethyl acetate. The organic layer was washed with water, dried and filtered and the solvent was removed to give 3.0 g of a crystalline residue. The obtained residue was chromatographed on silica gel (toluene : ethyl acetate) to give 2.02 g of a crystalline material (I-48) (83 %).

### Example 12 Compound (I-37)

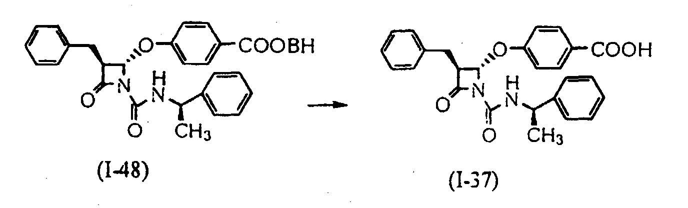

To a solution of 1.88 g of Compound (I-48) (3.08 mmol) in anisole (9.4 ml) was added 2.43 ml of CF₃COOH (10 eq) at 5 °C. The mixture was stirred for 3.5 hours at the same temperature, poured into diluted aqueous solution of sodium bicarbonate and extracted with methylene chloride. The organic layer was washed with water, dried and filtered, and the solvent was removed to give 10 g of an oil residue. The obtained residue was recrystallized from n-hexane to give 1.23 g of a crystalline material (I-37) (90 %).

### Example 13 Compound (I-39)

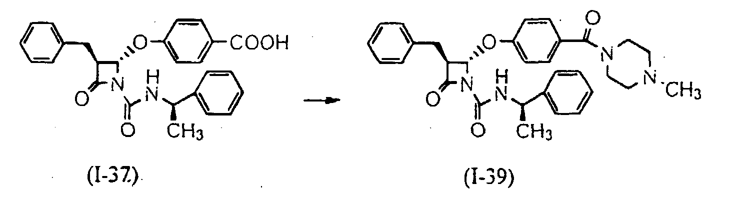

To a solution of 120 mg of Compound (I-37) (0.27 mmol) in methylene chloride (1.2 ml) were added 36 µl of 1-methyl-piperazine (1.2eq) and 62 mg of WSCD (1.2 eq). The mixture was stirred for 5 hours at 25 °C, poured into diluted aqueous solution of sodium bicarbonate and extracted with methylene chloride. The organic layer was washed with water, dried and filtered, and the solvent was removed to give 143 mg of an oil residue. The obtained residue was recrystallized from n-hexane to give 130 mg of a powder material (I-39) (92 %).

### Example 14 Compound (I-61)

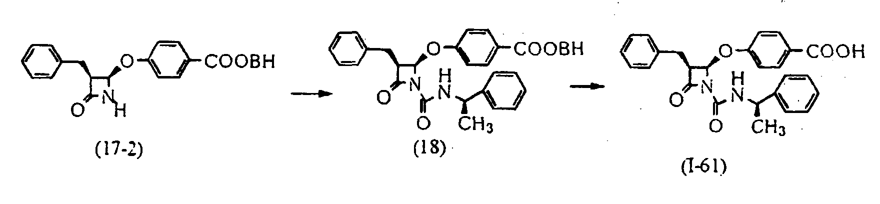

### (Step 1) Compound (18)

To a solution of 463 mg of Compound (17-2) (1.0 mmol) in methylene chloride (4.0 ml) were added 0.28 ml of R-(+)-phenyl-ethyl-isocyanate (2.0eq), 0.28 ml of triethylamine (2.0 eq) and catalytic amounts of DMAP. The mixture was stirred at 25 °C for 16 hours, poured into diluted hydrochloric acid and extracted with ethyl acetate. The organic layer was washed with water, dried and filtered, and the solvent was removed to give 0.55 g of a crystalline residue, The residue was chromatographed on silica gel (toluene : ethyl acetate) to give 0.50 g of a crystalline material (18) (82 %).
NMR:H¹,CDCl₃(δ),1.54(d,J=4.6Hz,3H),3.18-3.24(m,2H),3.82-3.94(m,1H), 4.96-5.12(m,1H),6.12(d,J=4.6Hz,1H),6.85(d,J=8.3Hz,1H),7.08-8.09(m,25H)

### (Step 2) Compound (I-61)

To a solution of 0.41 g of Compound (18) (0.67 mmol) in anisole (2.1 ml) was added 0.52 ml of CF₃COOH (10 eq) at 5 °C. The mixture was stirred for 5 hours at the same temperature, poured into diluted aqueous solution of sodium bicarbonate and extracted with methylene chloride. The organic layer was washed with water, dried and filtered, and the solvent was removed to give 3 g of an oil residue. The obtained residue was recrystallized from n-hexane : ether to give 270 mg of a crystalline material (I-61) (90 %).

### Example 15 Compound (I-62)

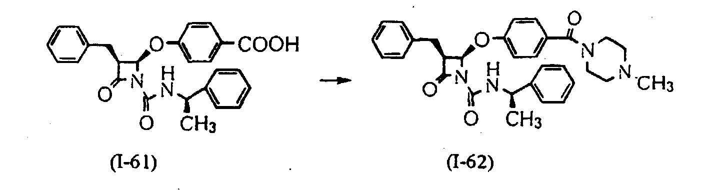

To a solution of 100 mg of Compound (I-61) (0.23 mmol) in methylene chloride (1.2 ml) were added 30 µl of 1-methyl-piperazine (1.2 eq) and 56 mg of WSCD (1.2 eq) at 5 °C. The mixture was stirred for 2.5 hours at 25 °C, poured into diluted aqueous solution of sodium bicarbonate and extracted with methylene chloride. The organic layer was washed with water, dried and filtered, and the solvent was removed to give 120 mg of an oil residue. The obtained residue was recrystallized from n-hexane to give 111 mg of a powder material (I-62) (94 %).

### Example 16 Compound (I-59)

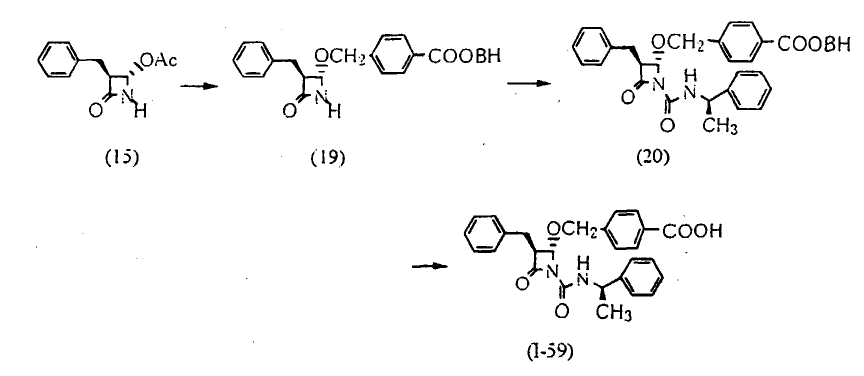

### (Step 1) Compound (19)

To a solution of 0.8 g of benzhydryl-4-hydroxy phenylacetate (1.0 eq) in tetrahydrofuran (2 ml) was added dropwise 1.25 ml of 2M t-BuMgCl/Et₂O (1.0eq) at 5 °C. At the same temperature, the mixture was stirred for 1.5 minutes and a solution of 0.55 g of Compound (15) (2.5 mmol) in tetrahydrofuran (3 ml) was added dropwise to the mixture. The mixture was stirred for 3 hours at 20 to 25 °C, poured into diluted hydrochloric acid and extracted with ethyl acetate. The organic layer was washed with water, dried and filtered, and the solvent was removed to give 1.40 g of an oil residue. The obtained residue was chromatographeci on silica gel (toluene : ethyl acetate) to give 0.69 g of Compound (19) (58 %).
NMR:H¹,CDCl₃(δ),2.79-3.17(m,2H),3.37-3.48(m,1H),4.39(s,2H) 4.84(d,J=1.0Hz,1H),6.26(br,1H), 7.11(s,1H), 7.23-8.10(m,19H)

### (Step 2) Compound (20)

To a solution of 0.66 g of Compound (19) (1.38 mmol) in methylene chloride (6.6 ml) were added 0.39 ml of R-(+)-phenyl-ethyl-isocyanate (2.0eq), 0.39 ml of triethylamine (2.0 eq) and catalytic amounts of DMAP. The mixture was stirred at 25 °C for 16 hours, poured into diluted hydrochloric acid and extracted with ethyl acetate, The organic layer was washed with water, dried and filtered, and the solvent was removed to give 1.0 g of a crystalline residue. The residue was chromatographed on silica gel (toluene : ethyl acetate) to give 0.61 g of a crystalline material (20) (70 %).
NMR:H¹,CDCl₃(δ),1.55(s,3H),2.79-3.16(m,2H),3.46-3.54(m,1H),4.81, 4.96 (ABq,J=14Hz,2H), 4.93-5.10(m,1H)5.12(d,J=1.7Hz,1H), 6.93(d,J=8.0Hz,1H), 7.05-8.10(m,25H)

### (Step 3) Compound (I-59)

To a solution of 0.55 g of Compound (20) (0.88 mmol) in anisole (2.0 ml) and methylene chloride (2.8 ml) was added 0.68 ml of CF₃COOH (10 eq) at 5 °C. The mixture was stirred for 5 hours at the same temperature, poured into diluted aqueous solution of sodium bicarbonate and extracted with methylene chloride. The organic layer was washed with water, dried and filtered, and the solvent was removed to give 3 g of an oil residue. The obtained residue was recrystallized from n-hexane : isopropyl ether to give 0.37 g of a crystalline material. The obtained material was chromatographed on silica gel (n-hexane : ethyl acetate) to give an oil residue and the residue was recrystallized from n-hexane to give 220 mg of a powder material (I-59) (54 %).

### Example 17 Compound (I-60)

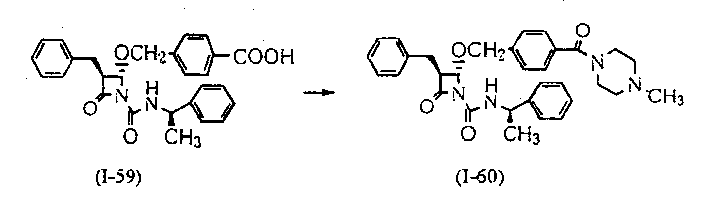

To a solution of 100 mg of Compound (I-59) (0.22 mmol) in methylene chloride (1.2 ml) were added 30 µl of 1-methyl-piperazine (1.2eq) and 55 mg of WSCD (1.2 eq). The mixture was stirred for 2.5 hours at 25 °C, poured into diluted aqueous solution of sodium bicarbonate and extracted with methylene chloride. The organic layer was washed with water, dried and filtered, and the solvent was removed to give 120 mg of an oil residue. The obtained residue was chromatographed on silica gel (n-hexane : ethyl acetate : methanol) to give an oil residue and recrystallized from n-hexane to give 67 mg of a powder material (I-60) (50 %).

### Example 18 Compound (I-144)

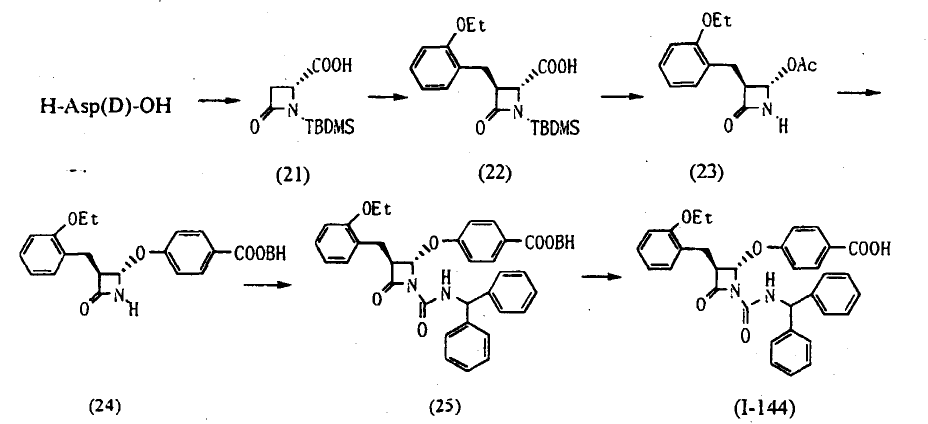

### (Step 1) (4R)-Carboxyl-N-(t-butyldimethylsilyl)-azetidine-2-one(21)

Compound (21) was synthesized from (D)-aspartic acid according to the method described in Tetrahedron Vol. 46 Nos. 13/14 PP. 4733-4748 1990, J. E. Boldwin et al.

### (Step 2) (3S)-2-Ethoxybenzyl-(4R)-carboxyl-N-(t-butyldimethylsilyl)-azetidine-2-one(22)

To a solution of 77 ml of 0.68 M LDA (2.1 eq) in THF was added dropwise a solution of 5.73 g of Compound (21) (25 mmol) in tetrahydrofuran (30 ml) at ―45 °C to ―25 °C over 15 minutes. The mixture was stirred for 2.5 hours at the same temperature and 10.75 g of 2-ethoxybenzylbromide (2.0 eq) was added to the mixture at ―38 °C to ―28 °C. The mixture was stirred for 2 hours at ―28 °C to ―15 °C, poured in a solution of N-hydrochloric acid and extract with ethyl acetate. The objective material was extracted with aqueous solution of sodium bicarbonate and with ethyl acetate at pH 3.0 successively. The organic layer was washed with water, dried and filtered, and the solvent was removed to give 7.82 g of a crystalline residue (22) (86 %).
NMR:H¹,CDCl₃(δ),0.22(s,6H),0.80(s,9H),1.41(t,3H,J=7.0Hz),2.90-3.30(m,2H), 3.50-3.70(m,1H),3.87(d,J=3.4Hz,1H),4.02(q,2H,J=7.0Hz),6.70-7.340(m,5H)

### (Step 3) (3S)-2-Ethoxybenzyl-4-acetoxy-azetidine-2-one(23)

To the mixture of 7.82 g of Compound (22) (21.5 mmol) in dimethylformamide (23.5 ml) was added 4.7 ml of acetic acid and was added 9.53 g of Pb(OAc)₄ (1.0 eq) at 25 °C. The mixture was stirred for 100 minutes at 50 °C to 55 °C and 16 ml of 1M n-Bu₄NF/THF (0.75 eq) was added to the mixture at 20 °C to 25 °C. The mixture was stirred for 2 hours at the same temperature, poured into diluted hydrochloric acid and extracted with ethyl acetate. The organic layer was washed with diluted aqueous solution of sodium bicarbonate and water successively, dried and filtered, and the solvent was removed to give 5.75 g of an oil residue. The obtained residue was chromatographed on silica gel (toluene : ethyl acetate) to give 2.43 g of an oil material, i.e., (3S)-2-ethoxybenzyl-(4S)-acetoxy-azetidine-2-one (23-1) (43 %), and 1.79 g of mixture of (23-1) and (3S)-ethoxybenzyl-(4R)-acetoxy-azetidine-2-one (23-2) (32 %).
(23-1)NMR:H¹,CDCl₃(δ),1.40(t,J=7.0Hz,3H),2.05(s,3H), 2.89-3.22(m,2H), 3.48-3.68 (m, 1H), 4.05 (q,J=7.0Hz,2H),5.61(d,J=1.2Hz,1H),6.42(br,1H),6.83-7.26(m,4H)

### (Step 4) (3S)-2-Ethoxybenzyl-(4S)-(4-benzhydrylcarboxyphenyl)oxy-azetidine-2-one(24)

To a solution of 5.69 g of benzhydryl-4-hydroxy benzoate (1.2 eq) in acetone (36 ml) was added dropwise 17 ml of N-NaOH (1.1 eq) at 5 °C to 10 °C. At the same temperature, the mixture was stirred for 10 minutes and a solution of 4.1 g of Compound (23-1,2) (15.6 mmol) in acetone (16 ml) was added dropwise to the mixture. The mixture was stirred for 1.5 hours at 10 °C to 15 °C, poured into ice-cooled water and extracted with ethyl acetate. The organic layer was washed with water, dried and filtered, and the solvent was removed to give 9.41 g of an oil residue. The obtained residue was chromatographed on silica gel (toluene : ethyl acetate) to give 4.82 g of a crystalline material (24-1) (61 %). From other fraction, 2.00 g of a crystalline material, i.e., (3S)-Ethoxybenzyl-(4R)-(4-benzhydrylcarboxyphenyl)oxy-azetidine-2-one was obtained (25 %).
(24-1)NMR:H¹,CDCl₃(δ),1.35(t,J=7.0Hz,3H),2.95-3.34(m,2H),3.58-3.65(m, 1H), 4.00 (q,J=7.0Hz,2H),5.49(d,J=0.9Hz,1H),6.43(br,1H),6.71-8.02(m,19H)

### (Step 5) Compound (25)

To a solution of 1.04 g of diphenylacetic acid (2.5 eq) in methylene chloride (10 ml) were added 0.69 ml of triethylamine (2.5eq) and 1.06 ml of diphenylphosphoryl amide (2.5 eq). The mixture was stirred for 3 hours at 25 °C and a solution of 1.00 g of Compound (24-1) (2.0 mmol) in methylene chloride (18.0 ml), 0.69 ml of triethylamine (2.5 eq) and catalytic amounts of DMAP were added to the mixture. The mixture was stirred at 25 °C for 24 hours, poured into diluted hydrochloric acid and extracted with ethyl acetate. The organic layer was washed with water, dried and filtered, and the solvent was removed to give 3.0 g of a residue. The residue was chromatographed on silica gel (toluene : ethyl acetate) to give 1.29 g of Compound (25) (91 %).
(25)NMR:H¹,CDCl₃(δ),1.28(t,J=7.2Hz,3H),2.94-3.34(m,2H),3.66-3.74(m, 1H), 3.94(q,J=7.2Hz,2H),5.83(d,J=1.3Hz,1H),6.14(br,1H),6.79-8.00(m,30H)

### (Step 6) Compound (I-144)

To a solution of 1.18 g of Compound (25) (1.64 mmol) in methylene chloride (6 ml) was added 1.27 ml of anisole and was added 1.27 ml of CF₃COOH (10 eq) at 25 °C. The mixture was stirred for 3.5 hours at the same temperature, poured into diluted aqueous solution of sodium bicarbonate and extracted with methylene chloride. The organic layer was washed with water, dried and filtered, and the solvent was removed to give 10 g of an oil residue. The obtained residue was recrystallized from n-hexane to give 0.84 g of a crystalline material (93 %). The obtained material was chromatographed on silica gel (n-hexane : ethyl acetate) to give a crystalline residue and the residue was recrystallized to give 825 mg of Compound (I-144) (91 %).

Other compounds are synthesized by the similar methods. The structures of compounds and physical properties are shown below.

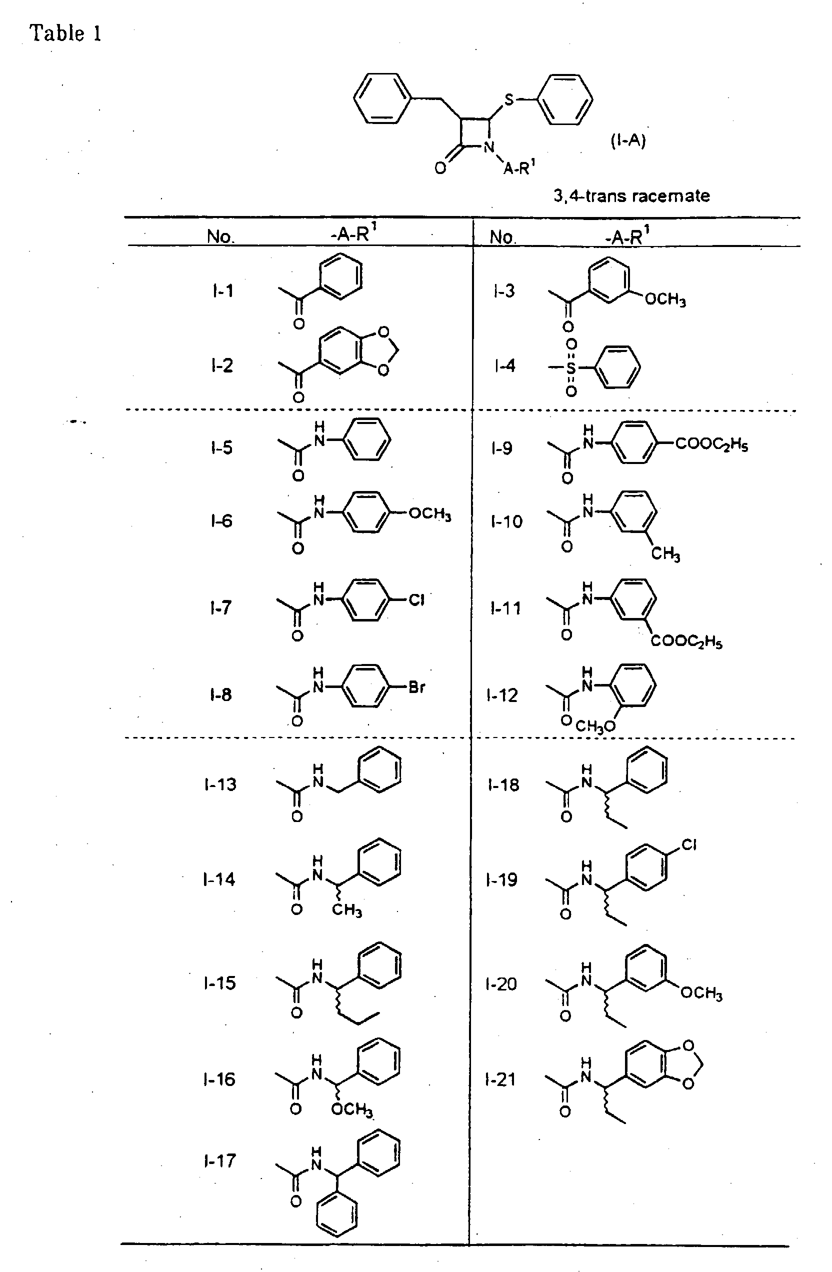

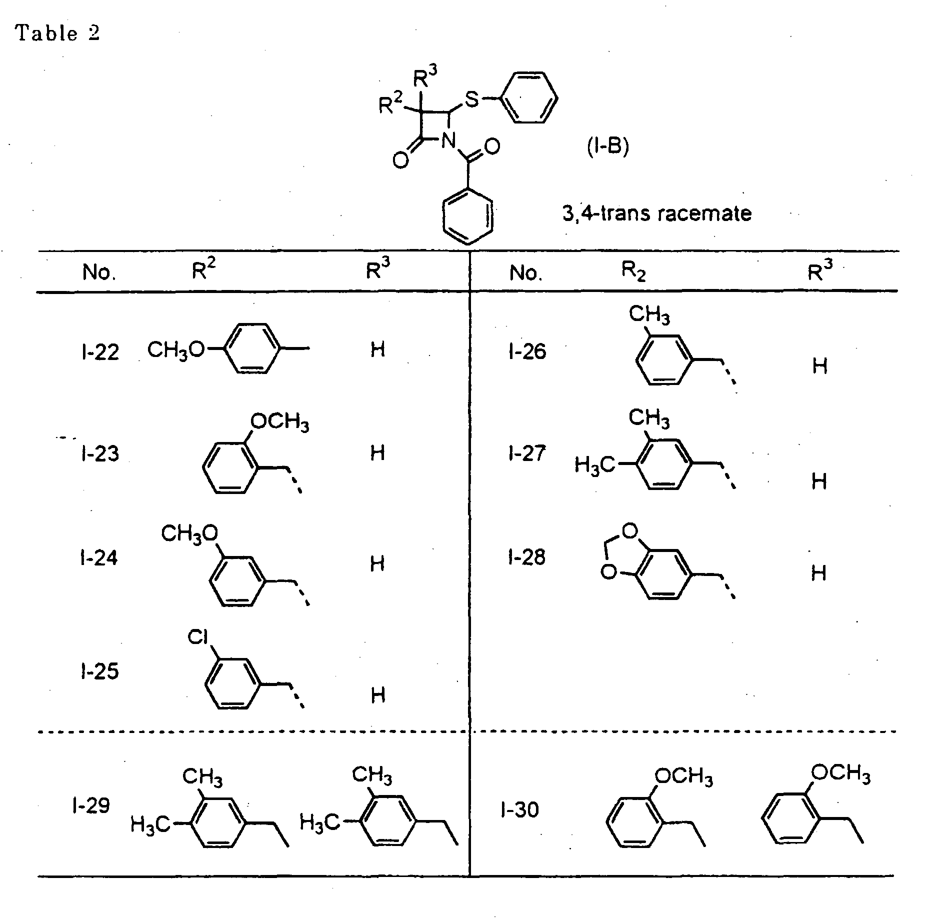

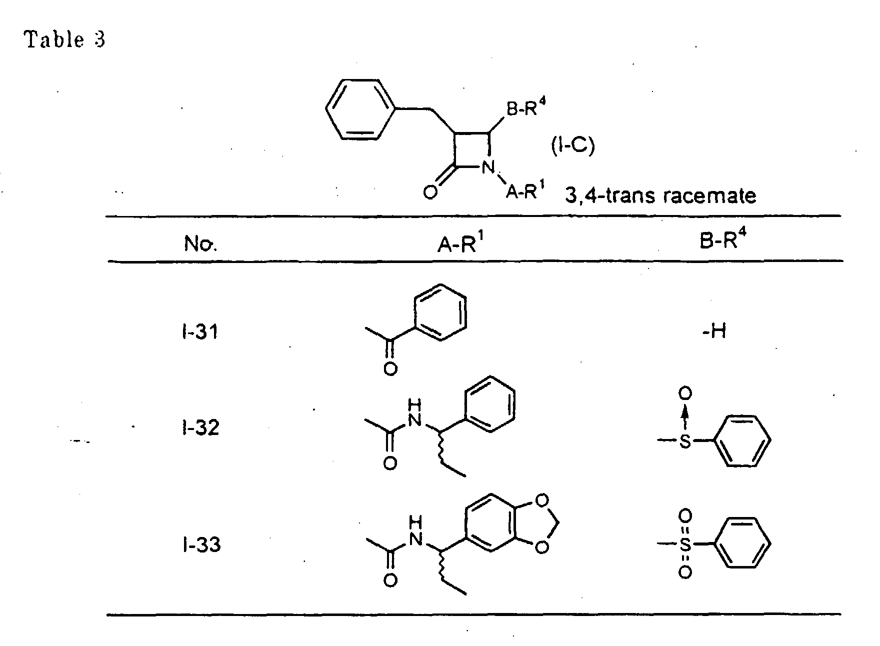

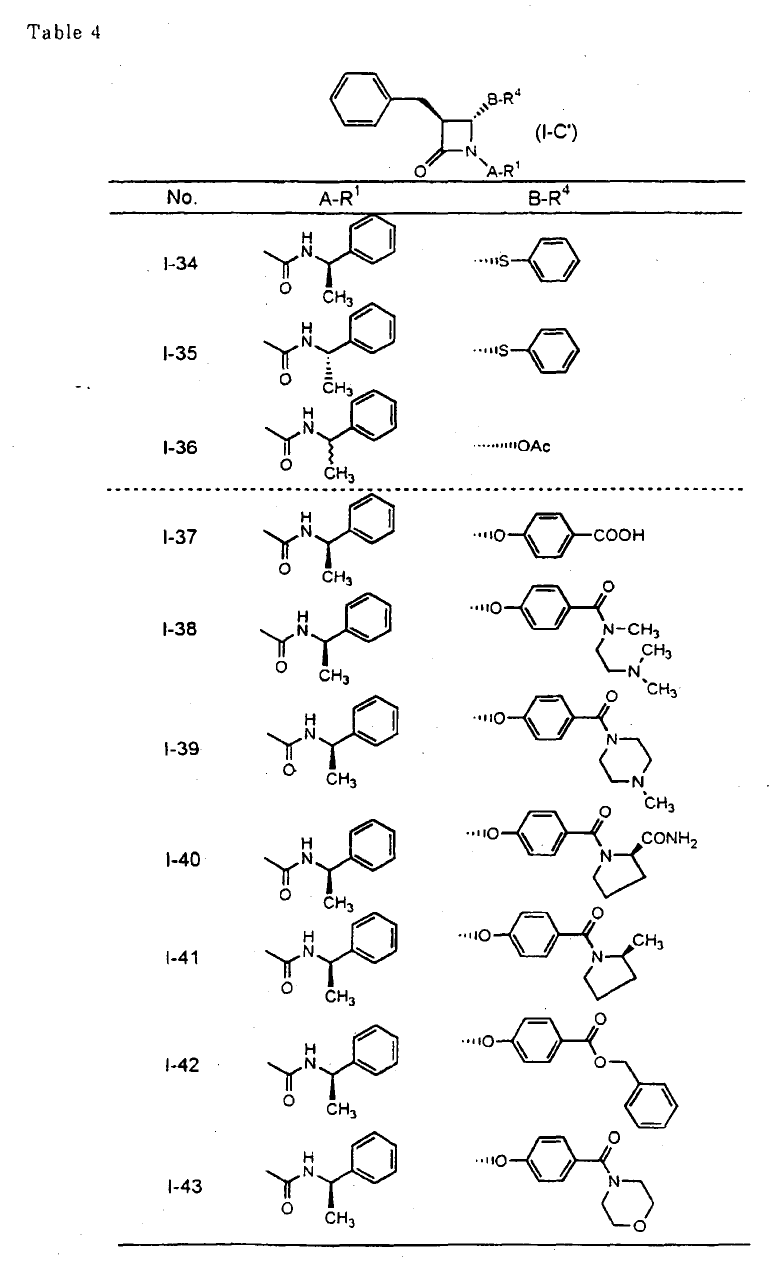

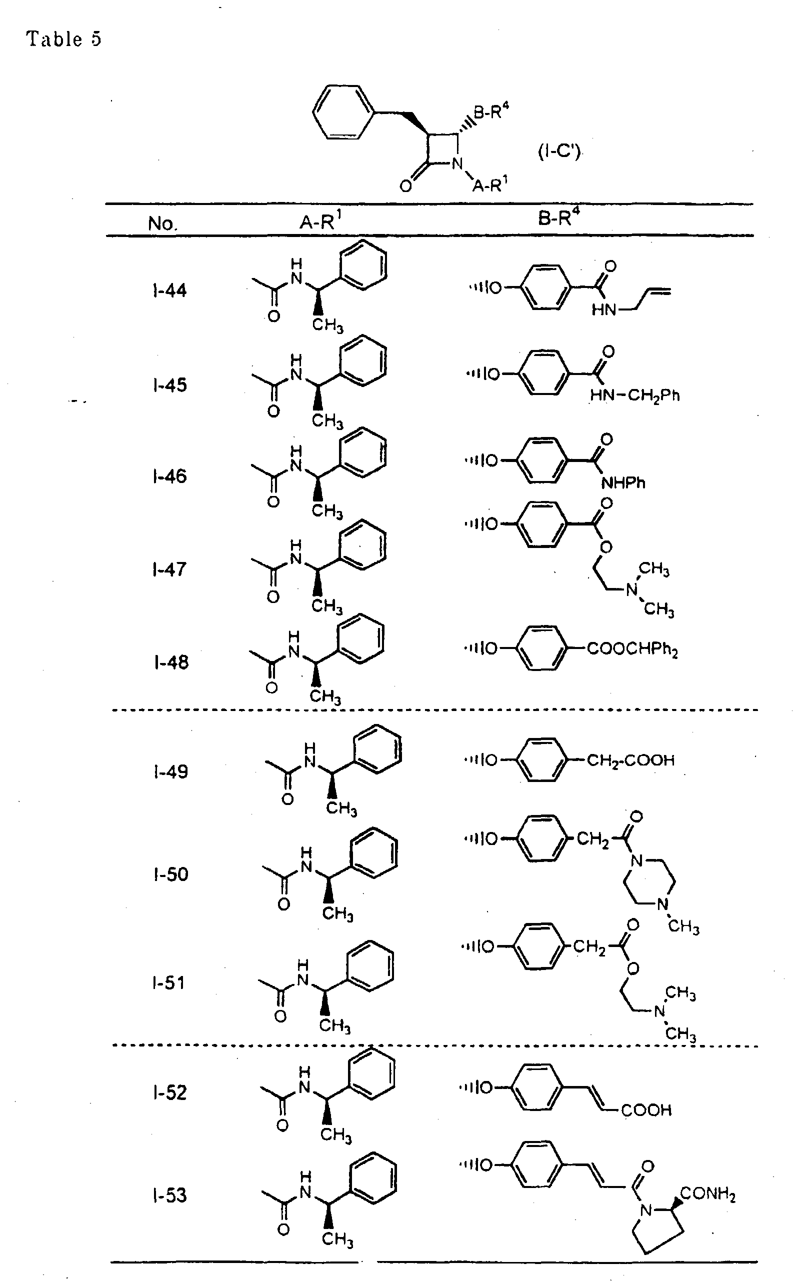

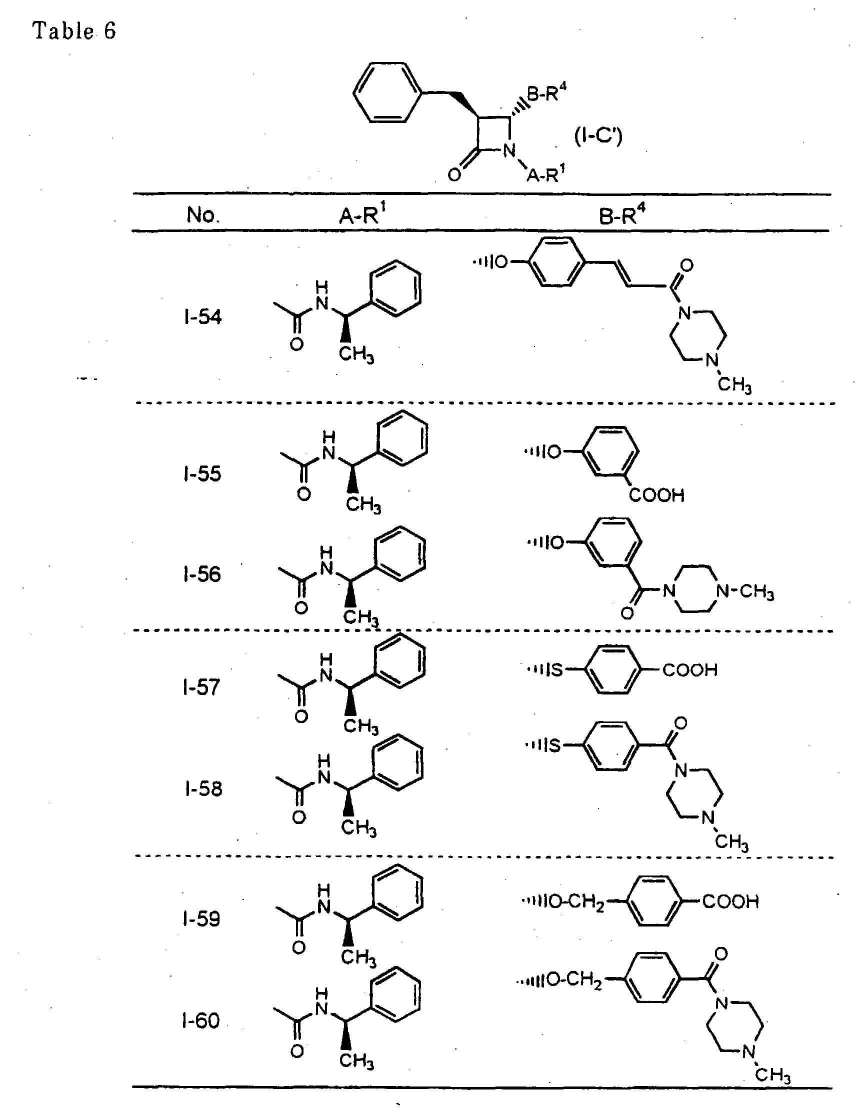

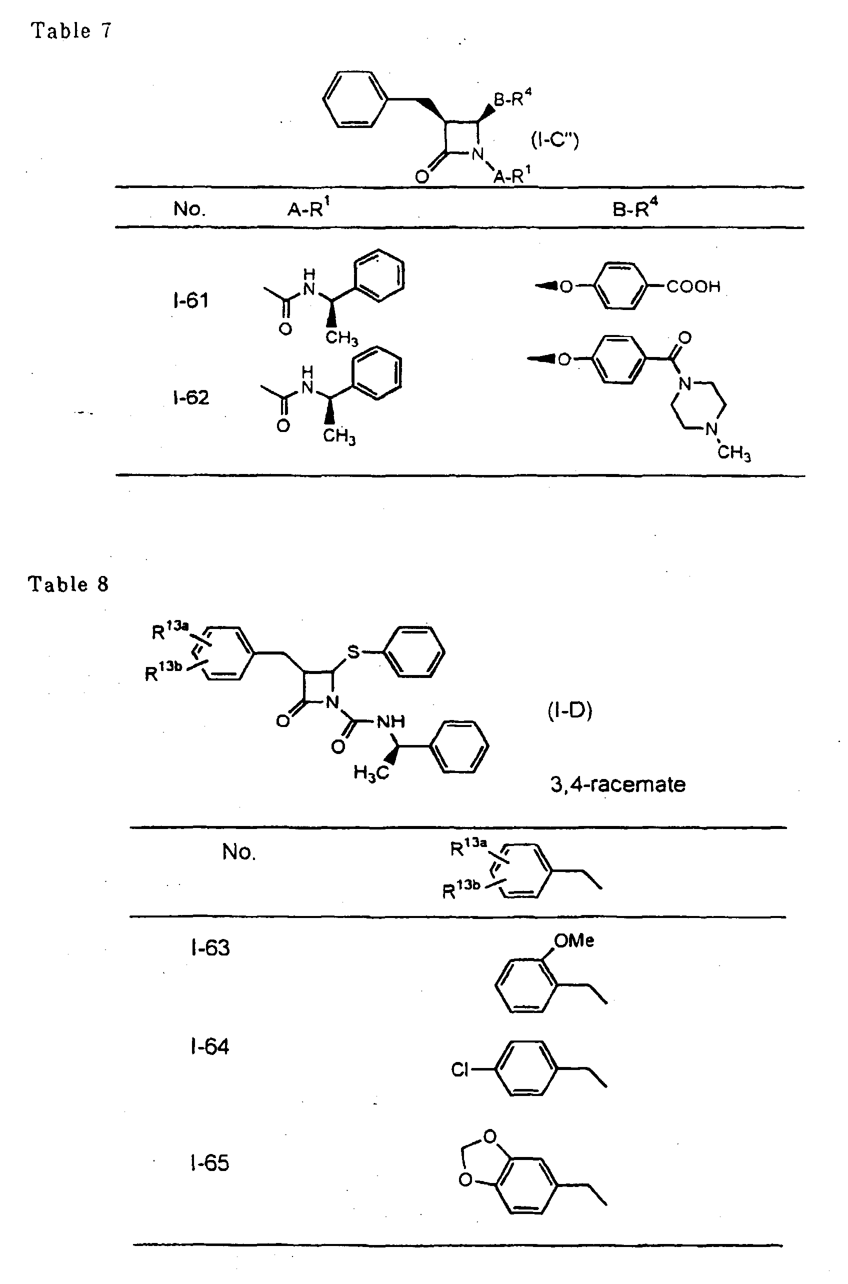

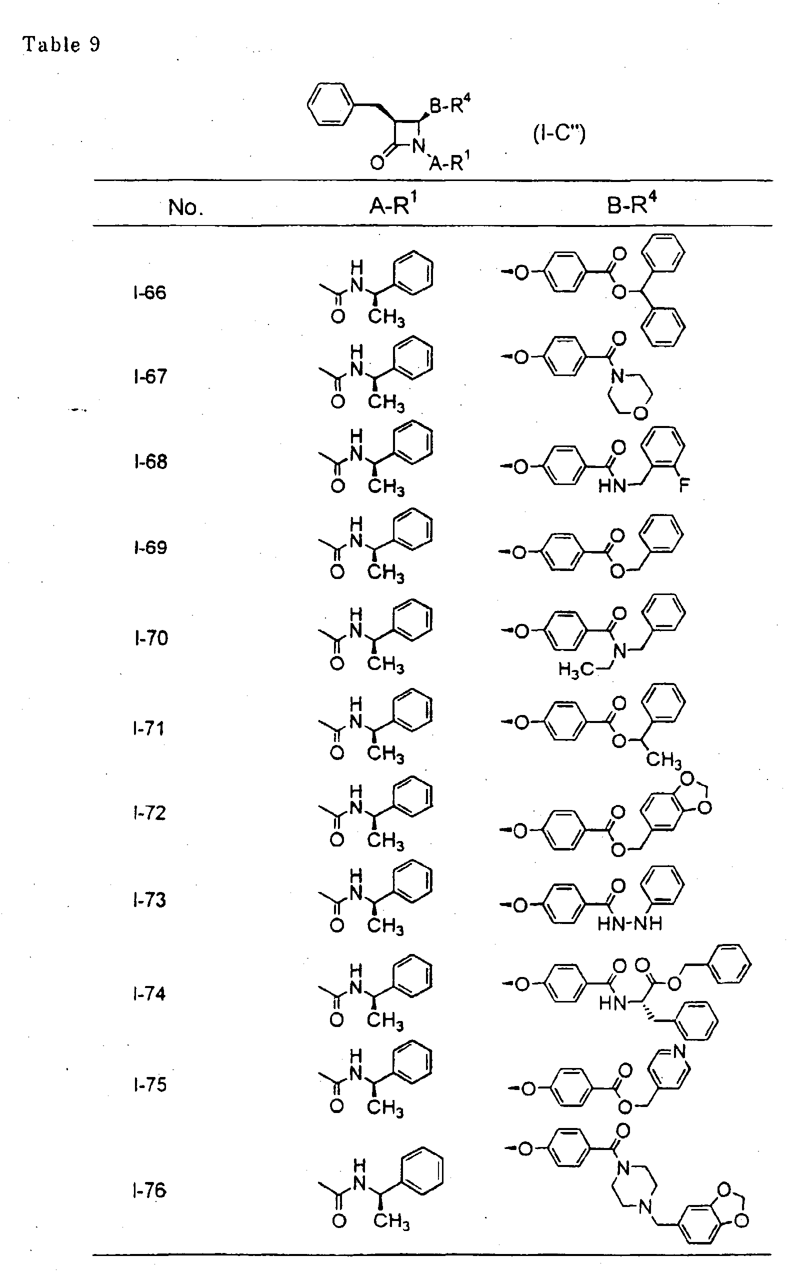

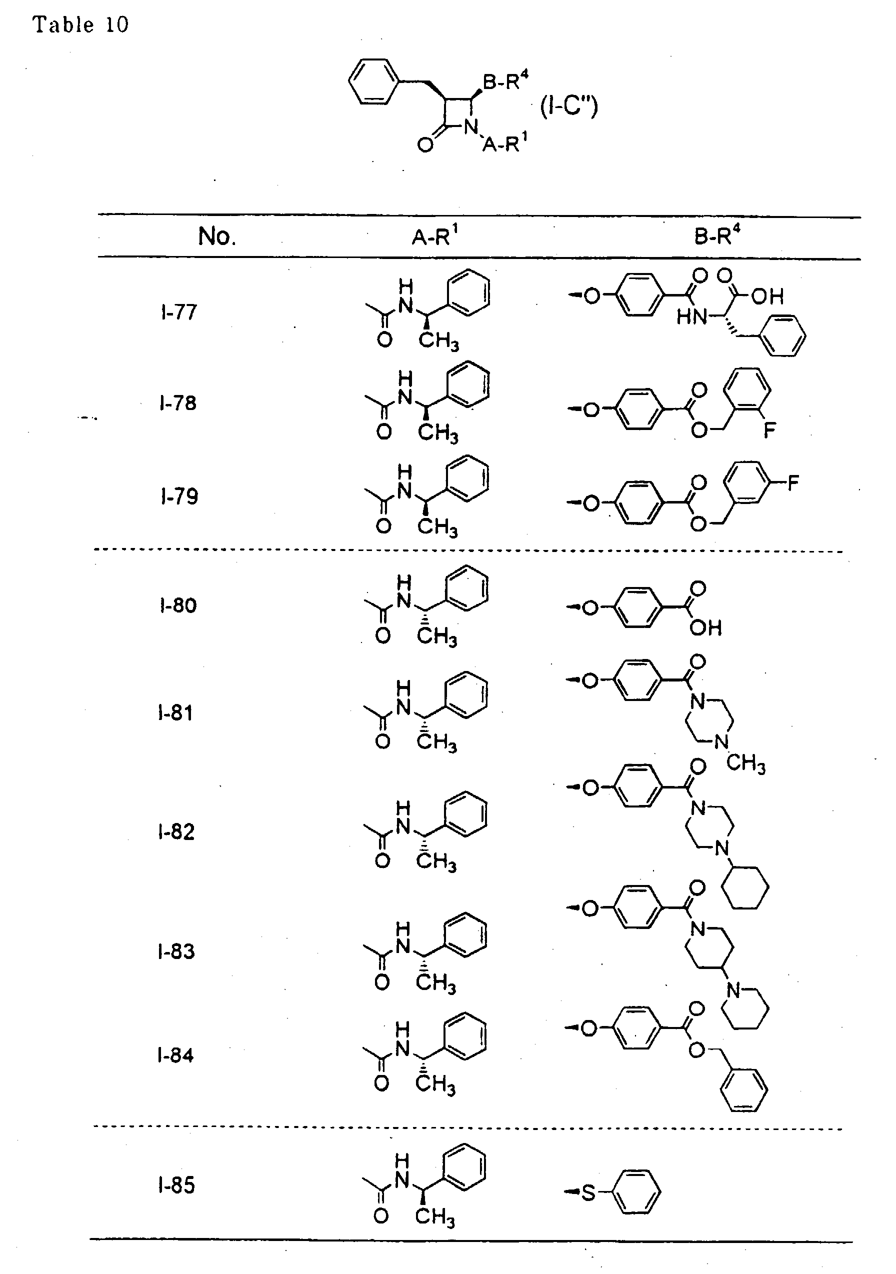

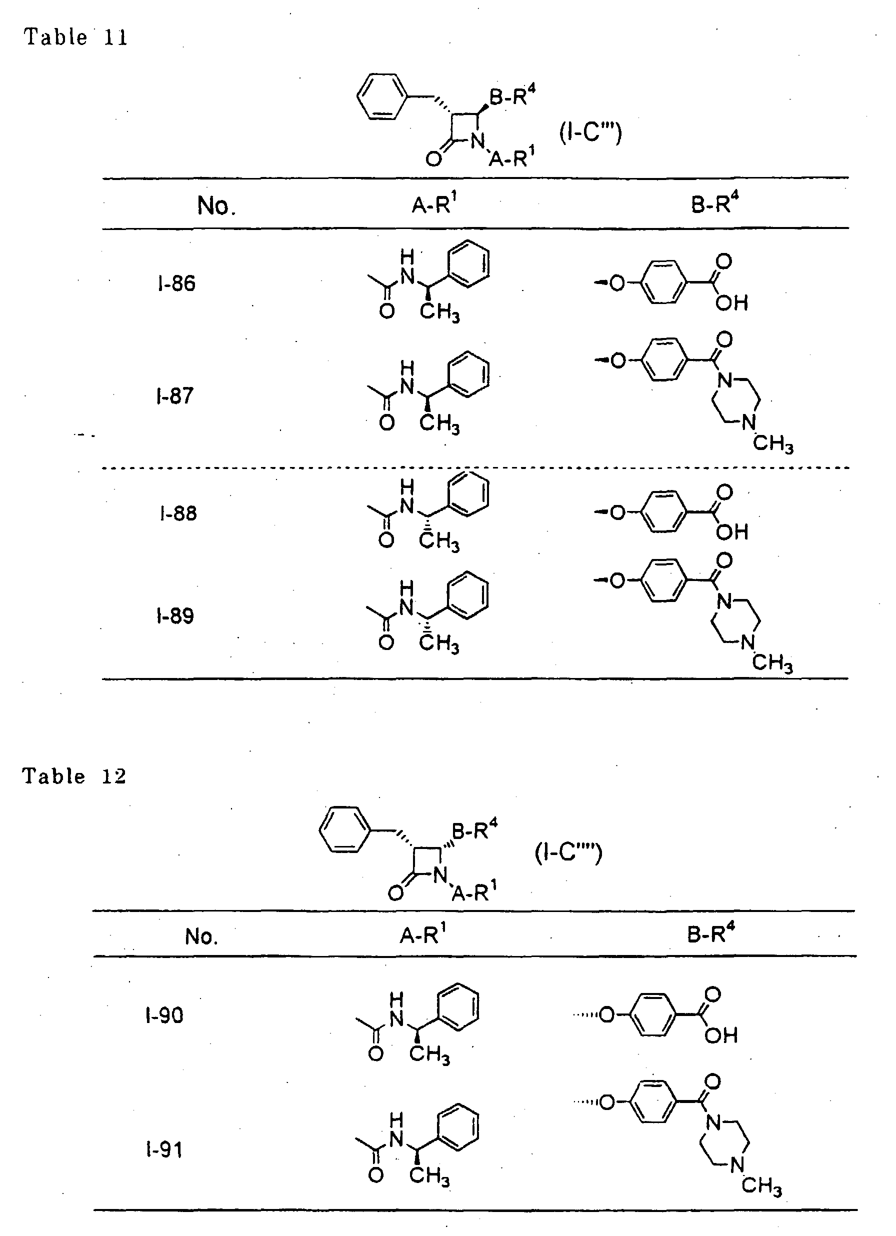

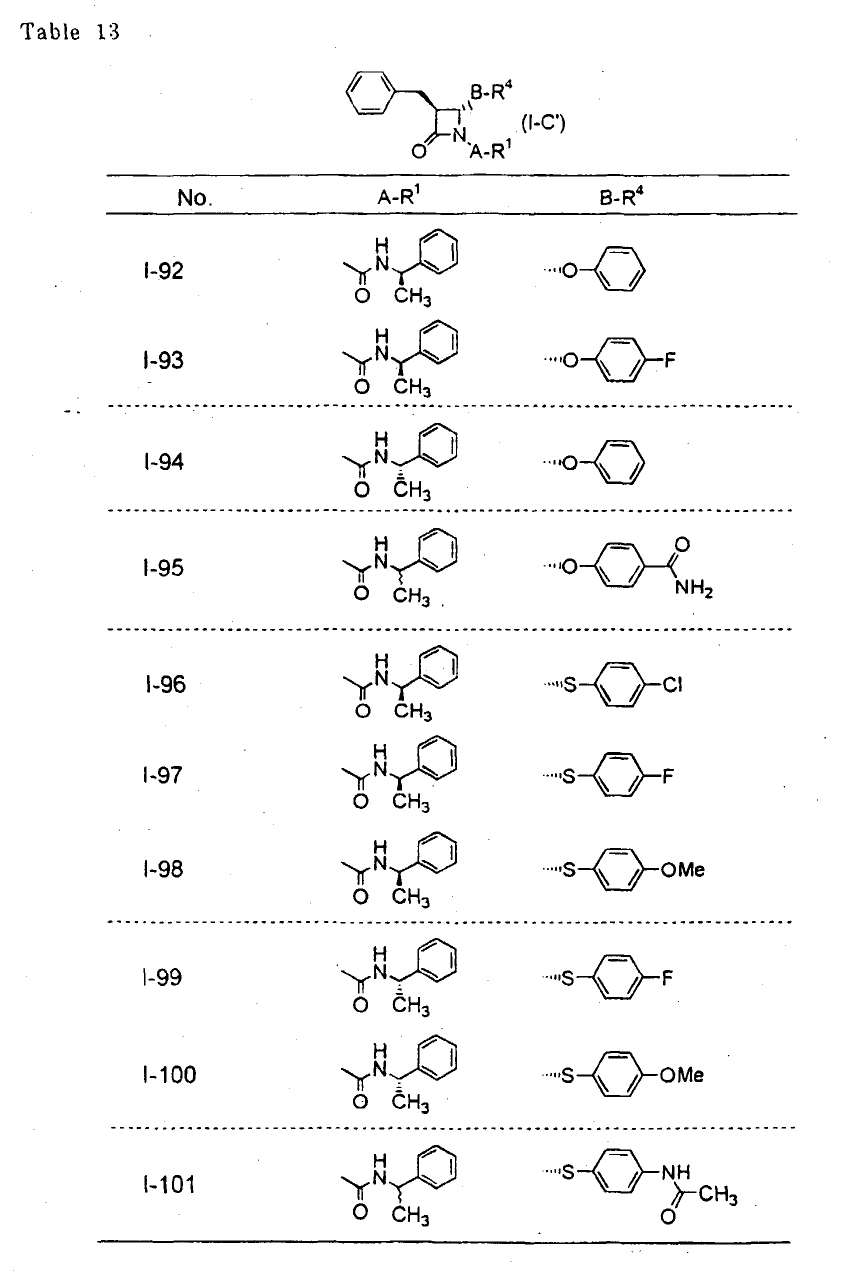

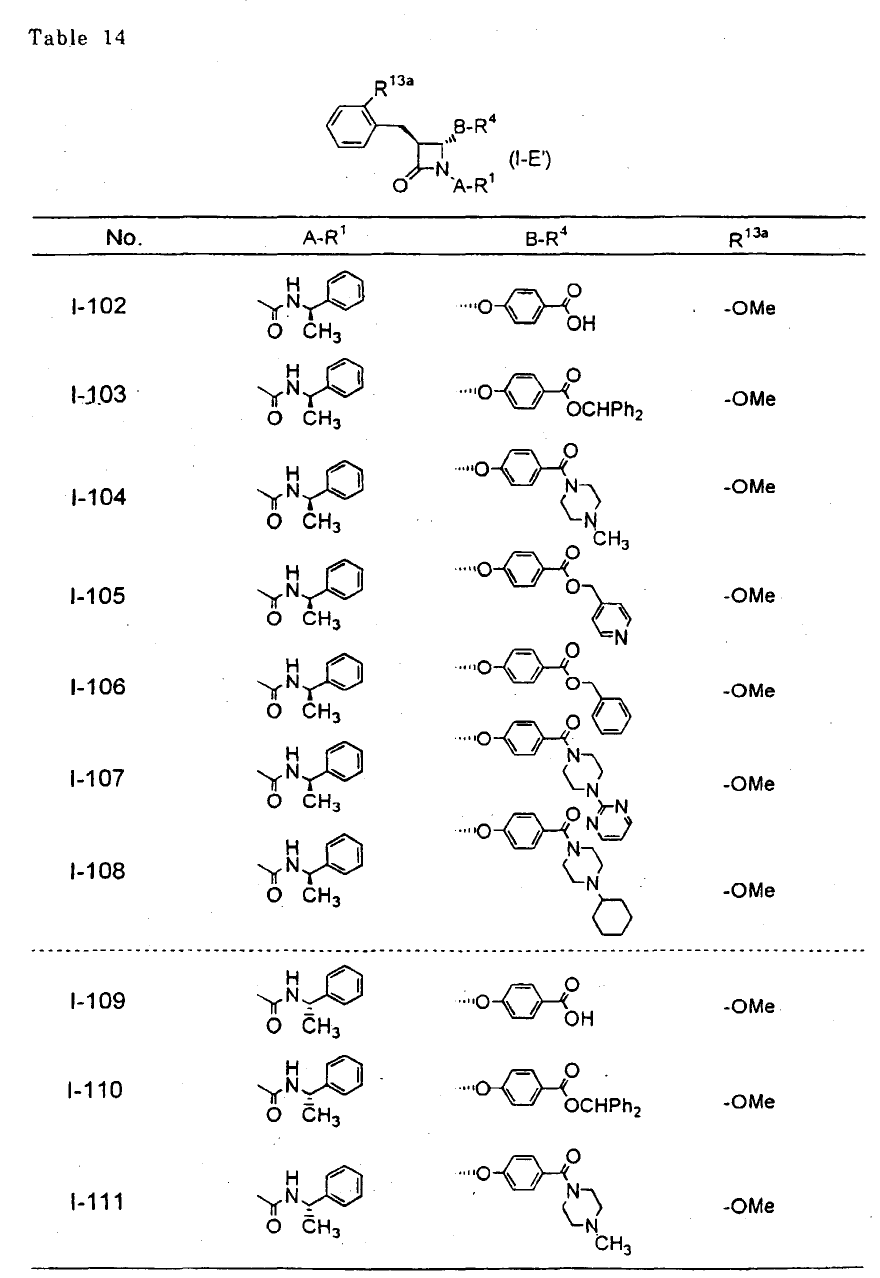

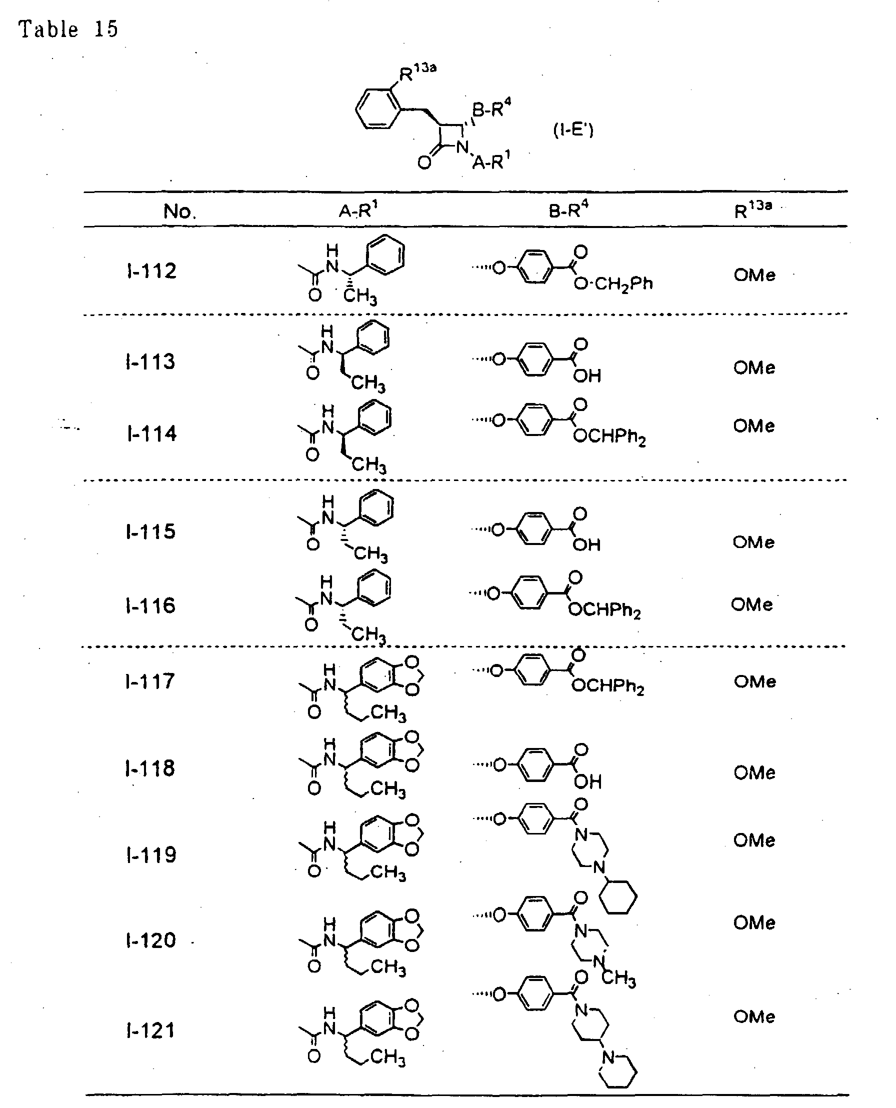

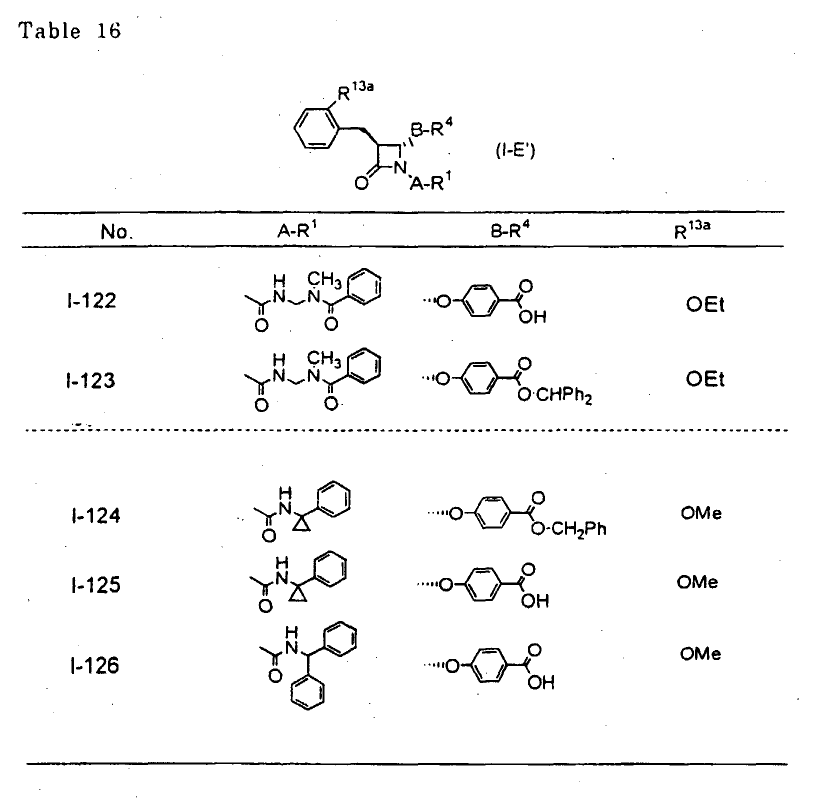

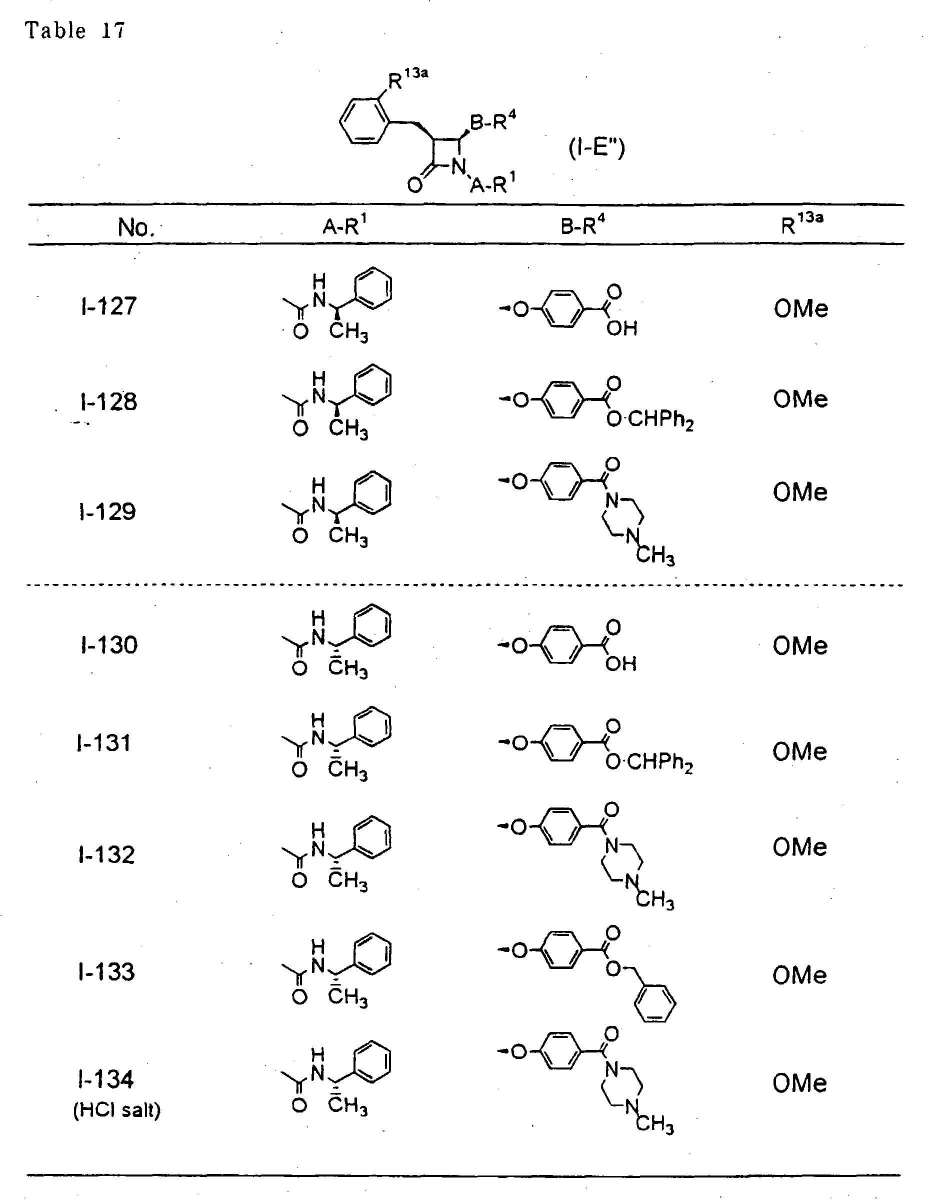

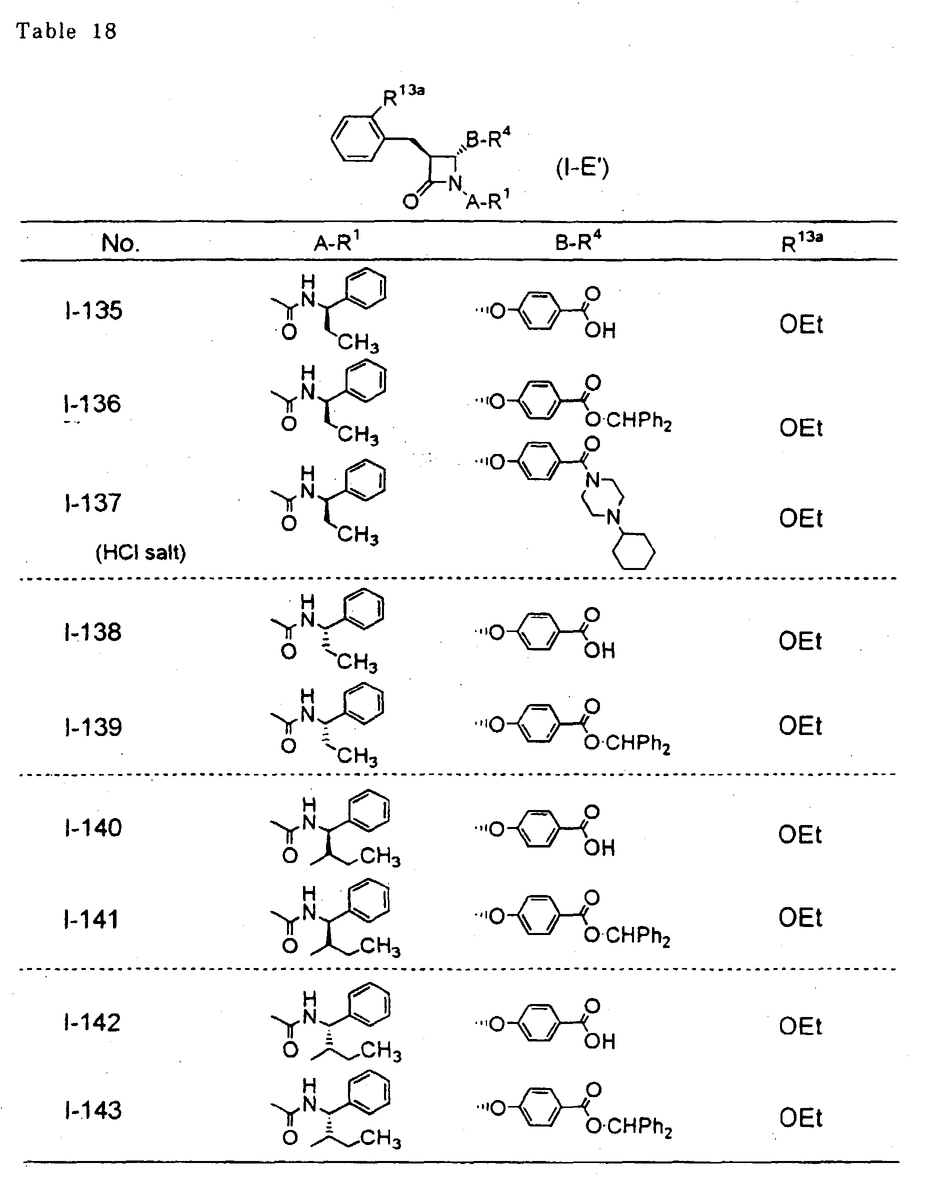

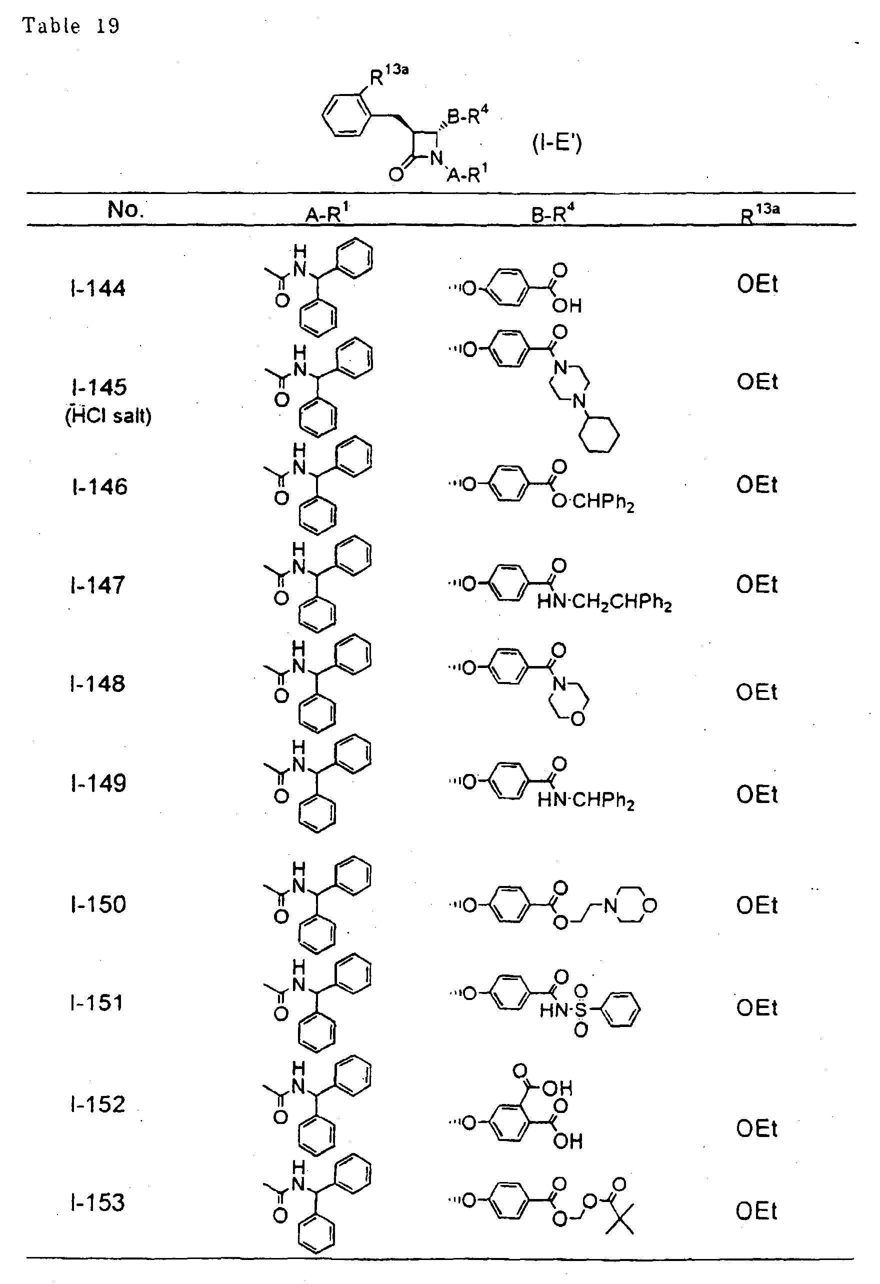

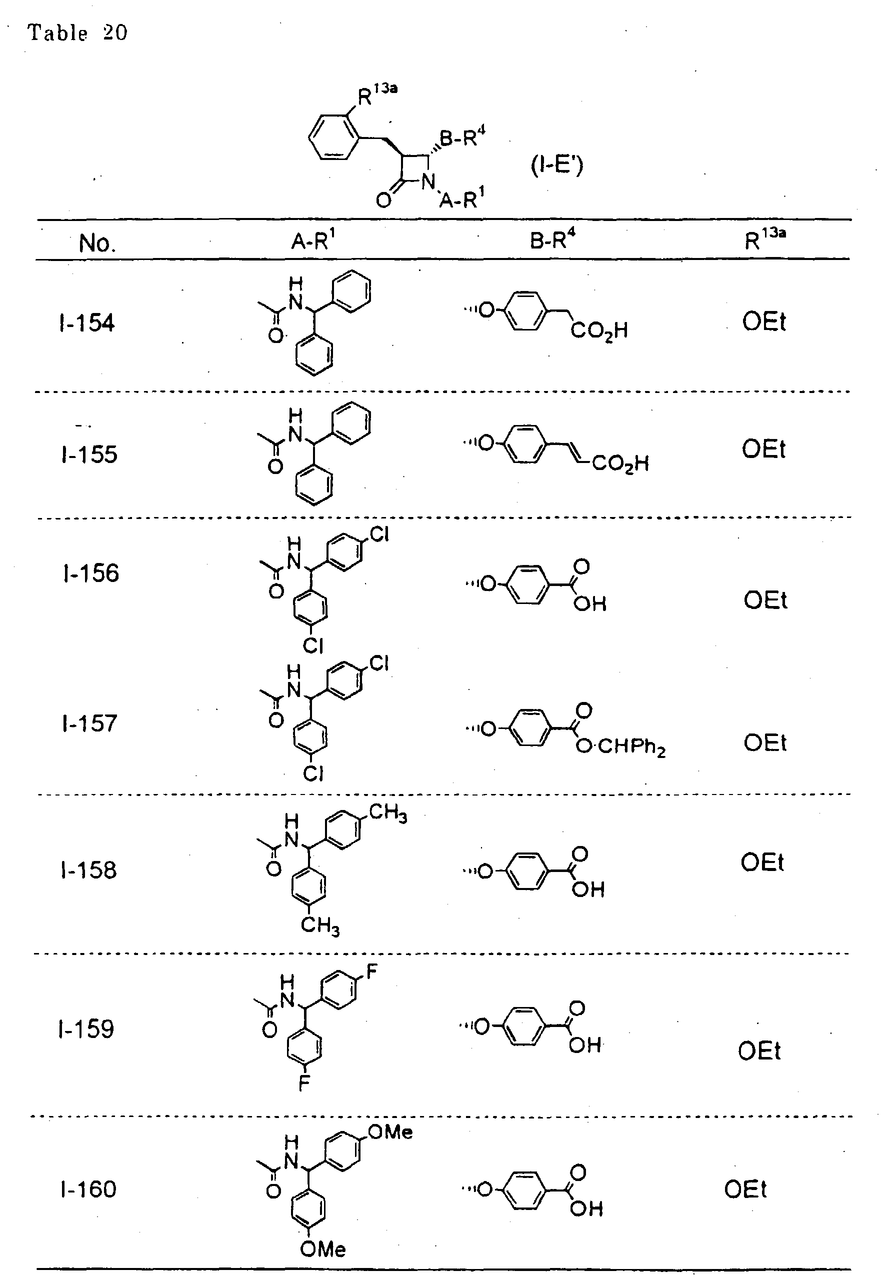

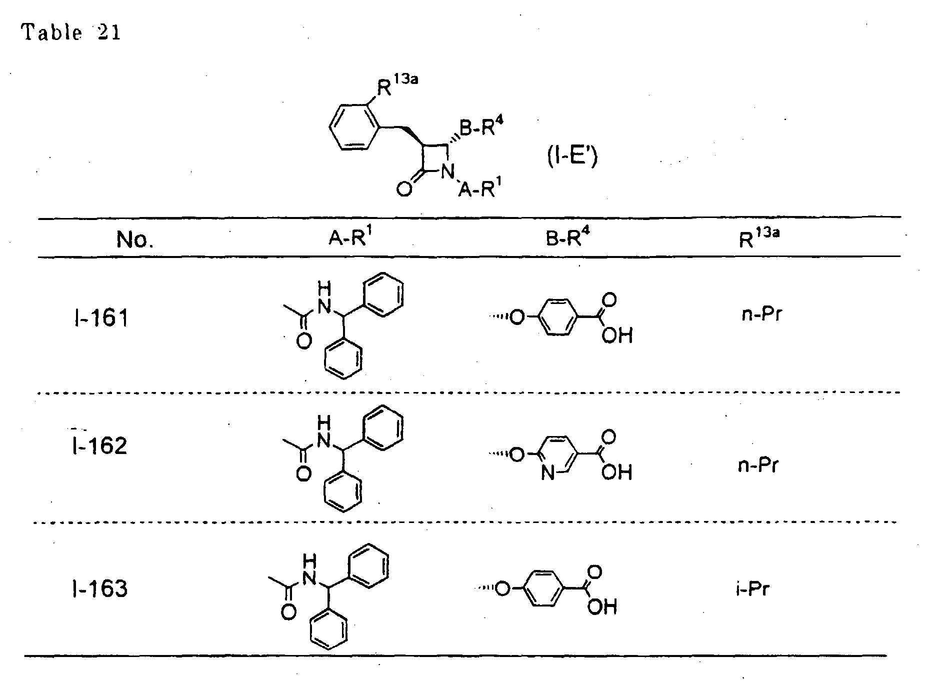

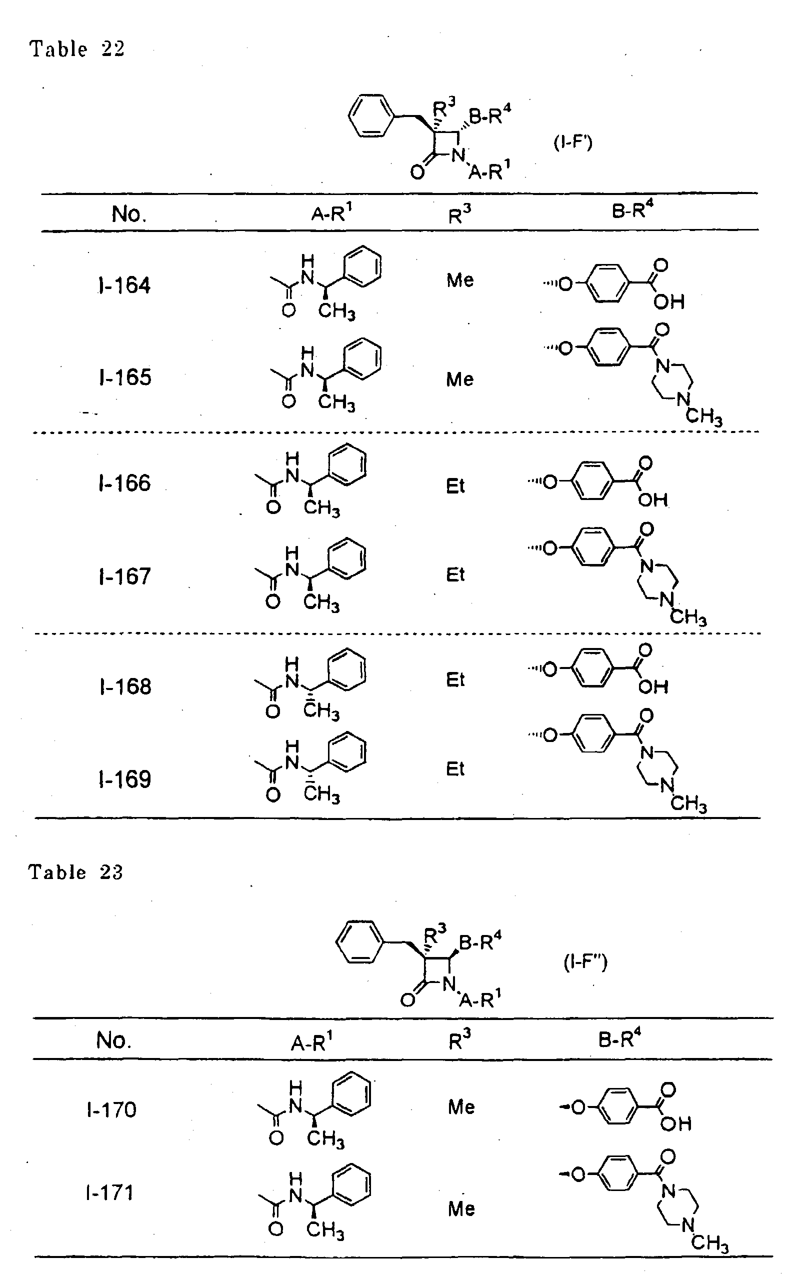

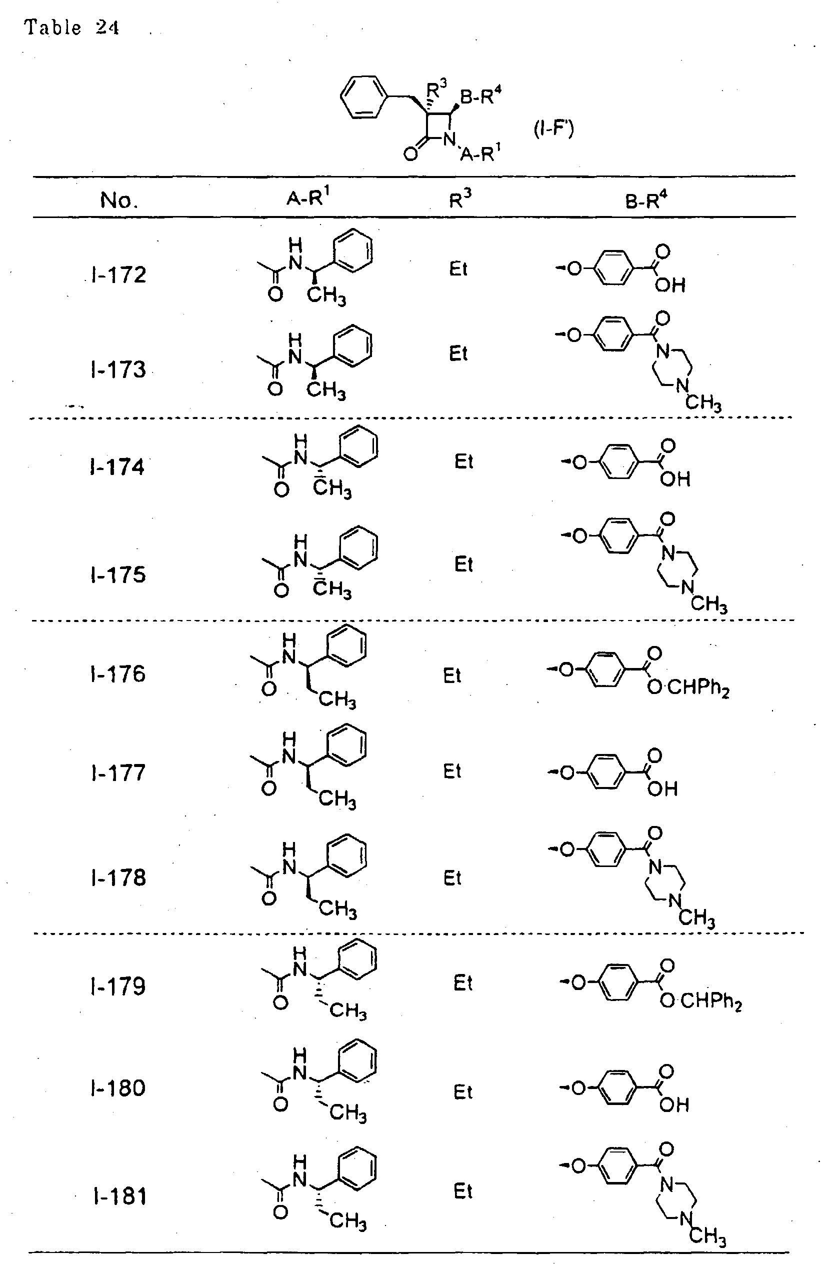

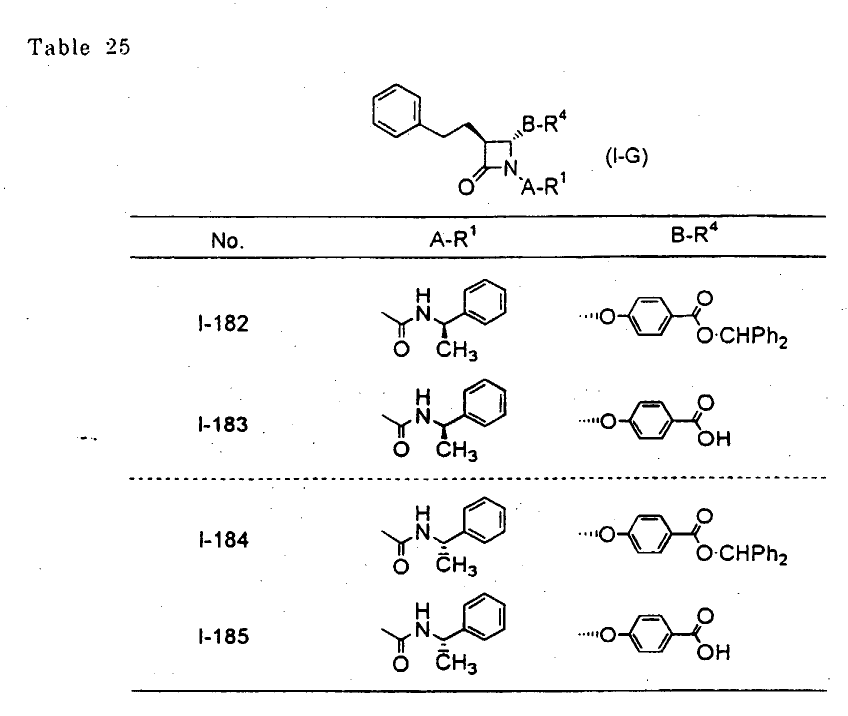

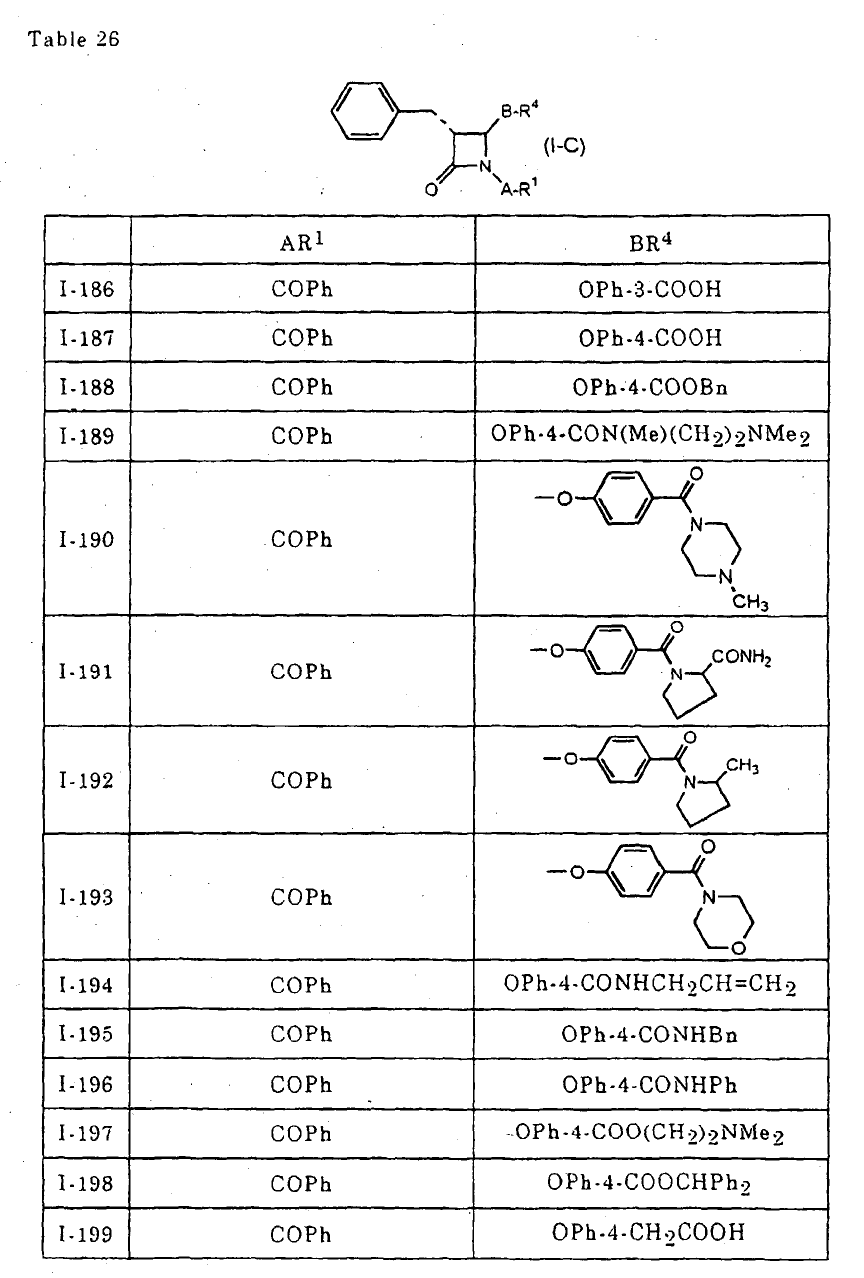

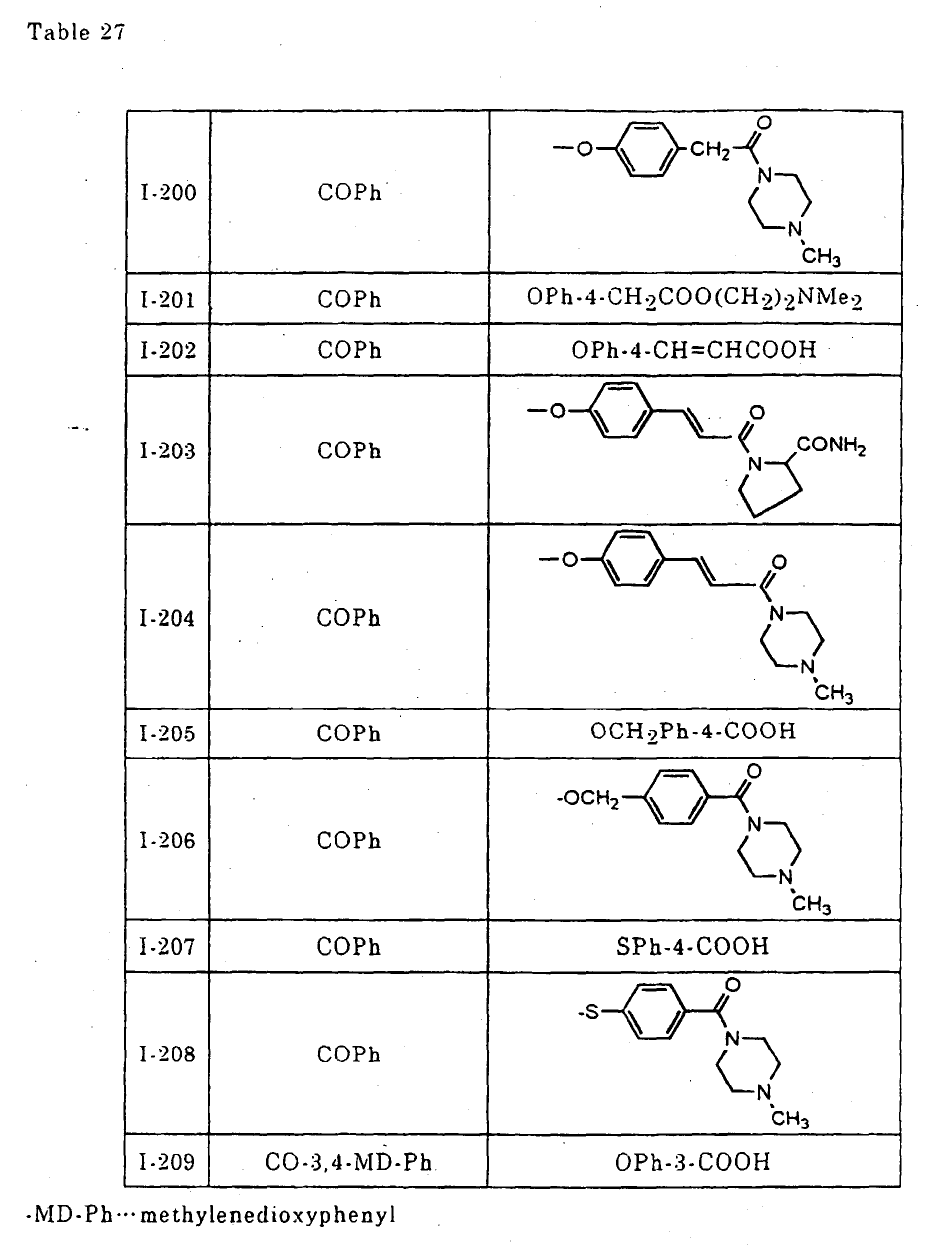

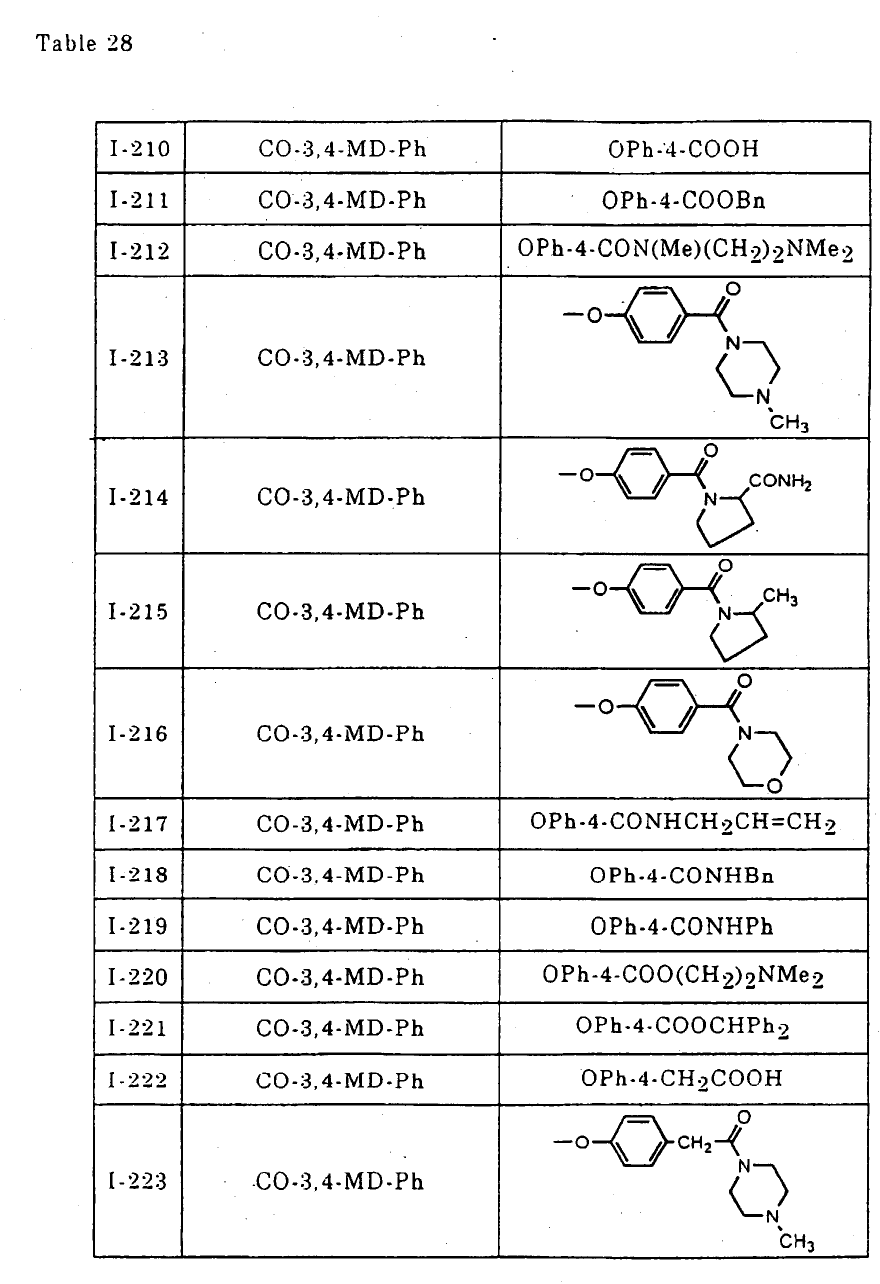

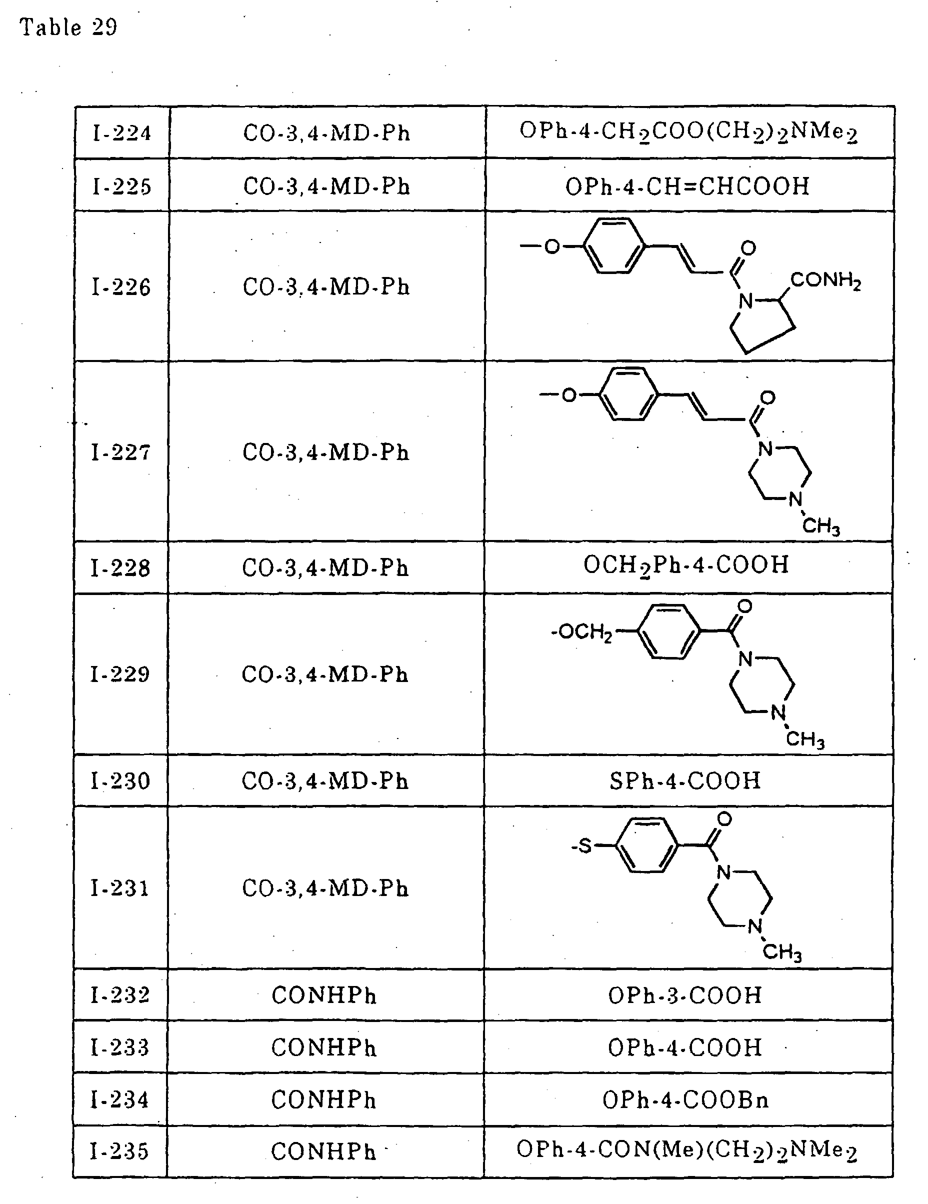

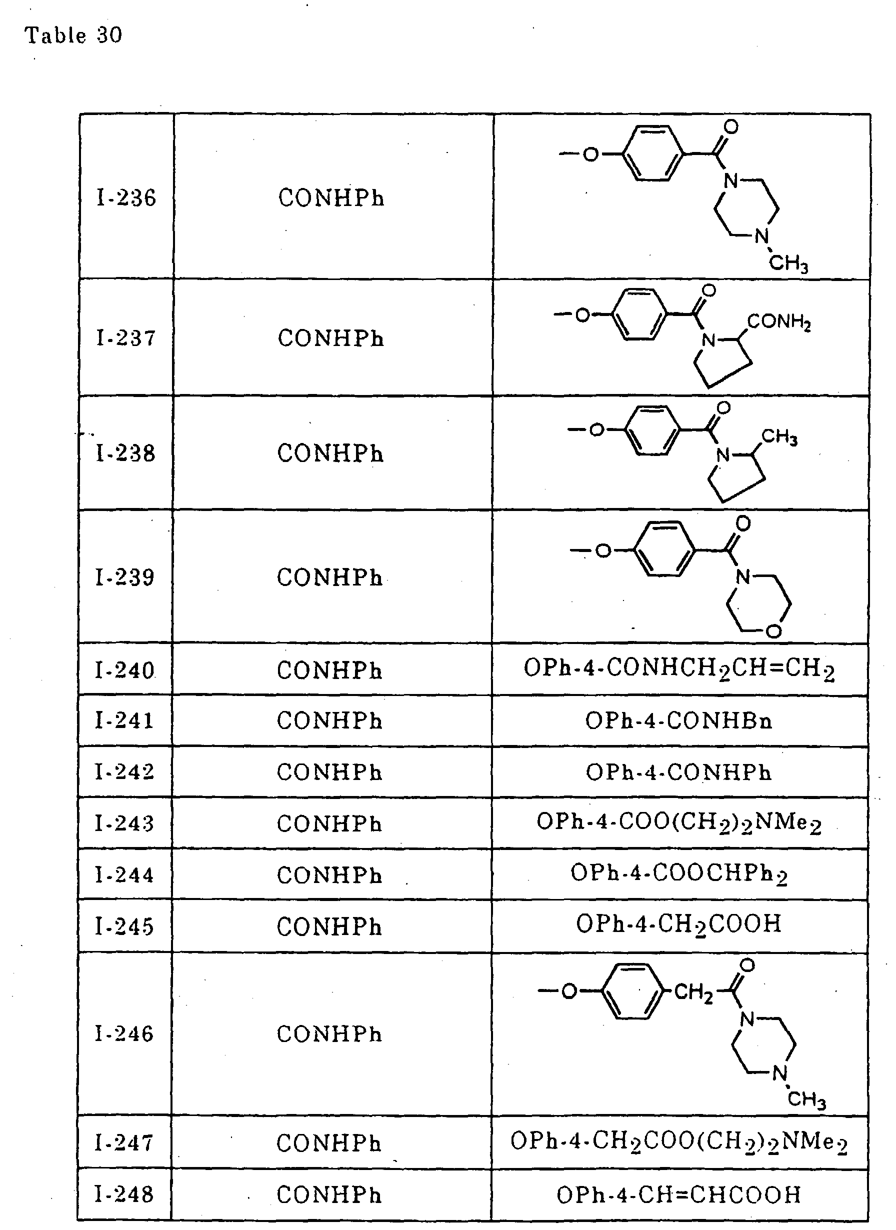

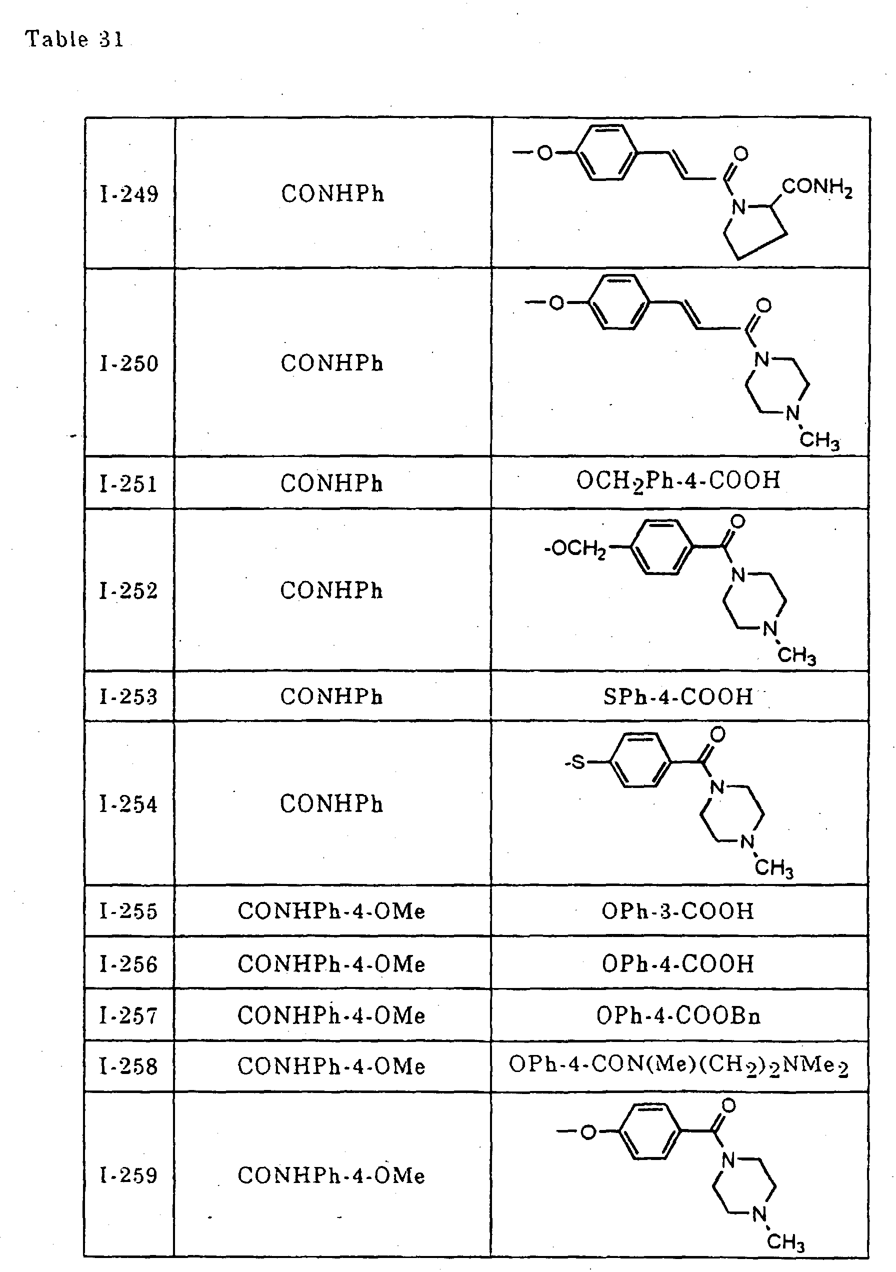

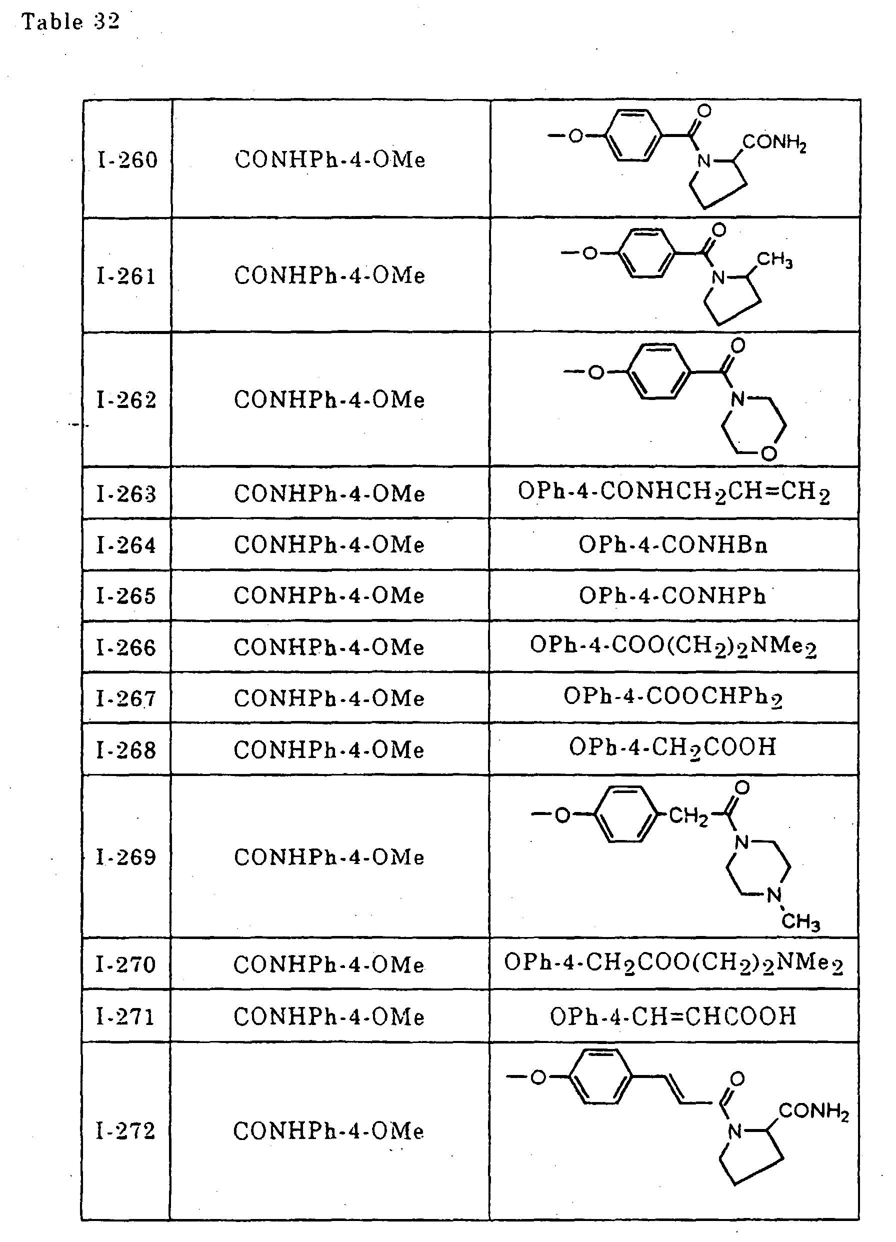

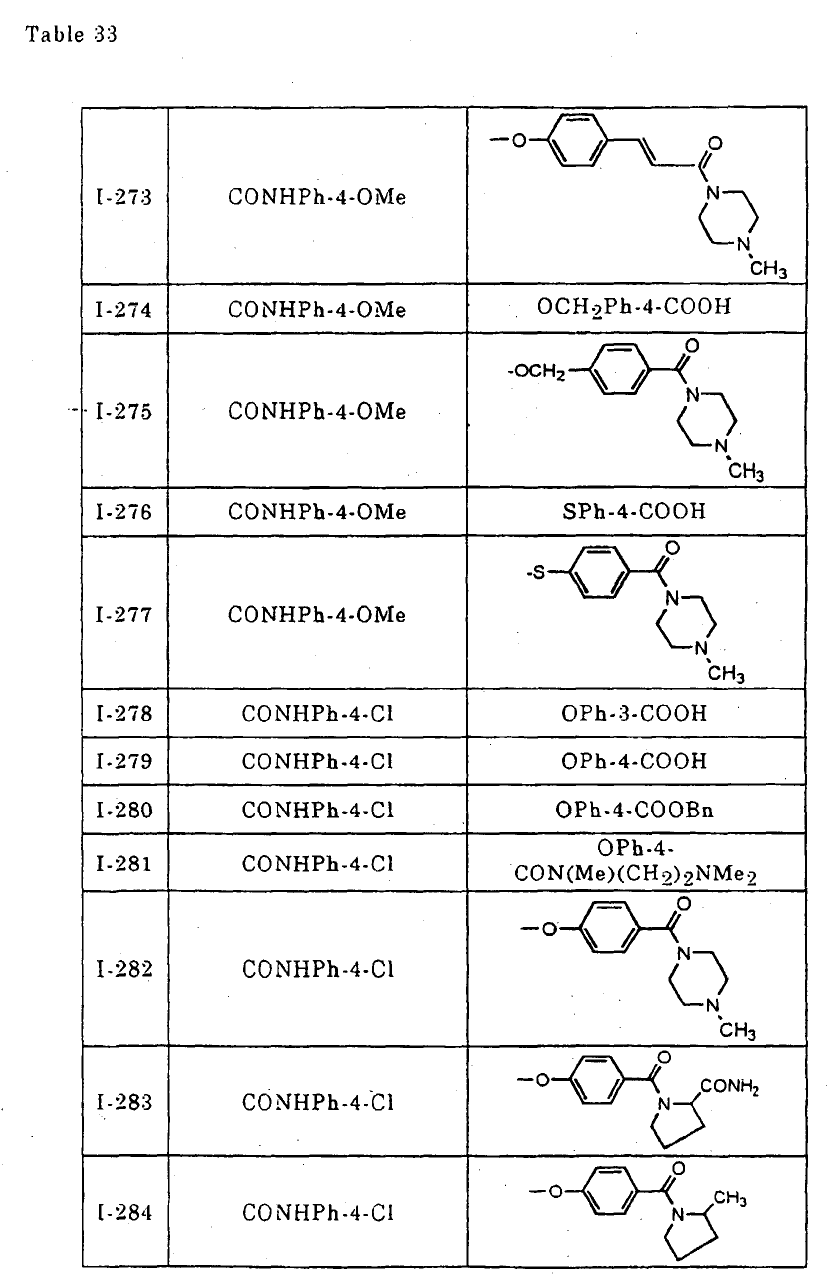

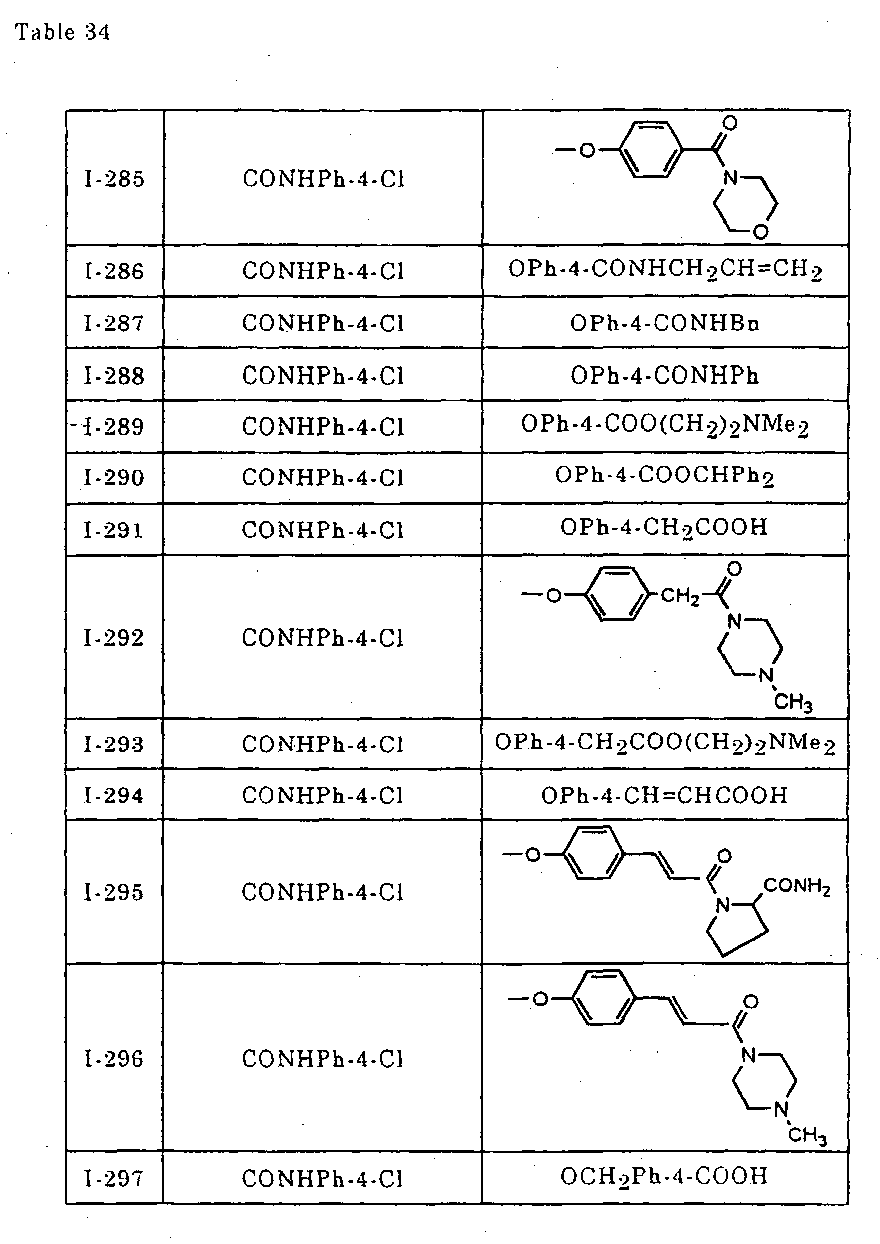

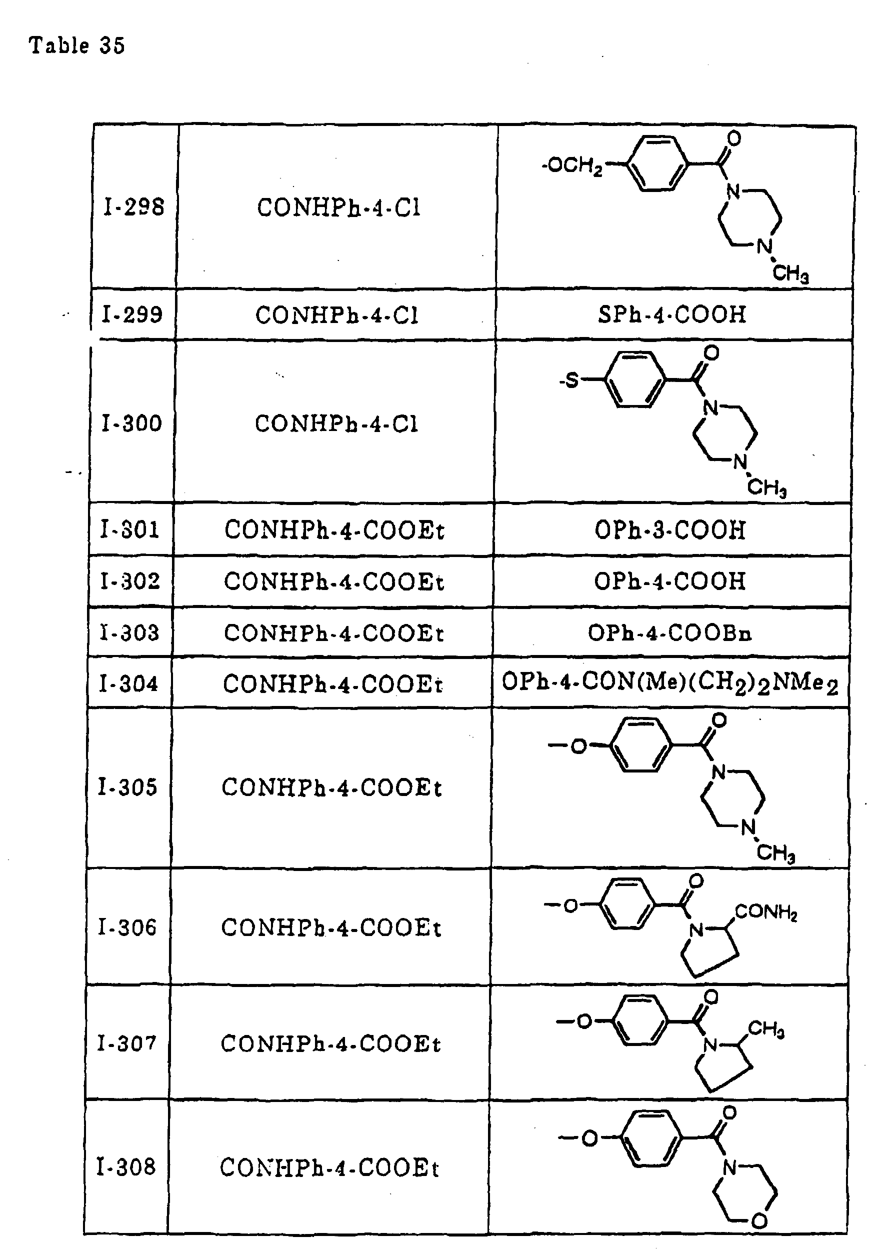

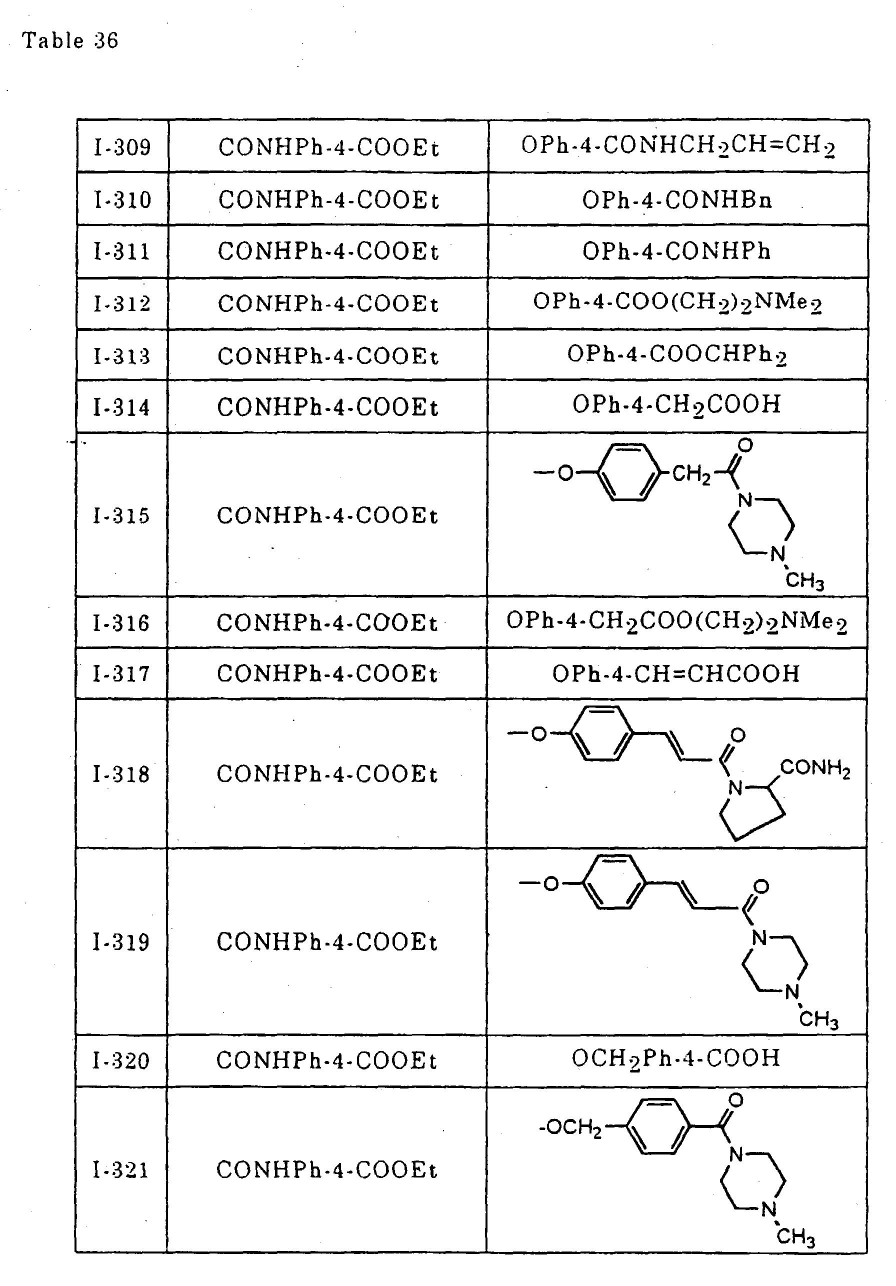

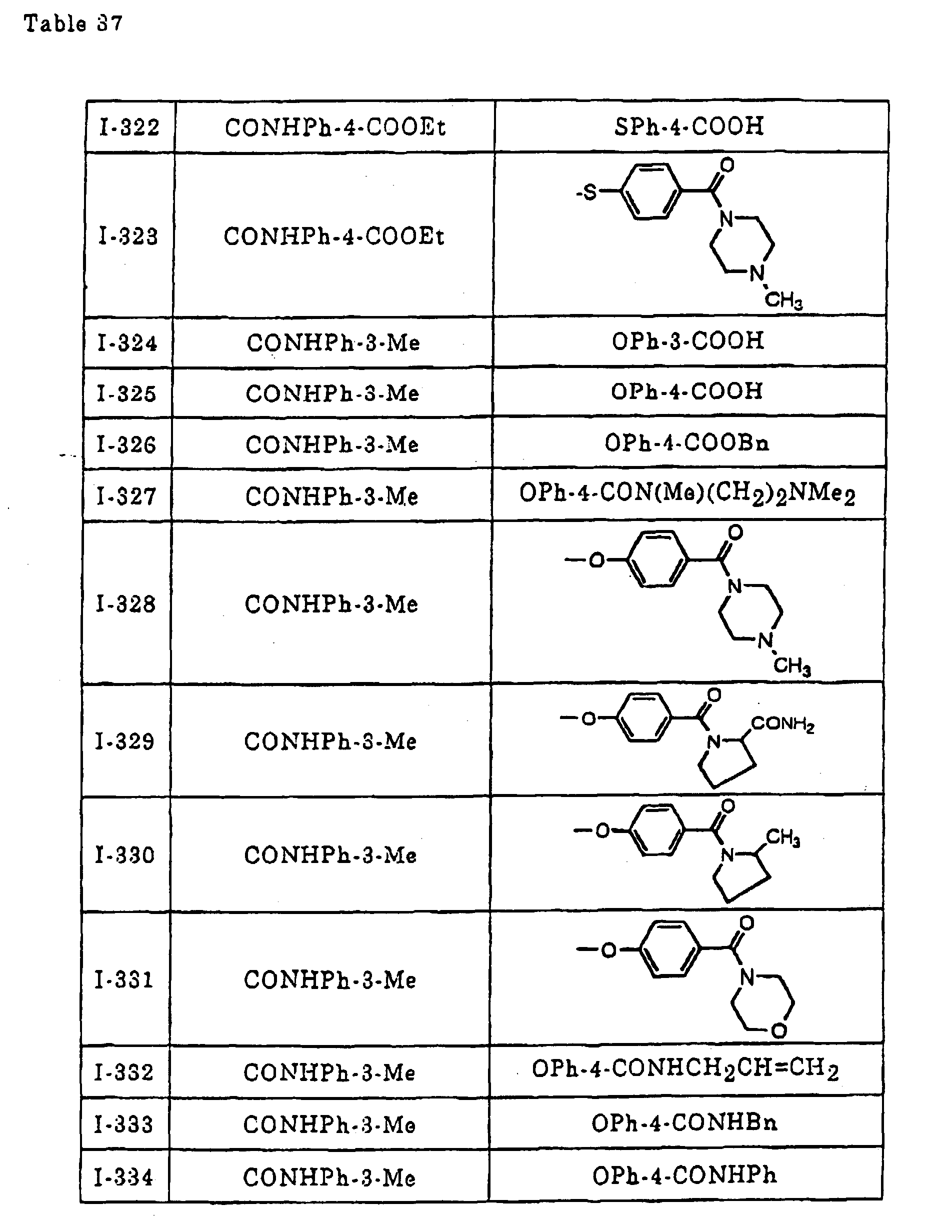

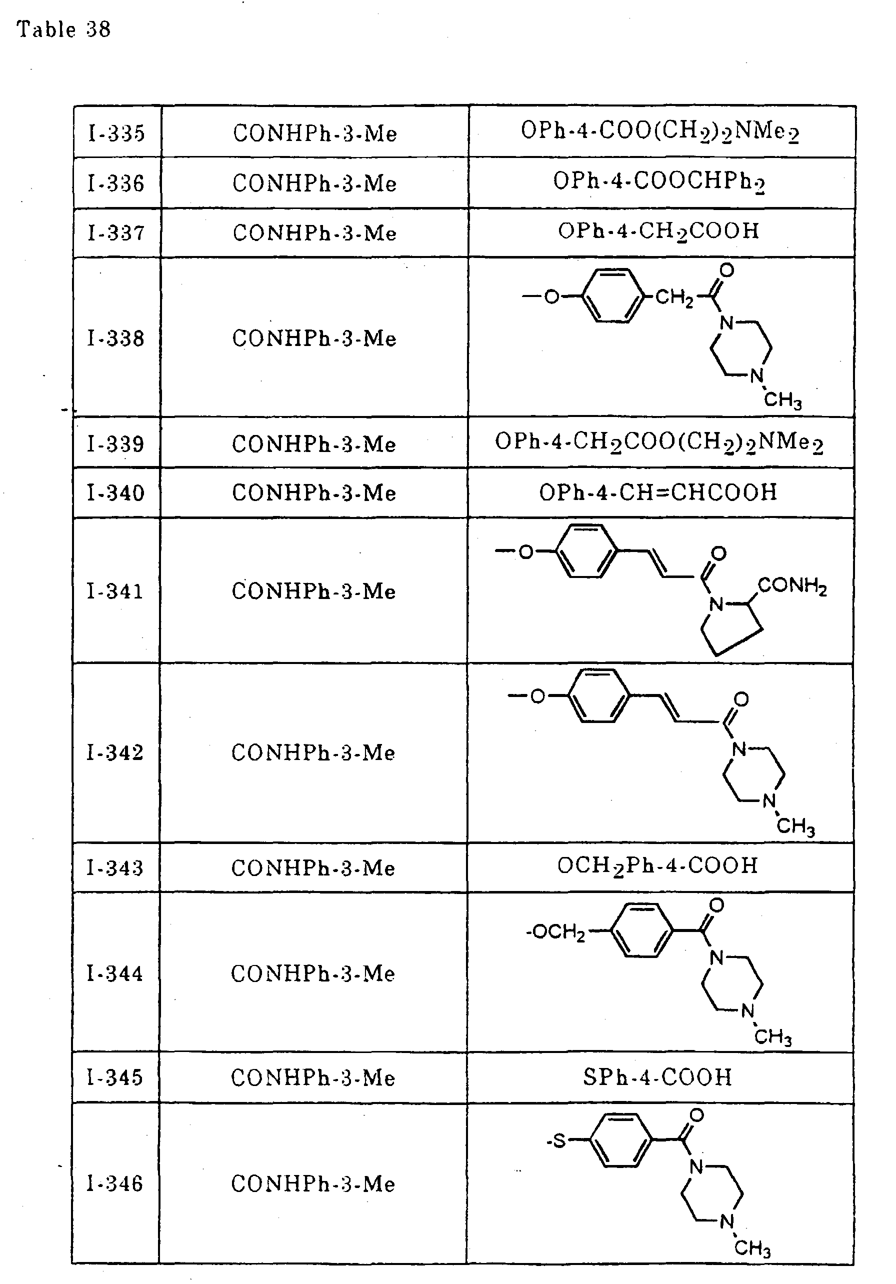

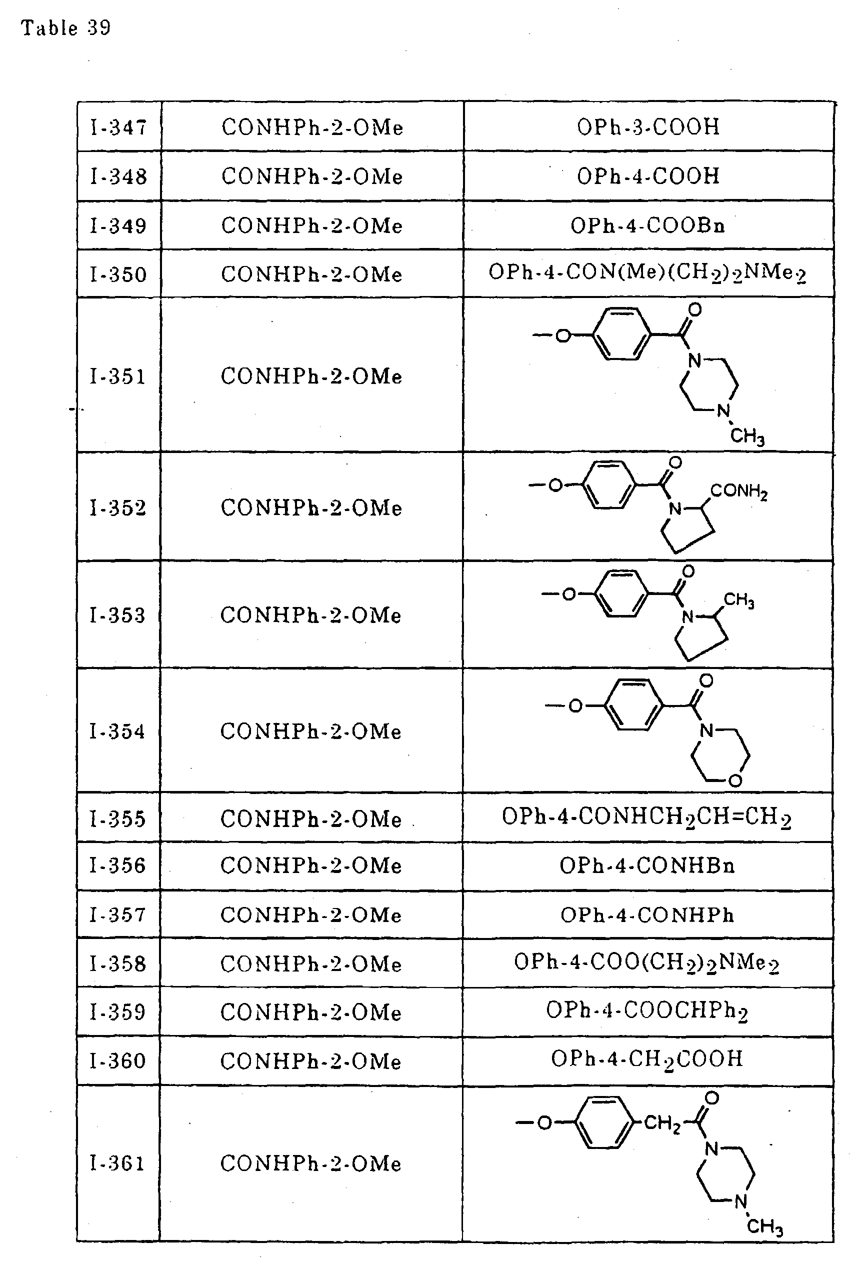

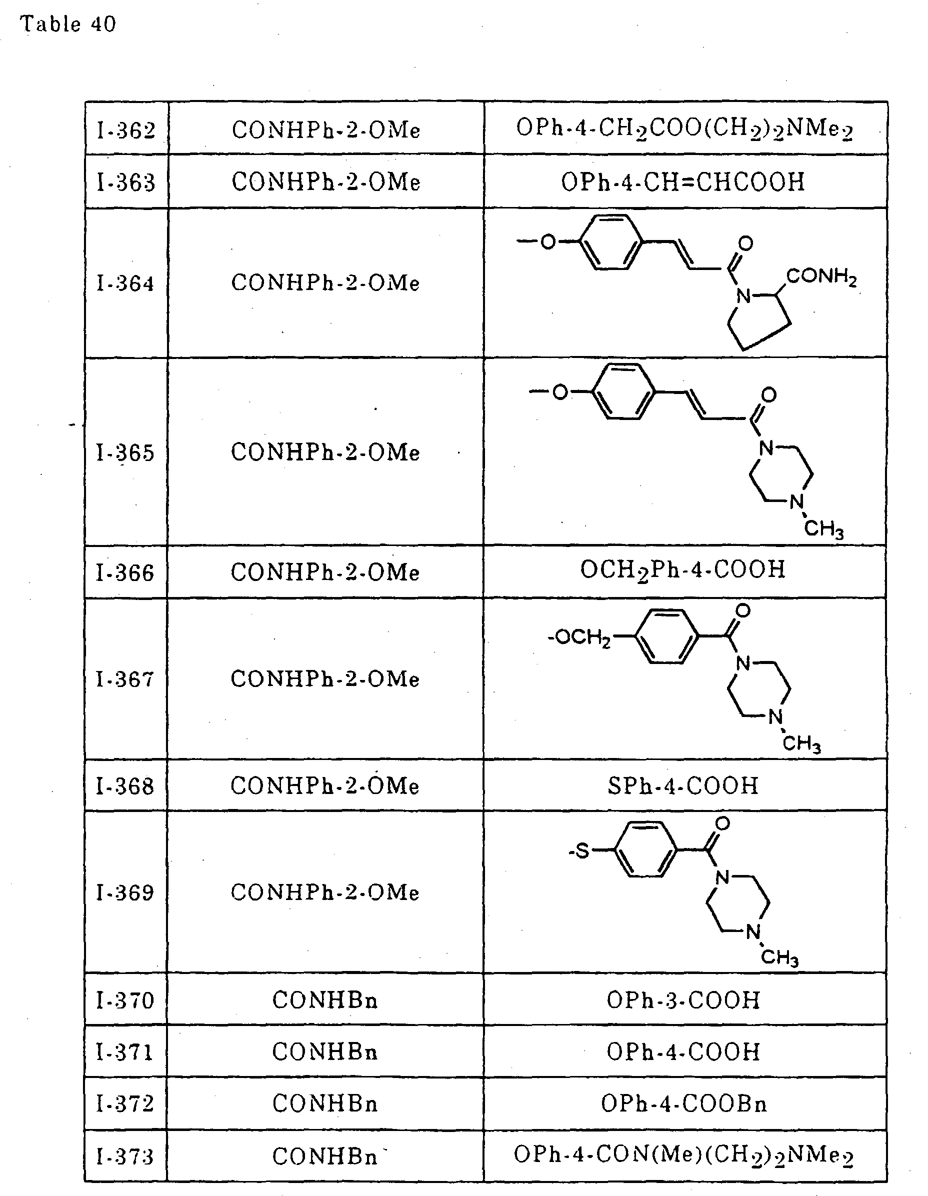

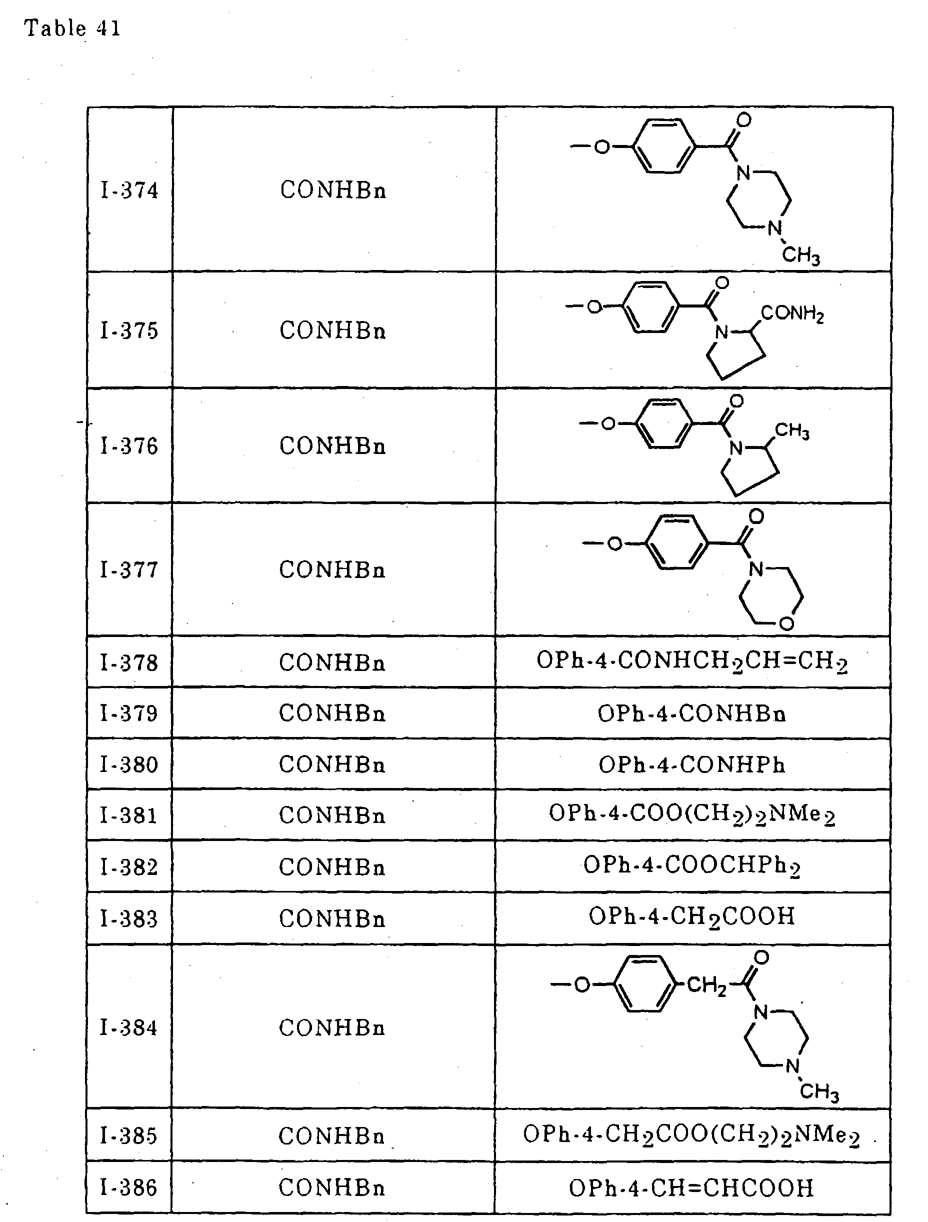

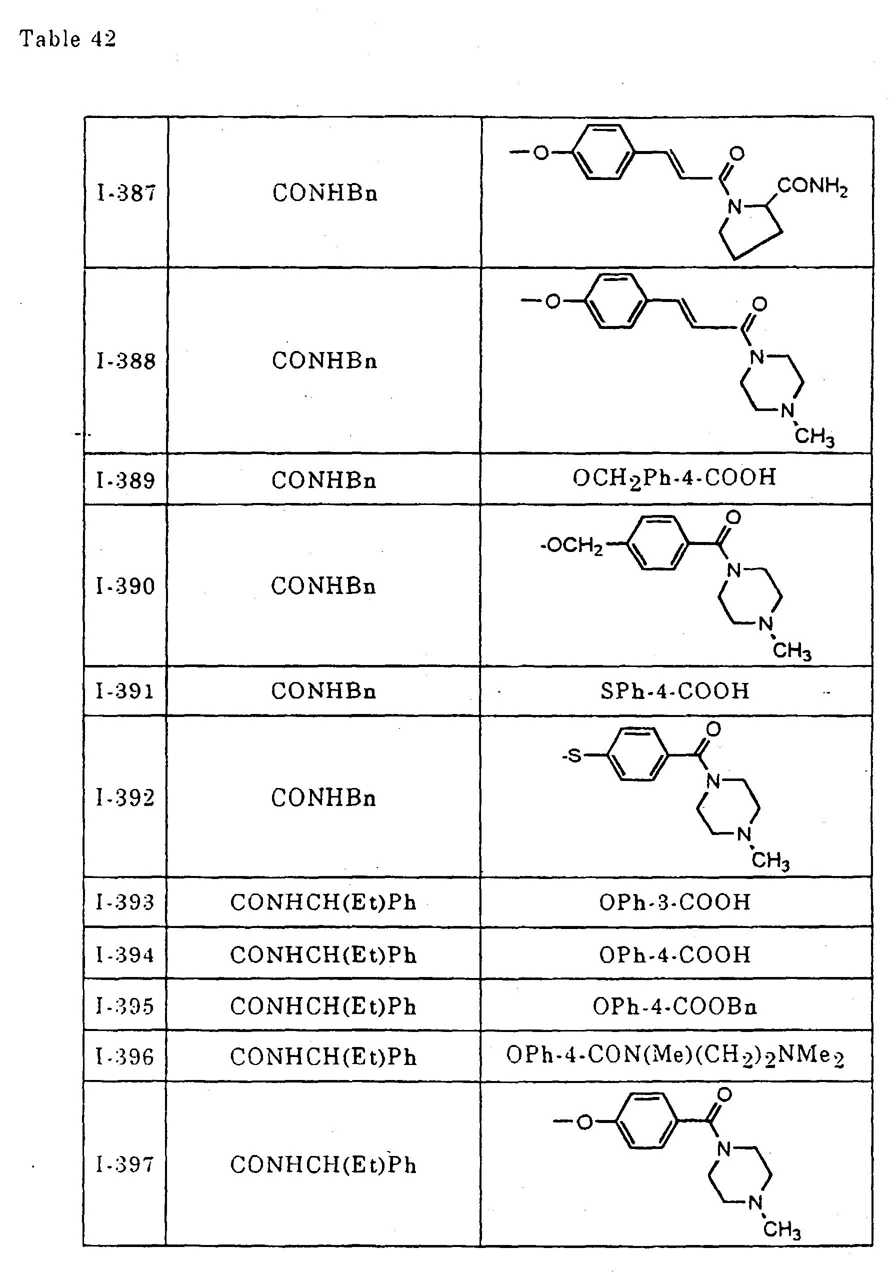

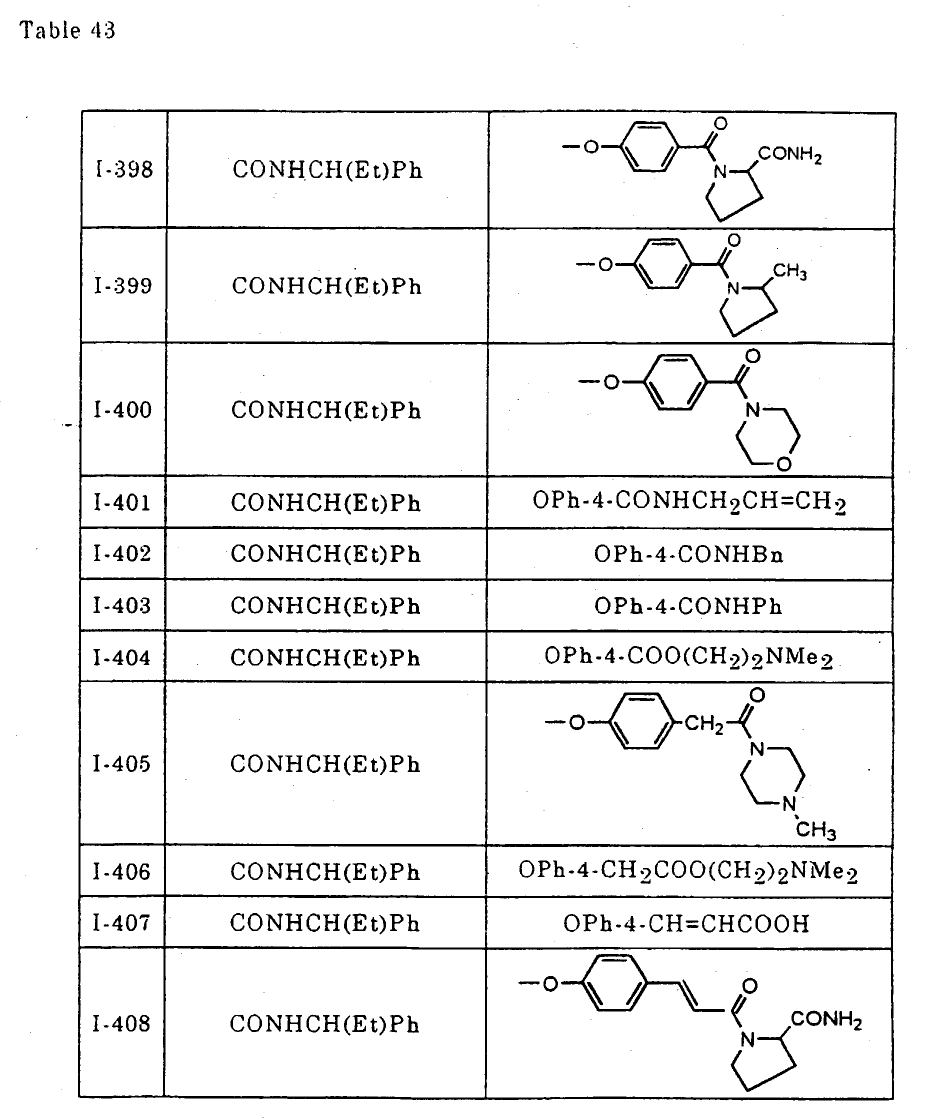

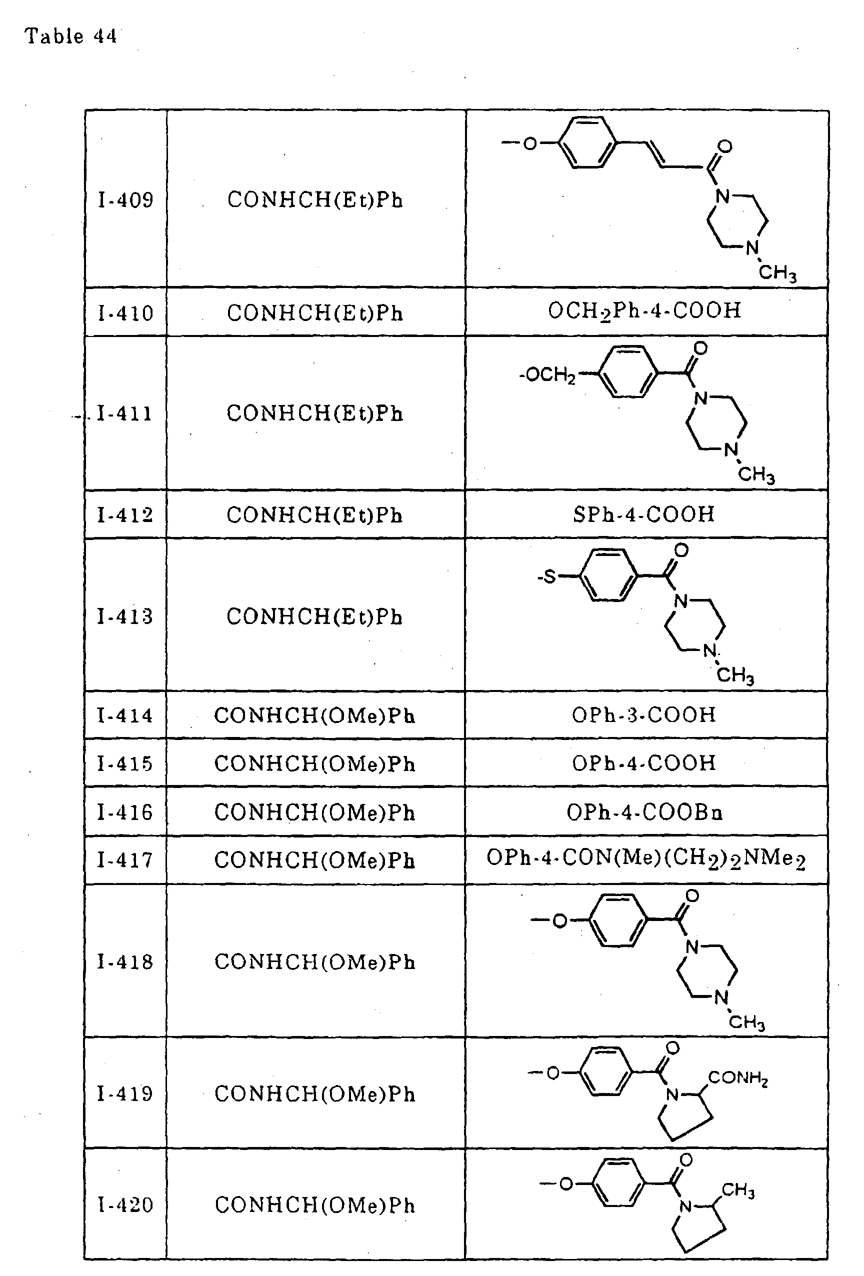

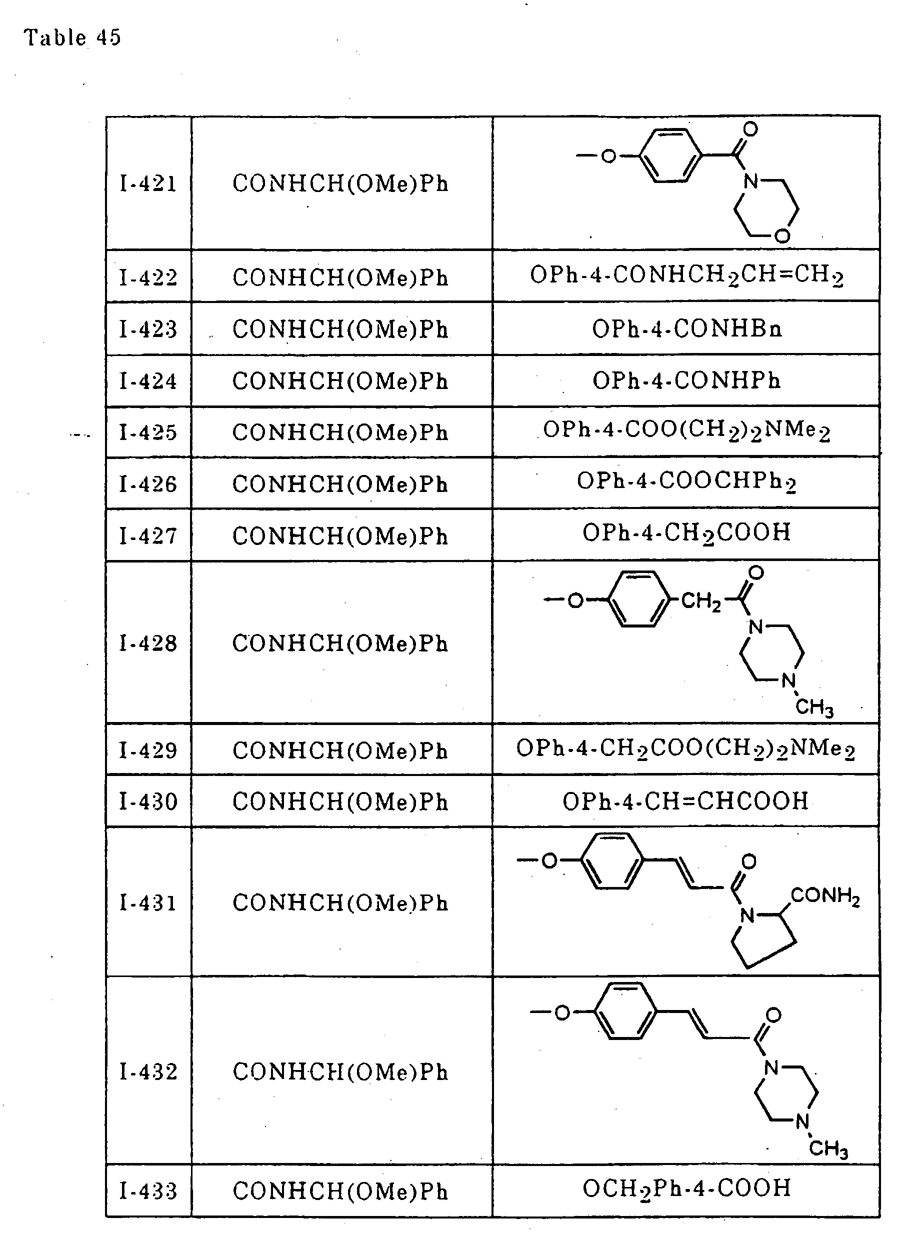

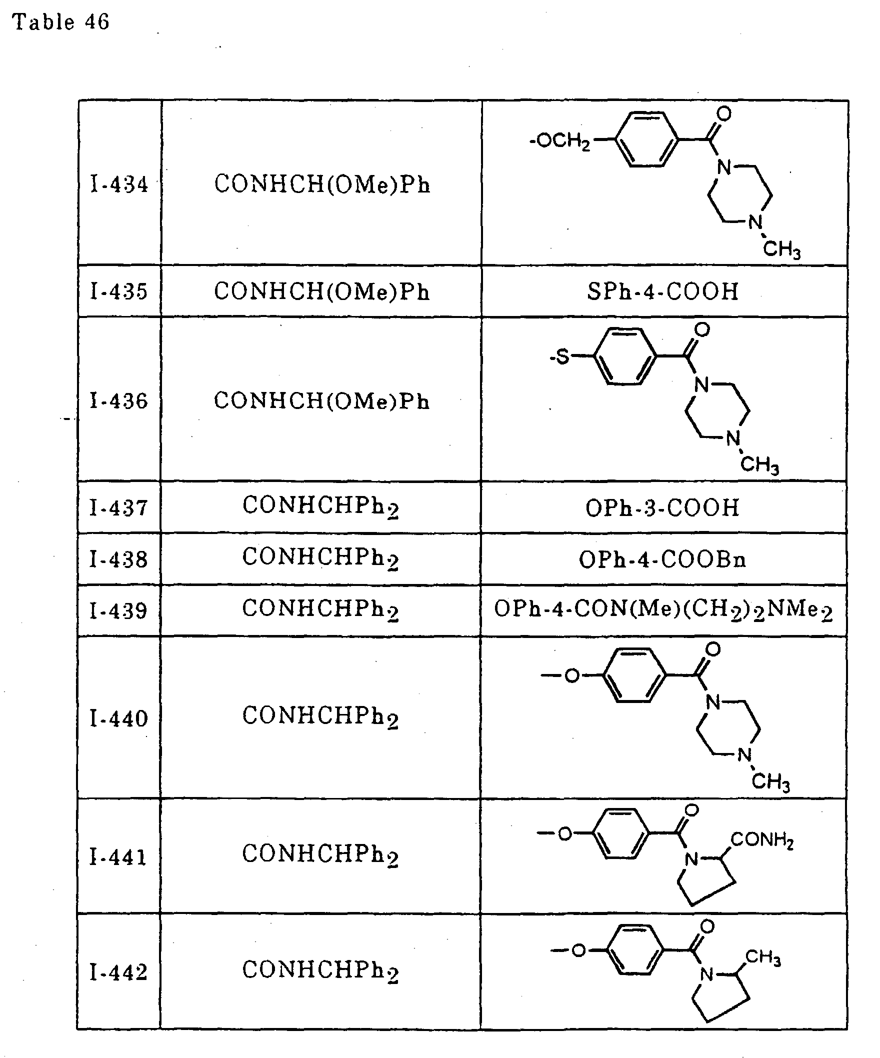

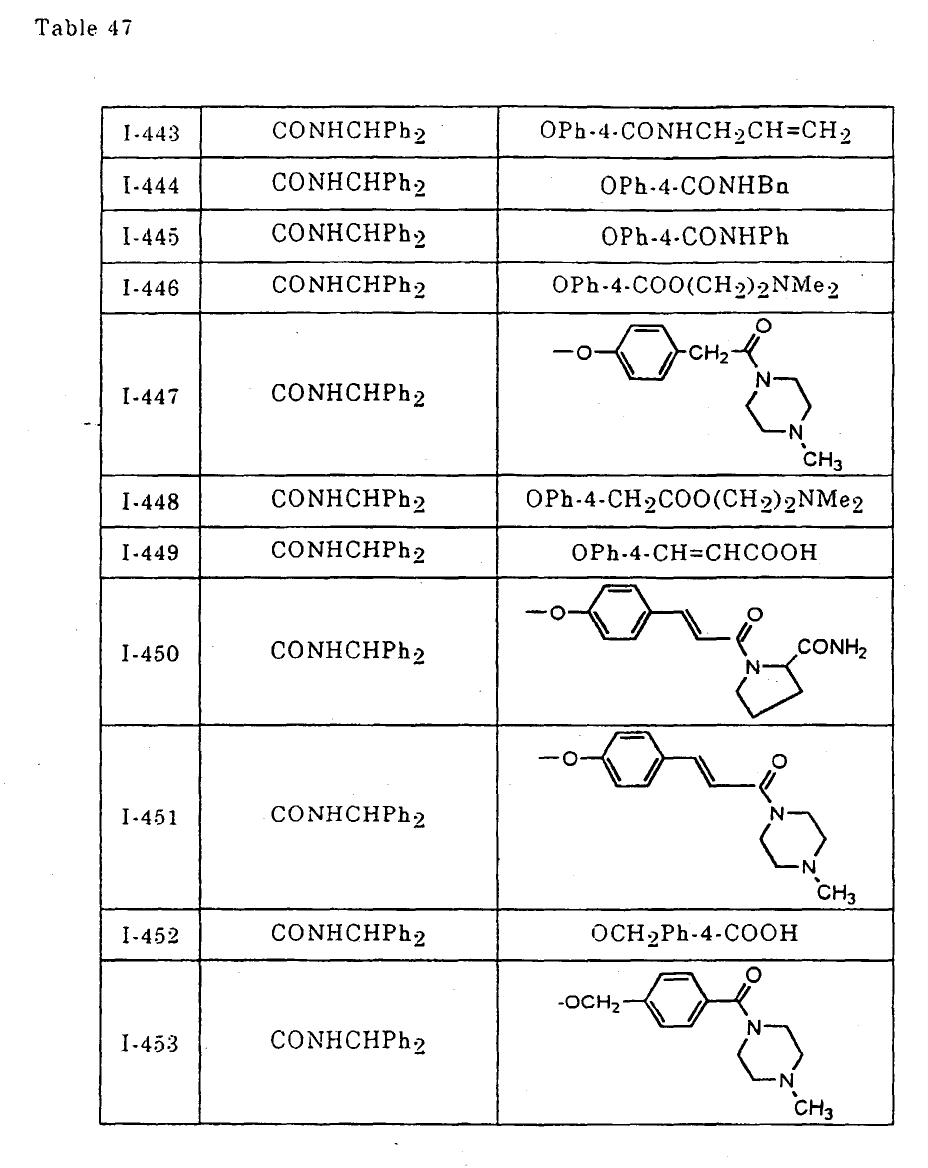

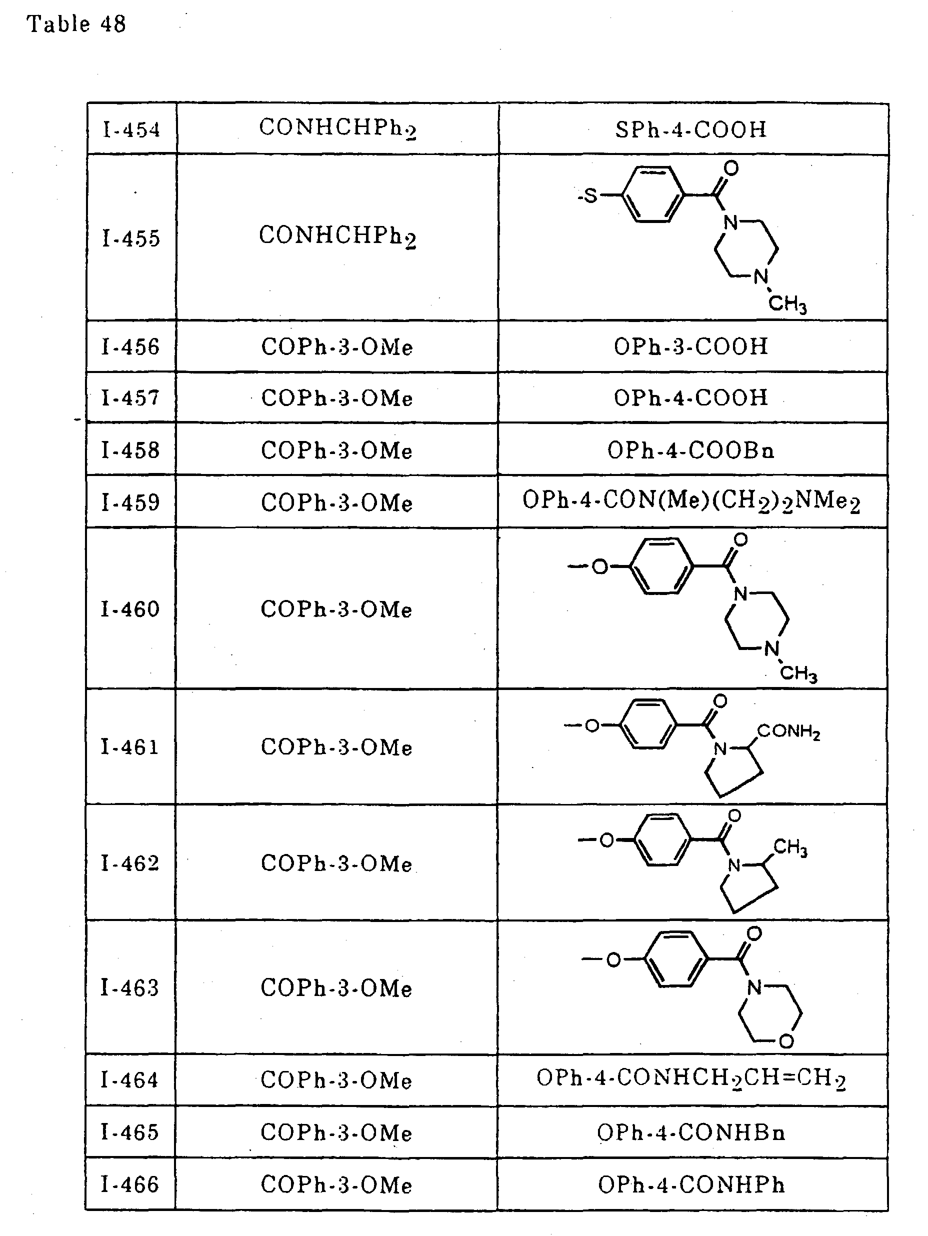

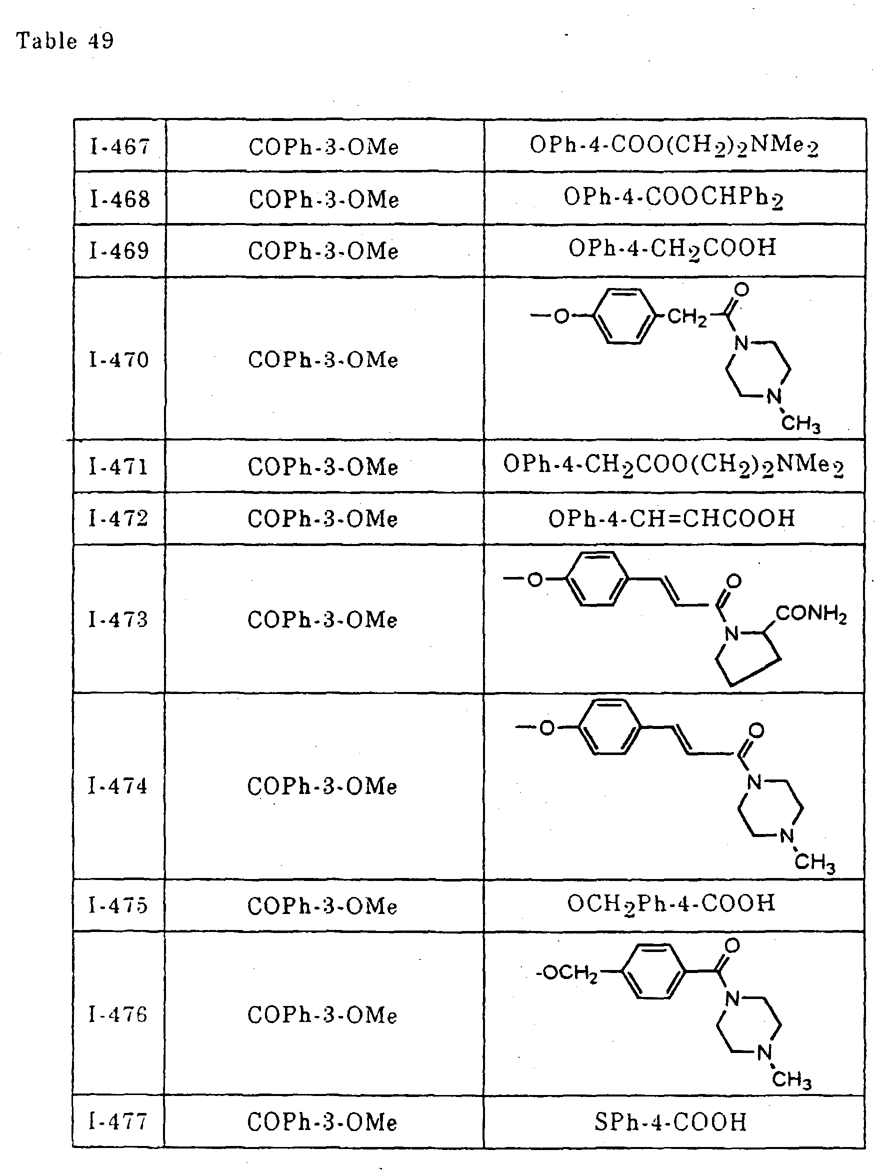

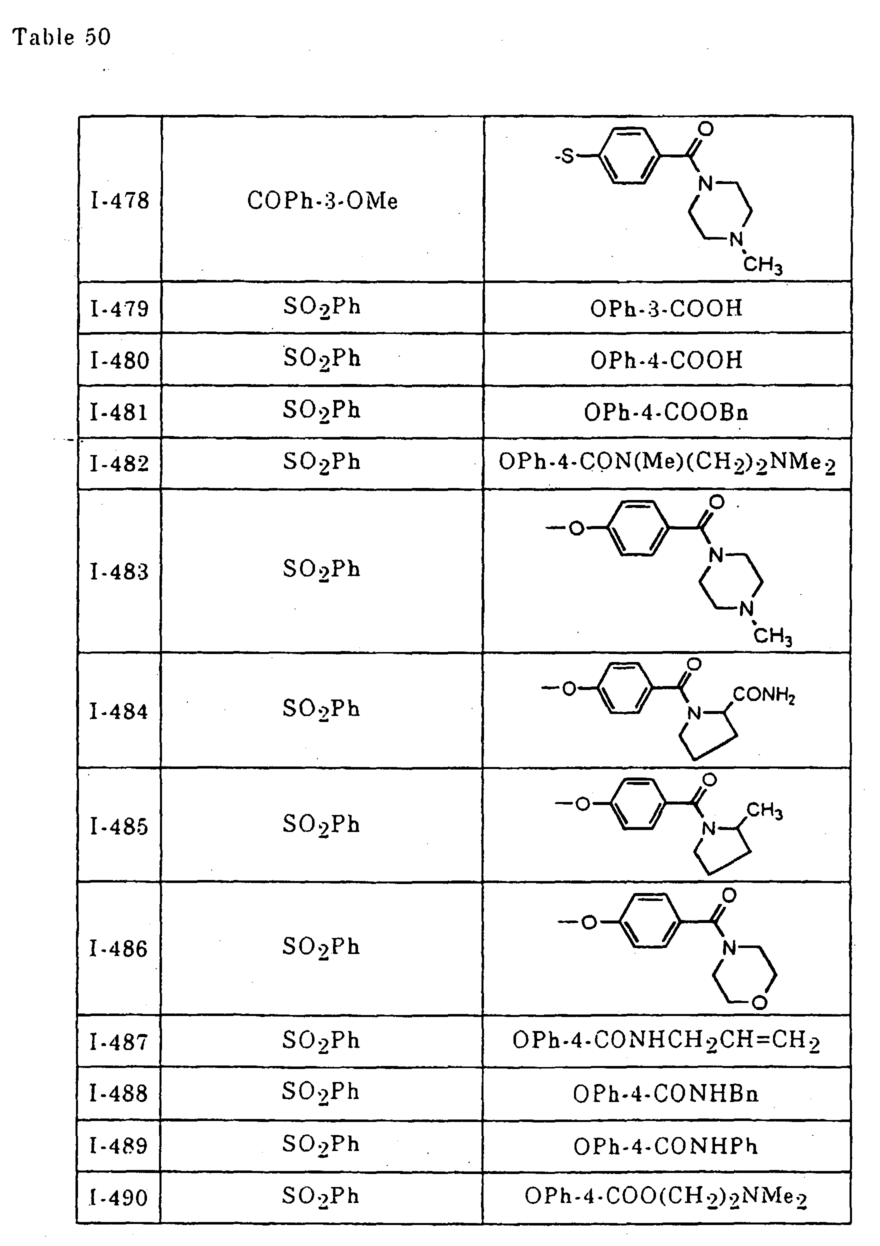

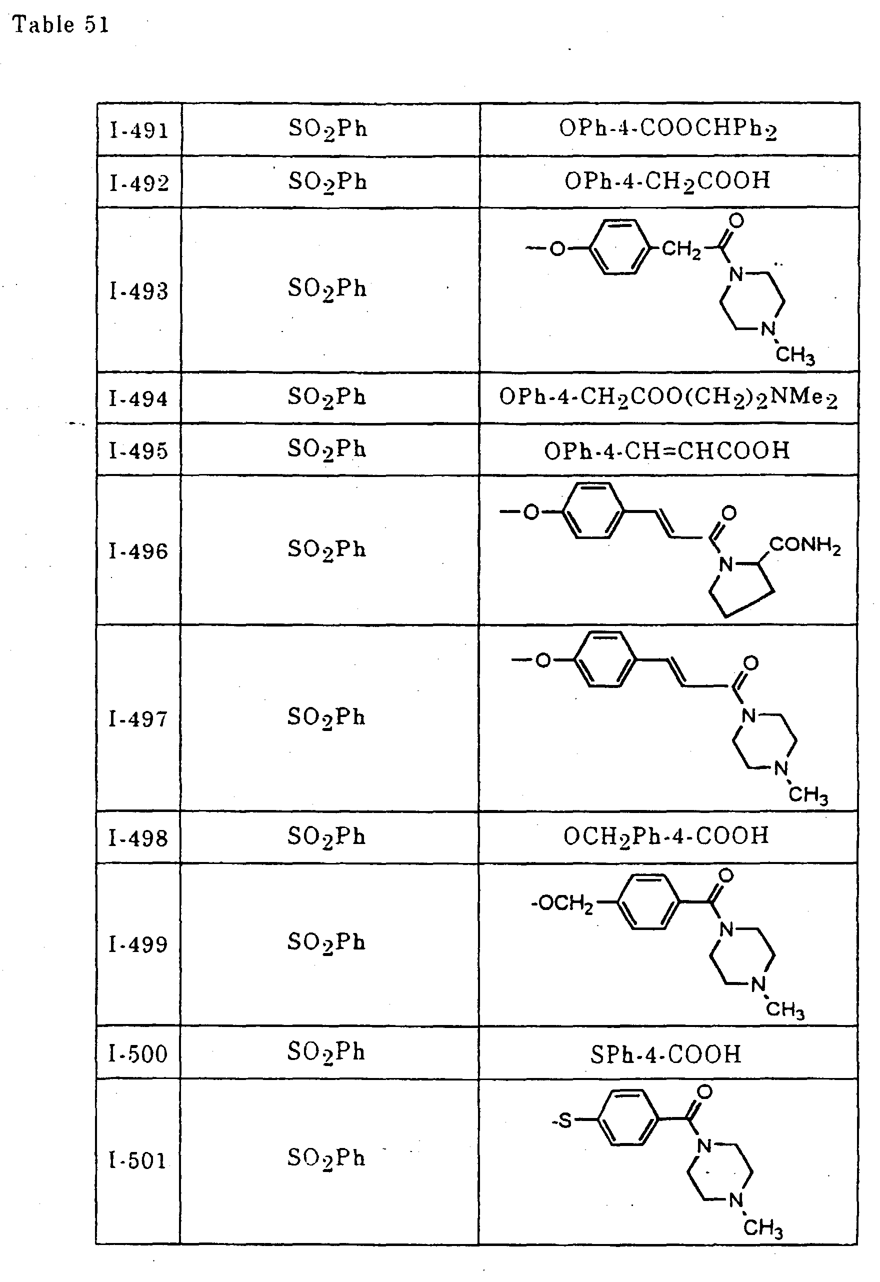

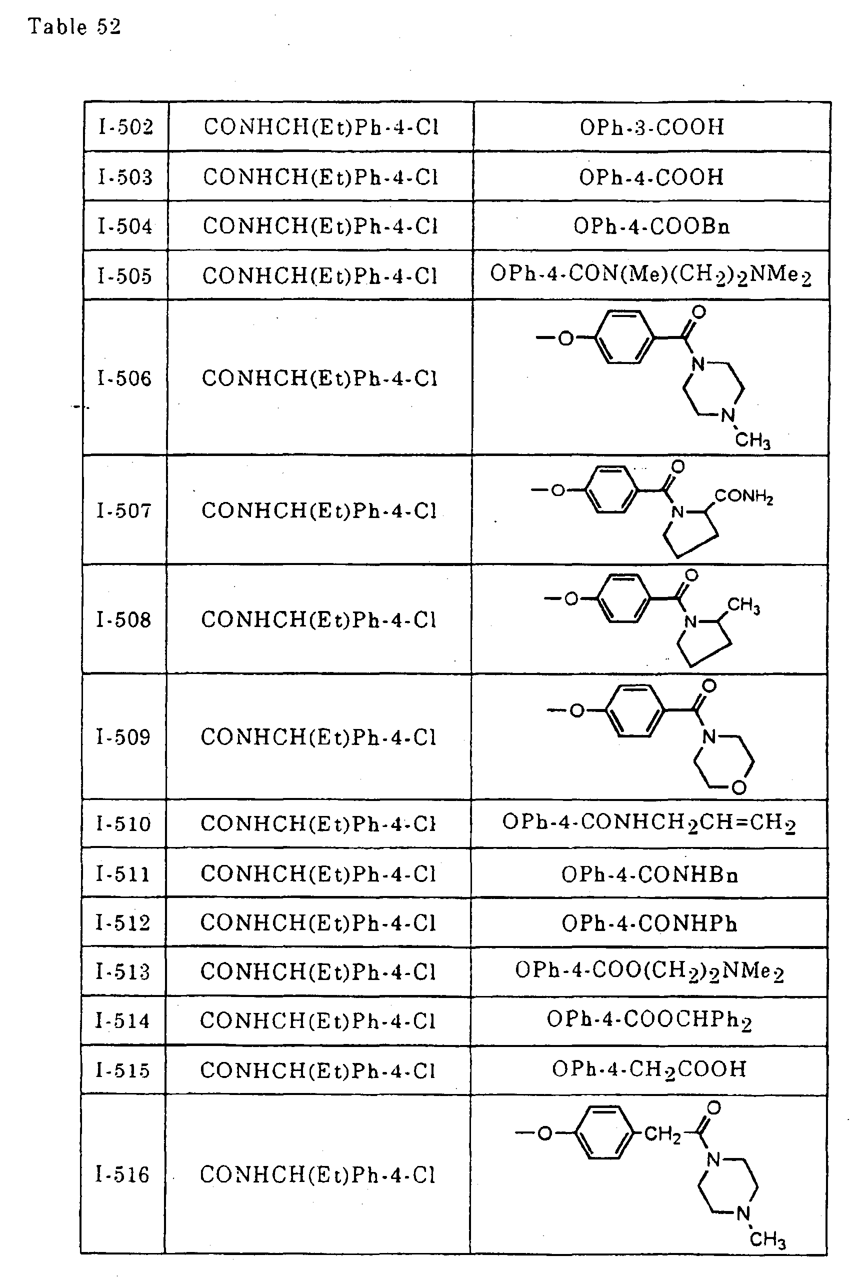

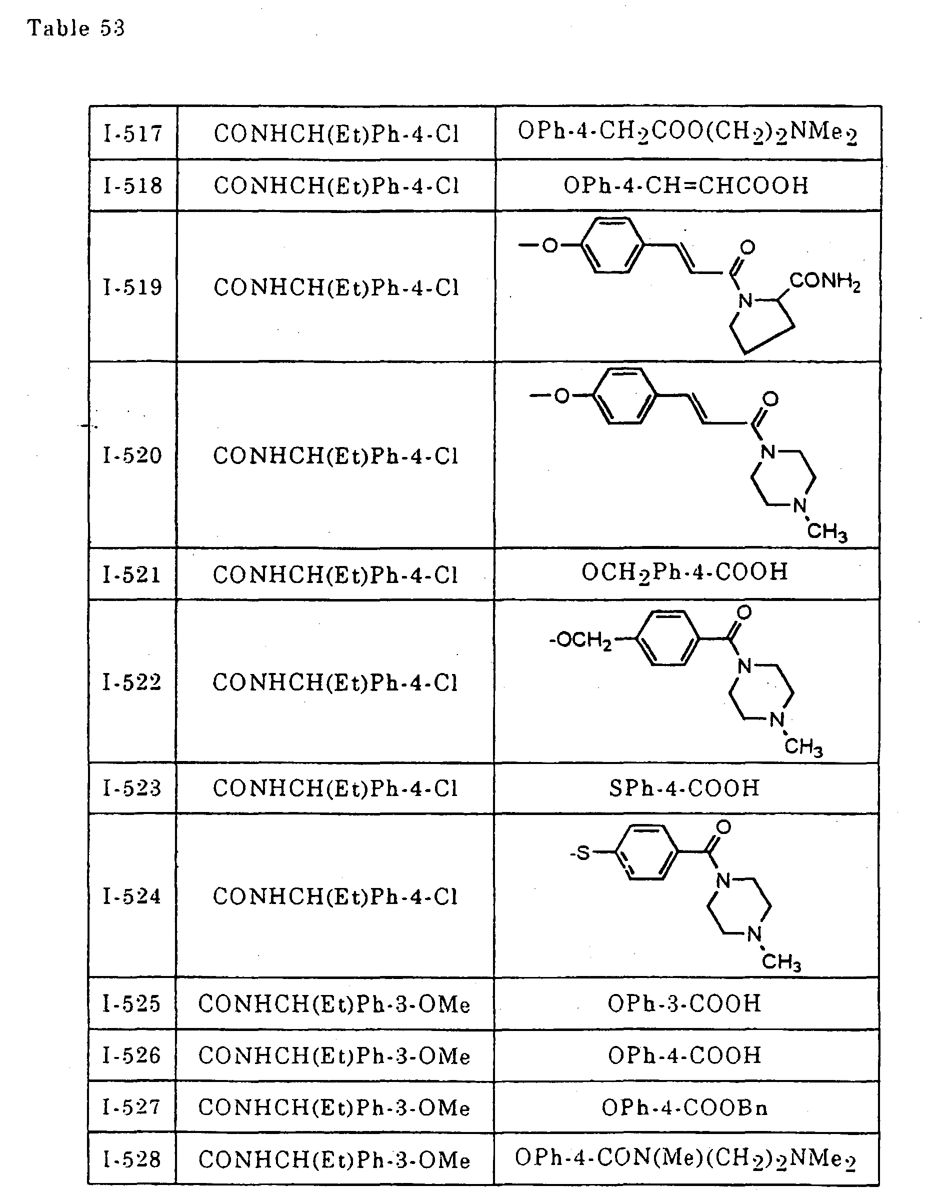

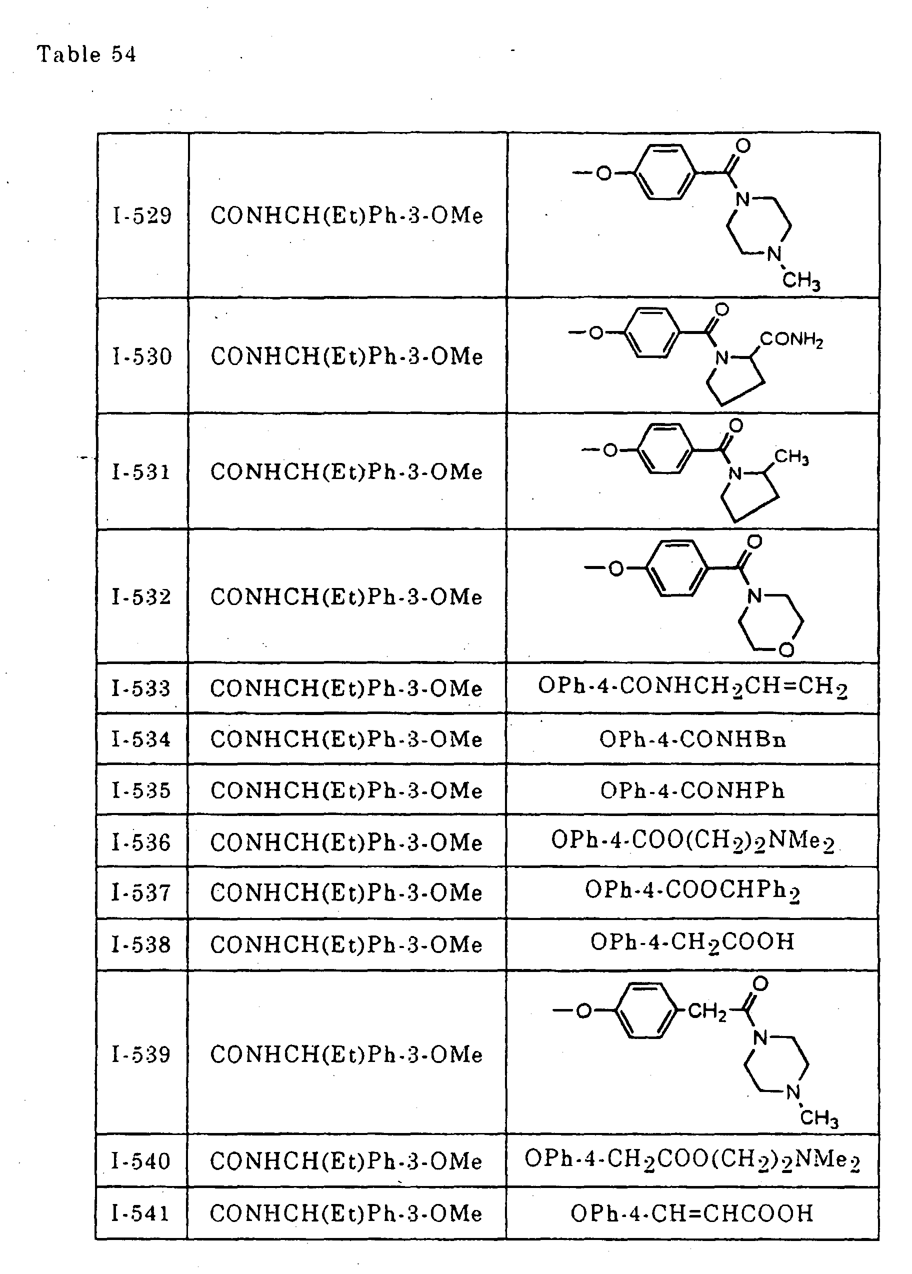

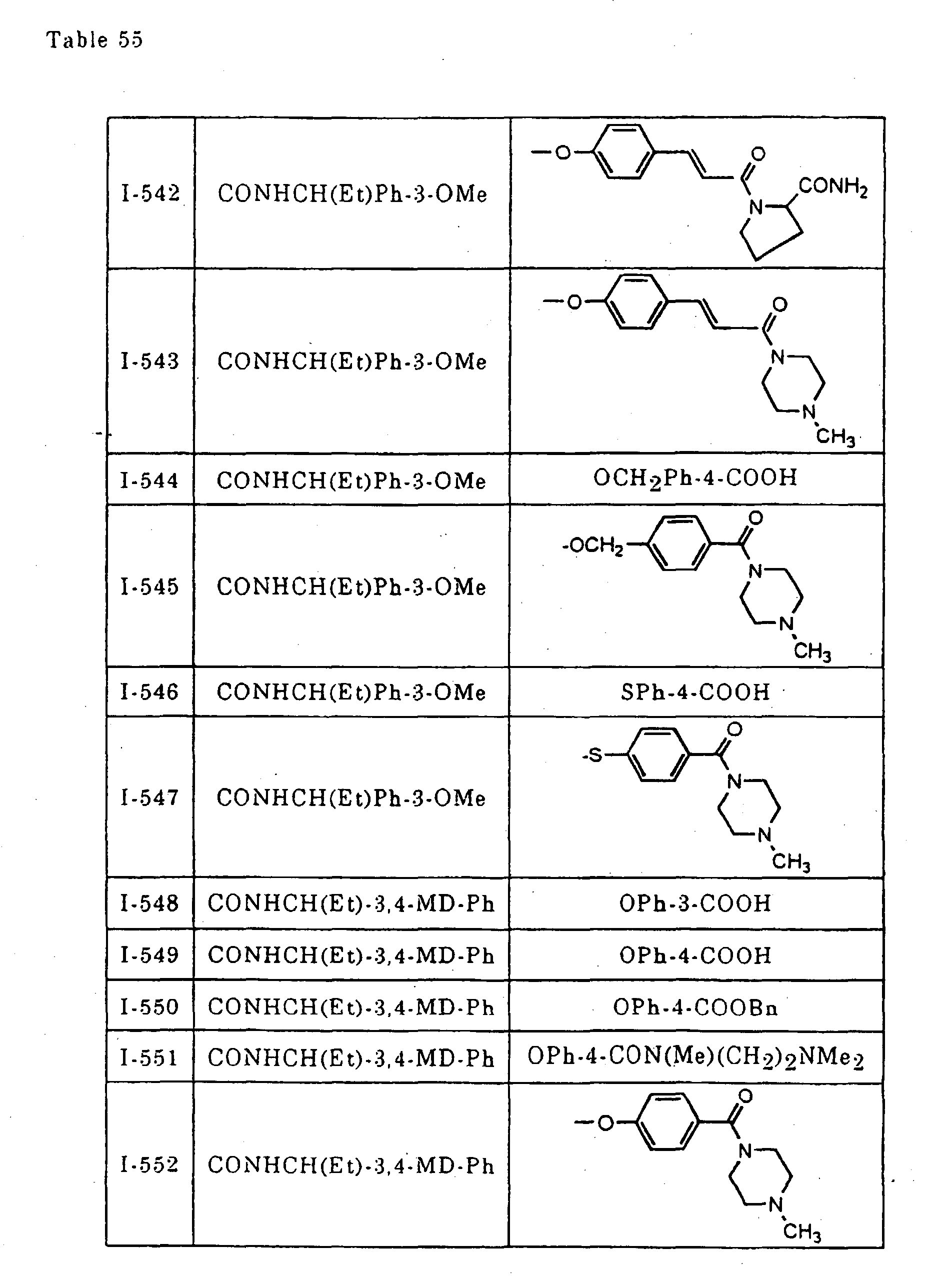

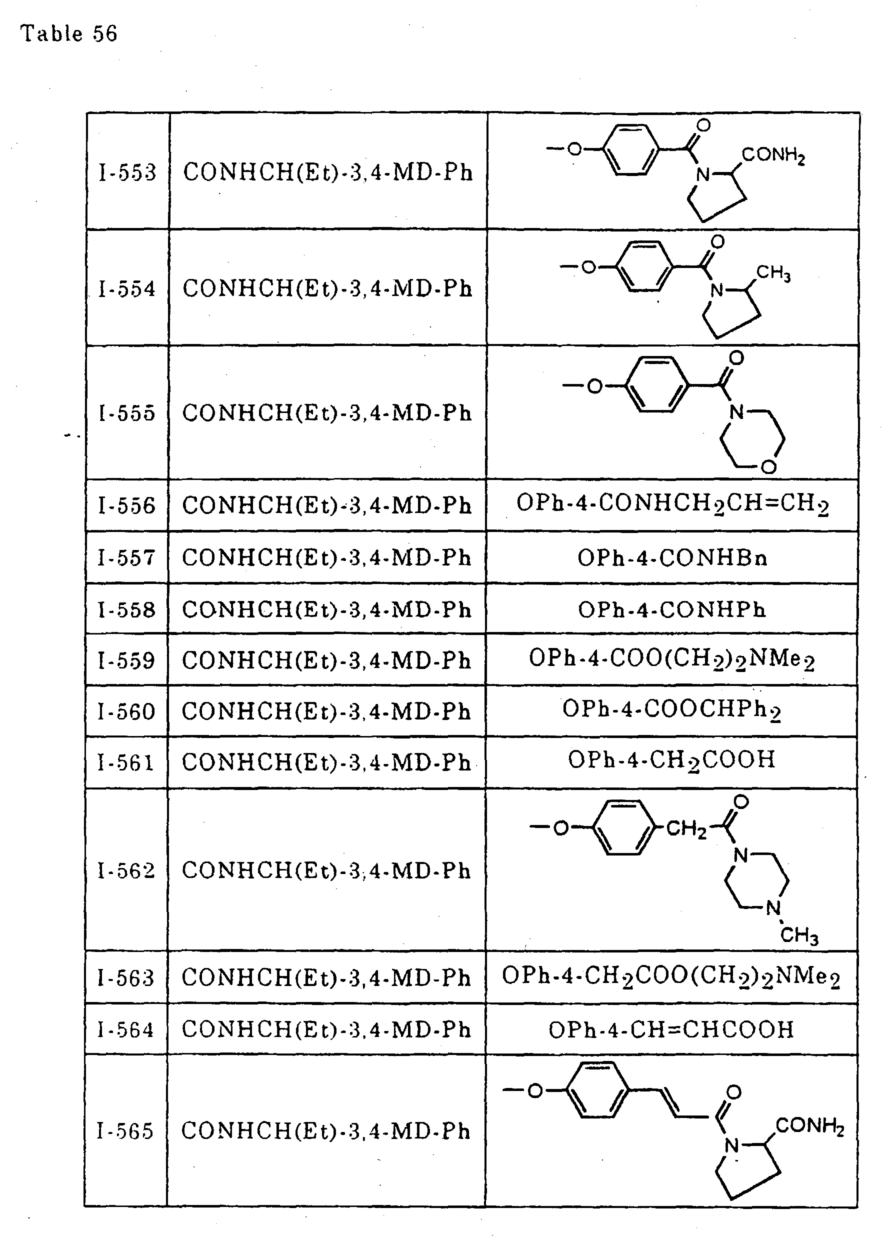

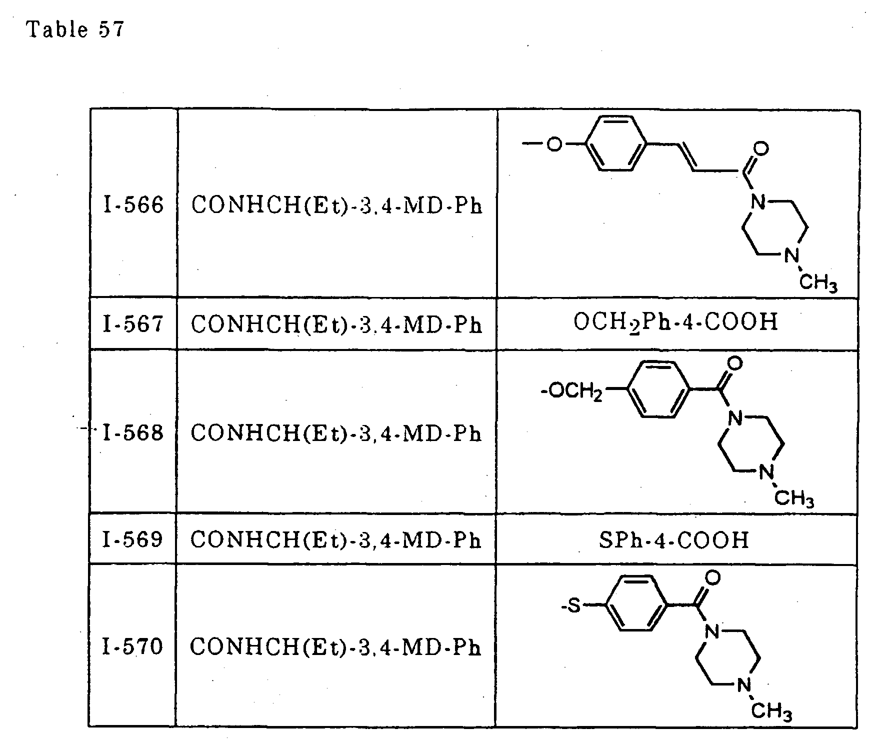

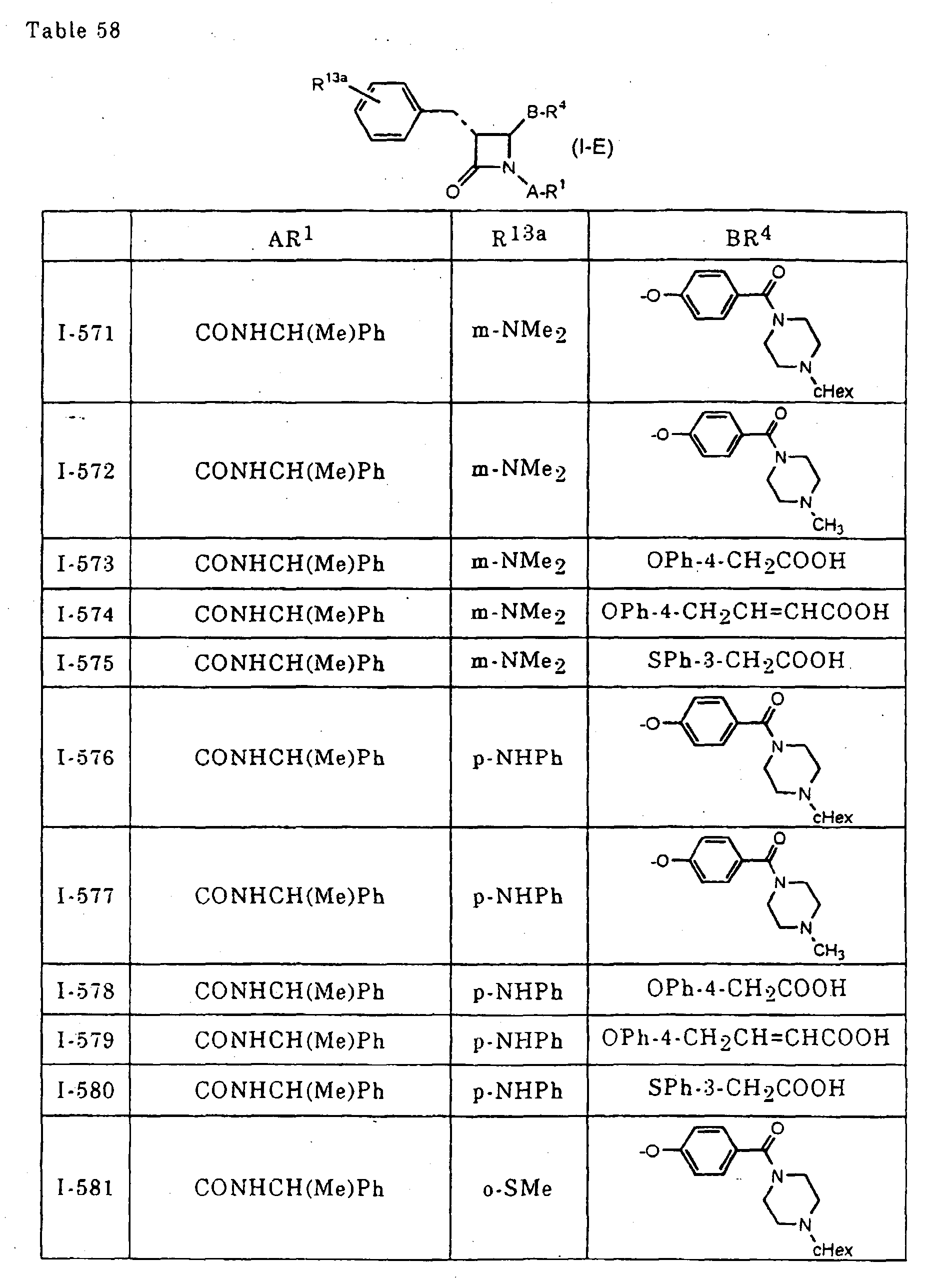

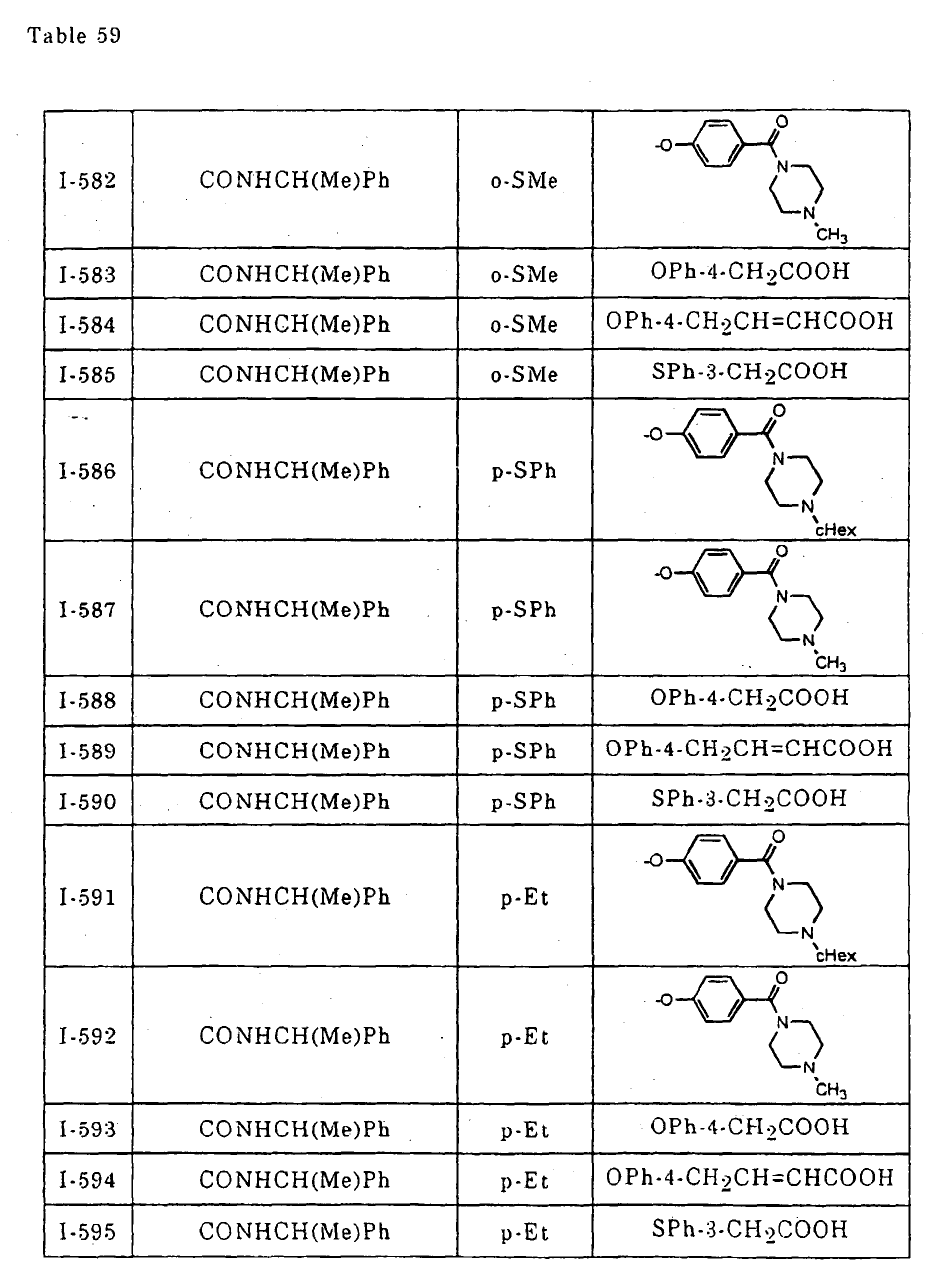

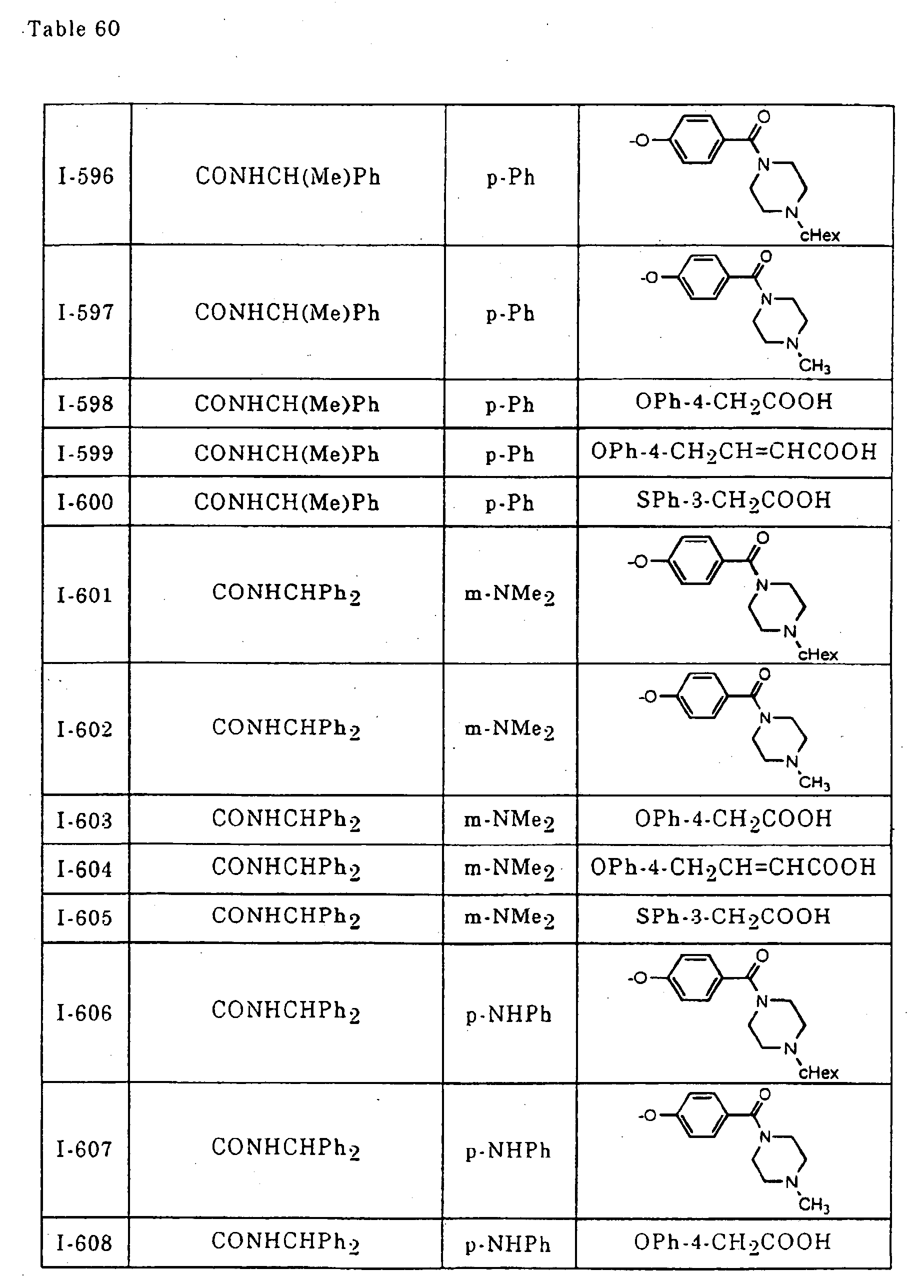

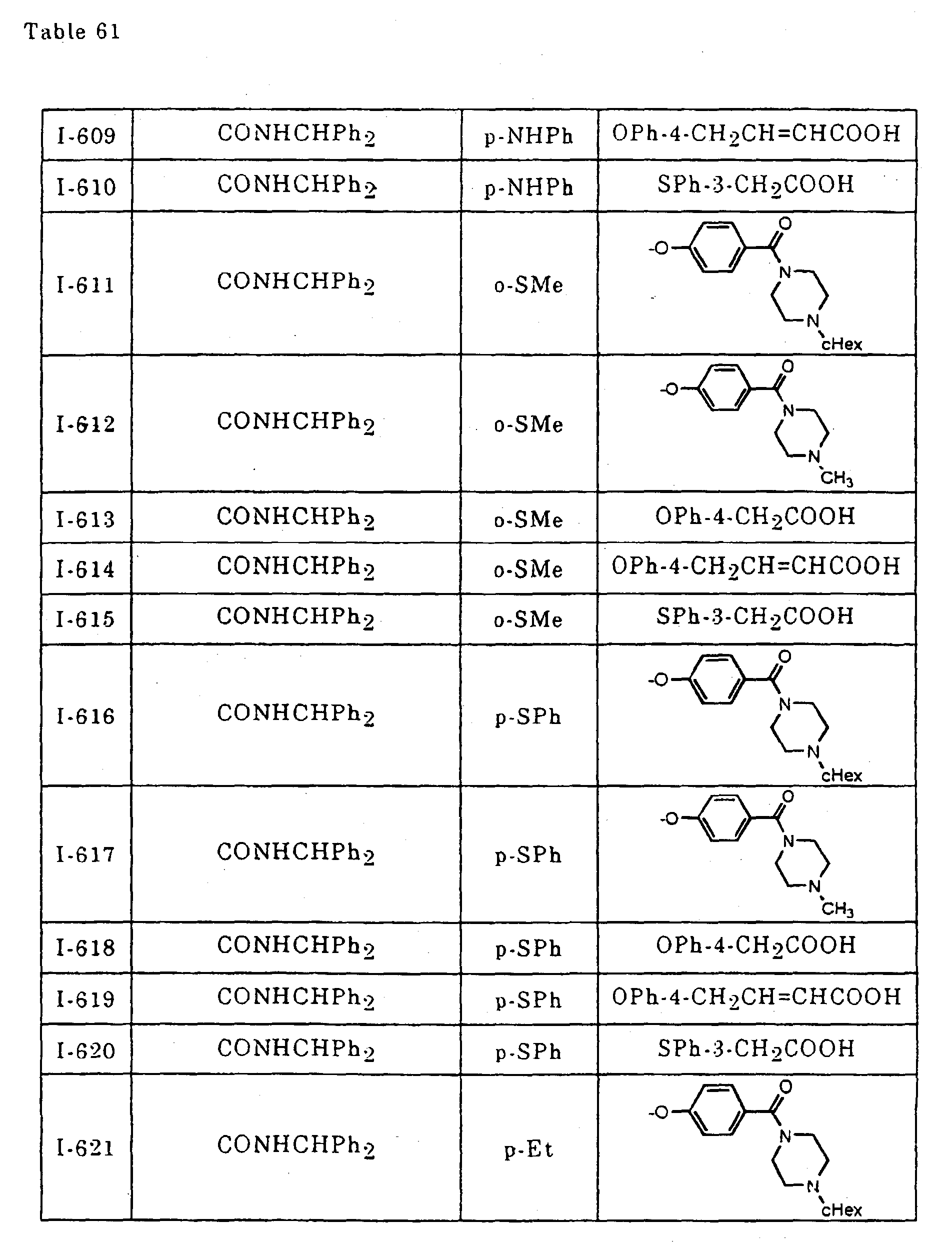

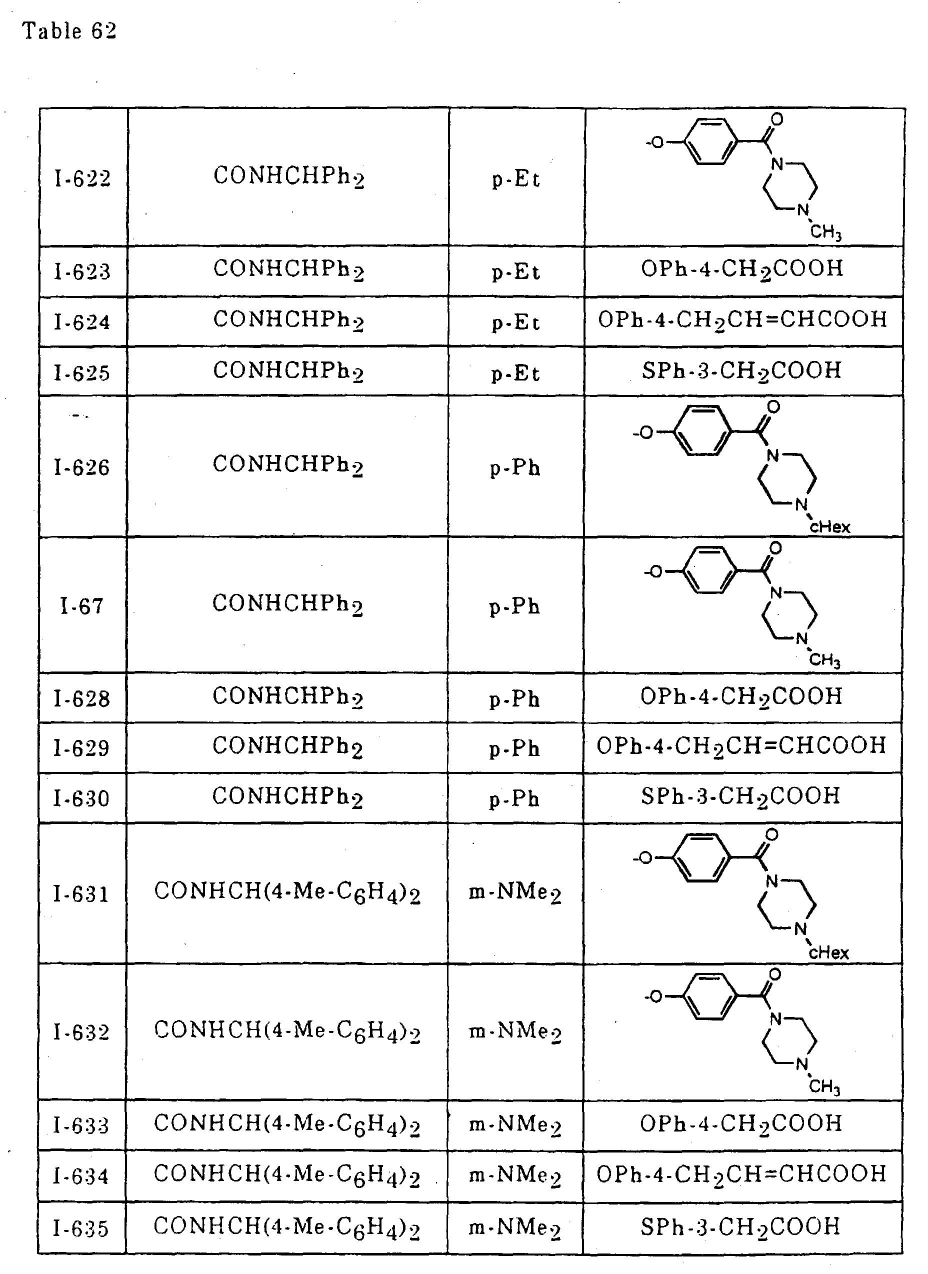

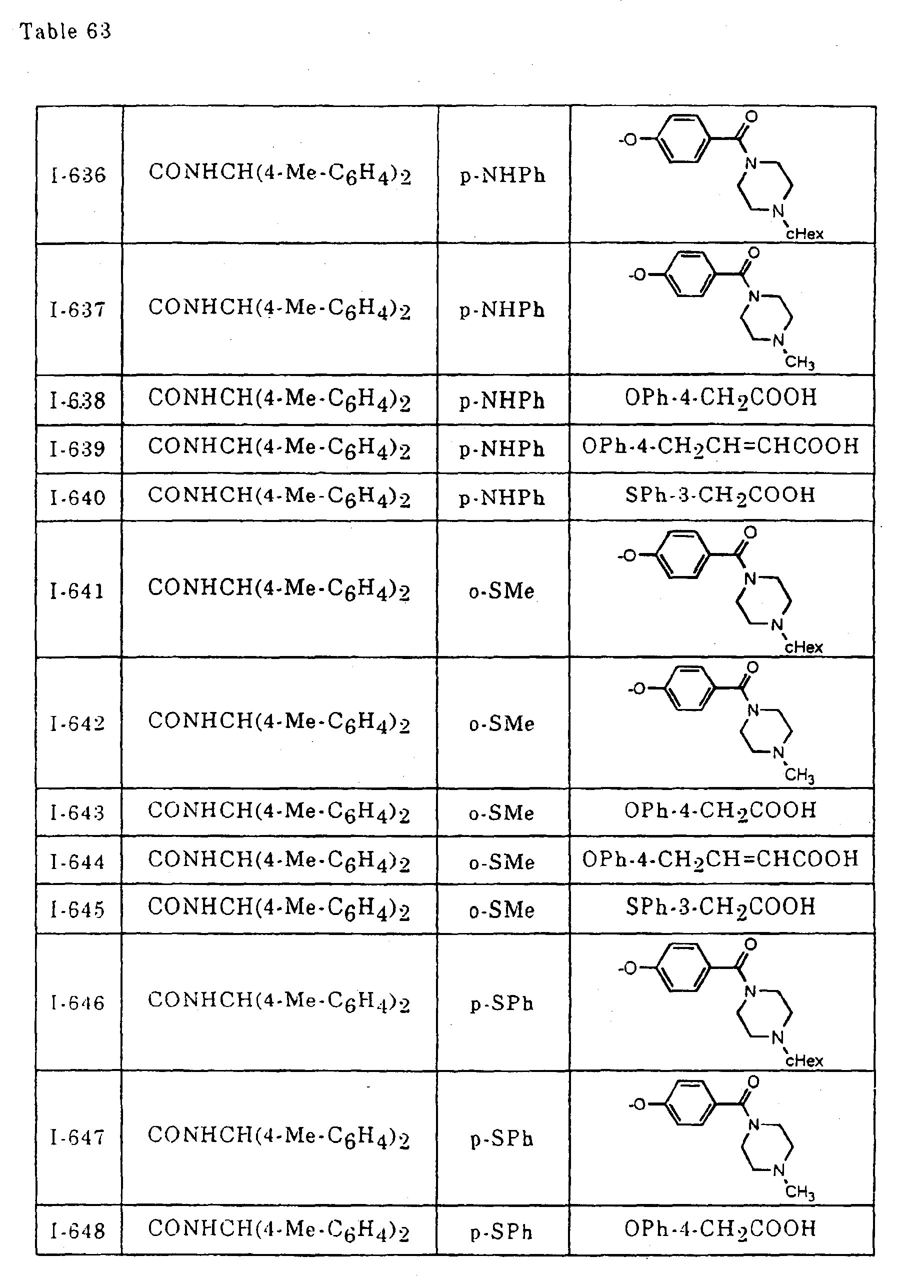

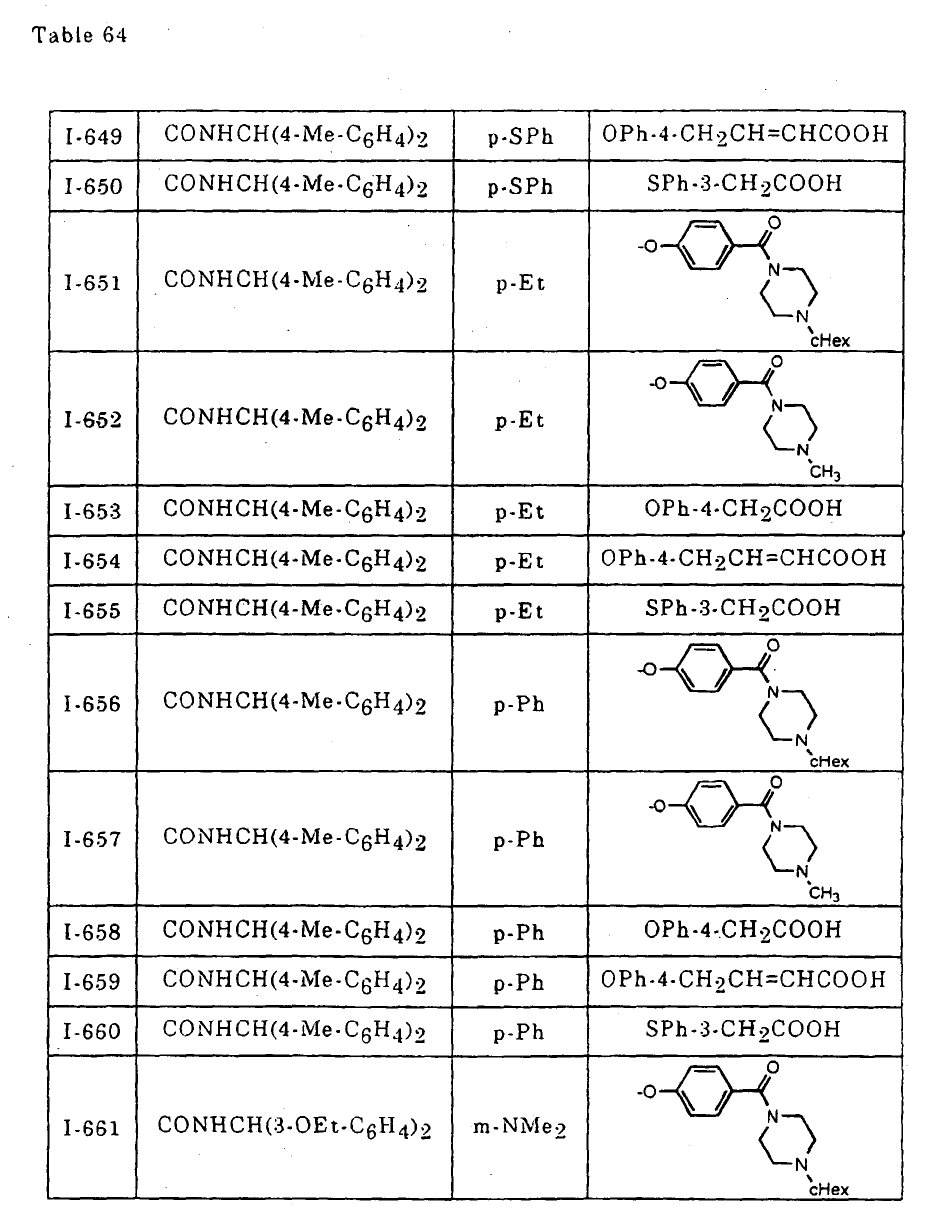

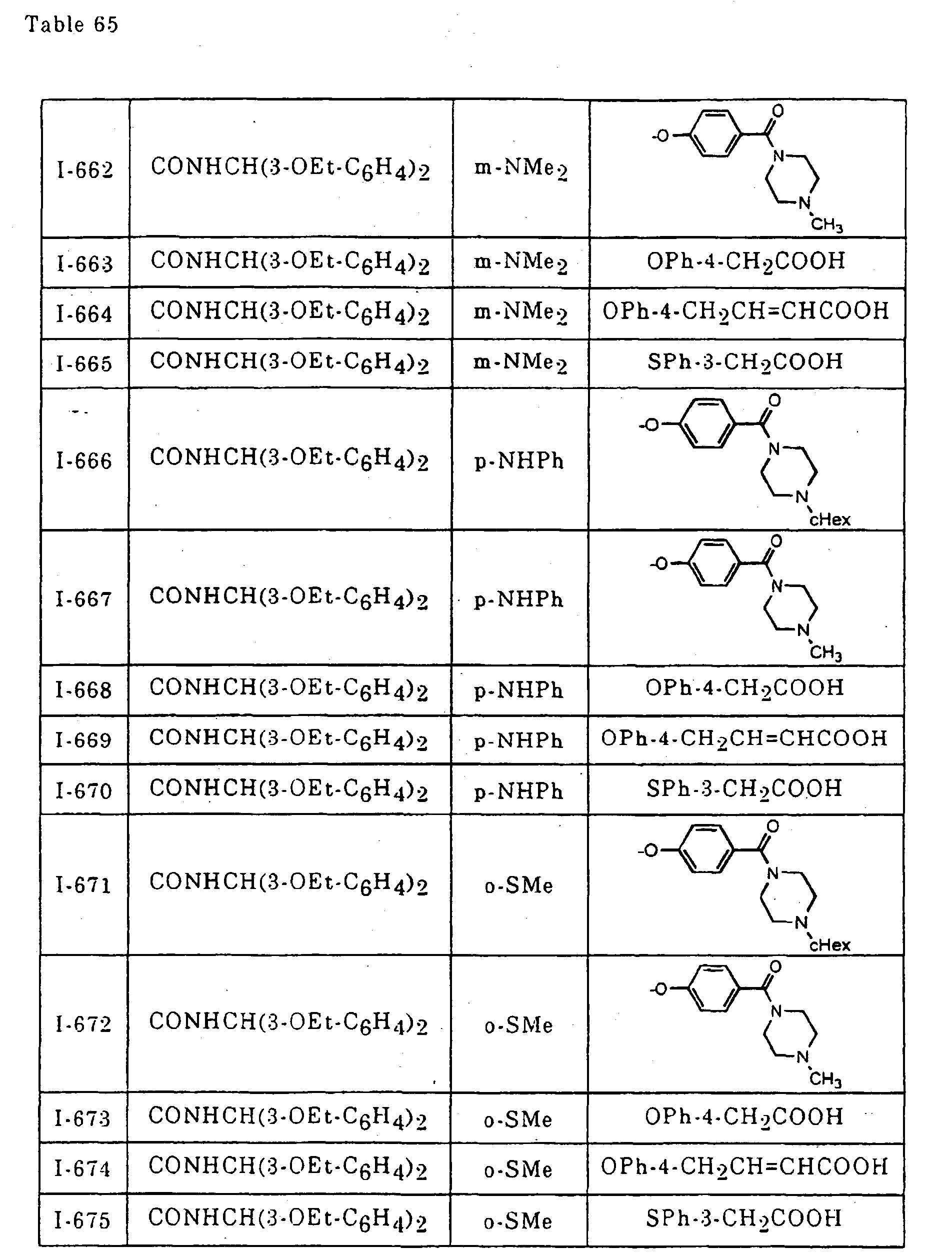

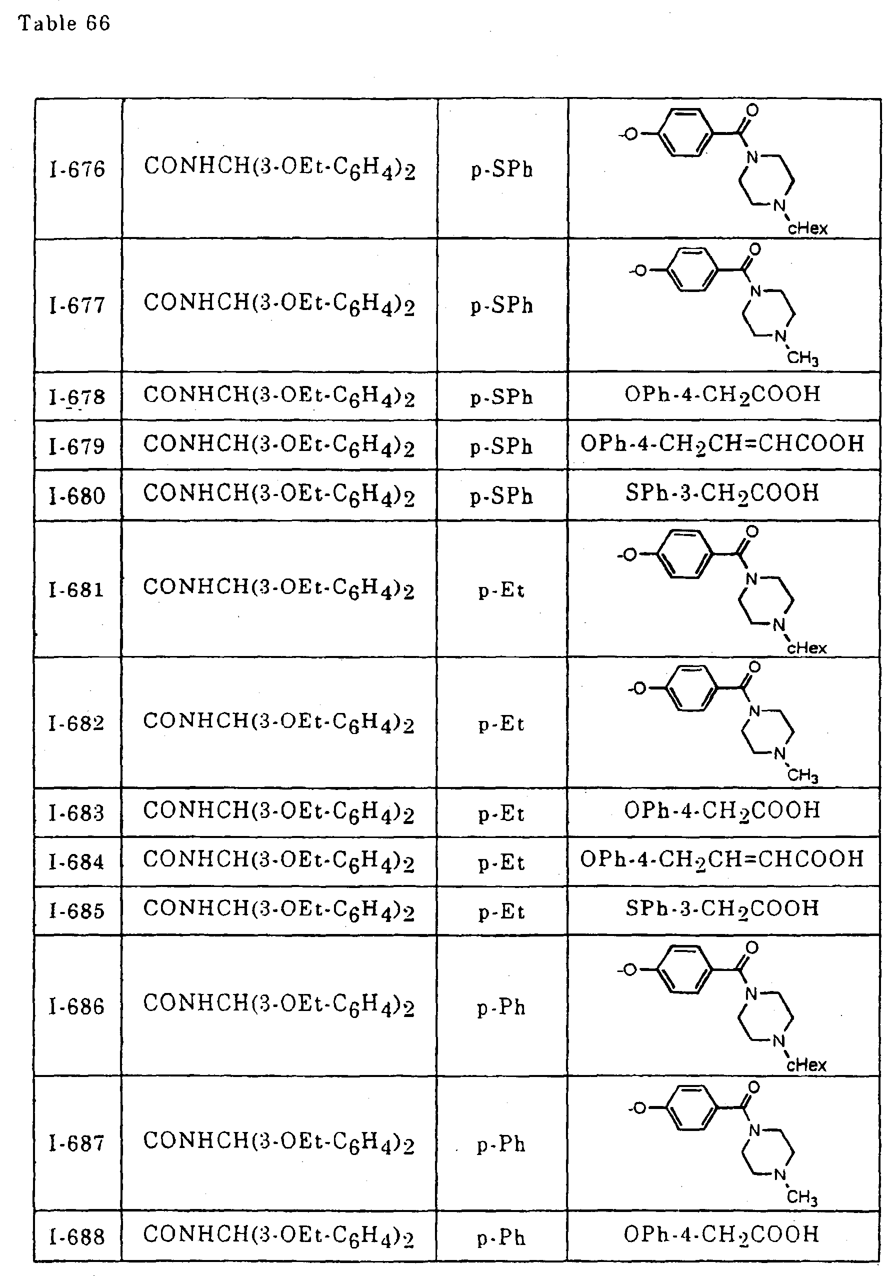

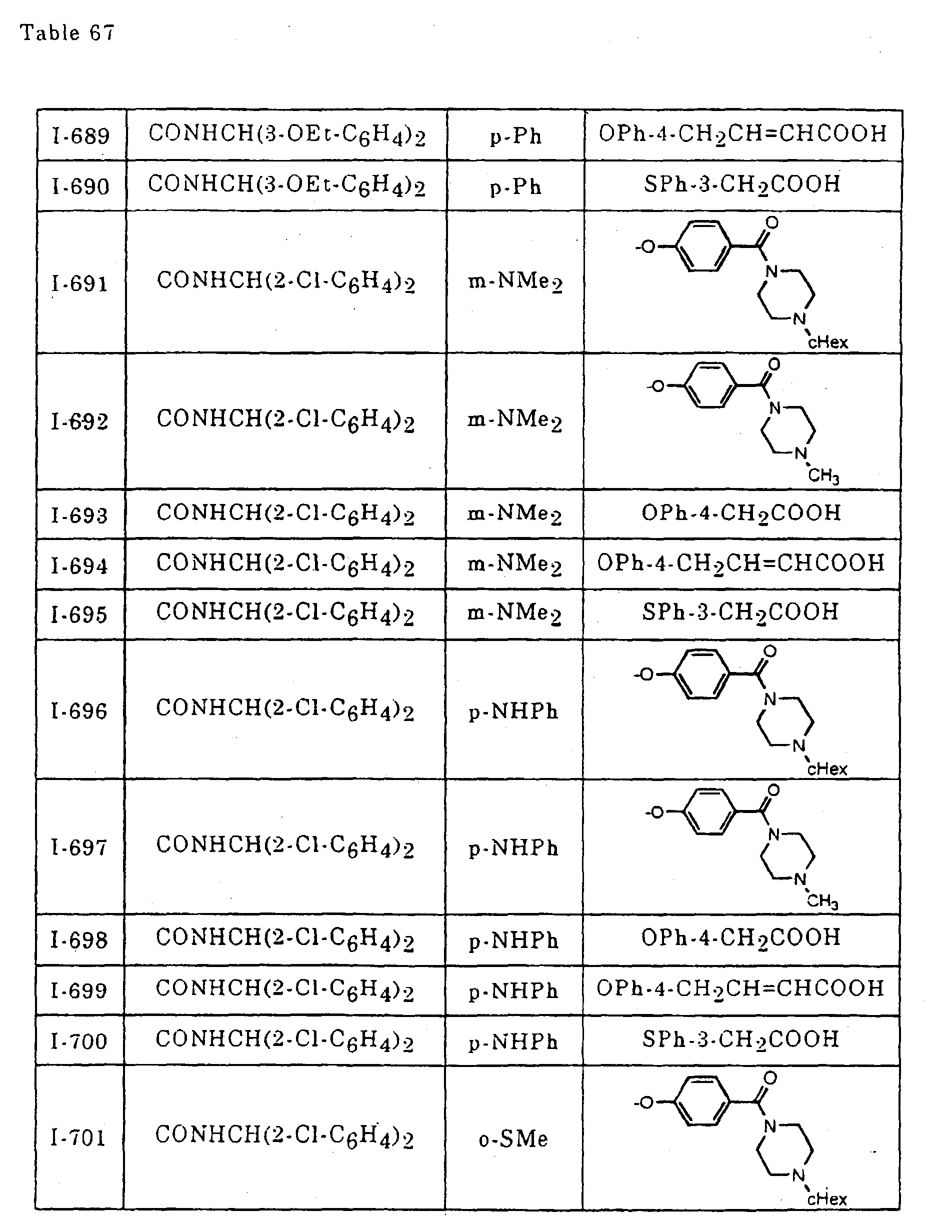

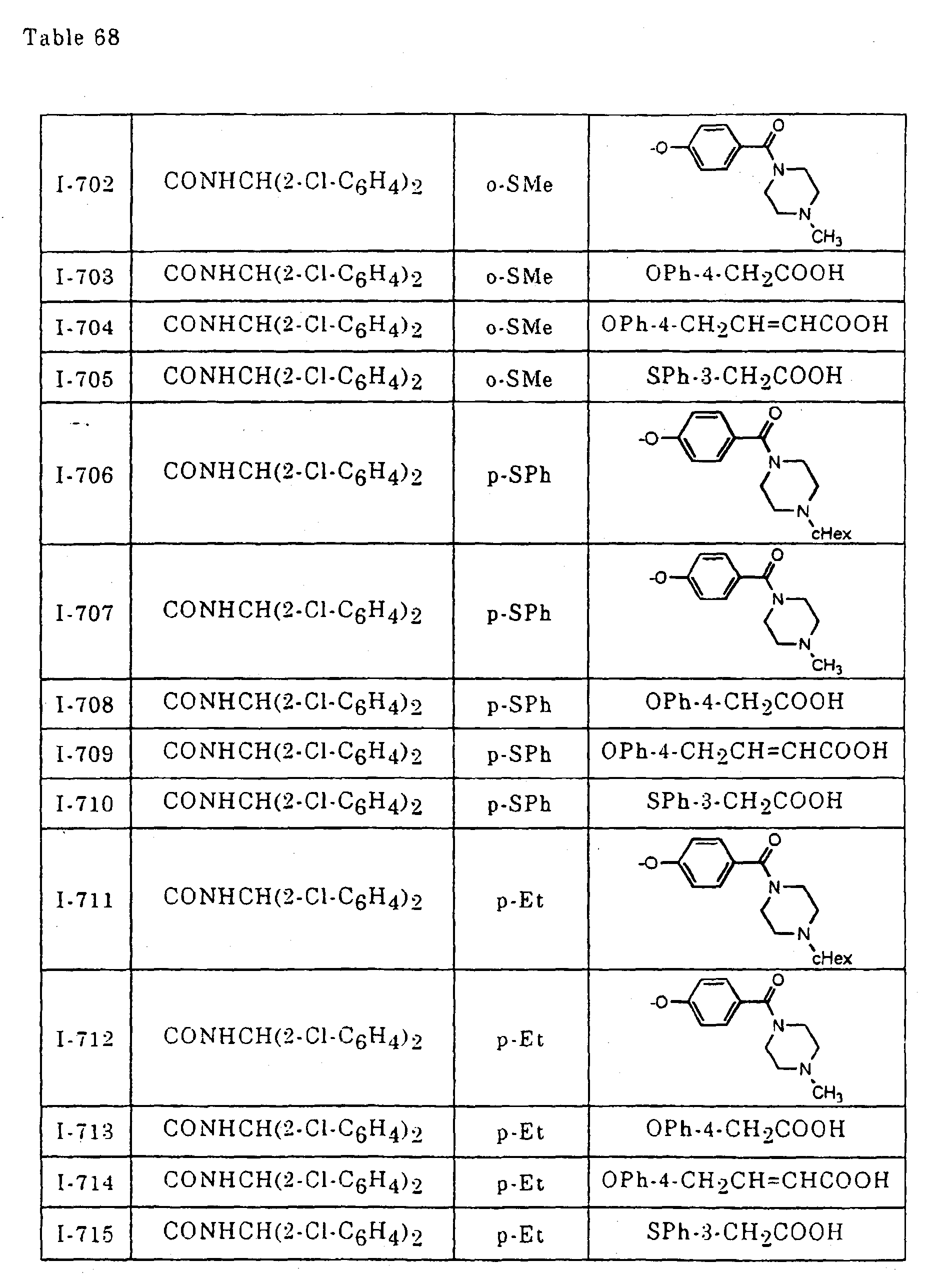

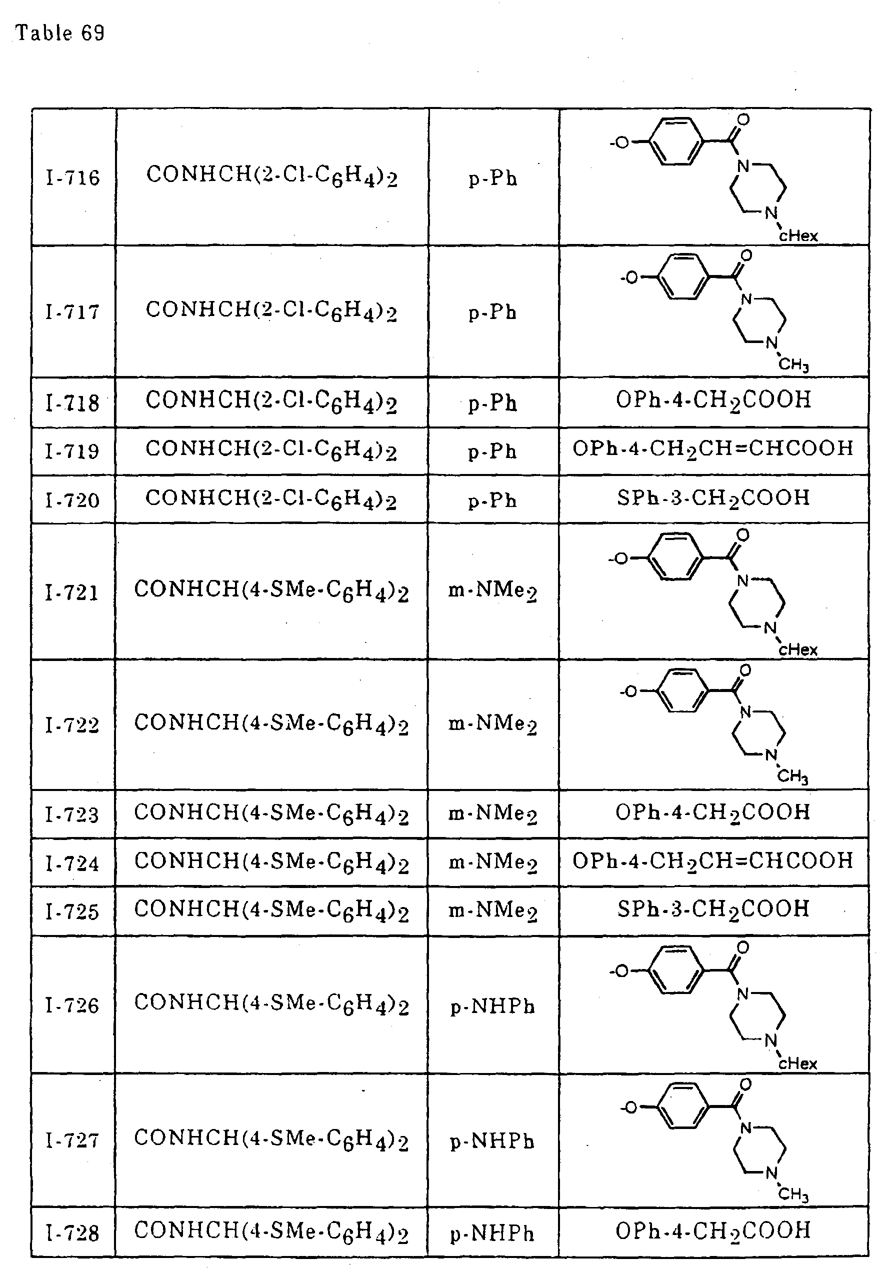

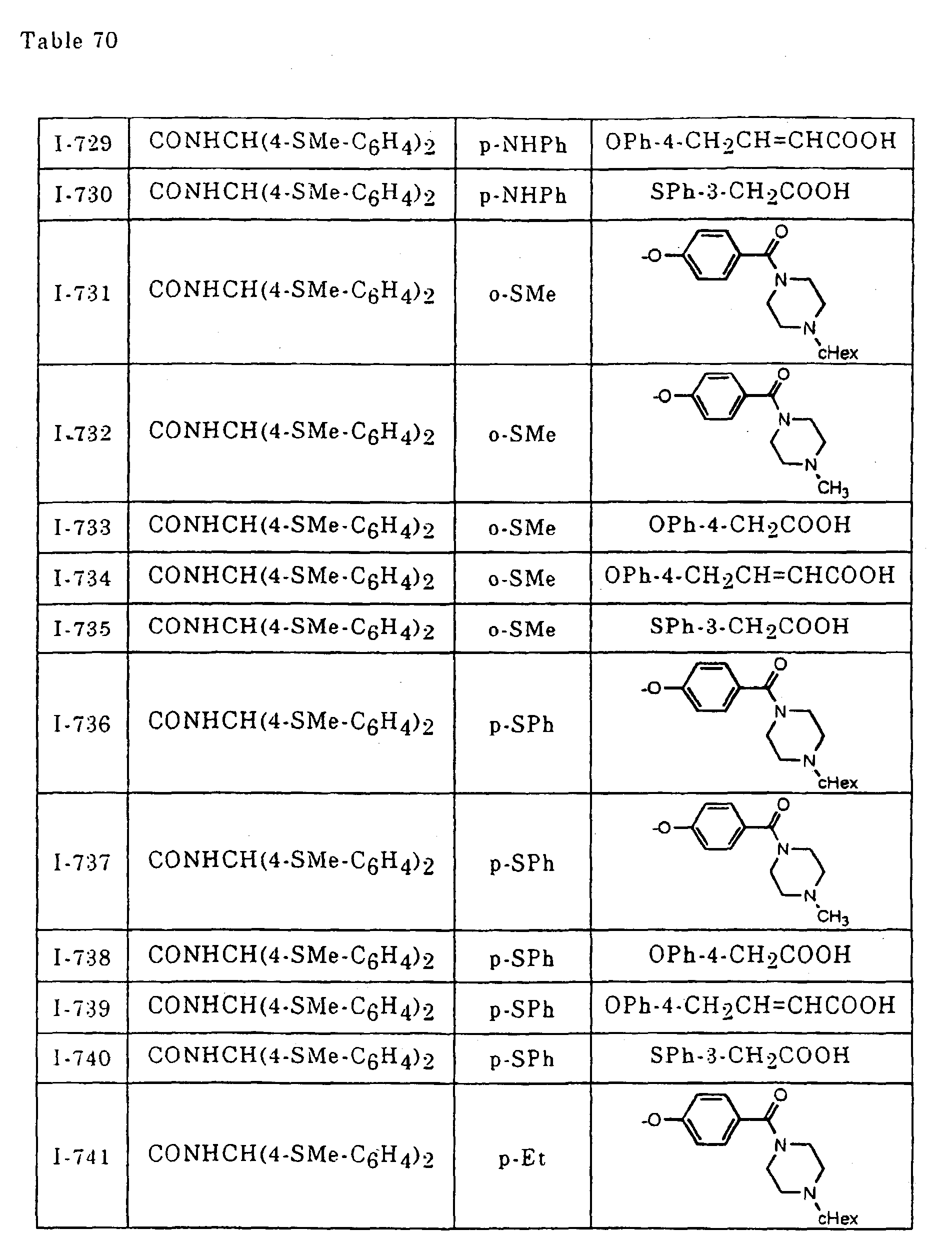

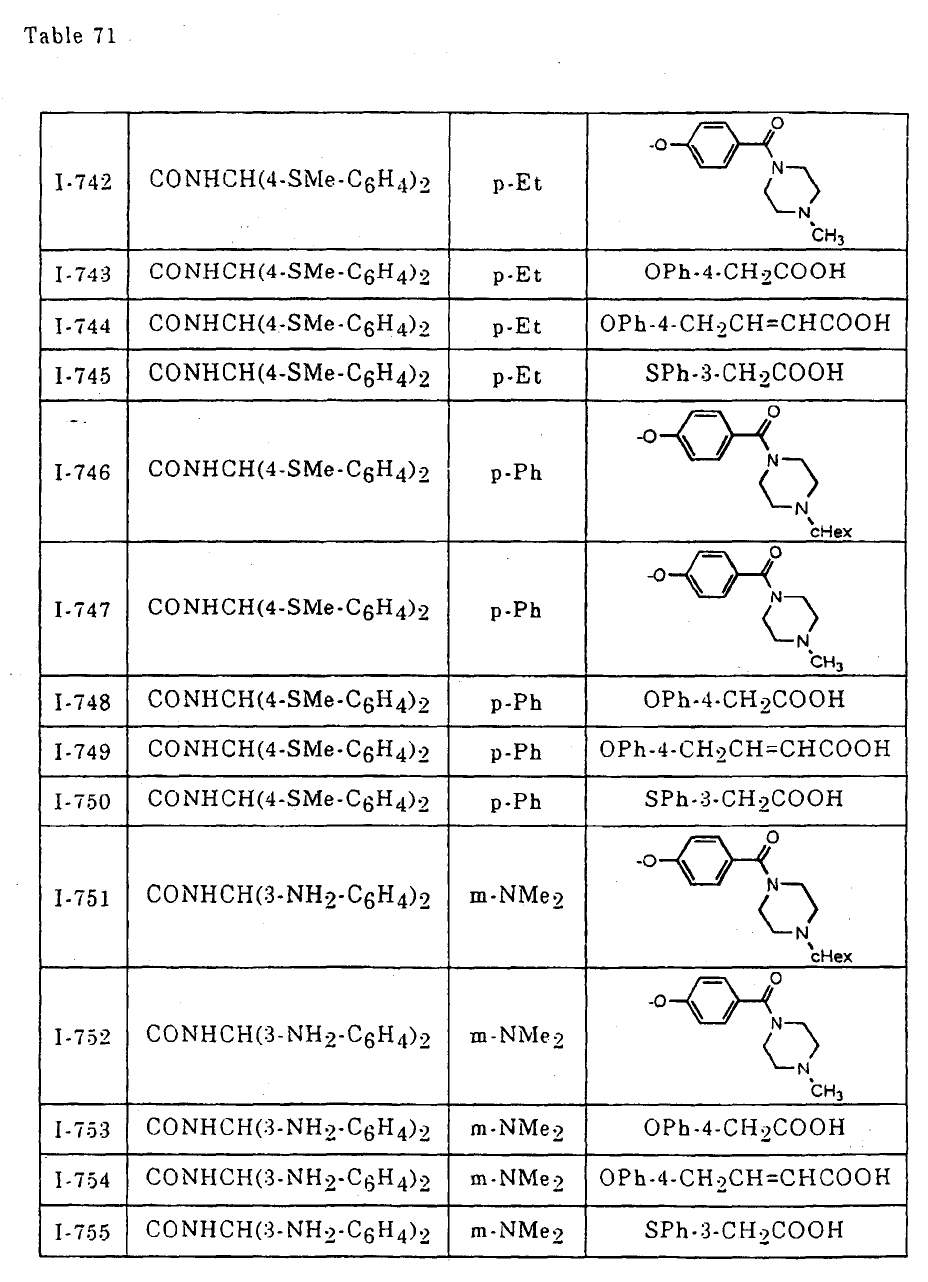

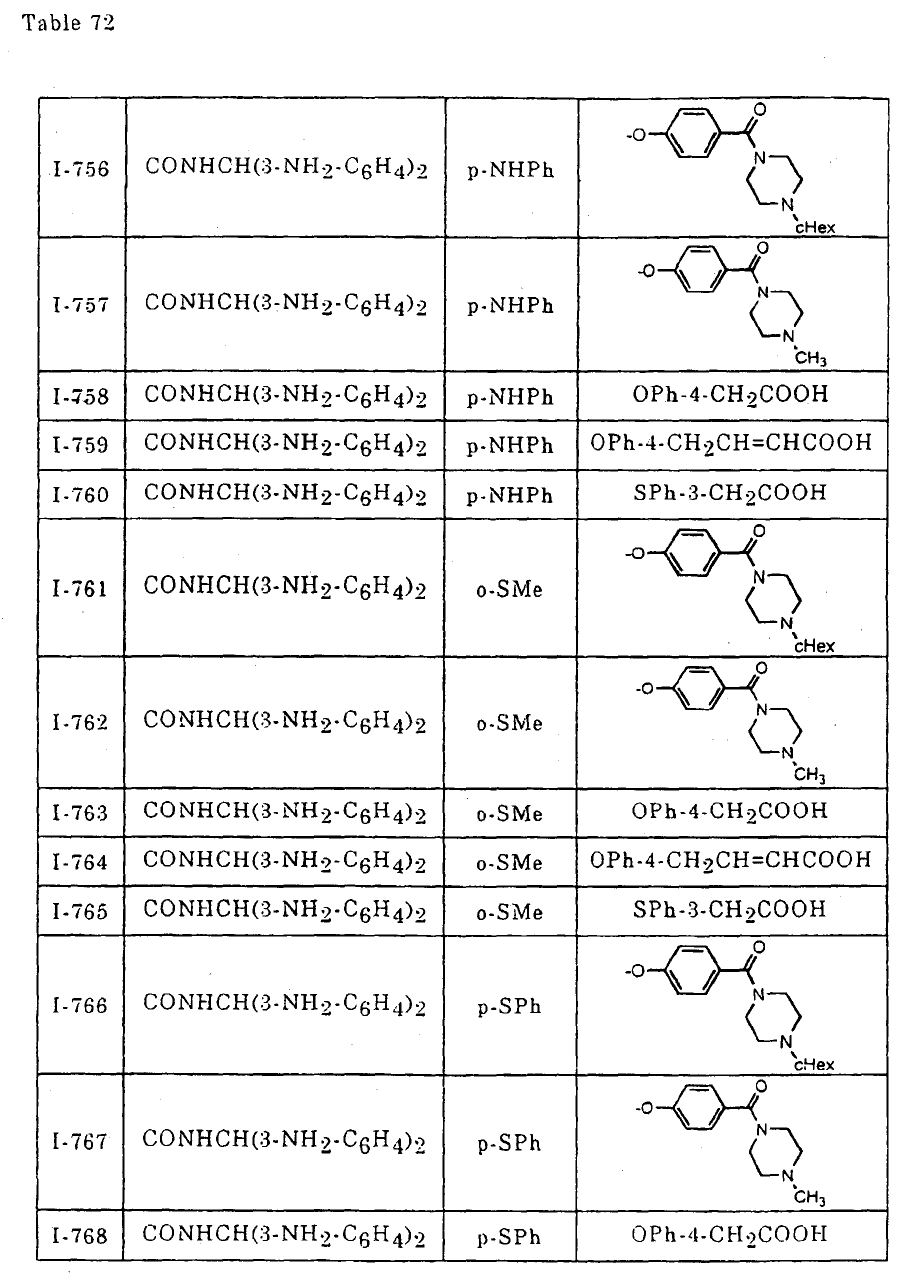

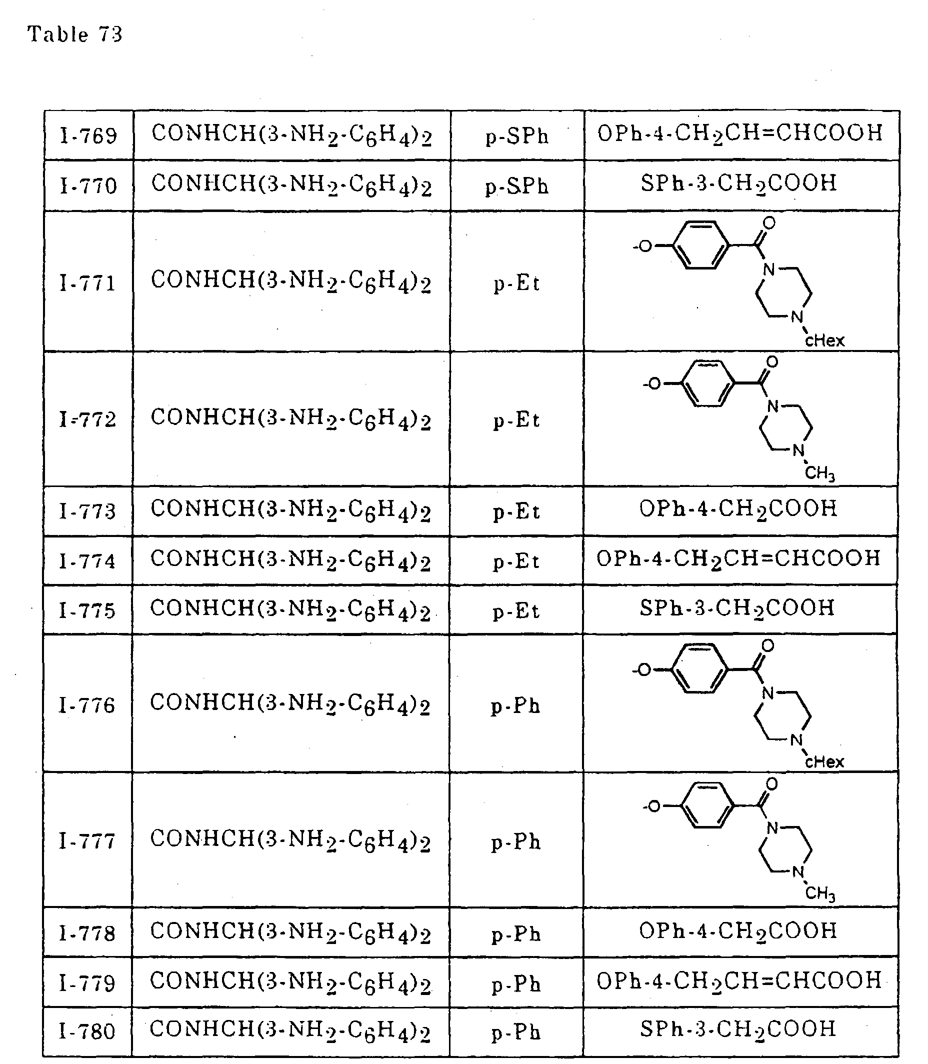

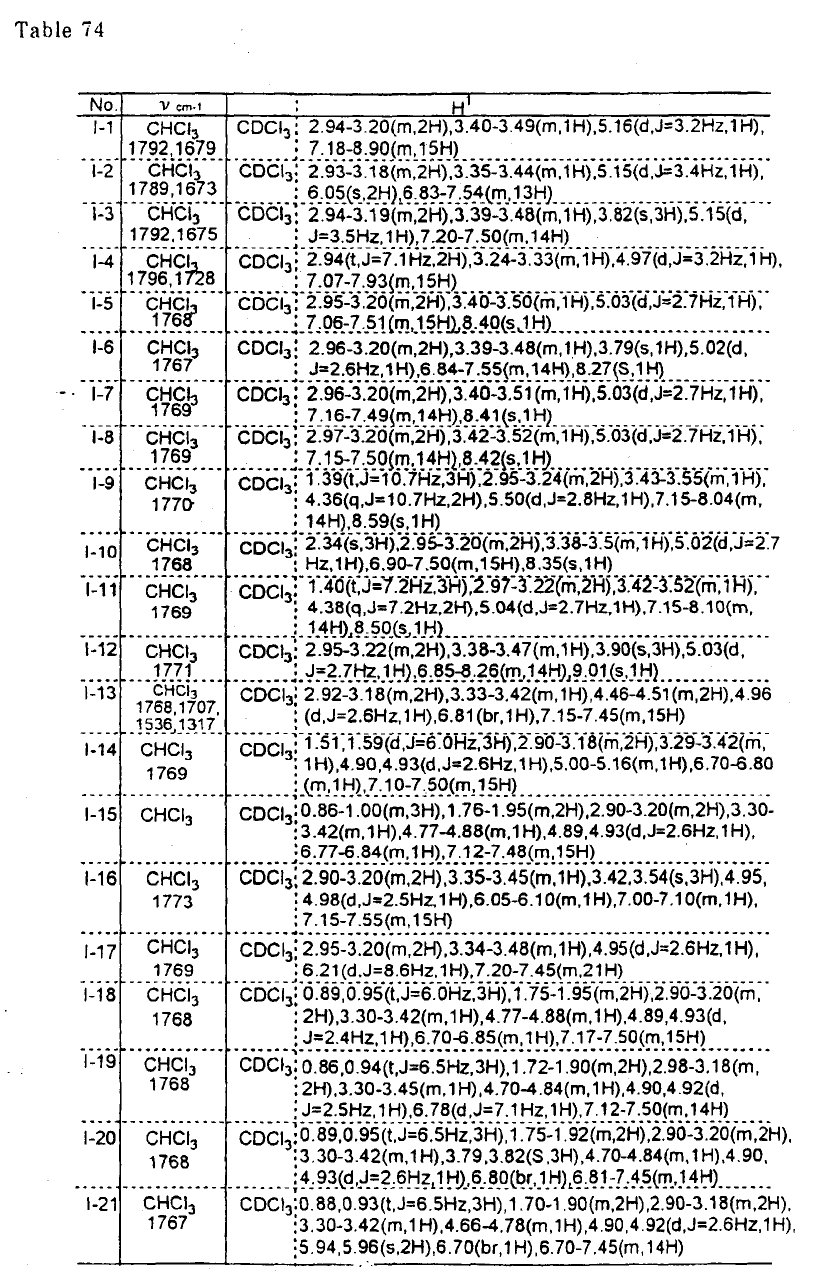

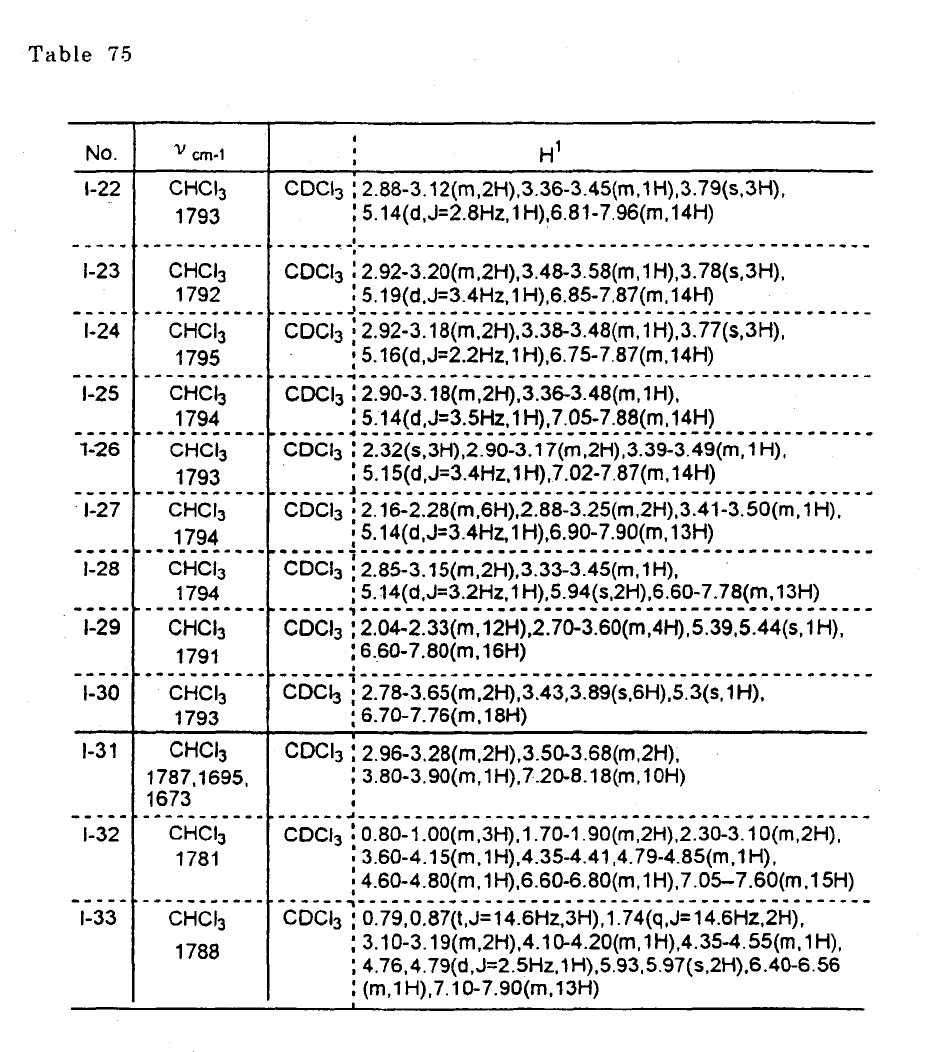

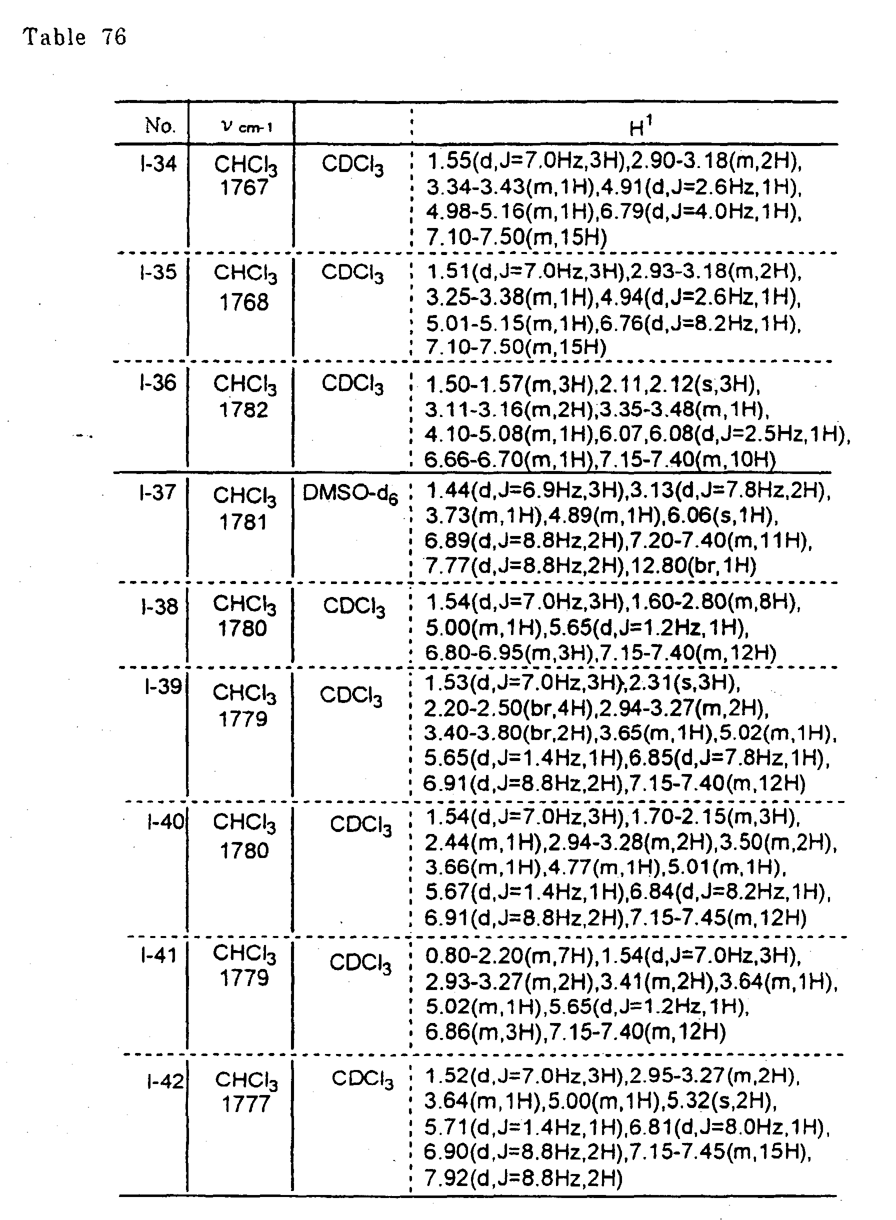

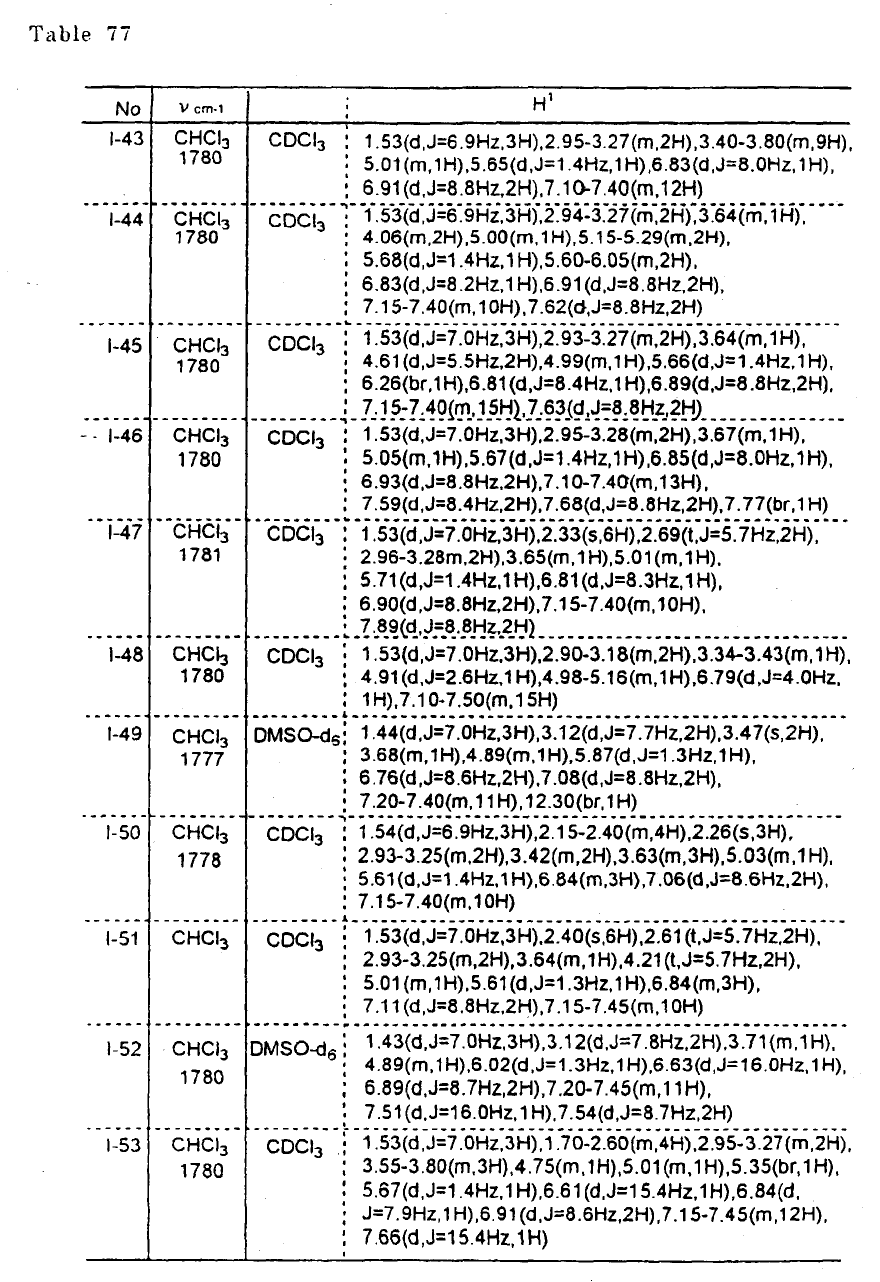

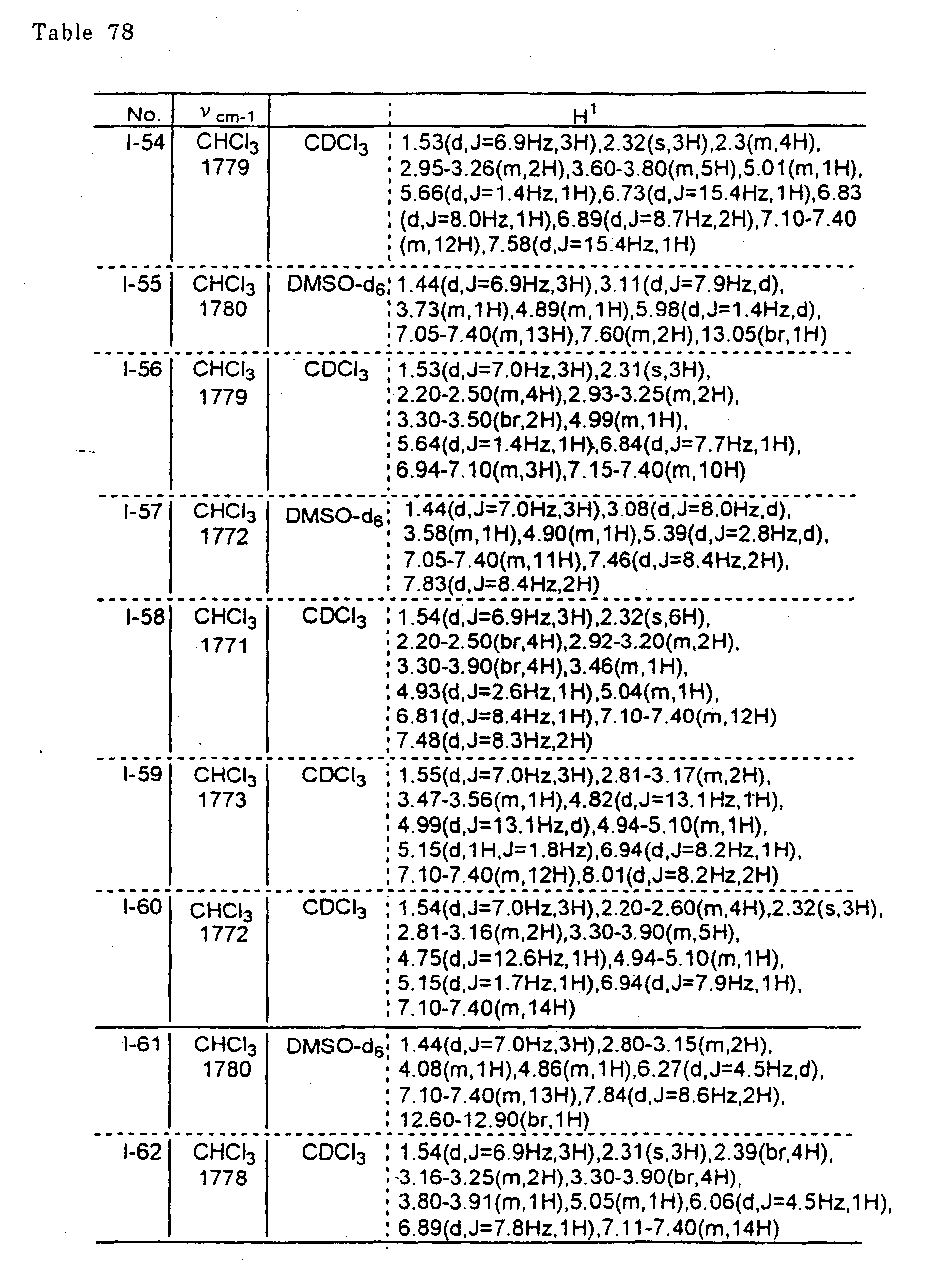

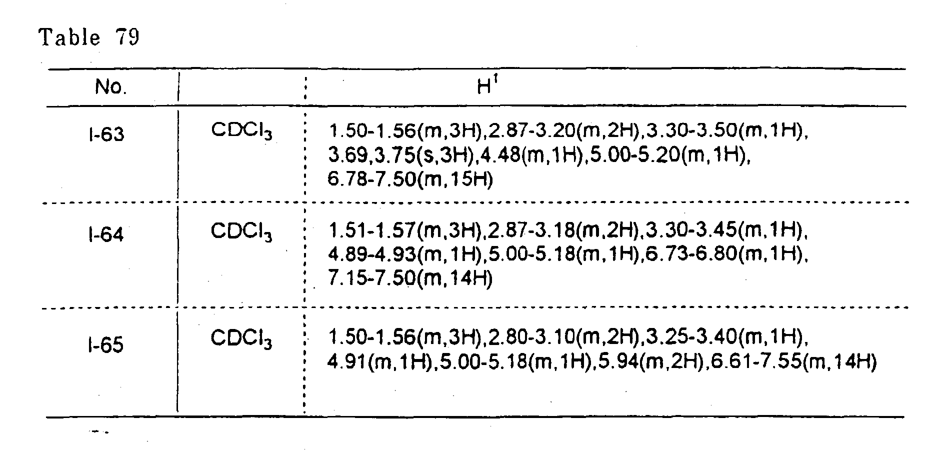

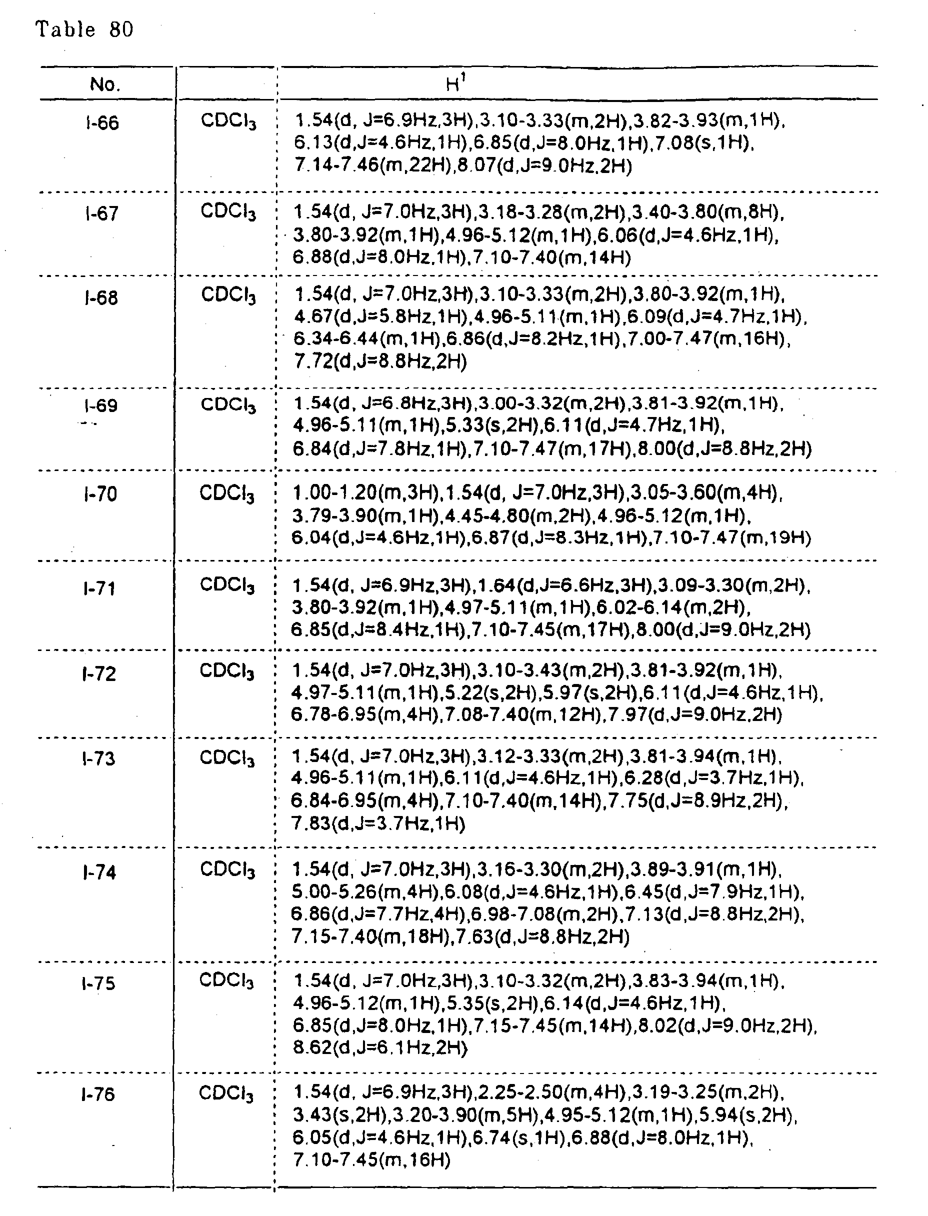

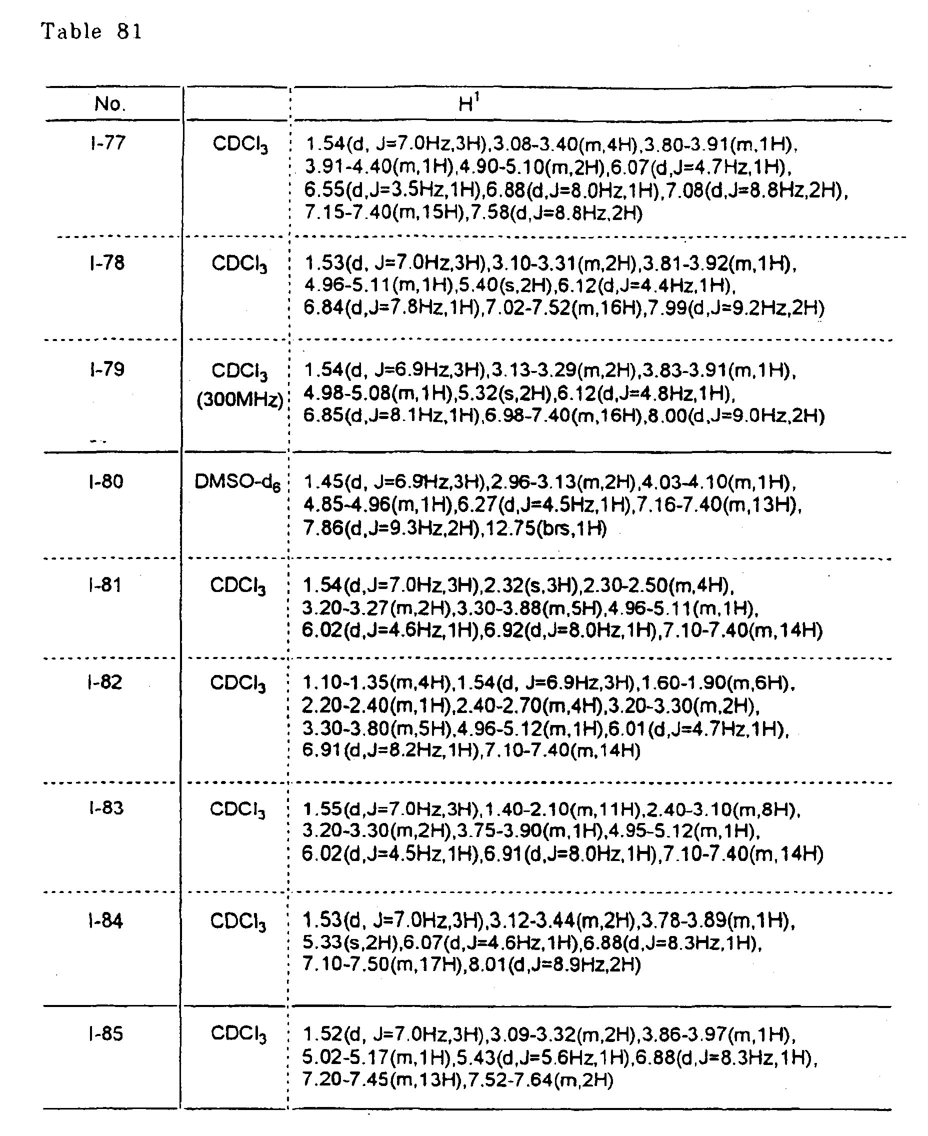

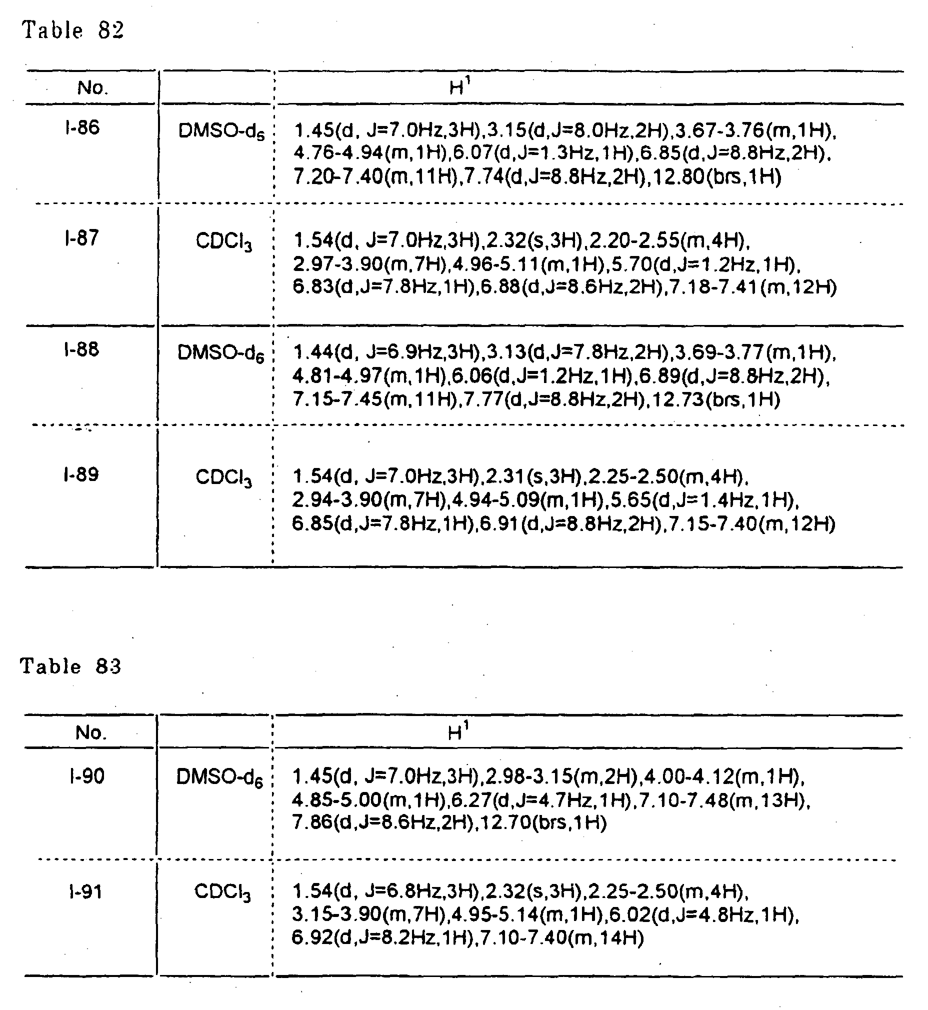

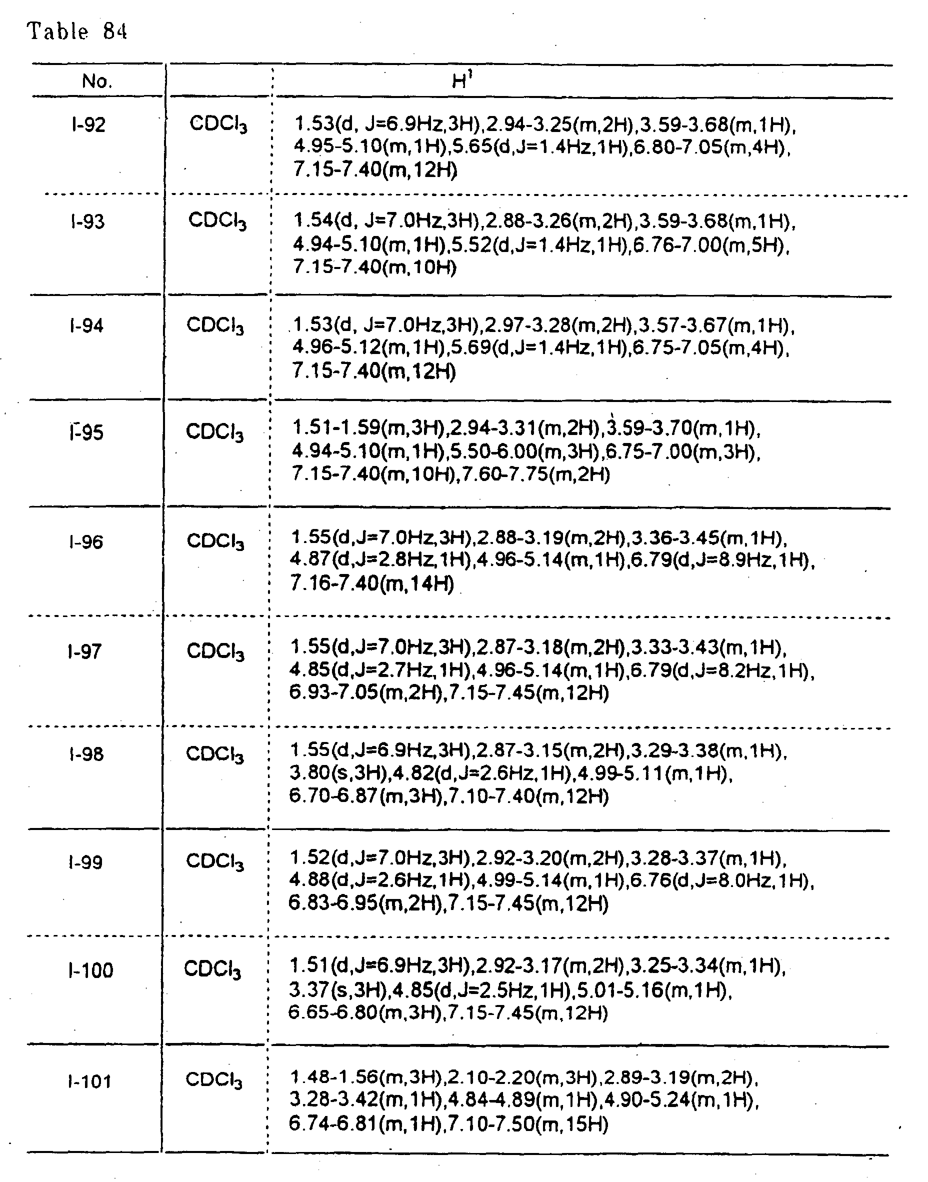

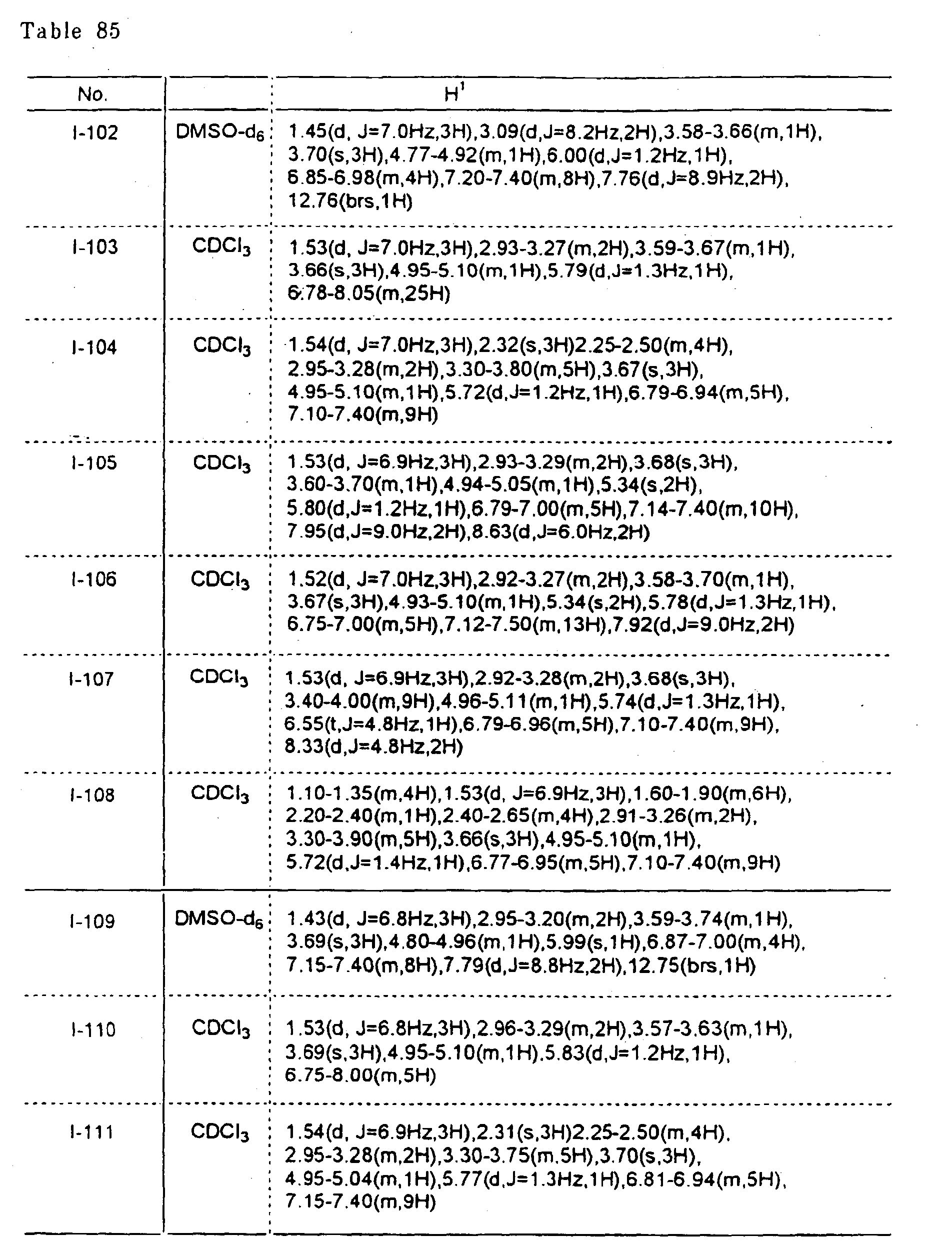

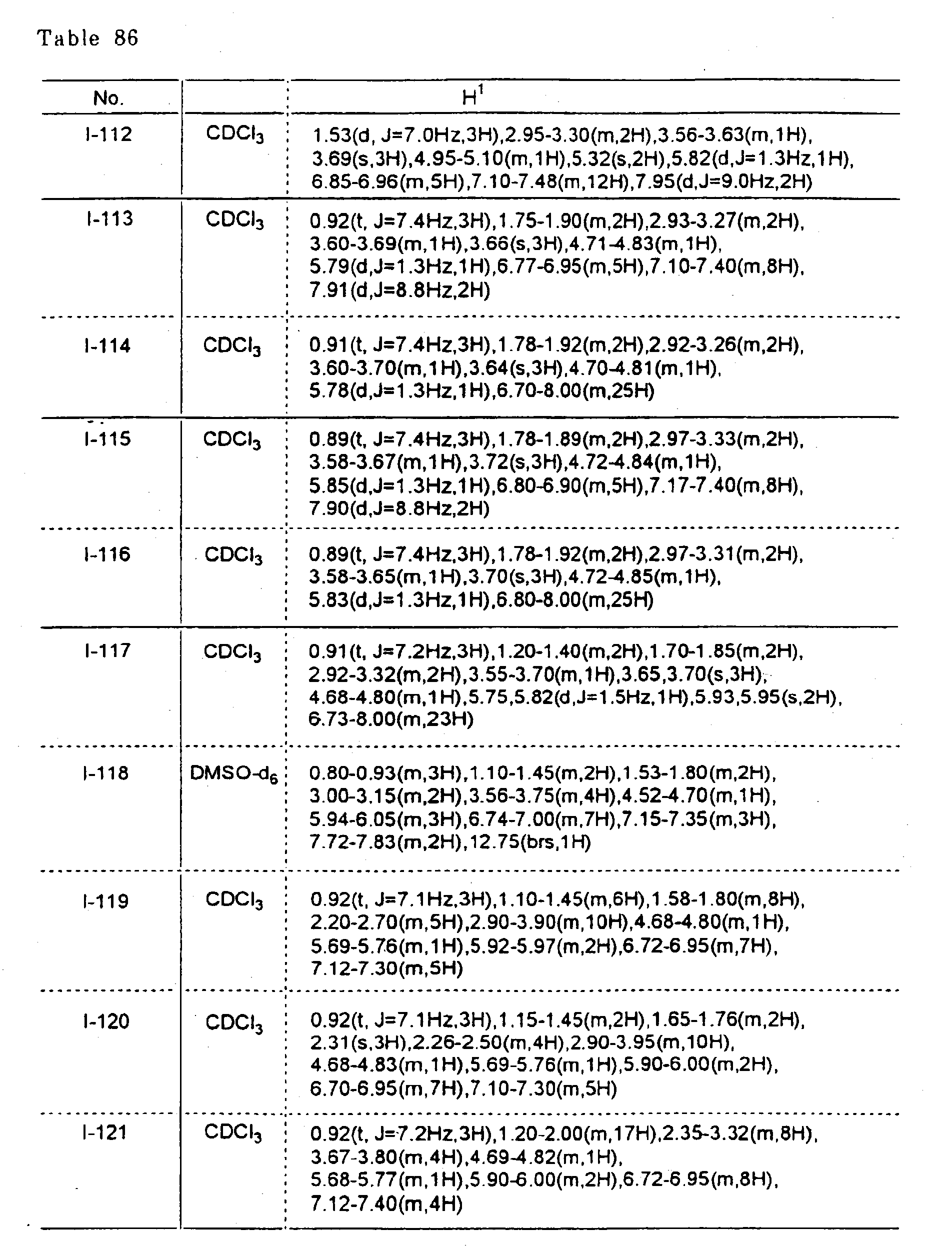

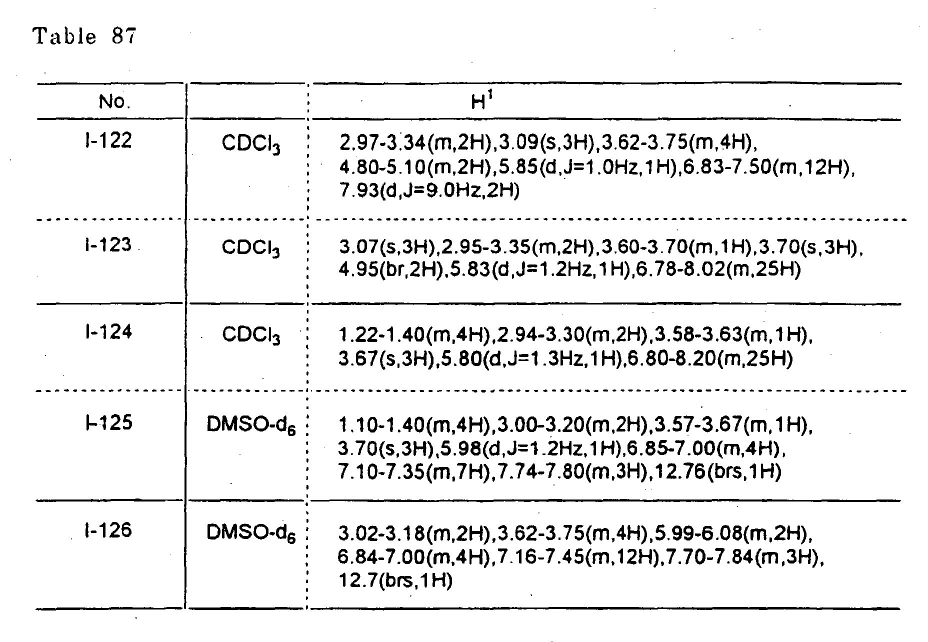

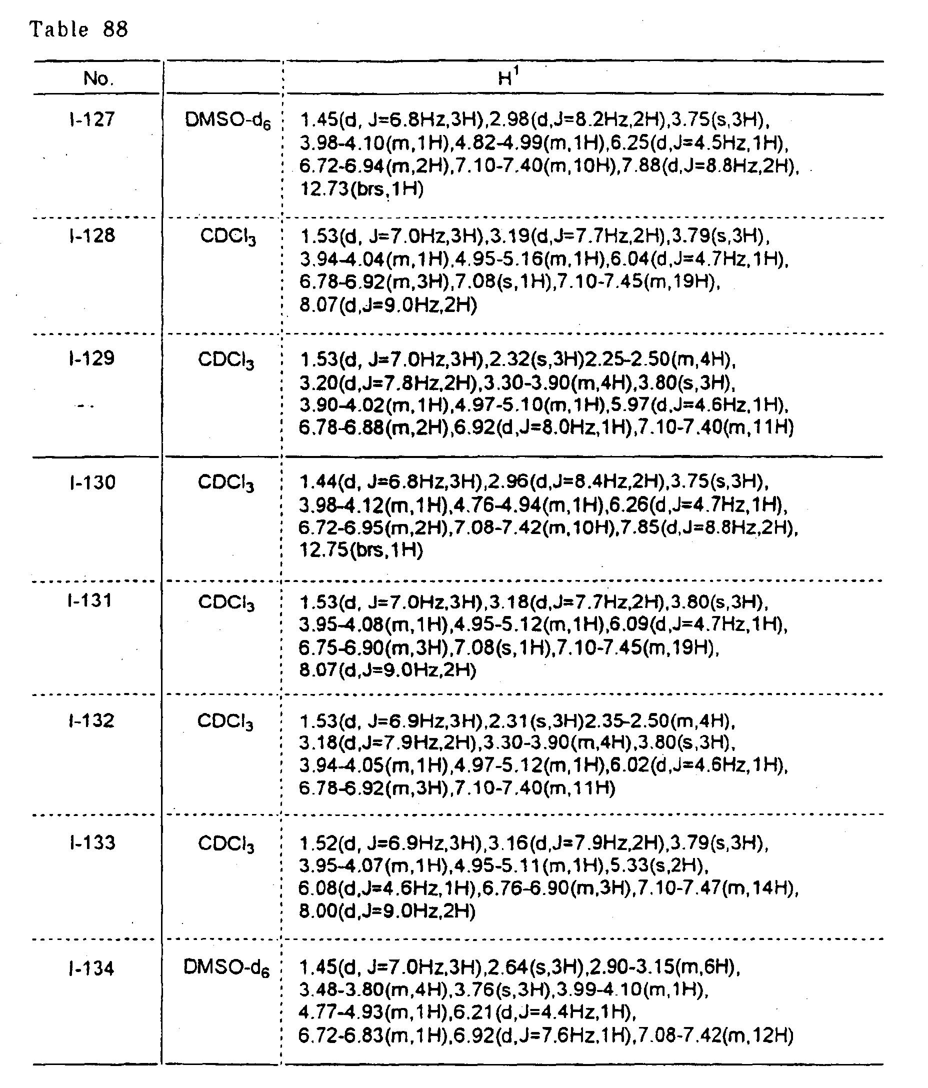

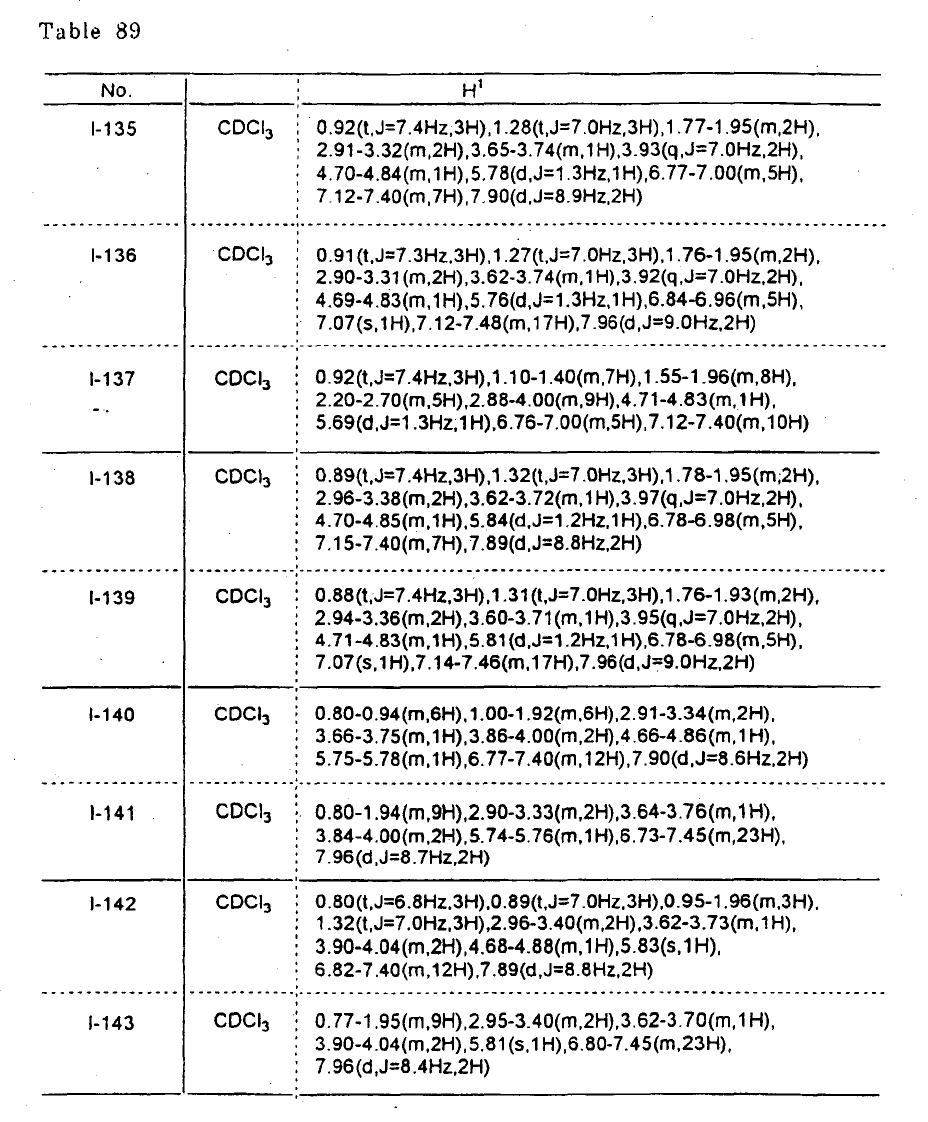

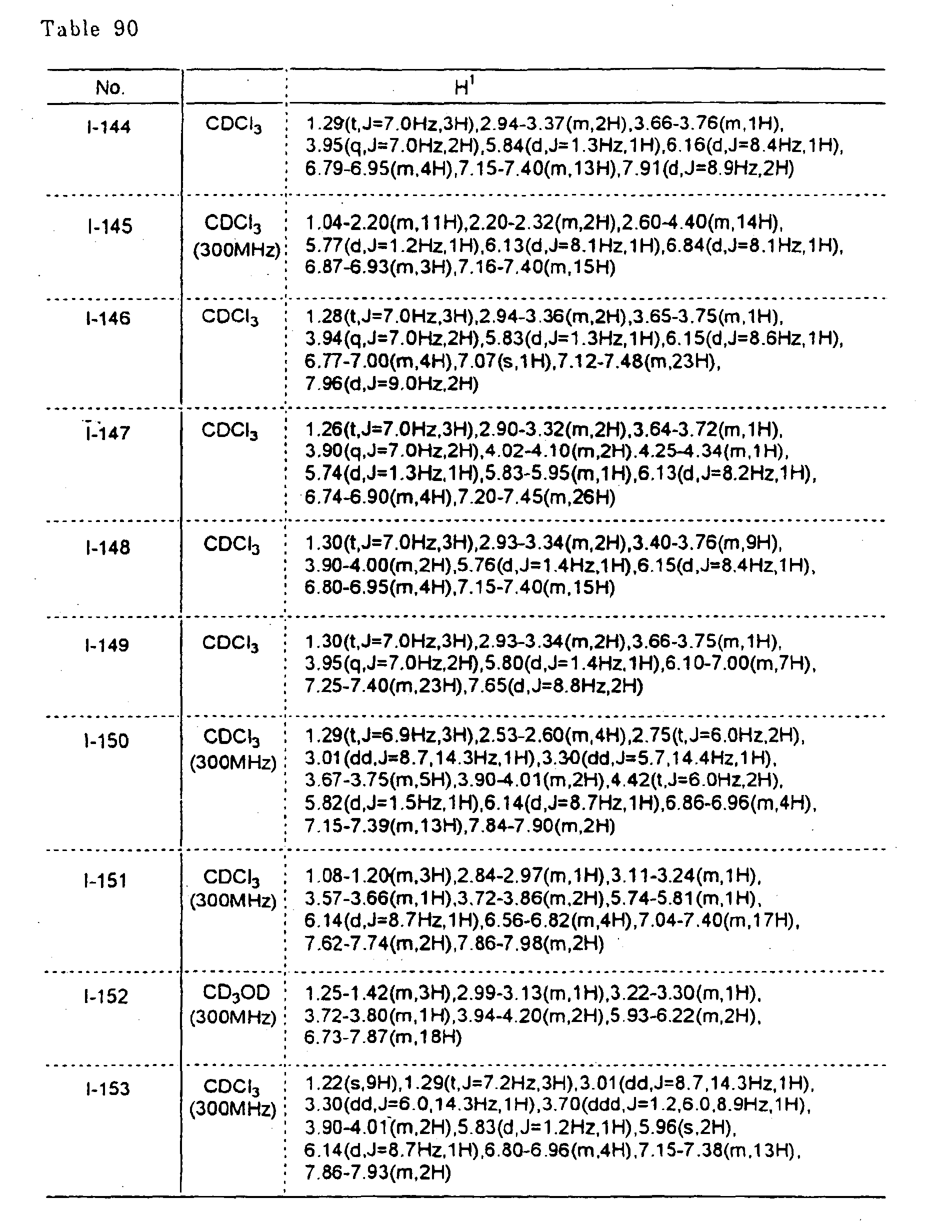

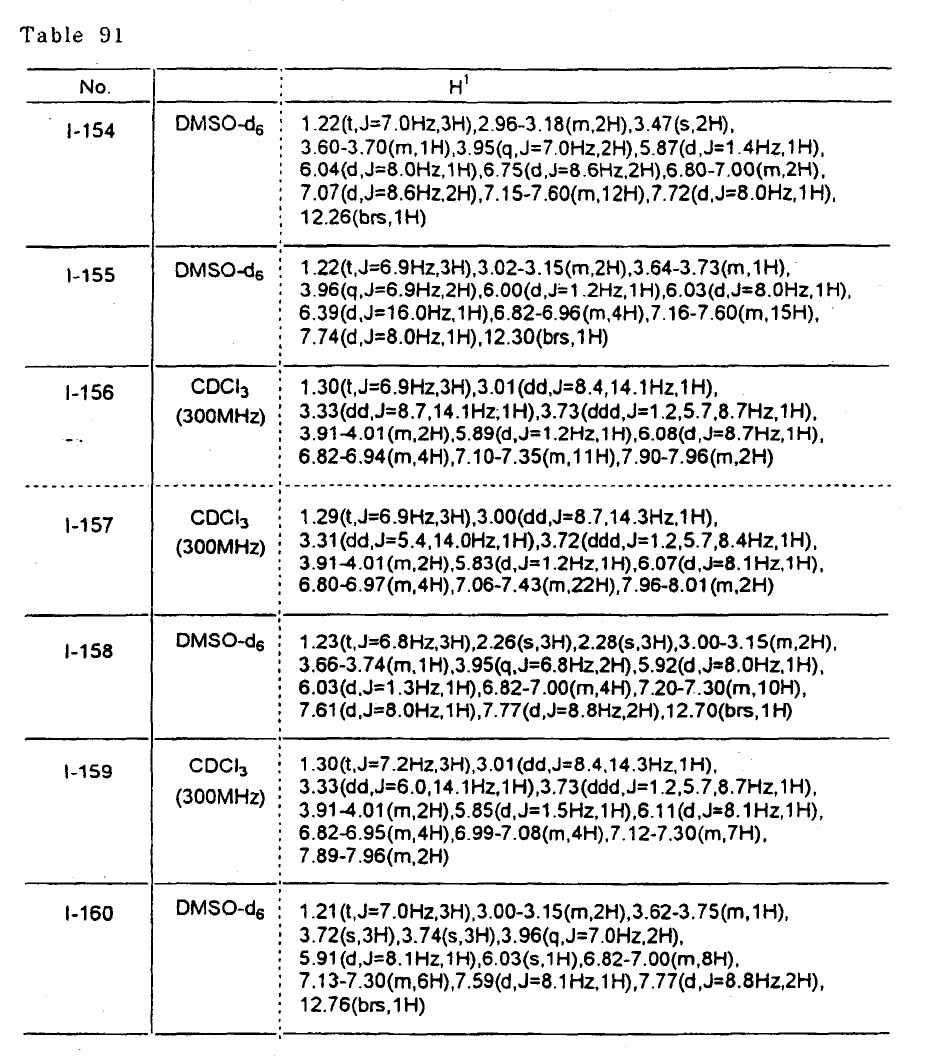

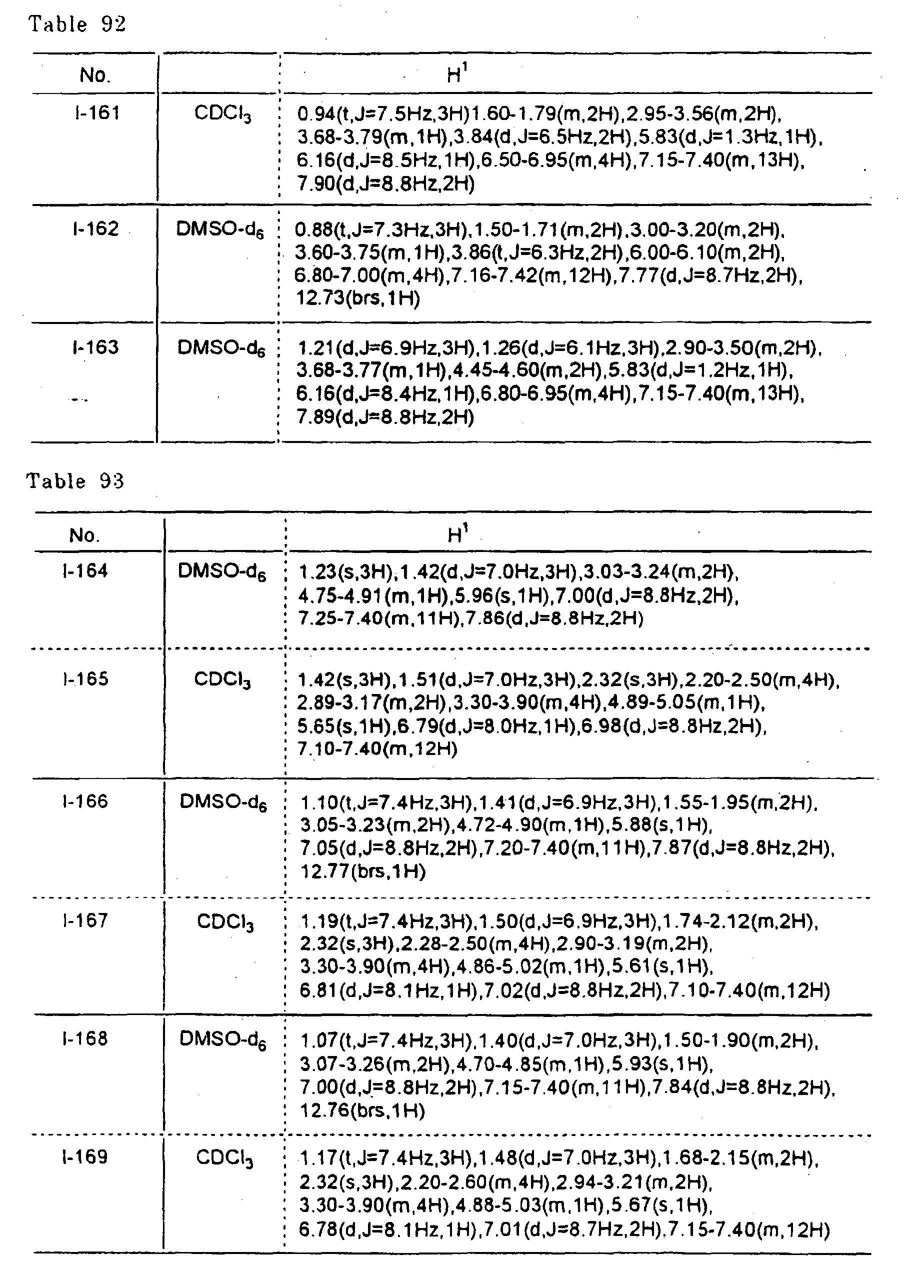

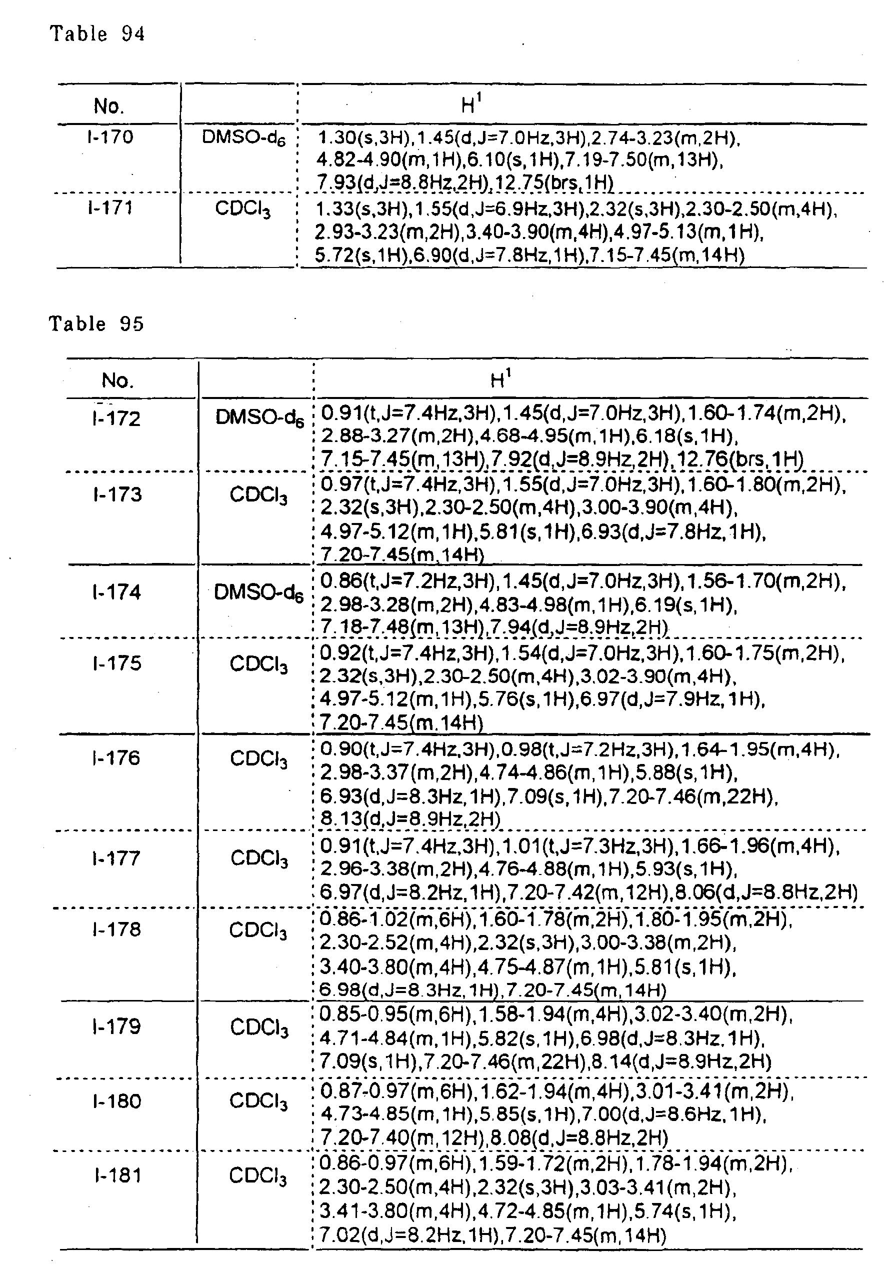

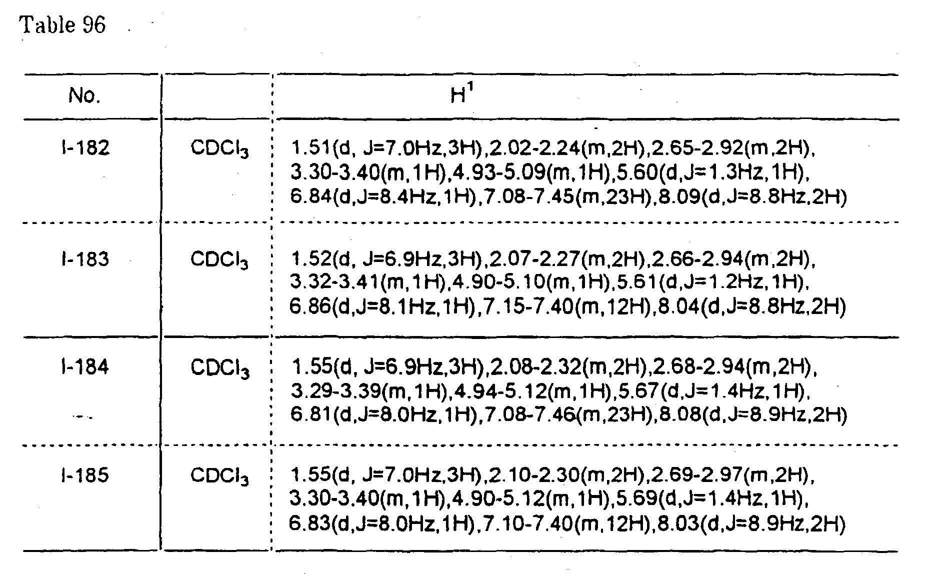

### Experiment 1 Chymase inhibitory activity

### (1) Preparation of Compound (I)

Compound (I) was dissolved in dimethylsulfoxide (DMSO) at 10⁻² M. Concentration of DMSO for activity measurement was 1 %.

### (2) Measurement of a chymase inhibitory activity

Compound (I) dissolved in DMSO and purified human chymase (Takai et al., Clinica Chimica Acta 265, 1997, 13-20) were added to a buffer (0.1M Tris-HCl, 1.8M NaCl pH8.0). After the mixture was treated for 30 minutes at 37 °C, Suc-Ala-Ala-Pro-Phe-pNA (BACHEM Feinchemikalien AG) was added at 0.5 mM as a substrate and the mixture was enzymatically reacted at 37 °C.

After the reaction, the absorption intensity (405 nm) of the solution was measured to calculate the inhibitory rate.

### (3) Result

Concentrations giving half-maximal inhibition (IC₅₀ of Compound (I) against human chymase activity are shown in Table 97.

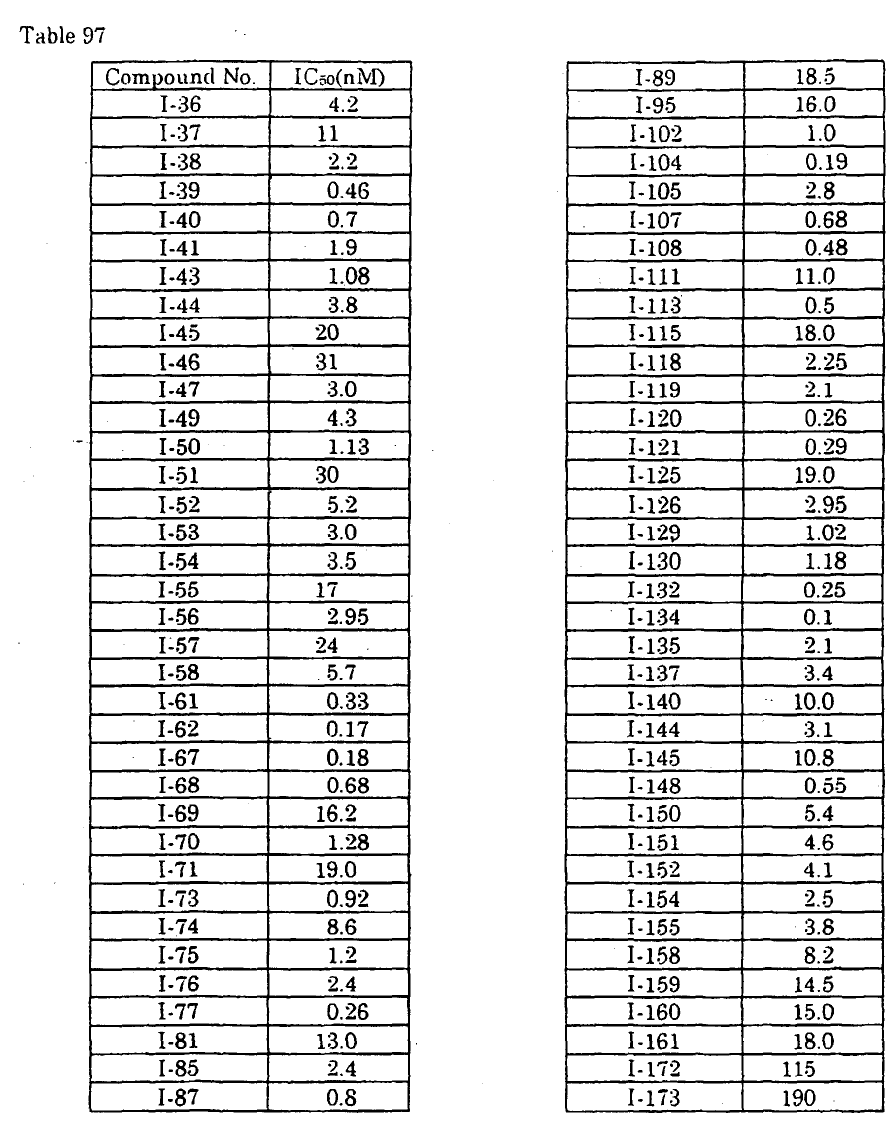

As shown in Table 97, the compounds of the present invention have a chymase inhibitory activity.

### Experiment 2 Cytokine production inhibitory activity

After human blood obtained in heparinized tube was layered on the Filcoll - Hypaque mixed solution (density=1.114, mono-poly separation solution, Dainippon Pharmaceutical Co., Ltd.), it was centrifuged to prepare mononuclear cells. The obtained mononuclear cells were suspended in a medium (Macrophage-SFM:GIBCO) to adjust 2 X 10⁶ cells/ml and cultivated in 48-well plates. Ten minutes later addition of the compound of the present invention, Concanavalin A (5 µg/ml) was added to stimulate cells. After forty-eight hours, IL-1β, IL-2, IL-4, IL-5, IL-6, TNF-α and IFNγ in the culture supernatant were quantified by ELISA method. The following kits were used for the quantification of these cytokines.
IL-1β: Quantikine(Trademark) Human IL-1β ELISA KIT (R&D system)
IL-2: Quantikine(Trademark) Human IL-2 ELISA KIT (R&D system)
IL-4: Quantikine(Trademark) Human IL-4 ELISA KIT (R&D system)
IL-5: Quantikine(Trademark) Human IL-5 ELISA KIT (R&D system)
IL-6: Quantikine(Trademark) Human IL-6 ELISA KIT (R&D system)
TNF-α: Quantikine(Trademark) Human TNF-α ELISA KIT (R&D system)
IFNγ: Quantikine(Trademark) Human IFNγ ELISA KIT (R&D system)

The results are shown in Table 98.

**Table 98**

| | IC₅₀ (µM) | |
|---|---|---|
| | I-39 | I-62 |
| IL-1β | 2.7 | 11.3 |
| IL-2 | 2.7 | 2.4 |
| IL-4 | 11.7 | 20.2 |
| IL-5 | 6.5 | 13.9 |
| IL-6 | 6.4 | 9.3 |
| TNF-α | 1.9 | 6.8 |
| IFNγ | 12.1 | 17.1 |

As shown in Table 98, the compounds of the present invention have a cytokine production inhibitory activity.

### Experiment 3 Inhibitory activity on other serine proteases

### 1) Trypsin

The mixture of 10 µl of bovine pancreas trypsin (1.5 µg/ml in 1 mM HCl, 20 mM CaCl₂, SIGMA), 80 µl of buffer (50mM Tris-HCl, 2 mM CaCl₂ pH 8.0) and 1 µl of the compound of the present invention (in DMSO) was incubated for 20 minutes at room temperature and for 10 minutes at 37 °C. The resultant mixture was reacted with 10 µl of a substrate (5 mM sucAAPRpNA (BACHEM Feinchemikalien AG) in 50 % DMSO) for about 60 minutes at 37 °C and the absorption intensity (405 nm) was measured.

### 2) Plasmin

The mixture of 10 µl of human serine plasmin (0.1 mg/ml in 1 mM HCl, 20 mM CaCl₂, SIGMA), 80 µl of buffer (50mM Tris-HCl, pH 7.5, 50 mM NaCl) and 1 µl of the compound of the present invention (in DMSO) was incubated for 20 minutes at room temperature and for 10 minutes at 37 °C. The resultant mixture was reacted with 10 µl of a substrate (5 mM Chromozyme PL(TosGPKpNA, Boehringer Mannheim) in H₂O) for about 30 minutes at 37 °C and the absorption intensity (405 nm) was measured.

### 3) Thrombin

The mixture of 10 µl of human plasma thrombin (1 U/ml in 10 mM Mes, pH 6.0, 0.1 M NaCl, SIGMA), 80 µl of buffer (0.1 M Tris-HCl, pH 8.0, 10 mM CaCl₂, 0.1 M NaCl) and 1 µl of the compound of the present invention (in DMSO) was incubated for 20 minutes at room temperature and for 10 minutes at 37 °C. The resultant mixture was reacted with 10 µl of a substrate (5 mM Chromozyme TH (TosGPRpNA, Boehringer Mannheim) in H₂O) for about 60 minutes at 37 °C and the absorption intensity (405 nm) was measured.

### 4) Elastase

The mixture of 10 µl of human neutrophils elastase (0.02 mg/ml in 50 mM Tris-HCl, pH 7.0, 2 mM CaCl₂, Athens research and technology), 80 µl of buffer (50mM Tris-HCl, pH 8.0, 2 mM CaCl₂) and 1 µl of the compound of the present invention (in DMSO) was incubated for 20 minutes at room temperature and for 10 minutes at 37 °C. The resultant mixture was reacted with 10 µl of a substrate (5 mM sucAAVpNA (BACHEM Feinchemikalien AG) in 50 % DMSO) for about 30 minutes at 37 °C and the absorption intensity (405 nm) was measured.

### 5) Cathepsin G

The mixture of 10 µl of human purulent sputum cathepsin (1.7 µg/ml in 1 mM HCl, 20 mM CaCl₂, CALBIOCHEM), 80 µl of buffer (50mM Tris-HCl, pH 7.5, 5.2 mM CaCl₂) and 1 µl of the compound of the present invention (in DMSO) was incubated for 20 minutes at room temperature and for 10 minutes at 37 °C. The resultant mixture was reacted with 10 µl of a substrate (5 mM sucAAPFpNA in DMSO, BACHEM Feinchemikalien AG) for about 60 minutes at 37 °C and the absorption intensity (405 nm) was measured.

The IC₅₀ values of each serine protease are calculated and compared with IC₅₀ value of chymase. The results are shown in Table 99.

**Table 99**

| | I-144 | | I-158 | |
|---|---|---|---|---|
| | IC₅₀(nM) | fold vs chymase | IC₅₀(nM) | fold vs chymase |
| Cathepsin G | 35.4 | 11 | 143.2 | 17 |
| elastase | >100000 | >30000 | 25000 | 3000 |
| trypsin | 25000 | 8000 | 6200 | 760 |
| thrombin | >100000 | >30000 | >100000 | >10000 |
| plasmin | >10000 | >3000 | >100000 | >10000 |

As shown in Table 99, the compounds of the present invention have a chymase-selective inhibitory activity.

### Formulation Example 1

| | |
|---|---|
| The compound of the present invention (Ia-1) | 15 mg |
| Starch | 15 mg |
| Lactose | 15 mg |
| Crystalline cellulose | 19 mg |
| Polyvinyl alcohol | 3 mg |
| Distilled water | 30 ml |
| Calcium stearate | 3 mg |

After all of the above ingredients except for calcium stearate were uniformly mixed, the mixture was crushed and granulated, and dried to give a suitable size of granules. After calcium stearate was added to the granules, tablets were formed by compression molding.

### Industrial Applicability

As explained in the above experiments, the compound of the present invention has a chymase inhibitory activity and/or cytokine production inhibitory activity. The compound of the present invention is very useful as a medicament for preventing and/or treating e.g. circulatory system diseases, inflammation, allergic diseases, rheumatics, asthma and atopy.

## Claims

1. A pharmaceutical composition for use as a chymase inhibitor comprising a compound of the formula (I): 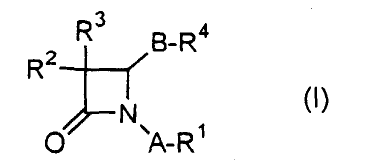
wherein A is a bond, -CO-, -COO-, -COCO-, -CONH- or -SO₂-,
R¹ is optionally substituted lower alkyl, optionally substituted lower alkenyl, optionally substituted lower alkynyl, optionally substituted cycloalkyl, optionally substituted cycloalkenyl or optionally substituted aryl, and R¹ may be hydrogen when A is a bond, -CO-, -COCO-, -CONH- or -SO₂-,
R² and R³ are each independently hydrogen, halogen, optionally substituted lower alkyl, optionally substituted lower alkoxycarbonyl, optionally substituted acyl, optionally substituted amino, optionally substituted carbamoyl or optionally substituted aryl. B is a bond, -S-, -O-, -S-S-, -SO- or -SO₂-, and
R⁴ is hydrogen, optionally substituted lower alkyl, optionally substituted aryl or optionally substituted heterocyclyl and R⁴ may be optionally substituted acyl when B is a bond, -S-, -O-, -SO- or -SO₂-,
prodrug, pharmaceutically acceptable salt or hydrate thereof.

2. The pharmaceutical composition for use as a chymase inhibitor as claimed in claim 1 wherein A-R¹ is 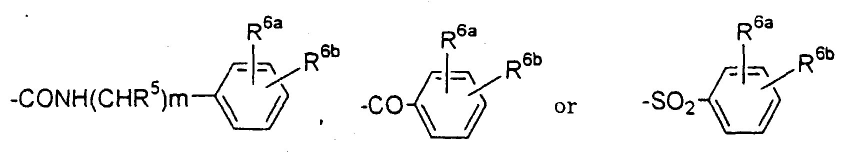
wherein R⁵ is hydrogen, optionally substituted lower alkyl, optionally substituted lower alkenyl, optionally substituted lower alkoxy or optionally substituted aryl, R^{6a} and R^{6b} are each independently hydrogen, halogen, hydroxy, lower alkyl, carboxy, lower alkoxycarbonyl, lower alkoxy, aryl, acyl, optionally substituted amino, aryloxy, lower alkylthio or heterocyclyl and R^{6a} and R^{6b} taken together may form lower alkylenedioxy, and m is 0 or 1,
R² and R³ are each independently hydrogen, optionally substituted phenyl or optionally substituted benzyl,
B-R⁴ is hydrogen, optionally substituted acyloxy, and 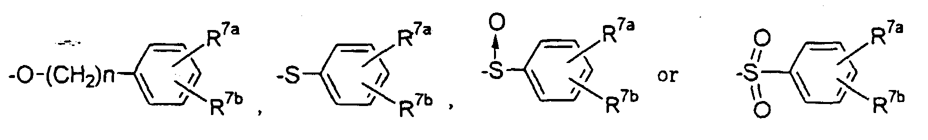
wherein R^{7a} and R^{7b} are each independently hydrogen, halogen, lower alkyl, lower alkoxy, lower alkenyl, amino, acylamino, 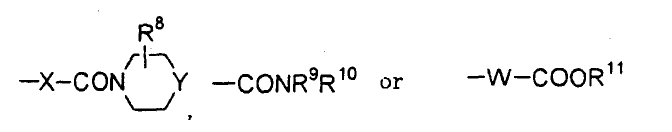
wherein X and W are each independently a bond, lower alkylene or lower alkenylene, Y is a bond, -CH₂-, -NR¹²- (wherein R¹² is hydrogen, cycloalkyl, heterocyclyl or lower alkyl optionally substituted with methylenedioxyphenyl) or -O-, R⁸ is hydrogen, optionally substituted lower alkyl or optionally substituted carbamoyl, R⁹, R¹⁰ and R¹¹ are each independently hydrogen, optionally substituted lower alkyl, optionally substituted lower alkenyl, optionally substituted amino, optionally substituted aryl or optionally substituted arylsulfonyl, and n is an integer of 0 to 6.

3. The pharmaceutical composition for use as a chymase inhibitor as claimed in claim 1 wherein A-R¹ is 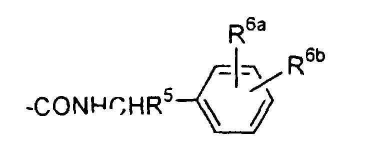
wherein R⁵ is C1 to C3 alkyl or optionally substituted phenyl wherein the substituent is halogen, lower alkyl or lower alkoxy, R^{6a} and R^{6b} are each independently hydrogen, halogen, lower alkyl or lower alkoxy.
R² is benzyl optionally substituted with lower alkoxy,
R³ is hydrogen,
B-R⁴ is acyloxy, 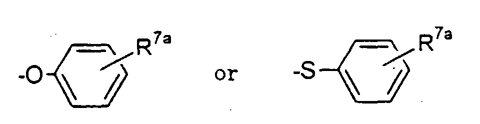
wherein R^{7a} is hydrogen, 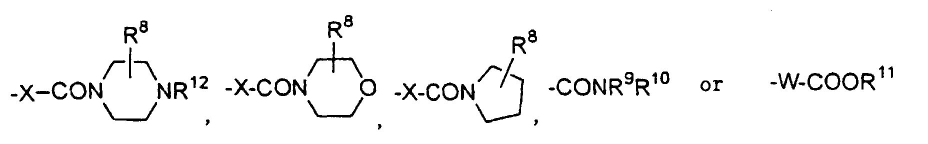
wherein X and W are each independently a bond, methylene or vinylene, R⁸ is lower alkyl or carbamoyl, R⁹ is hydrogen or optionally substituted lower alkyl, R¹⁰ is hydrogen, optionally substituted lower alkyl, lower alkenyl, lower alkylamino, arylamino, phenyl or arylsulfonyl, R¹¹ is hydrogen, optionally substituted lower alkyl or optionally substituted phenyl and R¹² is cycloalkyl or lower alkyl optionally substituted with methylenedioxyphenyl.

4. The pharmaceutical composition for use as a chymase inhibitor as claimed in claim 1
wherein A-R¹ is 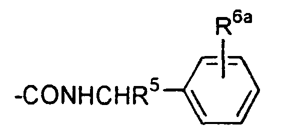
wherein R⁵ is C1 to C3 alkyl or 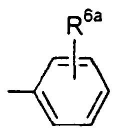 and all R^{6a} are the same and hydrogen, halogen, lower alkyl or lower alkoxy.

5. The pharmaceutical composition for use as a chymase inhibitor as claimed in claim 1
wherein A-R¹ is -CONHCHR⁵Ph wherein Ph is phenyl, R² is benzyl, R³ is C1 to C3 alkyl, B-R⁴ is 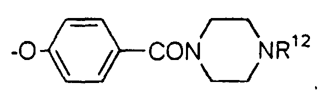 and R⁵ and R¹² are each independently C1 to C3 alkyl.

6. A pharmaceutical composition for use as a cytokine production inhibitor comprising the compound of the formula (I) according to claim 1, prodrug, pharmaceutically acceptable salt or hydrate thereof.

7. A pharmaceutical composition for use as a cytokine production inhibitor comprising the compound of the formula (I) according to claim 1 wherein A-R¹, R², R³ and B-R⁴ are the same as defined in claim 2, prodrug, pharmaceutically acceptable salt or hydrate thereof.

8. A pharmaceutical composition for use as a cytokine production inhibitor comprising the compound of the formula (I) according to claim 1 wherein A-R¹, R², R³ and B-R⁴ are the same as defined in claim 3, prodrug, pharmaceutically acceptable salt or hydrate thereof.

9. The pharmaceutical composition for use as a chymase inhibitor as claimed in any one of claims 1 to 5, which is for use as an anti-inflammatory agent.

10. The pharmaceutical composition for use as a cytokine production inhibitor as claimed in any one of claims 6 to 8, which is for use as an anti-inflammatory agent.

11. A method for preventing and/or treating diseases caused by chymase, comprising administering the compound of the formula (I) according to claim 1, prodrug, pharmaceutically acceptable salt or hydrate thereof.

12. Use of the compound of the formula (I) according to claim 1, prodrug, pharmaceutically acceptable salt or hydrate thereof for manufacturing a medicament for preventing and/or treating diseases caused by chymase.

13. A compound of the formula (I'): 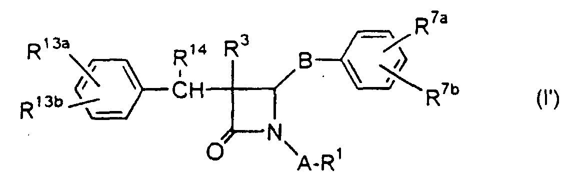
wherein A and R¹ are the same as defined in claim 1,
R³ is hydrogen, halogen, optionally substituted lower alkoxycarbonyl, optionally substituted acyl, optionally substituted amino, optionally substituted aryl or optionally substituted benzyl,
R^{13a} and R^{13b} are each independently hydrogen, halogen, hydroxy, optionally substituted lower alkyl, optionally substituted lower alkoxy, optionally substituted amino or optionally substituted lower alkylthio and R^{13a} and R^{13b} taken together may form lower alkylenedioxy,
R¹⁴ is hydrogen, hydroxy, lower alkyl, lower alkoxy or acyloxy,
R^{7a} is hydrogen, 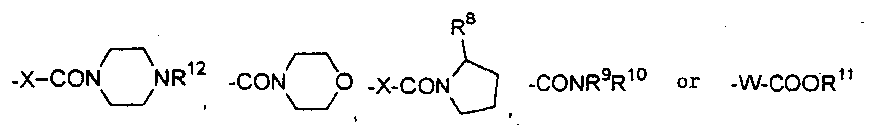
wherein X and W are each independently a bond, methylene or vinylene, R⁸ is methyl or carbamoyl, R⁹ is hydrogen or lower alkyl, R¹⁰ is optionally substituted lower alkyl (wherein the substituent is lower alkyl amino; phenyl optionally substituted with halogen; carboxy; or lower alkoxycarbonyl optionally substituted with aryl), lower alkenyl, lower alkylamino, phenylamino, phenyl or benzenesulfonyl, R¹¹ is hydrogen or optionally substituted lower alkyl (wherein the substituent is lower alkylamino; acyloxy; phenyl optionally substituted with halogen or methylenedioxy; or heterocyclyl), and R¹² is C1 to C3 alkyl or cyclohexyl,
R^{7b} is hydrogen, and B is O or S,
pharmaceutically acceptable salt or hydrate thereof.

14. A compound of the formula (I''): 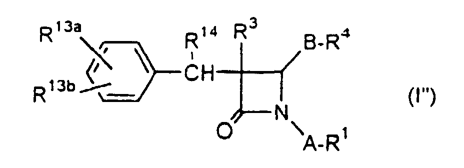
wherein B and R⁴ are the same as defined in claim 1,
A is -CO-, -CONH- or -SO₂-,
R¹ is optionally substituted lower alkyl or optionally substituted aryl,
R³ is hydrogen, halogen, lower alkyl, optionally substituted lower alkoxycarbonyl, optionally substituted acyl, optionally substituted amino, optionally substituted aryl or optionally substituted benzyl,
R^{13a} and R^{13b} are each independently hydrogen, halogen, hydroxy, optionally substituted lower alkyl, optionally substituted lower alkoxy, optionally substituted amino or optionally substituted lower alkylthio and R^{13a} and R^{13b} taken together may form lower alkylenedioxy,
R¹⁴ is hydrogen, hydroxy, lower alkyl, lower alkoxy or acyloxy, excluding a compound wherein B-R⁴ is optionally substituted aryloxy or optionally substituted acylthio and A is CONH,
prodrug, pharmaceutically acceptable salt or hydrate thereof.

15. Th 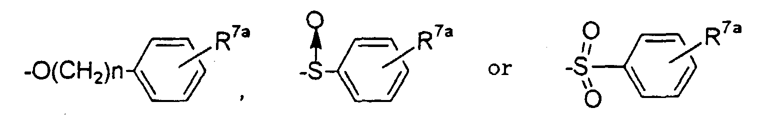 e compound as claimed in claim 14 wherein B-R⁴ is acyloxy,
wherein n is 0 or 1, R^{7a} is hydrogen, 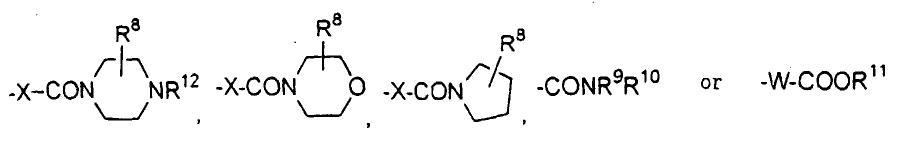
wherein X and W are each independently a bond, methylene or vinylene, R⁸ is lower alkyl or carbamoyl, R⁹ is hydrogen or optionally substituted lower alkyl, R¹⁰ is hydrogen, optionally substituted lower alkyl, lower alkenyl, lower alkylamino, arylamino, phenyl or arylsulfonyl, R¹¹ is hydrogen, optionally substituted alkyl or optionally substituted phenyl and R¹² is cycloalkyl or lower alkyl optionally substituted with methylenedioxyphenyl,
prodrug, pharmaceutically acceptable salt or hydrate thereof.

16. The compound as claimed in claim 13 or 14 wherein R³ is hydrogen, prodrug, pharmaceutically acceptable salt or hydrate thereof.

17. The compound as claimed in claim 13 or 14 wherein R^{13a} is hydrogen or C1 to C3 lower alkoxy at the *o*-position and R^{13b} is hydrogen, prodrug, pharmaceutically acceptable salt or hydrate thereof.

18. Any one of compounds selected from the group of
(a) 4-[3-Benzyl-4-oxo-1-(1-phenyl-ethylcarbamoyl)-azetidin-2-yloxy]-benzoic acid,
(b) 3-Benzyl-2-[4-(4-methyl-piperazine-1-carbonyl)-phenoxy]-4-oxo-azetidine-1-carboxylic acid (1-phenyl-ethyl)-amide,
(c) 3-Benzyl-2-[4-(2-carbamoyl-pyrrolidine-1-carbonyl)-phenoxy]-4-oxo-azetidine-1-carboxylic acid (1-phenyl-ethyl)-amide,
(d) 3-Benzyl-2-[4-(2-methyl-pyrrolidine-1-carbonyl)-phenoxy]-4-oxo-azetidine-1-carboxylic acid (1-phenyl-ethyl)-amide,
(e) 4-[3-(2-Methoxy-benzyl)-4-oxo-1-(1-phenyl-ethylcarbamoyl)-azetidin-2-yloxy]-benzoic acid,
(f) 4-[3-(2-Methoxy-benzyl)-4-oxo-1-(1-phenyl-ethylcarbamoyl)-azetidin-2-yloxy]-benzoic acid pyridin-4-ylmethyl ester,
(g) 4-[3-(2-Methoxy-benzyl)-4-oxo-1-(1-phenyl-ethylcarbamoyl)-azetidin-2-yloxy]-benzoic acid benzyl ester,
(h) 3-(2-Methoxy-benzyl)-2-oxo-4-[4-(4-pyrimidin-2-yl-piperazine-1-carbonyl)-phenoxy]-azetidine-1-carboxylic acid (1-phenyl-ethyl)-amide,
(i) 2-[4-(4-Cyclohexyl-piperazine-1-carbonyl)-phenoxy]-3-(2-methoxy-benzyl)-4-oxo-azetidine-1-carboxylic acid (1-phenyl-ethyl)-amide,
(j) 3-(2-Methoxy-benzyl)-2-[4-(4-methyl-piperazine-1-carbonyl)-phenoxy]-4-oxo-azetidine-1-carboxylic acid (1-phenyl-ethyl)-amide,
(k) 4-[1-(Benzhydryl-carbamoyl)-3-(2-ethoxy-benzyl)-4-oxo-azetidin-2-yloxy]-benzoic acid,
(l) 2-[4-(4-Cyclohexyl-piperazine-1-carbonyl)-phenoxy]-3-(2-ethoxy-benzyl)-4-oxo-azetidine-1-carboxylic acid benzhydryl-amide,
(m) 3-(2-Ethoxy-benzyl)-2-[4-(morpholine-4-carbonyl)-phenoxy]-4-oxo-azetidine-1-carboxylic acid benzhydryl-amide,
(n) {4-[1-(Benzhydryl-carbamoyl)-3-(2-ethoxy-benzyl)-4-oxo-azetidin-2-yloxy]-phenyl}-acetic acid,
(o) 3-{4-[1-(Benzhydryl-carbamoyl)-3-(2-ethoxy-benzyl)-4-oxo-azetidin-2-yloxy]-phenyl}-acrylic acid,
(p) 4-[1-(Di-p-tolylmethyl-carbamoyl)-3-(2-ethoxy-benzyl)-4-oxo-azetidin-2-yloxy]-benzoic acid,
(q) 4-[1-(Bis-4-fluoro-phenyl)-methyl-carbamoyl)-3-(2-ethoxy-benzyl)-4-oxo-azetidin-2-yloxy]-benzoic acid and
(r) 4-[1-{[Bis-(4-methoxy-phenyl)-methyl]-carbamoyl}-3-(2-ethoxy-benzyl)-4-oxo-azetidin-2-yloxy]-benzoic acid,
prodrug, pharmaceutically acceptable salt or hydrate thereof

19. A pharmaceutical composition comprising the compound according to any one of claims 13 to 18, prodrug, pharmaceutically acceptable salt or hydrate thereof.

20. The pharmaceutical composition as claimed in claim 19, which is for use as a chymase inhibitor.

21. The pharmaceutical composition as claimed in claim 19, which is for use as a cytokine production inhibitor.

22. The pharmaceutical composition as claimed in claim 19, which is for use as an anti-inflammatory agent.

23. A method for preventing and/or treating diseases caused by chymase comprising administering the compound according to any one of claims 13 to 18, prodrug, pharmaceutically acceptable salt or hydrate thereof.

24. Use of the compound according to any one of claims 13 to 18, prodrug, pharmaceutically acceptable salt or hydrate thereof for manufacturing a medicament for preventing and/or treating diseases caused by chymase.
